(19) Europäisches Patentamt
European Patent Office
Office européen des brevets

(11) EP 1 454 982 A1

(12) **EUROPEAN PATENT APPLICATION**
published in accordance with Art. 158(3) EPC

(43) Date of publication:
**08.09.2004 Bulletin 2004/37**

(21) Application number: **02786101.2**

(22) Date of filing: **13.12.2002**

(51) Int Cl.[7]: **C12N 15/00**, C12Q 1/68, A61K 38/17, A61P 35/00, C07K 14/47, C12P 21/02, C07K 16/18, G01N 33/566, G01N 33/50, G01N 33/15

(86) International application number:
**PCT/JP2002/013097**

(87) International publication number:
**WO 2003/052096 (26.06.2003 Gazette 2003/26)**

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority: **14.12.2001 JP 2001382053**
**21.02.2002 JP 2002045104**
**15.05.2002 JP 2002140111**
**18.11.2002 JP 2002333769**

(71) Applicant: **Takeda Chemical Industries, Ltd.**
**Osaka 541-0045 (JP)**

(72) Inventors:
- **HINUMA, Shuji**
**Tsukuba-shi, Ibaraki 305-0821 (JP)**
- **KOBAYASHI, Makoto**
**Kobe-shi, Hyogo 651-2276 (JP)**
- **ARAI, Toshimitsu**
**Suita-shi, Osaka 565-0823 (JP)**
- **FUKUSUMI, Shoji, Royal City Namiki 302**
**Tsukuba-shi, Ibaraki 305-0044 (JP)**
- **FUJII, Ryo**
**Tsukuba-shi, Ibaraki 305-0821 (JP)**
- **KOMATSU, Hidetoshi,**
**Takeda Kasuga Haitsu 803**
**Tsukuba-shi, Ibaraki 305-0821 (JP)**
- **MATSUMURA, Fumika,**
**Takeda Matsushiro Residence 613**
**Tsukuba-shi, Ibaraki 305-0035 (JP)**
- **KAWAMATA, Yuji,**
**Matsushiro 4-chome Danchi 2-203**
**Tsukuba-shi, Ibaraki 305-0035 (JP)**
- **OGI, Kazuhiro, Run Bini Umezono 206**
**Tsukuba-shi, Ibaraki 305-0045 (JP)**

(74) Representative: **Lewin, John Harvey**
**Takeda Euro IP Department,**
**11-12 Charles II Street**
**London SW1Y 4QU (GB)**

(54) **MTEHOD OF ANALYZING GENE EXPRESSION**

(57) The present invention intends to provide a method for analyzing gene expression, a novel use for a receptor protein and the like. Specifically, the present invention provides a method for analyzing gene expression, which is characterized by identifying genes, which are characteristically promoted or suppressed in certain cells or tissues by quantitatively analyzing the individual expressed amounts of a plural number of genes collectively, an assay kit employed in the method and the like. The present invention also provides a method of screening compounds or salts thereof which alter the binding properties of a novel G protein coupled receptor protein comprising an amino acid sequence identical or substantially identical to the amino acid sequence represented by SEQ ID NO: 9 or a salt thereof with a ligand, characterized by the use of the protein or salt thereof and a protein (such as a lectin) showing an affinity for a sugar chain which is one of the ligands.

EP 1 454 982 A1

## Description

### TECHNICAL FIELD

The present invention relates to a method of analyzing gene expression wherein the expression of multiple genes is analyzed collectively, to a gene expression analysis kit used therefor and the like. The present invention also relates to a method of specifying disease-associated genes employing said gene expression analysis method and analysis kit. Furthermore, the present invention relates to a method of diagnosing specific diseases by analyzing expressed amounts and mutations of disease-associated genes, and to drugs comprising specified gene DNA or gene products thereof. In addition, the present invention relates to uses for a human-derived G protein-coupled receptor protein (dJ287G14.2 receptor) and a polynucleotide encoding therefor. Moreover, the present invention relates to a novel mouse-derived G protein-coupled receptor protein (dJ287G14.2 receptor), to a polynucleotide encoding therefor and to uses for these. In addition, the present invention relates to a method for screening EDG-1 receptor agonists and antagonists or EDG-2 agonists and antagonists.

### BACKGROUND ART

G protein-coupled receptor proteins are present on the various functional cell surfaces of cells and organs in vivo, and play an important physiological role as targets for molecules such as hormones, neurotransmitters, bioactive substances and the like which regulate the functions of these cells and organs. When it binds with a bioactive substance a receptor transmits a signal inside the cell, and this signal stimulates a variety of reactions such as cell activation or suppression. Elucidation of substances which regulate complex functions within various cells and organs in vivo and their relationships with specific receptor proteins and G protein-coupled receptor proteins offers an extremely useful tool for developing drugs closely associated with the functions of various cells and organs in vivo: about 45% of existing drugs act on receptor proteins on cell membranes, and of these the G protein-coupled receptor proteins are known to play an important role (Drews, J., *Science* 287, 1960 (2000)).

Ion channels are membrane proteins, which penetrate the cell membrane and play a role in cell response by regulating membrane permeation of ions. Because they are ion-selective they are known as $Na^+$, $K^+$, $Ca^{2+}$ and $Cl^-$ channels and the like, and are classified as either potential-dependent ($Na^+$, $K^+$, $Ca^{2+}$) or receptor-dependent (nicotinic cholinergic receptor, GABA receptor) depending on the channel opening and closing mechanism. These ion channels have many physiological functions in vivo. For example, the $K^+$ channel is a membrane protein which selectively allows permeation of $K^+$ ions, and in vivo it is closely associated with important physiological functions including cell resting membrane potential formation, repolarization, and regulating frequency of action potential occurrence. The $Cl^-$ channel exhibits a variety of physiological functions including regulation of neuromuscular excitability, cell volume regulation, and electrolyte and water transport in the epithelial cell membranes. Thus, the various ion channels are drug targets because they have a variety of physiological functions.

Some receptors in the body also have enzyme activity, such as the tyrosine kinase receptors for example. Because this enzyme activity serves a variety of physiological functions, tyrosine kinase receptors have become the target of many drugs. Other gene families include transcription factors, transporters, protein kinases, protein phosphatases, proteases, heat shock proteins; ATPases, DNA-binding proteins and the like, which constitute families of tens or hundreds and are drug targets.

A variety of genes (about 400 G protein-coupled receptors, about 100 tyrosine kinase receptors and about 150 ion channels) have already been identified and cloned. However, the functions of all of these genes have not been elucidated, and there are many proteins whose functions are unknown. Elucidating the functions of these proteins is the first step in developing novel drugs.

Methods, which have been used for analyzing genes expressed in vivo, include the Northern blot method, differential display method and RT-PCR method. However, there are limits on the number of genes that can be analyzed at once, and it has been difficult to process samples containing many genes. Sensitivity has also been low for quantification.

A method that has been developed in recent years for analyzing all genes expressed in vivo is the microarray method, in which thousands or tens of thousands of pieces of DNA data are synthesized or spotted on a glass slide or other chip, target DNA derived from the RNA under analysis is hybridized, and the amount of transcripted gene is measured using the resulting hybrid as the indicator, but sensitivity has not been particularly high for quantification.

Methods of assaying specific mRNA include the TaqMan method and the like, and for example a method of assaying hTERT mRNA is disclosed in Kokai No. 2001-204483. The TaqMan method is also known as one method of SNP analysis, in which primers and probes, which recognize the SNP, are designed and can be used to analyze not only expressed amounts but also disease-related mutations.

A protein having the identical amino acid sequence as the dJ287G14.2 receptor used in the present invention and DNA encoding therefor have been described (WO 2001/18207). However, the functions of these G protein-coupled receptor proteins and their physiological ligands have not been elucidated.

An EDG-1 receptor (*Biochem Biophys Res Commun* 1997 Nov 26; 240(3): 737-41), EDG-2 receptor (*Biochem Biophys Res Commun* 1997 Feb 24; 231(3): 619-22), EDG-3 receptor (*Cell* 1999 Oct 29; 99(3): 301-12), EDG-5 receptor (*J Biol Chem* 1999 Dec 10; 274(50): 35343-50) and EDG-8 receptor (*Biochemistry* 2001 Nov 20; 40(46): 14053-60) have been described, along with the fact that these receptors are expressed in vascular tissue. However, it has not been explained to what degree each receptor is expressed in vascular and other cells. Clear expression of EDG-4, EDG-6 and EDG-7 in vascular tissue is unknown.

Notwithstanding, until now there has been no idea for assaying the expression of many genes belonging to a specific family all at once with high sensitivity, or any assay system for realizing such an idea. Consequently if there were a system for assaying the expression of many genes belonging to a specific family all at once with high sensitivity, it would be useful in such areas as specifying disease genes and diagnosing and treating specific diseases, since genes which are highly expressed specifically in certain disease cells or the like could be identified relatively and quantitatively.

## DISCLOSURE OF THE INVENTION

Namely, as shown below, the present invention provides a method for analyzing gene expression, a method of diagnosis, an assay kit used in these methods, and proteins and uses for proteins whose functions are elucidated by such methods or kit.

[1] A method for analyzing gene expression, wherein genes whose expression is characteristically promoted or inhibited in certain cells or tissue are identified by quantitatively analyzing the individual expressed amounts of multiple genes collectively.

[2] The method according to [1], wherein expression analysis is performed collectively on multiple genes belonging to a specific gene family in order to identify the genes in that family whose expression is characteristically promoted or inhibited in certain cells or tissue by computing expressed amounts as absolute values.

[3] The method according to [1], wherein an amplification reaction is performed by bringing an mRNA sample which may contain multiple mRNA targets into contact at multiple reaction sites with individual amplification reagents each of which comprises a primer pair corresponding to a particular mRNA target, and gene expression analysis is performed by measuring the amounts produced in the resulting amplification product.

[4] The method according to [1], wherein an amplification reaction is performed by bringing an mRNA sample which may contain multiple mRNA targets into contact, at the respective reaction sites of a reaction device having multiple reaction sites, with individual amplification reagents each of which comprises a primer pair corresponding to a particular mRNA target, and gene expression analysis is performed by measuring the amounts produced in the resulting amplification product.

[5] The method according to [2], wherein the specific gene family is the G protein-coupled receptor gene family.

[6] The method according to [2], wherein the specific gene family is the tyrosine kinase receptor gene family.

[7] The method according to [2], wherein the specific gene family is the ion channel gene family.

[8] The method according to [2], wherein the specific gene family is a gene family associated with either transcription factors, transporters, protein kinases, protein phosphatases, proteases, heat shock proteins, ATPases or DNA-binding proteins.

[9] A drug comprising a gene or product of a gene, which is specified by a method described in any of [1] to [8], and the expression of which is characteristically promoted or inhibited in certain cells or tissue.

[10] The method according to [3] or [4], wherein the reaction device is a plate having multiple wells as reaction sites.

[11] The method according to [10], wherein the plate is a 96-well or 384-well plate.

[12] The method according to [3] or [4], wherein 10-800 primer pairs are used.

[13] The method according to [3] or [4], wherein 10-300 primer pairs are used.

[14] The method according to [3], [4], [12] or [13], wherein the amplification reaction is a polymerase chain reaction.

[15] The method according to [14], wherein SNP analysis is performed.

[16] The method according to [3] or [4], wherein the produced amounts of amplification products are measured using probes, which are complementary or substantially complementary to said amplification products.

[17] The method according to [16], wherein the probes are probes which hybridize with mRNA.

[18] The method according to [17], wherein the probes are fluorescence labeled probes.

[19] The method according to [3] or [4], wherein a normal human-derived mRNA sample and an mRNA sample derived from a patient with a specific disease are used as the mRNA samples.

[20] The method according to [19], wherein mRNA whose expression is promoted or inhibited in an mRNA sample derived from a disease patient is specified, and a gene encoding said mRNA is designated as a disease-associated gene for that disease.

[21] The method according to [20], wherein the specific gene family is the G protein-coupled receptor protein gene family, and wherein a cancer-related gene is specified by using mRNA derived from a cancer patient.

[22] A primer pair kit comprising two or more pairs of primers each consisting of a first primer which is complementary or substantially complementary to one chain of an exon region of a target gene sequence and a second primer which is complementary or substantially complementary to the other chain of the exon region of the target gene sequence.

[23] The primer pair kit according to [22], wherein the target gene is a human G protein-coupled receptor protein gene, tyrosine kinase receptor gene or ion channel gene.

[24] The primer pair kit according to [23], composed of 10-800 primer pairs.

[25] The primer pair kit according to [23], composed of 10-300 primer pairs.

[26] The primer pair kit according to [23], for purposes of specifying disease-associated genes.

[27] An mRNA assay kit having each reaction site in a reaction device with multiple reaction sites filled with an individual amplification reagent comprising a primer pair corresponding to a particular mRNA target.

[28] The kit according to [27], also comprising a fluorescent probe.

[29] The kit according to [28], also comprising Tth DNA polymerase.

[30] A method of diagnosing a patient's disease using the method according to any of [1] to [4] or the assay kit according to [27] by assaying the mRNA of multiple target disease genes which may be contained in an mRNA sample collected from the patient, or by measuring the mutated amount of said mRNA.

[31] The diagnostic method according to [30], wherein cancer is diagnosed by identifying cancer-associated human G protein-coupled receptor protein genes.

[32] A drug comprising an agonist, antagonist or antibodies to the gene product of a gene identified by the diagnostic method according to [30], or DNA encoding said gene product.

[33] The drug according to [32], which is a cancer therapy drug.

[34] A method of administering an agonist, antagonist or antibodies to the gene product of a gene identified by the diagnostic method according to [30] or DNA encoding said gene product in order to treat diseases involving that gene.

[35] The treatment method according to [34], wherein the disease is cancer.

It was discovered using the method for analyzing gene expression of the present invention that the dJ287G14.2 receptor is highly expressed in prostate cancer cells. Based on this finding, the present invention provides a novel use for a receptor protein or partial peptide or salt thereof (dJ287G14.2 receptor), for polynucleotides encoding that receptor protein or partial peptide thereof (DNA, RNA and derivatives thereof) and for antibodies to that receptor protein or partial peptide or salt thereof and the like. A novel mouse-derived dJ287G14.2 receptor, polynucleotides (DNA, RNA and derivatives thereof) encoding that receptor protein or partial peptide thereof, and antibodies to that receptor protein or partial peptide or salt thereof and the like are also provided.

Namely,

[36] A drug containing a receptor protein which contains an amino acid sequence identical to or substantially identical to the amino acid sequence represented by SEQ ID NO: 9, or a partial peptide or salt thereof;

[37] The drug according to [37], which is a birth inducer or a preventative and/or therapeutic drug for cancer, prostatomegaly, male gonad dysfunction, infertility, premature birth, endometriosis, cirrhosis of the liver, hepatitis, hepatic insufficiency or calculus;

[38] The drug according to [37], wherein the cancer is prostate cancer, non-small cell carcinoma, ovarian cancer, stomach cancer, bladder cancer, breast cancer, cervical cancer, colon cancer, rectal cancer or large intestinal cancer;

[39] A diagnostic drug containing antibodies to a protein which contains an amino acid sequence identical to or substantially identical to the amino acid sequence represented by SEQ ID NO: 9, or to a partial peptide or salt thereof;

[40] The diagnostic drug according to [40], which is a diagnostic drug for cancer; prostatomegaly, male gonad dysfunction, infertility, premature birth, endometriosis, cirrhosis of the liver, hepatitis, hepatic insufficiency or calculus;

[41] A drug containing antibodies to a protein which contains an amino acid sequence identical to or substantially identical to the amino acid sequence represented by SEQ ID NO: 9, or to a partial peptide or salt thereof;

[42] The drug according to [41], which is a birth inducer or a preventative and/or therapeutic drug for cancer, prostatomegaly, male gonad dysfunction, infertility, premature birth, endometriosis, cirrhosis of the liver, hepatitis, hepatic insufficiency or calculus;

[43] A method of screening compounds or salts of compounds which alter the binding properties of a G protein-coupled receptor protein containing an amino acid sequence identical to or substantially identical to the amino acid sequence represented by SEQ ID NO: 9 or of a salt thereof with a protein showing affinity for a sugar chain, characterized by the use of (1) the aforementioned G protein-coupled receptor protein, or a partial peptide or salt thereof, and (2) the protein showing affinity for a sugar chain;

[44] The screening method according to [43], wherein the protein showing affinity for a sugar chain is a protein showing affinity for an asparagine-linked sugar chain or a serine/threonine-linked sugar chain;

[45] The screening method according to [43], wherein the protein showing affinity for a sugar chain is a lectin;

[46] The screening method according to [43], wherein the protein showing affinity for a sugar chain is concanavalin A, lentil lectin, pea lectin, datura lectin, Maackia Amurensis lectin or phytohemagglutinin;

[47] A screening kit for compounds or salts of compounds which alter the binding properties of a G protein-coupled receptor protein containing an amino acid sequence identical to or substantially identical to the amino acid sequence represented by SEQ ID NO:9 or of a salt thereof with a protein showing affinity for a sugar chain, characterized in that it contains (1) the aforementioned G protein-coupled receptor protein, or a partial peptide or salt thereof, and (2) the protein showing affinity for a sugar chain;

[48] A compound or salt of a compound which alters the binding properties of a protein showing affinity for a sugar chain with a G protein-coupled receptor protein containing an amino acid sequence identical to or substantially identical to the amino acid sequence represented by SEQ ID NO: 9 or with a salt thereof, obtained using the screening method according to [46] or the screening kit according to [47];

[49] A drug containing a compound or salt thereof according to [48];

[50] The drug according to [49], which is a birth inducer or a preventative and/or therapeutic drug for cancer, prostatomegaly, male gonad dysfunction, infertility, premature birth, endometriosis, cirrhosis of the liver, hepatitis, hepatic insufficiency or calculus;

[51] The drug according to [50], wherein the cancer is prostate cancer, non-small cell carcinoma, ovarian cancer, stomach cancer, bladder cancer, breast cancer, cervical cancer, colon cancer, rectal cancer or large intestinal cancer;

[52] A method of screening birth inducers or compounds or salts of compounds for preventing and/or treating cancer, prostatomegaly, male gonad dysfunction, infertility, premature birth, endometriosis, cirrhosis of the liver, hepatitis, hepatic insufficiency or calculus which alter the expressed amount of a G protein-coupled receptor protein containing an amino acid sequence identical to or substantially identical to the amino acid sequence represented by SEQ ID NO: 9, characterized by the use of a polynucleotide containing a polynucleotide encoding the aforementioned G protein-coupled receptor protein or a partial peptide thereof;

[53] A screening kit birth inducers or compounds or salts of compounds for preventing and/or treating cancer, prostatomegaly, male gonad dysfunction, infertility, premature birth, endometriosis, cirrhosis of the liver, hepatitis, hepatic insufficiency or calculus which alter the expressed amount of a G protein-coupled receptor protein containing an amino acid sequence identical to or substantially identical to the amino acid sequence represented by SEQ ID NO: 9, wherein is contained a polynucleotide containing a polynucleotide encoding the aforementioned G protein-coupled receptor protein or a partial peptide thereof;

[54] A birth inducer or compound or salt of a compound for preventing and/or treating cancer, prostatomegaly, male gonad dysfunction, infertility, premature birth, endometriosis, cirrhosis of the liver, hepatitis, hepatic insufficiency or calculus which alters the expressed amount of a G protein-coupled receptor protein containing an amino acid sequence identical to or substantially identical to the amino acid sequence represented by SEQ ID NO: 9 or of a partial peptide thereof, obtained using the screening method according to [52] or the screening kit according to [53];

[55] A birth inducer or preventative and/or therapeutic agent for cancer, prostatomegaly, male gonad dysfunction, infertility, premature birth, endometriosis, cirrhosis of the liver, hepatitis, hepatic insufficiency or calculus, containing a compound or salt thereof according to [54];

[56] The agent according to [55], wherein the cancer is prostate cancer, non-small cell carcinoma, ovarian cancer, stomach cancer, bladder cancer, breast cancer, cervical cancer, colon cancer, rectal cancer or large intestinal cancer;

[57] A signal transmission enhancing agent for a G protein-coupled receptor protein containing an amino acid sequence identical to or substantially identical to the amino acid sequence represented SEQ ID NO: 9, containing a protein showing affinity for a sugar chain;

[58] The agent according to [57], which is a birth inducer or preventative and/or therapeutic agent for cancer, prostatomegaly, male gonad dysfunction, infertility, premature birth, endometriosis, cirrhosis of the liver, hepatitis, hepatic insufficiency or calculus;

[59] The agent according to [58], wherein the cancer is prostate cancer, non-small cell carcinoma, ovarian cancer, stomach cancer, bladder cancer, breast cancer, cervical cancer, colon cancer, rectal cancer or large intestinal cancer;

[60] A birth inducer or preventative and/or therapeutic agent for cancer, prostatomegaly, male gonad dysfunction, infertility, premature birth, endometriosis, cirrhosis of the liver, hepatitis, hepatic insufficiency or calculus, containing a polynucleotide which contains a polynucleotide encoding a G protein-coupled receptor protein containing an amino acid sequence identical to or substantially identical to the amino acid sequence represented by SEQ ID NO: 9, or a partial peptide thereof;

[61] The agent according to [60], wherein the cancer is prostate cancer, non-small cell carcinoma, ovarian cancer, stomach cancer, bladder cancer, breast cancer, cervical cancer, colon cancer, rectal cancer or large intestinal cancer;

[62] A diagnostic drug for cancer, prostatomegaly, male gonad dysfunction, infertility, premature birth, endometriosis, cirrhosis of the liver, hepatitis, hepatic insufficiency or calculus, containing a polynucleotide which contains a polynucleotide encoding a G protein-coupled receptor protein containing an amino acid sequence identical to or substantially identical to the amino acid sequence represented by SEQ ID NO: 9, or a partial peptide thereof;

[63] The diagnostic drug according to [62], wherein the cancer is prostate cancer, non-small cell carcinoma, ovarian cancer, stomach cancer, bladder cancer, breast cancer, cervical cancer, colon cancer, rectal cancer or large intestinal cancer;

[64] A birth inducer or preventative and/or therapeutic agent for cancer, prostatomegaly, male gonad dysfunction, infertility, premature birth, endometriosis, cirrhosis of the liver, hepatitis, hepatic insufficiency or calculus, containing an antisense polynucleotide which contains a nucleotide sequence or part of a nucleotide sequence complementary to a polynucleotide containing a polynucleotide which encodes a G protein-coupled receptor protein containing an amino acid sequence identical to or substantially identical to the amino acid sequence represented by SEQ ID NO: 9, or a partial peptide thereof;

[65] The agent according to [64], wherein the cancer is prostate cancer, non-small cell carcinoma, ovarian cancer, stomach cancer, bladder cancer, breast cancer, cervical cancer, colon cancer, rectal cancer or large intestinal cancer;

[66] A diagnostic drug for cancer, prostatomegaly, male gonad dysfunction, infertility, premature birth, endometriosis, cirrhosis of the liver, hepatitis, hepatic insufficiency or calculus, containing an antisense polynucleotide which contains a nucleotide sequence or part of a nucleotide sequence complementary to a polynucleotide containing a polynucleotide which encodes a G protein-coupled receptor protein containing an amino acid sequence identical to or substantially identical to the amino acid sequence of SEQ ID NO: 9, or a partial peptide thereof;

[67] The diagnostic drug according to [66], which is a diagnostic drug for cancer;

[68] The diagnostic drug according to [67], wherein the cancer is prostate cancer, non-small cell carcinoma, ovarian cancer, stomach cancer, bladder cancer, breast cancer, cervical cancer, colon cancer, rectal cancer or large intestinal cancer;

[69] A method of preventing and/or treating cancer, prostatomegaly, male gonad dysfunction, infertility, premature birth, endometriosis, cirrhosis of the liver, hepatitis, hepatic insufficiency or calculus, or a method of inducing birth, characterized in that an effective dose of a receptor protein containing an amino acid sequence identical to or substantially identical to the amino acid sequence represented by SEQ ID NO: 9, or a partial peptide or salt thereof, is administered to mammals;

[70] A method of preventing and/or treating cancer, prostatomegaly, male gonad dysfunction, infertility, premature birth, endometriosis, cirrhosis of the liver, hepatitis, hepatic insufficiency or calculus, or a method of inducing birth, characterized in that an effective dose of antibodies to a protein containing an amino acid sequence identical to or substantially identical to the amino acid sequence represented by SEQ ID NO: 9, or to a partial peptide or salt thereof, is administered to mammals;

[71] A method of preventing and/or treating cancer, prostatomegaly, male gonad dysfunction, infertility, premature birth, endometriosis, cirrhosis of the liver, hepatitis, hepatic insufficiency or calculus, or a method of inducing birth, characterized in that an effective dose of a compound or salt thereof which alters the binding properties of a G protein-coupled receptor protein containing an amino acid sequence identical to or substantially identical to the amino acid sequence represented by SEQ ID NO: 9, or of a partial peptide or salt thereof, with a protein showing affinity for a sugar chains, is administered to mammals;

[72] A method of preventing and/or treating cancer, prostatomegaly, male gonad dysfunction, infertility, premature birth, endometriosis, cirrhosis of the liver, hepatitis, hepatic insufficiency or calculus, or a method of inducing birth, characterized in that an effective dose of a compound or salt thereof which alters the expressed amount of a G protein-coupled receptor protein containing an amino acid sequence identical to or substantially identical to the amino acid sequence represented by SEQ ID NO: 9 is administered to mammals;

[73] A method of preventing and/or treating cancer, prostatomegaly, male gonad dysfunction, infertility, premature birth, endometriosis, cirrhosis of the liver, hepatitis, hepatic insufficiency or calculus, or a method of inducing birth, characterized in that an effective dose of a protein or salt thereof showing affinity for a sugar chain is administered to mammals;

[74] A method of preventing and/or treating cancer, prostatomegaly, male gonad dysfunction, infertility, premature birth, endometriosis, cirrhosis of the liver, hepatitis, hepatic insufficiency or calculus, or a method of inducing birth, characterized in that an effective dose of an antisense polynucleotide containing a nucleotide sequence or part of a nucleotide sequence complementary to a polynucleotide encoding a protein or partial peptide of a protein containing an amino acid sequence identical to or substantially identical to the amino acid sequence represented by SEQ ID NO: 9 is administered to mammals;

[75] A method of preventing and/or treating cancer, prostatomegaly, male gonad dysfunction, infertility, premature birth, endometriosis, cirrhosis of the liver, hepatitis, hepatic insufficiency or calculus, or a method of inducing birth, characterized in that an effective dose of a polynucleotide containing a polynucleotide which encodes a G protein-coupled receptor protein containing an amino acid sequence identical to or substantially identical to the amino acid sequence represented by SEQ ID NO: 9, or a partial peptide thereof, is administered to mammals;

[76] The use of a receptor protein containing an amino acid sequence identical to or substantially identical to the amino acid sequence represented by SEQ ID NO: 9, or a partial peptide or salt thereof, to manufacture a birth inducer or a preventative and/or therapeutic agent for cancer, prostatomegaly, male gonad dysfunction, infertility, premature birth, endometriosis, cirrhosis of the liver, hepatitis, hepatic insufficiency or calculus;

[77] The use of antibodies to a receptor protein containing an amino acid sequence identical to or substantially identical to the amino acid sequence represented by SEQ ID NO: 9, or to a partial peptide or salt thereof, to manufacture a birth inducer or a preventative and/or therapeutic agent for cancer, prostatomegaly, male gonad dysfunction, infertility, premature birth, endometriosis, cirrhosis of the liver, hepatitis, hepatic insufficiency or calculus;

[78] The use of a compound or salt thereof which alters the binding properties of a protein showing affinity for a sugar chain with a G protein-coupled receptor protein containing an amino acid sequence identical to or substantially identical to the amino acid sequence represented by SEQ ID NO: 9, or with a partial peptide or salt thereof, to manufacture a birth inducer or a preventative and/or therapeutic agent for cancer, prostatomegaly, male gonad dysfunction, infertility, premature birth, endometriosis, cirrhosis of the liver, hepatitis, hepatic insufficiency or calculus;

[79] The use of a compound or salt thereof which alters the expressed amount of a G protein-coupled receptor protein containing an amino acid sequence identical to or substantially identical to the amino acid sequence represented by SEQ ID NO: 9 to manufacture a birth inducer or a preventative and/or therapeutic agent for cancer, prostatomegaly, male gonad dysfunction, infertility, premature birth, endometriosis, cirrhosis of the liver, hepatitis, hepatic insufficiency or calculus;

[80] The use of a protein or salt thereof showing affinity for a sugar chain to manufacture a birth inducer or a preventative and/or therapeutic agent for cancer, prostatomegaly, male gonad dysfunction, infertility, premature birth, endometriosis, cirrhosis of the liver, hepatitis, hepatic insufficiency or calculus;

[81] The use of an antisense polynucleotide containing a nucleotide sequence or part of a nucleotide sequence complementary to a polynucleotide which encodes a protein containing an amino acid sequence identical to or substantially identical to the amino acid sequence represented by SEQ ID NO: 9 or a partial peptide thereof to manufacture a birth inducer or a preventative and/or therapeutic agent for cancer, prostatomegaly, male gonad dysfunction, infertility, premature birth, endometriosis, cirrhosis of the liver, hepatitis, hepatic insufficiency or calculus;

[82] The use of a polynucleotide containing a polynucleotide encoding a G protein-coupled receptor protein containing an amino acid sequence identical to or substantially identical to the amino acid sequence represented by SEQ ID NO: 9 or a partial peptide thereof to manufacture a birth inducer or a preventative and/or therapeutic agent for cancer, prostatomegaly, male gonad dysfunction, infertility, premature birth, endometriosis, cirrhosis of the liver, hepatitis, hepatic insufficiency or calculus;

[83] A G protein-coupled receptor protein containing an amino acid sequence identical to or substantially identical to the amino acid sequence represented by SEQ ID NO: 13, SEQ ID NO: 15, SEQ ID NO: 17, SEQ ID NO: 19, SEQ ID NO: 21, SEQ ID NO: 23, SEQ ID NO: 25 or SEQ ID NO: 27, or a salt thereof;

[84] A G protein-coupled receptor protein consisting of an amino acid sequence represented by SEQ ID NO: 13, SEQ ID NO: 15, SEQ ID NO: 17, SEQ ID NO: 19, SEQ ID NO: 21, SEQ ID NO: 23, SEQ ID NO: 25 or SEQ ID NO: 27, or a salt thereof;

[85] A polynucleotide containing the polynucleotide encoding the G protein-coupled receptor protein according to [81];

[86] DNA consisting of a nucleotide sequence represented by SEQ ID NO: 14, SEQ ID NO: 16, SEQ ID NO: 18, SEQ ID NO: 20, SEQ ID NO: 22, SEQ ID NO: 24, SEQ ID NO: 26 or SEQ ID NO: 28;

[87] A recombinant vector containing the polynucleotide according to [83];

[88] A transformant transformed by the recombinant vector according to [87];

[89] A method of manufacturing the G protein-coupled receptor protein or salt thereof according to [83], wherein the transformant according to [88] is cultured in order to produce the G protein-coupled receptor protein or salt thereof according to [83];
[90] Antibodies to the G protein-coupled receptor protein or salt thereof according to [83];
[91] DNA, which hybridizes under highly stringent conditions with DNA, consisting of a nucleotide sequence represented by SEQ ID NO: 14, SEQ ID NO: 16, SEQ ID NO: 18, SEQ ID NO: 20, SEQ ID NO: 22, SEQ ID NO: 24, SEQ ID NO: 26 or SEQ ID NO: 28;
[92] A polynucleotide containing a nucleotide sequence or part of a nucleotide sequence complementary to the polynucleotide according to [85].

It was also discovered using the method for analyzing gene expression of the present invention that EDG-1 receptors are highly expressed in vascular endothelial cells and EDG-2 receptors in vascular smooth muscle cells. Based on this finding, the present invention provides a method of screening EDG-1 receptor agonists and antagonists using vascular endothelial cells or vascular smooth muscle cells, or for EDG-2 receptor agonists or antagonists using vascular smooth muscle cells.

[93] A method for screening EDG-1 receptor agonists or antagonists, characterized by the use of vascular endothelial cells.
[94] The screening method according to [93], wherein the EDG-1 receptor is a G protein-coupled receptor protein containing an amino acid sequence identical to or substantially identical to the amino acid sequence represented by SEQ ID NO: 38, or a partial peptide or salt thereof.
[95] A screening kit for EDG-1 receptor agonists or antagonists, wherein are contained vascular endothelial cells.
[96] An EDG-1 receptor agonist or antagonist obtained using a screening method according to [93] or a screening kit according to [95].
[97] A preventative and/or therapeutic agent for arteriosclerosis, myocardial infarction, cerebral infarction or ischemic disease, containing an EDG-1 receptor agonist obtained using the screening method according to 93 above or the screening kit according to [85].
[98] A method of screening EDG-2 receptor agonists or antagonists, characterized by the use of vascular smooth muscle cells.
[99] The screening method according to [98], wherein the EDG-2 receptor is a G protein-coupled receptor protein containing an amino acid sequence identical to or substantially identical to the amino acid sequence represented by SEQ ID NO: 40, or a partial peptide or salt thereof.
[100] A kit for screening EDG-2 receptor agonists or antagonists, wherein are contained vascular smooth muscle cells.
[101] An EDG-2 receptor agonist or antagonist obtained using the screening method according to [98] or the screening kit according to [100].
[102] A preventative and/or therapeutic agent for arteriosclerosis, myocardial infarction, cerebral infarction or ischemic disease, containing an EDG-2 receptor agonist or antagonist obtained using the screening method according to [98] or the screening kit according to [100].
[103] A method of preventing and/or treating arteriosclerosis, myocardial infarction, cerebral infarction or ischemic disease, characterized in that an effective dose of an EDG-1 receptor agonist obtained using the screening method according to [93] or the screening kit according to [95], or an EDG-2 receptor antagonist obtained using the screening method according to [98] or the screening kit according to [100], is administered to mammals.
[104] The use of an EDG-1 receptor agonist obtained using the screening method according to [93] or the screening kit according to [95] or an EDG-2 receptor antagonist obtained using the screening method according to [98] or the screening kit according to [100] to manufacture a preventative and/or therapeutic agent for arteriosclerosis, myocardial infarction, cerebral infarction or ischemic disease, are provided by the present invention.

## BRIEF DESCRIPTION OF THE DRAWINGS

Figure 1 shows dJ287G14.2 expression in various cell lines.
Figure 2 shows the distribution of dJ287G14.2 receptor mRNA expression in various human tissues.
Figure 3 shows the amino acid sequence of the dJ287G14.2 receptor.
Figure 4 is a continuation of Figure 3, showing the amino acid sequence of the dJ287G14.2 receptor.
Figure 5 shows the nucleotide sequence of DNA encoding the dJ287G14.2 receptor.
Figure 6 is a continuation of Figure 5, showing the nucleotide sequence of DNA encoding the dJ287G14.2 receptor.
Figure 7 is a continuation of Figure 6, showing the nucleotide sequence of DNA encoding the dJ287G14.2 receptor.

Figure 8 is a continuation of Figure 7, showing the nucleotide sequence of DNA encoding the dJ287G14.2 receptor.

Figure 9 shows the results of an investigation into the effects of lectins on the amount of cAMP produced in CHO cells expressing a dJ287G14.2-GFP fused protein, with the horizontal axis indicating lectin concentration and the vertical axis cAMP production, where ConA is concanavalin A, Lentil is lentil lectin, Pea is pea lectin, and FSK is forskolin (Mean ± s.d. (n = 2)).

Figure 10 shows the results of an investigation into the effects of lectins on the amount of cAMP produced in mock CHO cells, with the horizontal axis indicating lectin concentration and the vertical axis cAMP production (mean values for n = 2), where ConA is concanavalin A, Lentil is lentil lectin, Pea is pea lectin, and FSK is forskolin (mean ± s.d. (n = 2)).

Figure 11 shows the results of observation with a confocal microscope (Leica) of the fluorescent image of GFP in a dJ287G14.2-GFP fused protein expressed in CHO cells.

Figure 12 shows the results of observation with a confocal microscope (Leica) of the fluorescent image of GFP when concanavalin A was added to dJ287G14.2-GFP fused protein expressed in CHO cells.

## BEST MODE FOR CARRYING OUT THE INVENTION

The present invention is described in detail below.

The present invention is characterized in that a gene the expression of which is characteristically promoted or inhibited in specific cells or tissue is identified by quantitatively analyzing the individual expressed amounts of multiple genes collectively, and in that the expression of multiple genes belonging to a specific gene family is analyzed collectively in order to identify a gene in that gene family the expression of which is characteristically promoted or inhibited in specific cells or tissue by computing the expressed amount thereof as an absolute value. To say that a gene's expression is characteristically promoted or inhibited signifies here that the gene's expression is greater or less to a physiologically significant degree than its expression in normal cells or tissue. In the present invention there are no particular limits on the gene families which are targeted, but they may be selected for example from the G protein-coupled receptor gene family, tyrosine kinase receptor gene family or ion channel gene family, or from gene families associated with transcription factors, transporters, protein kinases, protein phosphatases, proteases, heat shock proteins, ATPases, DNA-binding proteins or the like.

"Multiple genes" signifies 2 or more genes, and there is no particular upper limit as long as the number is practicable, but normally it is 2 to tens of thousands or preferably 2 to 1000 or more preferably 10 to 800 or ideally 10 to 300.

More specifically, the gene expression analysis of the present invention is carried out by bringing an mRNA sample which may contain multiple target mRNAs into contact with amplification reagents each of which contains a pair of primers corresponding to an individual target mRNA at multiple reaction sites or preferably the reaction sites of a reaction device having multiple reaction sites, performing an amplification reaction, and measuring produced amounts of the amplification products. This method is explained below.

(mRNA sample)

One mode of the present invention is a method of analyzing gene expression by assaying the mRNA of multiple target genes, which may be contained in an mRNA sample. The "mRNA sample" here signifies a sample which contains mRNA and which is used for measuring the types and amounts of mRNA contained in the sample.

More specifically, the mRNA samples used in the present invention are samples used for analyzing the expressed level of specific genes in the sample, and are for example samples collected from the tissues of humans or other mammals (such as rats, mice, rabbits, sheep, pigs, cows, cats, dogs, monkeys and the like) or cultured cell strains thereof. Examples of such tissues include the brain, various parts of the brain (such as the olfactory bulb, amygdaloid nucleus, basal ganglia, hippocampus, thalamus, hypothalamus, subthalamic nucleus, cerebral cortex, medulla oblongata, cerebellum, occipital lobe, frontal lobe, temporal lobe, putamen, caudate nucleus, cerebral gland and substantia nigra), spinal cord, pituitary gland, stomach, pancreas, kidneys, liver, gonads, thyroid, gall bladder, bone marrow, adrenal gland, skin, muscles, lungs, digestive tract (such as the large and small intestine), blood vessels, heart, thymus, spleen, submandibular gland, peripheral blood, peripheral blood cells, prostate, testicles, testes, ovaries, placenta, uterus, bone, joints, skeletal muscle and the like. Using such an mRNA sample it is possible to analyze the expressed level of a target gene at the site from which the mRNA sample was collected by assaying target mRNA contained in the sample.

Namely, with the present invention it is possible to measure in one operation from an mRNA sample containing multiple mRNAs whether and how much target mRNA is produced. In particular, it is easy using an mRNA sample from a patient suffering from a particular disease to specify a gene (such as a GPCR gene) associated with that disease. In particular, when specifying G protein-coupled receptors, tyrosine kinase receptors and ion channel genes involved in multiple gene-associated diseases such as cancers which are thought to involve multiple genes, it is easy to specify the associated genes or proteins with the present invention because the expressed levels of all genes can be measured with one assay operation.

Assaying expressed gene levels and computing absolute values for expressed gene levels can be accomplished according to the target mRNA assay method described below.

(Reaction device with multiple reaction sites)

There are no particular limits on the number of multiple reaction sites as long as there are two or more, but normally there are 2 to tens of thousands or preferably 2 to 1000 or more preferably 10 to 800 or still more preferably 10 to 300.

In order to amplify target mRNAs which may be contained in an mRNA sample in the present invention, the sample is reacted all at once with individual amplification reagents each of which contains a pair of primers corresponding to a target mRNA. Consequently, in the present invention reaction of the mRNA sample with the individual amplification reagents is performed at individual reaction sites, preferably using a reaction device having multiple reaction sites. There are no limits on the composition and structure of the reaction device used in the present invention as long as it has two or more reaction sites to allow simultaneous reaction of the mRNA sample with the individual amplification reagents. Desirable examples of the amplification device used in the present invention include plates having multiple wells, reaction devices equipped with multiple glass slides and reaction devices equipped with multiple test tubes. Plates having multiple wells can be used by preference out of consideration of test space and operability. The plates can be selected depending on the number of primer pairs used, but commercially available 96-well and 384-well plates can be used by preference. However, a reaction device equipped with a desired number of reaction spites corresponding to the number of primer pairs can also be used. The method of the present invention can also be realized using 2 or more commercially available 96-well or 384-well plates.

(Amplification reagents)

Each of the amplification reagents used in the present invention comprises for example a primer pair corresponding to a target mRNA, a probe corresponding to a target mRNA, DNA polymerase, buffer and the like.

As used here, "a primer pair corresponding to a target mRNA" signifies a pair of primers consisting of a first primer complementary to or substantially complementary to one chain of the exon region of a target gene sequence encoding a target mRNA, and a second primer complementary to or substantially complementary to the other chain of the exon region of the target gene sequence. In the present invention, the presence or absence of mRNA transcripted from target genes in an mRNA sample and the transcripted amounts thereof can be detected all at once by using at least two or more of such primer pairs. Consequently; the greater the number of primer pairs used, the more efficient the assay method of the present invention.

In the assay method of the present invention, there are no particular limits on the group of primer pairs used because it can be adjusted according to the number of target mRNAs, but for example it may consist of 10 to 800 primer pairs. In another mode of the present invention, the group of primer pairs used consists of 10 to 300 primer pairs. When the number of primer pairs is large, different sets of amplification reagents can be assigned to a number of plates (such as 2 to 10 plates), and the amplification reaction performed multiple times.

According to a preferred mode of the present invention, the target gene is a gene encoding a G protein-coupled receptor, tyrosine kinase receptor, ion channel or the like, and thus the target mRNA is gene mRNA belonging to the G protein-coupled receptor, tyrosine kinase receptor, ion channel or other family. In this case, an amplification reaction is performed by bringing amplification reagents each comprising a primer pair corresponding to gene mRNA belonging to a target G protein-coupled receptor, tyrosine kinase receptor, ion channel or other family into contact with an mRNA sample in the respective reaction sites of a reaction device, and the mRNA amplification products are assayed in order to measure the expressed amounts of genes belonging to the G protein-coupled receptor, tyrosine kinase receptor, ion channel and other families which were contained in the mRNA sample.

In a more preferred mode of the present invention, if all primer pairs are prepared corresponding to gene mRNA belonging to all known G protein-coupled receptor, tyrosine kinase receptor, ion channel and other families, and amplification reagents comprising the respective primer pairs are set in each reaction site, the degree to which gene mRNA belonging to any G protein-coupled receptor, tyrosine kinase receptor, ion channel or other family is produced in an mRNA sample can be assessed with a single assay operation. Of course, it is also possible when necessary to assign different sets of amplification reagents to different plates, and perform the assay reaction multiple times.

At present, the following G protein-coupled receptors (or genes) are known:

(1) Acetylcholine receptors: $M_1$; $M_2$; $M_3$; $M_4$; $M_5$

(2) Adenosine receptors: $A_1$; $A_{2A}$, $A_{2B}$, $A_3$
(3) Adrenoceptors: α1A; α1B; α1D; α2A; α2B; α2C; β1; β2; β3
(4) Angiotensin receptors: AT1; AT2
(5) Bombesin receptors: BB1; BB2; bb3
(6) Bradykinin receptors: $B_1$; $B_2$
(7) Calcitonin, amylin, CGRP and adrenomedullin receptors:
(8) Cannabinoid receptors: CB 1; CB2
(9) Chemokine receptors: CCR1; CCR2; CCR3; CCR4; CCR5; CCR6; CCR7; CCR8; CCR9; CCR10; CXCR1; CXCR2; CXCR3; CXCR4; CXCR5; $CX_3CR1$; XCR1
(10) Cheniotactic receptors: C3a; C5a; fMLP
(11) Cholecystokinin and gastrin receptors: $CCK_1$; $CCK_2$
(12) Corticotropin-releasing factor receptors: $CRF_1$; $CRF_2$
(13) Dopamine receptors: D1; D2; D3; D4; D5
(14) Endothelin receptors: $ET_A$; $ET_B$
(15) Galanin receptors: GAL1; GAL2; GAL3
(16) Glutamate receptors: $mglu_1$; $mglu_2$; $mglu_3$; $mglu_4$; $mglu_5$; $mglu_6$; $mglu_7$; $mglu_8$
(17) Glycoprotein hormone receptors: FSH; LSH; TSH
(18) Histamine receptors: $H_1$; $H_2$; $H_3$; $H_4$
(19) 5-HT receptors: 5-$HT_{1A}$; 5-$HT_{1B}$; 5-$HT_{1D}$; 5-$ht_{1B}$; 5-$ht_{1F}$; 5-$HT_{2A}$; 5-$HT_{2F}$; $5HT_{2C}$; 5-$HT_3$; 5-$HT_4$; 5-$ht_{5A}$; 5-$ht_{5B}$; 5-$HT_6$; 5-$HT_7$
(20) Leukotriene receptors: BLT, $CysLT_1$, $CysLT_2$
(21) Lysophospholipid receptors: edg1; edg2; edg3; edg4
(22) Melanocorlin receptors: $MC_1$; $MC_2$; $MC_3$; $MC_4$; $MC_5$
(23) Melatonin receptors: $MT_1$; $MT_2$; $MT_3$
(24) Neuropeptide Y receptors: $Y_1$; $Y_2$; $Y_4$; $Y_5$; $Y_6$
(25) Neurotension receptors: NTS1; NTS2
(26) Opioids: DOP; KOP; MOP; NOP
(27) P2Y receptors:$P2Y_1$; $P2Y_2$; $P2Y_4$; $P2Y_6$; $P2Y_{11}$; $P2Y_{12}$
(28) Peroxisome proliferator-activated receptors: PPAR-α; PPAR-β; PPAR-γ
(29) Prostanoid receptors: DP; FP; IP; TP; $EP_1$; $EP_2$; $EP_3$; $EP_4$
(30) Protease-activated receptors: PAR1; PAR2; PAR3; PAR4
(31) Somatostatin receptors: $sst_1$; $sst_2$; $sst_3$; $sst_4$; $sst_5$
(32) Tachykinin receptors: $NK_1$; $NK_2$; $NK_3$
(33) Thyrotropin-releasing hormone receptors: $TRH_1$; $TRH_2$
(34) Urotensin-II receptors:
(35) Vasoactive intestinal peptide and pituitary adenylate cyclase activating peptide receptors: $VPAC_1$; $VPAC_2$; $PAC_1$
(36) Vasopressin and oxytocin receptors: $V_{1a}$; $V_{1b}$; $V_2$; OT. At present the following genes belonging to the tyrosine kinase receptor, ion channel gene and other families are known:
(37) Ion channels: $Na^+$ channels (Type I; Type II/Type IIA; Type III; SCL11/NaG; PN1; NaCh6; NaDRG; SkM1/μ1, SkM2), $K^+$ channels (Kv; EAG; KQT; IRK; ROMK; GIRK; $K_{ATP}$ and the like), $Ca^{2+}$ channels (α1G; α1E; α1S; α1C; α1D; α1B; α1A; IP3; ryanodine receptor and the like), $Cl^-$ channels ($GABA_A$; $GABA_C$; glycine receptor; C1C0; C1C1; CFTR and the like), non-selective cation channels (nAChR; 5-$HT_3$; NMDA; AMPA; $P_{2X}$ATP; CNG and the like) and others
(38) Tyrosine kinase receptors: insulin receptors; EGF receptors and the like.

In another mode of the present invention, it is possible by using a group of primer pairs corresponding to a group of mRNAs of genes belonging to a G protein-coupled receptor, tyrosine kinase receptor or ion channel family to specify in one assay operation the genes belonging to the G protein-coupled receptor, tyrosine kinase receptor, ion channel or other family which are highly expressed among the genes belonging to the G protein-coupled receptor, tyrosine kinase receptor, ion channel or other family.
For example, targets for drug creation can be classified into three types: (1) proteins (GPCR, intranuclear receptors, ion channels and the like) which bind low molecular weight substances, (2) enzymes with catalytic activity towards low molecular weight or high molecular weight substrates, and (3) proteins that bind to macromolecules such as proteins, nucleic acids and polysaccharides. Following this classification, genes can be grouped as follows.

(1) Genes associated with the following proteins, which bind low molecular weight substances:

Adrenaline receptors; acetylcholine receptors; histamine receptors; dopamine receptors; serotonin receptors; glutamine receptors, endothelin receptors; vasopressin receptors; serotonin transporters; glucocorticoid receptors; estrogen receptors; $Ca^{2+}$ channels; $Na^+$ channels; $Cl^-$ channels

(2) Genes associated with the following enzymes having catalytic activity towards low molecular weight or high molecular weight substrates:

HMG-CoA reductase; angiotensin convertase; thromboxane synthase; proton pump; aldose reductase; cyclooxygenase; phosphodiesterase; protein phosphorylase; protein dephosphorylase; HIV reverse transcriptase; fungal squalene epoxidase; DNA gyrase; beta-lactamase

(3) Genes associated with the following proteins, which bind to proteins, nucleic acids, polysaccharides and other macromolecules:

Insulin receptor; erythropoietin receptor; G-CSF receptor; growth hormone receptor; interferon receptor; growth factor receptor HER2; TNF receptor; platelet integrin GPIIb/IIIa
The pair of primers for amplifying the target mRNA can be easily designed by a person having ordinary skill in the field based on the sequence of the target GPCR gene (see for example *Genome Res.* 1996 Oct, 6(10): 986-94).

The method used to assay mRNA in the present invention can be used for a variety of purposes other than hGPCR function analysis. For example, a specific disease can be diagnosed by using a set of multiple amplification regents comprising pairs of primers, which detect mRNA produced by the known disease gene. Because the expressed level of each gene can be measured accurately by the present invention, it offers the advantage of more accurate diagnosis than prior methods.
Next, probes corresponding to the target mRNAs used in the present invention can be designed easily by a person having ordinary skill in the field based on the target sequence (see for example *Genome Res.* 1996 Oct, 6(10): 986-94). Suitable oligonucleotide probes for use in the present invention have a length of preferably about 15 to about 50 nucleotides or more preferable about 25 to about 35 nucleotides. The oligonucleotide probes can be labeled by the incorporation of a detectable chemical substance or the like by biochemical, immunochemical or chemical means. Useful labels include $^{32}P$ and other radioactive isotopes, fluorescamine, fluorescein isothiocyanate and other fluorescent substances, luminol, luciferin and other luminous substances, beta-galatosidase, peroxidase, alkaliphosphatase and other enzymes, biotin and antibodies and the like. Examples of DNA polymerase which can be used in the present invention include heat-resistant DNA polymerase having reverse transcription and 5'-->3' exonuclease activity, such as rTth DNA polymerase and the like.
Well-known or commercially available buffers can be used in the present invention (for example PE Biosystems buffer, see *Genome Res.* 1996 Oct, 6(10): 986-94).

(Amplification of mRNA)

In the analysis method of the present invention, target mRNA, which may be contained in an mRNA sample, is amplified using an amplification reagent comprising a primer pair for amplifying said target mRNA. In a preferred mode of the present invention, amplification of the target mRNA is accomplished using a conventional polymerase chain reaction (PCR) (see U.S. Patents Nos. 4,683,195, 4,683,202 and 4,965,188 and the like).
mRNA can be amplified by reversely transcribing the target mRNA using for example a virus reverse transcriptase, and then amplifying the resulting cDNA. In a more preferred mode, mRNA is amplified using a reverse transcriptase polymerase chain reaction (RT-PCR) (see U.S. Patents Nos. 5,310,652, 5,322,770, 5,561,058, 5,641,864, 5,693,517 and the like).
A variety of mRNA amplification methods other than the aforementioned polymerase chain reaction can be used in the present invention. Such amplification methods include for example strand-displacement amplification (U. S. Patent No. 5,455,166 and the like), transcription-based amplification system (TAS) (U.S. Patents Nos. 5,437,990, 5,409,818, 5,399,491 and the like) and self-sustained sequence replication (3SR) (WO 92/08800 and the like).
The'conditions for these amplification reactions can be easily designed by a person having ordinary skill in the field according to the types of reagents used and the like.

(Methods of assaying target mRNA)

Next, the amount of the aforementioned mRNA amplification product produced is assayed in the analysis method of the present invention. Preferably the amplification product is assayed by a method employing a probe. In a preferred mode, it is assayed by a method employing a probe labeled with a fluorescent substance.

In a more preferred mode of the present invention, the target mRNA is assayed using the "TaqMan method" or "5' nuclease assay method" (*Proceedings of the National Academy of Sciences U.S.A.,* Vol. 88, pp. 7276-7280 (1991); U.S. Patents Nos. 5,210, 015, 5,487,972, 5,804,375 and the like). In the TaqMan assay method, a probe labeled at the 5'-terminal is used. The 3'-terminal of this probe is also modified to prevent the probe from acting as a primer for DNA synthesis. Modification can be by addition of a phosphate base, fluorescent substance or the like to the end. Amplification of target mRNA is accomplished using DNA polymerase having 5'-->3' exonuclease activity, such as Tth DNA polymerase. The probe, which hybridizes with target mRNA downstream from the primer, is degraded by the 5'-->3' exonuclease activity of the DNA polymerase during the amplification reaction. Each time a new target region is amplified, the probe is degraded and label substance released. The amount of target mRNA can be measured indirectly by assaying this released label substance.

Well-known methods can be used to quantitatively detect the released label substance. In a preferred method, the probe is labeled at the 5'- and 3'-terminals with two fluorescent substances, one of which can quench the fluorescence of the other substance. The fluorescence of this probe is quenched by the interaction of the two fluorescent substances as the probe hybridizes with the template DNA, but it emits fluorescence as it is broken down by the 5'-->3' exonuclease activity of the DNA polymerase. As the amplification reaction progresses the fluorescence increases and this increase is monitored.

A sample containing target mRNA is assayed based on a "standard curve" which is prepared by amplifying a sample containing a previously known quantity of target mRNA. The standard curve is used to calculate the number of input copies, which are derived from signals emitted during amplification. Consequently, an unknown number of copies of a target sequence in a sample is estimated by calculating the number of copies which were determined in advance to emit a signal equivalent to what is observed. Next, the concentration of the target sequence in the sample can be calculated from the number of input copies determined before reaction and the size of the sample (see Kokai No. 2001-204483 and the like).

An internal standard can also be used to eliminate experimental errors. Assay methods based on amplification which use internal standards are disclosed in U.S. Patents Nos. 5,219,717 and 5,476,774 and the like. Various methods are known for improving the specificity of the amplification reaction, such as that described for example in European Patent Application No. 0866,071 and the like.

(Primer pair kit and assay kit)

The present invention a also relates to a primer pair kit and mRNA assay kit for performing the method of the present invention.

The primer pair kit of the present invention is a kit comprising two or more pairs of primers each consisting of a first primer which is complementary or substantially complementary to one chain of the exon region of a target gene sequence and a second primer which is complementary or substantially complementary to the other chain of the exon region of a target gene sequence. The assay method of the present invention can be performed rapidly if such a kit is prepared beforehand. A preferred primer pair kit of the present invention has a pair of primers corresponding to mRNA transcripted from a G protein-coupled receptor protein gene. In one mode of the present invention, the primer pair kit comprises a group of primer pairs consisting of 10 to 800 primer pairs or 10 to 300 primer pairs.

In a more preferred mode of the present invention, an mRNA assay kit is provided wherein each of the reaction sites of a reaction device having multiple reaction sites is filled with an amplification reagent which comprises a primer pair corresponding to a target mRNA. Preferably, the target gene in this kit is a disease-associated gene. More preferably, the kit also comprises a fluorescent probe, and still more preferably it comprises Tth DNA polymerase. As necessary, these kits may also contain for example chemicals which catalyze synthesis of primer elongation products, substrate nucleoside triphosphate, means used for labeling (such as avidin-enzyme conjugate, enzyme substrate and pigment base for example if the label is avidin), suitable buffers for the PCR or hybridization reaction and the like.

(Means of diagnosing diseases and drugs for treating those diseases)

Next, gene expression analysis can be performed according to the method of (30) above of the present invention using an mRNA sample taken from a patient, and a disease from which the patient suffers diagnosed by detecting the characteristic expression of a specific disease-associated gene. With the present invention, it is possible by collectively analyzing the expression of genes belonging to a specific disease-associated gene facility (such as a specific disease-associated GPCR gene family) to specify in one operation the gene out of multiple specific disease-associated genes whose expression is characteristic.

Consequently, drugs containing agonists, antagonists or antibodies to gene products of a gene specified in this way or DNA encoding such gene products are particularly useful for patients with such a diagnosis. In the present invention multiple genes with abnormal expression can be specified, and the expression levels of those genes can even be assayed accurately. As a result, multiple agonists, antagonists or antibodies can be selected as suitable for prescription to the patient, and the prescribed amounts can be adjusted according to the expressed level of the disease-associated gene. In other words, with the present invention it is possible to devise so-called tailor-made drugs, which are prescribed only for that individual patients.

More specifically, if the physiological function of a ligand cannot be relied upon because a particular receptor protein is reduced in the body of a patient (deficiency of the receptor protein), the action of the ligand can be adequately restored by (1) administering the receptor protein to the patient to replace the missing receptor protein, or (2) increasing the amount of receptor protein in the patient's body by either (i) inducing expression by administering DNA encoding the receptor protein of the present invention to the patient, or (ii) inducing expression by inserting DNA encoding the receptor protein of the present invention into target cells, and transplanting the cells into the patient.

The drug of the present invention is effective for preventing or treating diseases involving a specific gene, and is useful for example for preventing and/or treating central disorders (such as Alzheimer's disease, dementia, eating disorders and the like); endocrine disorders (such as hypertension, gonad dysfunction, thyroid dysfunction, pituitary dysfunction and the like); metabolic disorders (such as diabetes, abnormal lipid metabolism, hyperlipidemia and the like), cancer (such as non-small cell carcinoma, ovarian cancer, prostate cancer, stomach cancer, bladder cancer, breast cancer, cervical cancer, colon cancer, rectal cancer and the like) and others.

When the genetic product of a gene specified by the present invention (such as a receptor protein), an agonist, antagonist or antibodies thereto, or DNA encoding that gene is used as the aforementioned preventative and/or therapeutic agent, it can be prepared by conventional means.

When DNA (sometimes abbreviated below as the DNA of the present invention) is used as the aforementioned preventative or therapeutic agent, such DNA can be applied by ordinary means either alone or after insertion into a suitable vector such as a retrovirus vector, adenovirus vector, adenovirus-associated virus vector or the like. Such DNA can be administered with a gene gun or hydrogel catheter or other catheter, either as is or together with adjuvants for promoting intake.

For example, the drug used in the present invention can be used orally in the form of tablets with a sugar coating as necessary, capsules, elixir, microcapsules or the like, or parenterally as an injection of a suspension or sterile solution with water or another pharmacologically acceptable liquid or the like. For example, the drug of the present invention can be manufactured by mixing with physiologically acceptable known carriers, flavorings, excipients, vehicles; preservatives, stabilizers, binders and the like in the unit dose form required by generally accepted preparation practice. The amount of the active ingredient in such preparations is such as to provide an appropriate dose within the indicated range.

Additives which can be mixed into tablets, capsules and the like include for example gelatin, corn starch, tragacanth, gum arabic or other binders, crystal cellulose and other excipients, corn starch, gelatin, alginic acid or other swelling agents, magnesium stearate or other lubricants, sucrose, lactose, saccharin or other sweeteners, and peppermint, *akamono* oil, cherry or other flavorings. When the dispensing unit form is capsules, a liquid carrier such as fat or oil can be included in addition to the aforementioned types of ingredients. A sterile composition for injection may be formulated by ordinary means of preparation such as dissolving or suspending the active substance and sesame seed oil, coconut oil or another naturally-produced vegetable oil or the like in a vehicle such as injection water. Physiological saline for example or an isotonic solution containing glucose or another adjuvant (such as D-sorbitol, D-mannitol, sodium chloride or the like) can be used as the aqueous solution for injection, and suitable solubilizers such as alcohols (ethanol for example), polyalcohols (propylene glycol or polyethylene glycol for example), non-ionic surfactants (polysorbate 80™ or HCO-50 for example) may be included. Sesame seed oil, soybean oil or the like for example can be used as an oily liquid, and may be used in conjunction with a solubilizer such as benzyl benzoate, benzyl alcohol or the like.

The aforementioned preventative and/or therapeutic agent may also be combined with buffers (such as phosphate buffer or sodium acetate buffer), analgesics (such as benzalkonium chloride, procaine hydrochloride and the like), stabilizers (such as human serum albumin, polyethylene glycol and the like), preservatives (such as benzyl alcohol, phenol and the like), antioxidants and the like. The formulated injection liquid is normally packed in suitable ampoules.

Since the resulting preparation is stable and low toxic, it can be administered for example to humans and other mammals (such as rats, mice, rabbits, sheep, pigs, cows, cats, dogs, monkeys and the like).

The dosage of the preventative and/or therapeutic agent of the present invention differs depending on the subject of administration, organ, symptoms, method of administration and the like, but for oral administration it is normally about 0.1 mg to 100 mg or preferably about 1.0 to 50 mg or more preferably about 1.0 to 20 mg a day for example in the case of a cancer patient (weight 60 kg). For parenteral administration, the single administered dose differs depending on the subject of administration, organ, symptoms, method of administration and the like, but in the case of injection normally about 0.01 to 30 mg or preferably about 0.1 to 20 mg or more preferably about 0.1 to 10 mg a day for example should be administered by intravenous injection in the case of a cancer patient (weight 60 kg). In the case of other animals, the dose can be converted from the 60 kg dose.

The dose of the DNA of the present invention differs depending on the subject of administration, organ, symptoms and administration method, but for oral administration it is normally about 0.1 mg to 100 mg or preferably about 1.0 to 50 mg or more preferably about 1.0 to 20 mg a day for example in the case of a cancer patient (weight 60 kg). For parenteral administration, the single administered dose differs depending on the subject of administration, organ, symptoms, method of administration and the like, but for example in the case of injection normally about 0.01 to 30 mg or preferably about 0.1 to 20 mg or more preferably about 0.1 to 10 mg a day for example should be administered by intravenous injection in the case of a cancer patient (weight 60 kg). In the case of other animals, the dose can be converted from the 60 kg dose.

A novel use for a receptor protein comprising an amino acid sequence identical to or substantially identical to the amino acid sequence represented by SEQ ID NO: 9 for example which is obtained using the aforementioned gene expression analysis method of the present invention is explained below.

(Receptor protein)

The receptor protein used in the present invention is a receptor protein (sometimes referred to hereunder as the receptor protein of the present invention) comprising an amino acid sequence identical or substantially identical to the amino acid sequence represented by SEQ ID NO: 9. The protein having the amino acid sequence represented by SEQ ID NO: 9 is the human-derived dJ287G14.2 receptor protein. The human-derived dJ287G14.2 receptor has an amino acid sequence identical to that of the protein described in WO 2001/18207.

The present inventors confirmed for the first time that a protein exhibiting an affinity for a sugar chains such as an asparagine-linked sugar chain or serine/threonine-linked sugar chain (such as concanavalin A (ConA), lentil lectin, pea lectin, Datura lectin, Maackia Amurensis lectin, phytohemagglutinin and other lectins for example) is one of the ligands of the dJ287G14.2 receptor. The receptor protein of the present invention may be a protein derived from any cells (such as retinal cells, spleen cells, nerve cells, glia cells, pancreatic beta cells, bone marrow cells, mesangial cells, Langerhans' cells, epidermal cells, epithelial cells, endothelial cells, fibroblasts, fibrocytes, muscle cells, fat cells, immune cells (such as macrophages, T cell, B cells, natural killer cells, mast cells, neutrophils, basophils, acidophils, monocytes or luekocytes), megakaryocytes, synovial cells, cartilage cells, bone cells, osteoblasts, osteoclasts, mammary gland cells, liver cells or interstitial cells, or precursor cells, stem cells or cancer cells of these cells (such as breast cancer cell lines (GI-101), colon cancer cell lines (CX-1, GI-112), lung cancer cell lines (LX-1, GI-117), ovarian cancer cells lines (GI-102), prostate cancer cell lines) or the like) of humans and other mammals (such as guinea pigs, rats, mice, rabbits, pigs, sheep, cows, monkeys and the like), or from any tissues wherein these cells are present, such as the brain, various parts of the brain (such as the olfactory bulb, amygdaloid nucleus, basal ganglia, hippocampus, thalamus, hypothalamus, subthalamic nucleus, cerebral cortex, medulla oblongata, cerebellum, occipital lobe, frontal lobe, temporal lobe, putamen, caudate nucleus, cerebral gland and substantia nigra), spinal cord, pituitary gland, stomach, pancreas, kidneys, liver, gonads, thyroid, gall bladder, bone marrow, adrenal gland, skin, muscles, lungs, digestive tract (such as the large and small intestine), blood vessels, heart, thymus, spleen, submandibular gland, peripheral blood, peripheral blood cells, prostate, testicles, testes, ovaries, placenta, uterus, bone, joints, skeletal muscle and the like, or from blood cells or cultured cells thereof (such as MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01 and the like), or it may be a synthetic protein.

In these Specifications, a "substantially identical amino acid sequence" signifies an amino acid sequence having about 50% or greater or preferably about 60% or greater or more preferably about 70% or greater or even more preferably about 80% or greater or still more preferably about 90% or greater or most preferably about 95% or greater homology with the compared amino acid sequence.

A protein or the like comprising an amino acid sequence substantially identical to the amino acid sequence represented by SEQ ID NO: 9 for example and having substantially the same activity as a protein comprising the amino acid sequence represented by SEQ ID NO: 9 for example is preferred as the protein comprising an amino acid sequence substantially identical to the amino acid sequence represented by SEQ ID NO: 9.

"Substantially the same activity" refers for example to ligand binding activity, signal transmission action and the like. Substantially the same means that these activities are the same in character. Consequently, the ligand binding activity, signal transmission action or other activity is preferably equivalent (such as about 0.01 to 100 times or preferably about 0.5 to 20 times or more preferably about 0.5 to 2 times), but quantitative factors such as the degree of activity and the molecular weight of the protein may be different.

Ligand binding activity, signal transmission action and other activity can be measured according to well-known methods, and for example can be measured according to the ligand determination method and screening method described below.

Moreover, a protein comprising (1) an amino acid sequence having 1 or 2 or more (preferably about 1 to 30 or more preferably 1 to 10 or still more preferably several (1 to 5)) amino acids deleted from the amino acid sequence represented by SEQ ID NO: 9, SEQ ID NO: 13, SEQ ID NO: 15, SEQ ID NO: 17, SEQ ID NO: 19, SEQ ID NO: 21, SEQ ID NO: 23, SEQ ID NO: 25 or SEQ ID NO: 27, (2) an amino acid sequence having 1 or 2 or more (preferably about 1 to 30 or more preferably 1 to 10 or still more preferably several (1 to 5)) amino acids added to the amino acid sequence represented by SEQ ID NO: 9, SEQ ID NO: 13, SEQ ID NO: 15, SEQ ID NO: 17, SEQ ID NO: 19, SEQ ID NO: 21, SEQ ID NO: 23, SEQ ID NO: 25 or SEQ ID NO: 27, or (3) an amino acid sequence having 1 or 2 or more (preferably about 1 to 30 or more preferably 1 to 10 or still more preferably several (1 to 5) amino acids replaced by other amino acids in to the amino acid sequence represented by SEQ ID NO: 9, SEQ ID NO: 13, SEQ ID NO: 15, SEQ ID NO: 17, SEQ ID NO: 19, SEQ ID NO: 21, SEQ ID NO: 23, SEQ ID NO: 25 or SEQ ID NO: 27, or (4) an amino acid sequence which is a combination of these or the like can also be used as the receptor protein of the present invention.

Following peptide marking conventions, the left end of the receptor protein of the present specifications is the N terminus (amino terminus) and the right end is the C terminus (carboxyl terminus). The C terminus of the receptor protein of the present invention may be a carboxyl group (-COOH), carboxylate ($-COO^-$), amide ($-CONH_2$) or ester (-COOR).

A methyl, ethyl, n-propyl, isopropyl or n-butyl or other $C_{1-6}$ alkyl group for example, a cyclopentyl, cyclohexyl or other $C_{3-8}$ cycloalkyl group for example, a phenyl, alpha-naphthyl or other $C_{6-12}$ aryl group for example, a benzyl, phenethyl or other phenyl-$C_{1-2}$ alkyl group for example or an alpha-naphthylmethyl or other alpha-naphthyl-$C_{1-2}$ alkyl group or other $C_{7-14}$ aralkyl group or else a pivaloyl oxymethyl group or the like widely used as an ester for oral use can be used as the R in the ester here.

When the receptor protein of the present invention has a carboxyl group (or carboxylate) somewhere other than the C terminus, it is included in the receptor protein of the present invention if the carboxyl group is amidified or esterified. The aforementioned ester of the C terminus or the like for example can be used as the ester in this case.

The aforementioned protein in which the amino group of the methionine residue of the N terminus is protected by a protective group (such as a formyl group, acetyl or other $C_{2-6}$ alkanoyl or other $C_{1-6}$ acyl group or the like), in which a glutamyl group produced by nicking of the N end in vivo is pyroglutaminated, or in which a substitutional group (such as an -OH, -SH, amino group, imidazole group, indole group, guanidino group or the like) on a side chain of an amino acid in the molecule is protected by a suitable protective group (such as a formyl group, acetyl or other $C_{2-6}$ alkanoyl or other $C_{1-6}$ acyl group or the like) is included as the receptor protein of the present invention, as are composite proteins such as so-called glycoproteins having bound sugar chains.

Specific examples of the receptor protein of the present invention include for example a human-derived dJ287G14.2 receptor protein consisting of the amino acid sequence represented by SEQ ID NO: 9, a mouse-derived dJ287G14.2 receptor protein consisting of an amino acid sequence represented by SEQ ID NO: 13, SEQ ID NO: 15, SEQ ID NO: 17, SEQ ID NO: 19, SEQ ID NO: 21, SEQ ID NO: 23, SEQ ID NO: 25 or SEQ ID NO: 27 and the like.

The mouse-derived dJ287G14.2 receptor protein consisting of the amino acid sequence represented by SEQ ID NO: 13, SEQ ID NO: 15, SEQ ID NO: 17, SEQ ID NO: 19, SEQ ID NO: 21, SEQ ID NO: 23, SEQ ID NO: 25 or SEQ ID NO: 27 is a novel protein.

The receptor protein or salt thereof of the present invention may be manufactured from the aforementioned human or mammalian cells or tissue by well-known methods of purifying receptor proteins, or may be manufactured by culturing the transformant described below which comprises DNA encoding the receptor protein of the present invention. It may also be manufactured according to the protein synthesis methods described below or corresponding methods.

In the case of manufacture from human or mammalian tissue or cells, the human or mammalian tissue or cells are first homogenized, then extracted with acid or the like, and the extract can be purified and isolated by a combination of chromatographic methods such as reverse-phase chromatography, ion exchange chromatography and the like.

(Partial peptide of the receptor protein)

The partial peptide of the present invention (sometimes abbreviated hereunder as "partial peptide") may be any peptide having a partial amino acid sequence of the receptor protein of the present invention, but for example of the molecules of the receptor protein of the present invention, sites which are exposed outside the cell membrane and have receptor binding activity substantially equivalent to that of the receptor protein of the present invention and the like are used.

Specifically, partial peptides of the receptor protein of the present invention having the amino acid sequence represented by SEQ ID NO: 9 are peptides which include a part analyzed by hydrophobic plot analysis as being an extracellular region (hydrophilic site). Peptides comprised in part of a hydrophobic site can be used in the same way.

Peptides, which separately comprise individual domains, can be used, as can peptides of a part, which comprises multiple domains simultaneously.

As for the number of amino acids in the partial peptide of the present invention, a peptide having an amino acid sequence of at least 20 or more or preferably 50 or more or more preferably 100 or more out of the constituent amino acid sequence of the receptor protein of the present invention or the like is preferred.

Substantially identical amino acid sequences are amino acid sequences having about 50% or greater or preferably about 60% or greater or more preferably about 70% or greater or even more preferably about 80% or greater or still more preferably about 90% or greater or most preferably about 95% or greater homology with these amino acid sequences.

"Substantially equivalent receptor binding activity" has the same significance here as above. "Substantially equivalent receptor binding activity" is measured in the same way here as above.

The partial peptide of the present invention may also have 1 or two or more (preferably about 1 to 10 or preferably several (1 to 5)) amino acids deleted from the aforementioned amino acid sequence, 1 or two or more (preferably 1 to 20 or more preferably 1 to 10 or still more preferably several (1 to 5)) amino acids added to the aforementioned amino acid sequence, or 1 or 2 or more (preferably about 1 to 10 or more preferably several or still more preferably 1 to 5) amino acids replaced by other amino acids in the aforementioned amino acid sequence.

The C terminus of the partial peptide of the present invention may be a carboxyl group (-COOH), carboxylate ($-COO^-$), amide ($-CONH_2$) or ester (-COOR).

As with the receptor protein of the present invention described above, the partial peptide of the present invention also includes those in which the amino group of the methionine residue of the N terminus is protected by a protective group, those in which a Gln produced by nicking of the N end in vivo is pyroglutaminated, and those in which a substitutional group on a side chain of an amino acid in the molecule is protected by a suitable protective group, as well as so-called glycopeptides having bound sugar chains and the like.

Examples of salts of the receptor protein or partial peptide thereof of the present invention include physiologically allowable salts with acids and bases. Physiologically allowable acid-added salts are particularly desirable. Such salts, which can be used, include salts with inorganic acids (such as hydrochloric acid, phosphoric acid, hydrobromic acid, sulfuric acid) and salts with organic acids (such as acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The partial peptide or salt thereof of the present invention can be manufactured by well-known methods of peptide synthesis or by nicking the GPCR of the present invention with a suitable peptidase. Peptide synthesis may be for example by either solid-phase or liquid-phase synthesis. That is, a partial peptide or amino acids, which may constitute the GPCR of the present invention, can be condensed with the residual part, and the target peptide manufactured by removing the protective groups if the product has protective groups. Conventional methods of condensing and removing protective groups include those described in a) through e) below.

a) M. Bodanszky and M.A. Ondetti, Peptide Synthesis, Interscience Publishers, New York (1966)
b) Schroeder and Luebke, The Peptide, Academic Press; New York (1965)
c) Izumiya, Nobuo et al, Fundamental and Experimental Peptide Synthesis, Maruzen (1975)
d) Yashima, Haruaki and Sakakibara, Shunpei, Biochemical Experimental Course 1, Protein Chemistry IV, 205 (1977)
e) Yashima, Haruaki ed, Drug Development Vol.14, Peptide Synthesis, Hirokawa Shoten

After the reaction the partial peptide of the present invention can be purified and isolated by a combination of solvent extraction, distillation, column chromatography, liquid chromatography, recrystallization and the like for example. If the partial peptide obtained by these methods is in free form, it can be converted to an appropriate salt by well-known methods, or conversely if it is obtained as a salt it can be converted to free form by well-known methods.

(Polynucleotide)

A polynucleotide encoding the receptor protein of the present invention may be any that contains a nucleotide sequence (DNA or RNA, preferably DNA) encoding the receptor protein of the present invention. This polynucleotide may be DNA, mRNA or other RNA encoding the receptor protein of the present invention, and may be double-stranded or single-stranded. If double-stranded it may be double-stranded DNA, double-stranded RNA or a DNA:RNA hybrid. If single-stranded, it may be a sense strand (namely a coding strand) or an antisense strand (namely a non-coding strand).

A polynucleotide encoding the receptor protein of the present invention can be used to assay mRNA of the receptor protein of the present invention by the well-known methods described in *Jikken Igaku Zokan*, "A new PCR and its application" 15(7), 1997 or methods conforming thereto for example.

DNA encoding the receptor protein of the present invention may be genome DNA, genome DNA library, cDNA derived from the aforementioned cells or tissue, cDNA library derived from the aforementioned cells or tissue or synthetic DNA. The vector used for the library may be a bacteriophage, plasmid, cosmid, phagemid or the like. Total RNA or an mRNA fraction prepared from the aforementioned cells or tissue can be amplified directly by a reverse transcriptase polymerase chain reaction (abbreviated hereunder as RT-PCR).

Specific examples of DNA encoding the receptor protein of the present invention include for example DNA containing a nucleotide sequence represented by SEQ ID NO: 8, SEQ ID NO: 10, SEQ ID NO: 14, SEQ ID NO: 16, SEQ ID NO: 18, SEQ ID NO: 20, SEQ ID NO: 22, SEQ ID NO: 24, SEQ ID NO: 26 or SEQ ID NO: 28, or DNA encoding a receptor protein having DNA which hybridizes under highly stringent conditions with DNA containing a nucleotide sequence represented by SEQ ID NO: 8, SEQ ID NO: 10, SEQ ID NO: 14, SEQ ID NO: 16, SEQ ID NO: 18, SEQ ID NO: 20, SEQ ID NO: 22, SEQ ID NO: 24, SEQ ID NO: 26 or SEQ ID NO: 28, and having substantially the same activity (such as ligand binding activity, signal transmission action or the like) as a receptor protein containing the amino acid sequence represented by SEQ ID NO: 9.

DNA or the like containing a nucleotide sequence having about 70% or greater or preferably about 80% or greater or more preferably about 90% or greater or most preferably about 95% or greater homology with a nucleotide sequence represented by SEQ ID NO: 8, SEQ ID NO: 10, SEQ ID NO: 14, SEQ ID NO: 16, SEQ ID NO: 18, SEQ ID NO: 20, SEQ ID NO: 22, SEQ ID NO: 24, SEQ ID NO: 26 or SEQ ID NO: 28 for example can be used as DNA which hybridizes under highly stringent conditions with DNA having a nucleotide sequence represented by SEQ ID NO: 8, SEQ ID NO: 10, SEQ ID NO: 14, SEQ ID NO: 16, SEQ ID NO: 18, SEQ ID NO: 20, SEQ ID NO: 22, SEQ ID NO: 24, SEQ ID NO: 26 or SEQ ID NO: 28.

Hybridization can be performed by well-known methods or methods conforming thereto, such as for example the method described in Molecular Cloning $2^{nd}$ (J. Sambrook et al., Cold Spring Harbor Lab. Press, 1989). When using a commercial library, the methods described in the attached manual may be followed. More preferably, highly stringent conditions can be followed.

These highly stringent conditions are for example a sodium concentration of about 19 to 40 mM or preferably 19 to 20 mM and a temperature of about 50 to 70°C or preferably about 60 to 65°C. A sodium concentration of about 19 mM and a temperature of about 65°C are particularly desirable.

The meaning of "a polynucleotide containing part of the nucleotide sequence of DNA encoding the receptor protein or part of a nucleotide sequence complementary to that DNA" encompasses not only DNA encoding a partial peptide of the present invention as described below, but also RNA.

According to the present invention, an antisense polynucleotide (nucleic acids) capable of inhibiting the replication or expression of the receptor protein gene can be designed and synthesized based on the nucleotide sequence information for DNA encoding the cloned or determined receptor protein. Such a polynucleotide (nucleic acids) can hybridize with the RNA of the receptor protein gene, and can either inhibit the synthesis or function of such RNA or else regulate and control expression of the receptor protein gene by interacting with the receptor protein-associated RNA. Polynucleotides complementary to selected sequences of receptor protein-associated RNA and polynucleotides which can hybridize specifically with receptor protein-associated RNA are useful for regulating and controlling expression of the receptor protein gene both in vivo and in vitro, and are useful for treating and diagnosing disease. The phrase "corresponds to" signifies having homology or being complementary to a nucleotide, nucleotide sequence or specific sequence of nucleic acids including a gene. What "corresponds" between a nucleotide, nucleotide sequence or nucleic acids and a peptide (protein) is normally amino acids of a peptide (protein) under control induced by the sequence of a nucleotide (nucleic acids) or its complement. The 5' hairpin loop, 5'6-base pair lipid, 5' non-translation region, polypeptide translation initiation codon, protein coding region, ORF translation termination codon, 3' non-translation region, 3' palindrome region and 3' hairpin loop of the receptor protein gene can be selected as desirable target regions, but so can any region of the receptor protein gene.

Methods which can be used to clone DNA which completely encodes the receptor protein or partial peptide thereof of the present invention (sometimes abbreviated together below as the receptor protein of the present invention) are to amplify it by PCR using synthetic DNA primers having a partial nucleotide sequence of the receptor protein of the present invention, or to select it by hybridization with DNA incorporated into a suitable vector and labeled with either synthetic DNA or a DNA fragment encoding some or all regions of the receptor protein of the present invention. Hybridization can be accomplished for example by the method described in Molecular Cloning $2^{nd}$ (J. Sambrook et al., Cold Spring Harbor Lab. Press, 1989) or the like. When a commercial library is used, the methods described in the attached manual can be employed.

(Antisense polynucleotide)

The relationship between the target nucleic acids and a polynucleotide complementary to at least part of the region of interest can be called an "antisense" relationship of a polynucleotide, which can hybridize with the object of interest. Examples of antisense polynucleotides include polynucleotides containing 2-deoxy-D-ribose, polynucleotides containing D-ribose, other types of polynucleotides which are N-glycosides of pyrimidine bases or purines, and other polymers having non-nucleotide frameworks (such as commercial proteins, nucleic acids and synthetic sequence-specific nucleic acid polymers) as well as other polymers containing special binding (when such polymers contain nucleotides which are arranged so as to allow base pairings and base attachments such as are found in DNA and RNA). These may be double-stranded DNA, single-stranded DNA, double-stranded RNA, single-stranded RNA or DNA:RNA hybrids, and may be unmodified polynucleotides (or unmodified oligonucleotides) or those with conventional modifications added, such as those having labels familiar in the field, those having caps, those that are methylated, those having one or more natural nucelotides replaced by analogues, those having intermolecular nucleotide modifications such as those with non-charged bonds (such as methylphosphonate, phosphotriester, phosphoramidate, carbamate or the like) and those having bonds with charge or sulfur-containing bonds (such as phosphorothioate, phosphorodithioate and the like) including for example proteins (nucleases, nuclease inhibitors, toxins, antibodies, signal peptides, poly-L-lysine and the like); sugars (such as monosaccharides and the like) and others with side-chain bases, those with intercalate bonds (such as acridine and psoralen), those with chelate compounds (such as metals, metals with radioactivity, boron, oxidizing metals and the like), those containing alkylating agents, or those having modified bonds (such as alpha anomer type nucleic acids). Here "nucleosides," "nucleotides" and "nucleic acids" may include not only those with purine or pyrimidine bases, but those with other modified heterocyclic bases. These modified bases may include methylated purine and pyrimidine, acylated purine and pyrimidine and other heterocyclic structures. Modified nucleotides may also have their sugar parts modified, and for example one or more hydroxyl groups may be replaced by halogens or aliphatic groups, or converted to ether, amine or other functional groups.

The antisense polynucleotide (nucleic acids) of the present invention is RNA, DNA or modified nucleic acids (RNA, DNA). Specific examples of modified nucleic acids include sulfur derivatives or thiophosphate derivatives of nucleic acids, and those with resistance to degradation of polynucleoside amide and olignucleoside amide, but they are not limited by these examples. The antisense nucleic acids of the present invention can preferably be designed according to the following principles. Namely, the antisense nucleic acids within the cell are made more stable, the cell permeability of the antisense nucleic acids is enhanced, affinity for the target sense chain is increased, and toxicity of the antisense nucleic acids is reduced if they have toxicity.

Many such modifications are known in the field, and are disclosed for example in J. Kawakami et al., *Pharm Tech Japan* Vol. 8, pp 247, 1992 and Vol. 8, pp 395, 1992; S.T. Crooke et al. ed., <u>Antisense Research and Applications</u>, CRC Press, 1993 and the like.

The antisense nucleic acids of the present invention may be altered or may contain modified sugars, bases or bonds, or they may be provided in a special form such as a liposome or microsphere in a form more suitable for gene therapy or with additions. Examples of such additions which can be used include polylysine and other polycations which serve to neutralize the charge of phosphate group structures, and lipids (such phospholipids, cholesterol and the like) which are hydrophobic and improve interaction with the cell membrane and increase incorporation of nucleic acids. Desirable lipids for addition include cholesterol and its derivatives (such as cholesteryl chloroformate and cholic acid). These may be attached to the 3'-terminal or 5'-terminal of the nucleic acids, and may be attached via bases, sugars or intermolecular nucleoside bonds. Other groups include the capping groups, which are positioned specifically at the 3'-terminal or 5'-terminal of the nucleic acids and serve to prevent degradation by exonucleases, Rnases and other nucleases. Examples of such capping groups include protective groups of hydroxyl groups known in the field, including polyethylene glycol, tetraethylene glycol and other glycols, but they are not limited to these examples.

The inhibitory activity of antisense nucleic acids can be studied using a transformant of the present invention, an in vivo or in vitro gene expression system of the present invention or an in vivo or in vitro translation system of the receptor protein. The nucleic acids can be applied to cells by a variety of conventional methods.

Since the antisense polynucleotide of the present invention can suppress the function of the protein of the present invention or the polynucleotide of the present invention (such as DNA) in vivo, it can be used for example as a preventative and/or therapeutic drug for disorders associated with dysfunction of the receptor protein of the present invention. Moreover, since the antisense polynucleotide of the present invention can also be used as a diagnostic oligonucleotide probe for investigating the presence and expression of the DNA of the present invention in tissue and cells, it can be used for diagnosing disorders associated with dysfunction of the receptor protein of the present invention.

(DNA encoding partial peptide)

The DNA encoding the partial peptide of the present invention may be any that contains a nucleotide sequence encoding the partial peptide of the present invention. It may be genome DNA, genome DNA library, cDNA derived from the aforementioned cells or tissue, cDNA library derived from the aforementioned cells or tissue, or synthetic DNA. The vector used for the library may be a bacteriophage, plasmid, cosmid, phagemid or the like. An mRNA fraction prepared from the aforementioned cells or tissue can be amplified directly by a reverse transcriptase polymerase chain reaction (abbreviated hereunder as RT-PCR).

Specifically, the following for example can be used as DNA encoding the partial peptide of the present invention:

(1) DNA having a partial nucleotide sequence of DNA containing a nucleotide sequence represented by SEQ ID NO: 8, SEQ ID NO: 10, SEQ ID NO: 14, SEQ ID NO: 16, SEQ ID NO: 18, SEQ ID NO: 20, SEQ ID NO: 22, SEQ ID NO: 24, SEQ ID NO: 26 or SEQ ID NO: 28;
(2) DNA having a partial nucleotide sequence of DNA encoding a protein which has DNA which hybridizes under highly stringent conditions with DNA containing a nucleotide sequence represented by SEQ ID NO: 8, SEQ ID NO: 10, SEQ ID NO: 14, SEQ ID NO: 16, SEQ ID NO: 18, SEQ ID NO: 20, SEQ ID NO: 22, SEQ ID NO: 24, SEQ ID NO: 26 or SEQ ID NO: 28 and which has substantially the same activity (such as ligand binding activity, signal transmission action or the like) as a protein peptide containing the amino acid sequence represented by SEQ ID NO: 9.

DNA or the like containing a nucleotide sequence having about 70% or greater or preferably about 80% or greater or more preferably about 90% or greater or most preferably about 95 % or greater homology with a nucleotide sequence represented by SEQ ID NO: 8, SEQ ID NO: 10, SEQ ID NO: 14, SEQ ID NO: 16, SEQ ID NO: 18, SEQ ID NO: 20, SEQ ID NO: 22, SEQ ID NO: 24, SEQ ID NO: 26 or SEQ ID NO: 28 for example can be used as DNA which hybridizes under highly stringent conditions with DNA containing a nucleotide sequence represented by SEQ ID NO: 8, SEQ ID NO: 10, SEQ ID NO: 14, SEQ ID NO: 16, SEQ ID NO: 18, SEQ ID NO: 20, SEQ ID NO: 22, SEQ ID NO: 24, SEQ ID NO: 26 or SEQ ID NO: 28.

(Antibodies)

Antibodies to the receptor protein or partial peptide or salt thereof of the present invention may be either polyclonal antibodies or monoclonal antibodies as long as they are capable of recognizing the receptor protein or partial peptide or salt thereof of the present invention or cells or tissue containing the receptor protein of the present invention.

Antibodies to the receptor protein or partial peptide or salt thereof of the present invention (sometimes abbreviated hereunder as the receptor protein or the like of the present invention) can be manufactured according to well-known methods of manufacturing antibodies or antiserum, using the receptor protein or the like of the present invention as the antigen.

(Preparation of monoclonal antibodies)

(a) Preparation of monoclonal antibody-producing cells

The receptor protein or the like of the present invention is administered to mammals at a site where antibody production from administration as possible, either by itself or together with a carrier or diluent. Complete Freund's adjuvant or incomplete Freund's adjuvant can be administered to enhance antibody productivity during administration. Administration is normally once every 2 to 6 weeks, for a total of about 2 to 10 administrations. The mammals used may be monkeys, rabbits, dogs; guinea pigs, mice, rats, sheep or goats for example, with mice and rats being preferred.

To produce monoclonal antibody-producing cells, individuals with confirmed antibody titer are selected from warm-blooded animals (such as mice) which were immunized with the antigen, spleens or lymph nodes are removed 2 to 5 days after the final immunization, and antibody-producing cells contained therein can be fused with myeloma cells to prepare a monoclonal antibody-producing hybridoma. Measurement of antibody titer in antiserum can be accomplished for example by first reacting the labeled receptor protein or the like described below with antiserum, and then measuring the activity of label bound to the antibodies. Fusing can be accomplished by known methods such as the method of Kohler and Milstein (*Nature* 256, p. 495 (1975)). Examples of fusion promoters include polyethylene glycol (PEG), sendai virus and the like, but preferably PEG is used.

Examples of myeloma cells include NS-1, P3U1, SP2/0 and the like, but preferably P3U1 is used. The preferred ratio of number of antibody-producing cells (spleen cells) to number of myeloma cells used is about 1:1 to 20: 1, and cell fusion can be performed efficiently if PEG (preferably PEG1000 to PEG6000) is added at a concentration of about 10 to 80%, and the cells incubated for about 1 to 10 minutes at about 20 to 40°C or preferably about 30 to 37°C.

Various methods can be used to screen the monoclonal antibody-producing hybridoma, and possible methods include for example a method of adding hybridoma culture supernatant to a solid phase (such as a microplate) on which the receptor protein or another antigen has been adsorbed either directly or together with a carrier, then adding protein A or anti-immunoglobulin antibodies (anti-mouse immunoglobulin antibodies are used if the cells used in cell fusion are mouse cells) labeled with a radioactive substance or enzyme, and detecting monoclonal antibodies bound to the solid phase, or a method of adding hybridoma culture supernatant to a solid phase on which anti-immunoglobulin antibodies or protein A have been adsorbed, then adding receptor protein or the like labeled with a radioactive substance or enzyme, and detecting monoclonal antibodies bound to the solid phase.

Well-known methods or methods conforming thereto can be used for selection of monoclonal antibodies, which is normally accomplished in an animal cell medium or the like to which HAT (hypoxanthine, aminopterin, thymidine) has been added. The medium for selection and breeding can be any medium in which the hybridoma can grow. For example, RPMI 1640 medium containing 1 to 20% or preferably 10 to 20% fetal calf serum, GIT medium (Wako Pure Chemical Industries, Ltd.) containing 1 to 10% fetal calf serum or serum-free medium for hybridoma culture (SFM-101, Nissui Pharmaceutical Co., Ltd.) or the like can be used. The culture temperature is normally 20 to 40°C or preferably about 37°C. Culture time is normally 5 days to 3 weeks or preferably 1 week to 2 weeks. Culture can normally be performed under 5% carbon dioxide. The antibody titer of the hybridoma culture supernatant can be measured in the same way as the aforementioned measurement of antibody titer in antiserum.

(b) Purification of monoclonal antibodies

Isolation and purification of monoclonal antibodies can be accomplished in the same way as ordinary isolation and purification of polyclonal antibodies using methods of isolating and purifying immunoglobulin (for example, salting out, alcohol precipitation, isoelectric point precipitation, electrophoresis, adsorption-desorption with an ion exchanger (such as DEAE), ultracentrifugation, gel filtration, or a specific purification method in which antibodies are obtained by collecting them alone with an antigen-bound solid phase or an active adsorber such as protein A or protein G, and then dissociating the bonds).

(Preparation of polyclonal antibodies)

The polyclonal antibodies of the present invention can be manufactured by well-known methods or methods conforming thereto. For example, they can be manufactured by creating a composite of an immunogen (protein of the present invention or other antigen) and a carrier protein, immunizing mammals as in the manufacture of monoclonal antibodies described above, collecting material containing antibodies to the receptor protein or the like of the present invention from the immunized animals, and isolating and purifying the antibodies.

For the composite of immunogen and carrier protein used to immunize the mammals, the type of carrier protein and proportions of carrier and hapten may be any type and any proportions as long as antibodies are produced efficiently in response to the immunized hapten linked to the carrier, but for example a method can be used in which bovine serum albumin, bovine thyroglobulin, keyhole limpet hemocyanin or the like is coupled at a weight ratio of about 0.1 to 20 or preferably about 1 to 5 units per 1 unit of hapten.

Various condensing agents can be used for coupling the hapten and the carrier, but glutaraldehyde and active ester reagents containing carbodiimide, maleimide active ester, thiol groups and dithiopyridil groups can be used.

The condensed product is administered to warm-blooded animals either alone or together with a carrier or diluent at a site where antibodies can be produced. Complete Freund's adjuvant or incomplete Freund's adjuvant can be administered to enhance antibody productivity during administration. Administration is normally once every 2 to 6 weeks, for a total of about 3 to 10 administrations.

The polyclonal antibodies can be collected from the blood, abdominal fluid or the like of mammals immunized as described above, preferably from the blood.

Polyclonal antibody titer in antiserum can be measured in the same way as the measurement of antibody titer in serum described above. The polyclonal antibodies can be isolated and purified in the same way as the isolation and purification of monoclonal antibodies described above using methods of isolating and purifying immunoglobulin.

(Uses for receptor protein, DNA and the like)

A protein showing affinity for an asparagine-linked sugar chain or a serine/threonine-linked sugar chain (such as concanavalin A, lentil lectin, pea lectin, datura stramonium lectin, sophora japonica lectin, phytohemagglutinin or another lectin or the like) is one ligand for the receptor protein of the present invention.

Consequently, the receptor protein of the present invention, the polynucleotide encoding therefor (sometimes abbreviated hereunder as the polynucleotide of the present invention), antibodies to the receptor protein of the present invention (sometimes abbreviated hereunder as antibodies of the present invention), the antisense polynucleotide to the DNA of the present invention (sometimes abbreviated hereunder as the antisense polynucleotide of the present invention) and the like have the following uses.

(1) Preventative and/or therapeutic agents for disorders associated with dysfunction of the receptor protein of the present invention

Since expression of the receptor protein of the present invention is seen in the prostate (cancer), placenta and liver, a) the receptor protein of the present invention or (b) a polynucleotide encoding the receptor protein of the present invention (such as DNA) can be used as a drug such as a birth inducer or a preventative and/or therapeutic agent for dysfunction of the receptor protein of the present invention, particularly prostate cancer and other cancers (non-small cell carcinoma, ovarian cancer, stomach cancer, bladder cancer, breast cancer, cervical cancer, colon cancer, rectal cancer, large intestinal cancer and the like), prostatomegaly, male gonad dysfunction, infertility, premature birth, endometriosis, cirrhosis of the liver, hepatitis, hepatic insufficiency or calculus.

For example, if the physiological function of a ligand cannot be relied upon because a particular receptor protein is reduced in the body of a patient (deficiency of the receptor protein), the action of the ligand can be adequately restored by (1) administering the receptor protein to the patient to replace the missing receptor protein, or (2) increasing the amount of receptor protein in the patient's body by either (i) inducing expression by administering DNA encoding the receptor protein of the present invention to the patient, or (ii) inducing expression by inserting DNA encoding the receptor protein of the present invention into target cells and transplanting the cells into the patient. In other words, a polynucleotide encoding the receptor protein of the present invention is useful as safe, low-toxicity birth inducer or preventative and/or therapeutic agent for dysfunction of the receptor protein, particularly prostate cancer and other cancers (non-small cell carcinoma, ovarian cancer, stomach cancer, bladder cancer, breast cancer, cervical cancer, colon cancer, rectal cancer, large intestinal cancer and the like), prostatomegaly, male gonad dysfunction, infertility, premature birth, endometriosis, cirrhosis of the liver, hepatitis, hepatic insufficiency and calculus.

When the receptor protein or polynucleotide encoding therefor of the present invention is used as the aforementioned drug, it can be prepared according to ordinary means.

(2) Diagnostic agent and diagnostic method employing the polynucleotide or antisense polynucleotide of the present invention

Since the polynucleotide (such as DNA) and antisense polynucleotide (such as antisense DNA) of the present invention can be used as probes to detect abnormalities (gene abnormalities) in DNA or mRNA encoding the receptor protein or partial peptide thereof of the present invention in humans or mammals (such as rats, mice, rabbits, sheep, pigs, cows, cats, dogs, monkeys and the like), they are useful as gene diagnosis agents for detecting damage, mutations or reduced expression of that DNA or mRNA, or increase, over-expression or the like of that DNA or mRNA.

The aforementioned gene diagnosis using the polynucleotide or antisense polynucleotide of the present invention can be accomplished for example by well-known Northern hybridization or PCR-SSCP methods (*Genomics* 5, 874-879 (1989); *Proceedings of the National Academy of Sciences of the United States of America* 86, 2766-2770 (1989)) or the like.

For example, if reduced expression or over-expression of the receptor protein of the present invention is detected by Northern hybridization, it is possible to diagnose a high likelihood of a disorder stemming from dysfunction or over-expression of the receptor protein of the present invention for example, particularly prostate cancer, other cancers (non-small cell carcinoma, ovarian cancer, stomach cancer, bladder cancer, breast cancer, cervical cancer, colon cancer, rectal cancer large intestinal cancer), prostatomegaly, male gonad dysfunction, infertility, premature birth, endometriosis, cirrhosis of the liver, hepatitis, hepatic insufficiency or calculus, or a high likelihood of such a disorder occurring in the future.

(3) Drugs containing the antisense polynucleotide of the present invention

The antisense polynucleotide of the present invention can be used as a birth inducer or as a preventative and/or therapeutic agent for disorders caused by over-expression or the like of the receptor protein of the present invention (such as prostate cancer and other cancers (non-small cell carcinoma, ovarian cancer, stomach cancer, bladder cancer, breast cancer, cervical cancer, colon cancer, rectal cancer large intestinal cancer), prostatomegaly, male gonad dysfunction, infertility, premature birth, endometriosis, cirrhosis of the liver, hepatitis, hepatic insufficiency, calculus and other disorders).

For example, when this antisense polynucleotide is used it can be used alone or first inserted into a suitable vector such as a retrovirus vector, adenovirus vector, adenovirus-associated virus vector or the like and applied by ordinary means. The antisense polynucleotide can be prepared alone or together with adjuvants for promoting ingestion or other physiologically acceptable carriers, and administered with a gene gun or a catheter such as a hydrogel catheter.

Moreover, the antisense polynucleotide can also be used as a diagnostic oligonucleotide probe for investigating the presence and expression of the DNA of the present invention in cells or tissue.

(4) Method of screening compounds, which alter the expressed amount of the receptor protein or partial peptide thereof of the present invention

When used as a probe, the DNA of the present invention can be used for screening compounds, which alter the expressed amount of the receptor protein or partial peptide thereof of the present invention.

Namely, the present invention provides for example a method of screening compounds which alter the expressed amount of the receptor protein or partial peptide thereof of the present invention by measuring the amount of mRNA of the receptor protein or partial peptide thereof of the present invention contained in for example (i) (1) blood, (2) specific organs or (3) tissue or cells isolated from organs of non-human mammals or in (ii) a transformant or the like.

Specifically, the amount of mRNA of the receptor protein or partial peptide thereof of the present invention is measured as follows.

(i) A drug (such as an anti-cancer drug) or physical stress (such as immersion stress, electric shock, brightness contrast or low temperature) or the like is applied to normal or disease-model non-human mammals (such as mice, rats, rabbits, sheep, pigs, cows, cats, dogs, monkeys or the like, or more specifically cancer-carrying mice), and after a fixed amount of time blood or specific organs (such as brains, lungs, large intestines, prostate glands or the like) or tissue or cells isolated from organs are obtained.

mRNA of the receptor protein or partial peptide thereof of the present invention contained in the resulting cells can be extracted from the cells or the like by ordinary methods and assayed using a method such as TaqMan PCR for example, and can also be analyzed by Northern blotting using well-known means.

(ii) A transformant expressing the receptor protein or partial peptide thereof of the present invention can be prepared according to the methods described above, and mRNA of the receptor protein or partial peptide thereof of the present invention contained in the transformant assayed and analyzed in the same way.

Compounds, which alter the expressed amount of the receptor protein or partial peptide thereof of the present invention, can be screened by:

(i) Administering a test compound to normal or disease model non-human mammals a fixed time before (30 minutes to 24 hours before, preferably 30 minutes to 12 hours before, more preferably 1 hour to 6 hours before) or a fixed time after (30 minutes to 3 days after, preferably 1 hour to 2 days after, more preferably 1 hour to 24 hours after) application of a drug or physical stress or simultaneously with the drug or physical stress, and assaying and analyzing the amount of mRNA of the receptor protein or partial peptide thereof of the present invention contained in cells a fixed time after administration (30 minutes to 3 days, preferably 1 hour to 2 days, more preferably 1 hour to 24 hours);

(ii) Mixing a test compound into the medium when culturing the transformant by ordinary methods, and assaying and analyzing the amount of mRNA of the receptor protein or partial peptide thereof of the present invention contained in the transformant after a fixed culture time (1 day to 7 days, preferably 1 day to 3 days, more preferably 2 days to 3 days).

A compound or salt thereof obtained using the screening method of the present invention is a compound having the action of altering the expressed amount of the receptor protein or partial peptide thereof of the present invention, and specifically it is a compound which either (a) increases the expressed amount of the receptor protein or partial peptide thereof of the present invention, thus augmenting cell stimulus activity via the receptor, or (b) decreases the expressed amount of the receptor protein or partial peptide thereof of the present invention, thus weakening the cell stimulus activity.

Examples of cell stimulus activity include (1) arachidonic acid release, (2) acetylcholine release, (3) intercellular $Ca^{2+}$ release, (4) intercellular cAMP production, (5) intercellular cGMP production, (6) inositolphosphoric acid production, (7) cell membrane potential fluctuation, (8) phosphorylation of intercellular proteins (such as MAP kinase), (9) activation of c-fos, (10) reduction of pH, (11) activation of Rho, Rac, Ras and other low molecular weight G proteins, and (12) activation of reporter genes (such as luciferase and the like) attached downstream from transcription factor CRE (cAMP responsive element), AP1, NFAT, SRE (serum responsive element) or the like, with promotion of intercellular cAMP production and the like being especially desirable.

Examples of the compound include peptides, proteins, non-peptidergic compounds, synthetic compounds, fermentation products and the like, and this compounds may be either a novel compound or a well-known compound.

Such compounds, which augment cell stimulus activity, are useful as safe, low toxic drugs for augmenting the physiological activity of the receptor protein or the like of the present invention.

Such compounds, which weaken cell stimulus activity, are useful as safe, low toxic drugs for depressing the physiological activity of the receptor protein or the like of the present invention.

As explained above, one ligand of the receptor protein of the present invention is a protein (such as a lectin) showing an affinity for a sugar chain. Consequently, a compound obtained by the aforementioned screening method which alters the expressed amount of the receptor protein of the present invention can be used as a birth inducer or as a preventative and/or therapeutic agent for prostate cancer and other cancers (non-small cell carcinoma, ovarian cancer, stomach cancer, bladder cancer, breast cancer, cervical cancer, colon cancer, rectal cancer, large intestinal cancer and the like), prostatomegaly, male gonad dysfunction, infertility, premature birth, endometriosis, cirrhosis of the liver, hepatitis, hepatic insufficiency, calculus or the like.

(5) Preventative and/or therapeutic drug for various disorders containing a compound which alters the expressed amount of the receptor protein or partial peptide thereof of the present invention

As mentioned above, the receptor protein of the present invention is thought to serve some important role in the body in central function or the like for example. Consequently, a compound which alters the expressed amount of the receptor protein or partial peptide thereof of the present invention can be used as a preventative and/or therapeutic agent for disorders associated with dysfunction of the receptor protein of the present invention.
When this compound is used as a preventative and/or therapeutic agent for disorders associated with dysfunction of the receptor protein of the present invention, it can be formulated according to ordinary means.

(6) Screening method and screening kit for compounds (agonists, antagonists and the like) which alter the binding properties of the receptor protein of the present invention with a ligand

By using the receptor protein or the like of the present invention or by constructing a system expressing a recombinant receptor protein or the like and using a receptor binding assay system employing this expression system, it is possible to efficiently screen for compounds (such as peptides, proteins, non-peptidergic compounds, synthetic compounds, fermentation products and the like) or salts thereof which alter the binding properties of the receptor protein or the like of the present invention with a ligand.
As described above, one example of a ligand for the receptor protein of the present invention is a protein (such as a lectin) showing an affinity for a sugar chain.
Moreover, a compound or salt thereof, which alters the binding properties of a protein showing affinity for a sugar chain with the receptor protein of the present invention, can also be used as a ligand. This compound or salt thereof, which alters the binding properties of a protein showing affinity for a sugar chain with the receptor protein of the present invention, can be obtained by applying the screening method of the present invention using the protein showing an affinity for a sugar chain for example as the ligand.
The compound or salt thereof, which alters the binding properties of a protein showing affinity for a sugar chain with the receptor protein of the present invention, should preferably be a low molecular weight synthetic compound and may be either a novel or a well-known compound. In particular, when screening antagonists to the receptor protein of the present invention, it is desirable to use low molecular weight synthetic compounds having agonist activity in place of proteins showing affinity for sugar chains.
Low molecular weight synthetic compounds are suitable for screening purposes because they are easier to label than proteins showing affinity for sugar chains. These proteins showing affinity for sugar chains and low molecular weight synthetic compounds are referred to collectively below as ligands.
Compounds which alter the binding properties of a ligand with the receptor protein of the present invention include (a) compounds having activity which promotes or activity which suppresses cell stimulus activity or the like via the receptor (so-called agonists to the receptor protein of the present invention), (b) compounds having no such cell stimulus activity (so-called antagonists to the receptor protein of the present invention), (c) compounds which strengthen the binding force of a ligand with the G protein-coupled receptor protein of the present invention, or (d) compounds which weaken the binding force of a ligand with the receptor protein of the present invention or the like (the compounds of (a) above should preferably be screened by the aforementioned ligand determination method).
Examples of cell stimulus activity include for example (1) arachidonic acid release, (2) acetylcholine release, (3) intercellular $Ca^{2+}$ release, (4) intercellular cAMP production, (5) intercellular cGMP production, (6) inositolphosphoric acid production, (7) cell membrane potential fluctuation, (8) phosphorylation of intercellular proteins (such as MAP kinase), (9) activation of c-fos, (10) reduction of pH, (11) activation of Rho, Rac, Ras and other low molecular weight G proteins, and (12) activation of reporter genes (such as luciferase) attached downstream from transcription factor CRE (cAMP responsive element), AP1, NFAT, SRE (serum responsive element) or the like, with promotion of intercellular cAMP production and the like being especially desirable.
That is, the present invention provides a method of screening compounds or salts thereof which alter the binding properties of a ligand with the receptor protein or partial peptide or salt thereof of the present invention, characterized by a comparison of (i) bringing the receptor protein or partial peptide or salt thereof of the present invention into contact with a ligand and (ii) bringing the receptor protein or partial peptide or salt thereof of the present invention into contact with a ligand and a test compound.

The screening method of the present invention is characterized in that for example the amount of binding of the ligand to the receptor protein or the like, cell stimulus activity and the like in cases (i) and (ii) are measured and compared.

More specifically, the present invention provides:

(i) A method of screening compounds or salts thereof which alter the binding properties of a ligand with the receptor protein or the like of the present invention, characterized in that the amount of binding of a labeled ligand with the receptor protein or the like is measured and compared when the labeled ligand is brought into contact with the receptor protein of the present invention and when the labeled ligand and a test compound are brought into contact with the receptor protein of the present invention;

(ii) A method of screening compounds or salts thereof which alter the binding properties of a ligand with the receptor protein or the like of the present invention, characterized in that the amount of binding of a labeled ligand with cells or a membrane fraction is measured and compared when the labeled ligand is brought into contact with cells or a membrane fraction of such cells containing the receptor protein of the present invention or the like, and when the labeled ligand and a test compound are brought into contact with cells or a membrane fraction of such cells containing the receptor protein of the present invention or the like;

(iii) A method of screening compounds or salts thereof which alter the binding properties of a ligand with the receptor protein or the like of the present invention, characterized in that the amount of binding of a labeled ligand with the receptor protein is measured and compared when the labeled ligand is brought into contact with the receptor protein or the like expressed on cell membranes by culturing of a transformant containing the DNA of the present invention, and when the labeled ligand and a test compound are brought into contact with the receptor protein expressed on cell membranes by culturing of a transformant containing the DNA of the present invention;

(iv) A method of screening compounds or salts thereof which alter the binding properties of a ligand with the receptor protein or the like of the present invention, characterized in that cell stimulus activity via a receptor is measured and compared when a compound (such as a ligand) which activates the receptor protein or the like of the present invention is brought into contact with cells containing the receptor protein or the like of the present invention, and when a compound which activates the receptor protein or the like of the present invention and a test compound are brought into contact with cells containing the receptor protein or the like of the present invention; and

(v) A method of screening compounds or salts thereof which alter the binding properties of a ligand with the receptor protein or the like of the present invention, characterized in that cell stimulus activity via a receptor is measured and compared when a compound (such as a ligand) which activates the receptor protein or the like of the present invention is brought into contact with the receptor protein or the like of the present invention expressed on cell membranes by culturing of a transformant containing the DNA of the present invention, and when a compound which activates the receptor protein or the like of the present invention and a test compound are brought into contact with the receptor protein or the like of the present invention expressed on cell membranes by culturing of a transformant containing the DNA of the present invention.

The screening method of the present invention is explained in detail below.

First, the receptor protein or the like of the present invention used in the screening method of the present invention may be any that contains the aforementioned receptor protein or the like of the present invention, but preferably it should be a cell membrane fraction of a mammalian organ containing the receptor protein or the like of the present invention. However, due to the extreme difficulty of obtaining human-derived organs, a human-derived receptor protein or the like made to be expressed in large quantities by means of a recombinant is suited for screening use.

The aforementioned methods can be used to manufacture the receptor protein of the present invention, but it is preferable that the DNA of the present invention be expressed in mammalian cells or insect cells. Complement DNA can be used for a DNA fragment encoding the target protein part, but this is not necessarily a constraint. For example, a gene fragment or synthetic DNA may also be used. For purposes of introducing a DNA fragment encoding the receptor protein of the present invention into host animal cells and causing them to be expressed efficiently, it is preferable that the DNA fragment be incorporated downstream from the polyhedrin promoter of the nuclear polyhedrosis virus (NPV) which belongs to the Baculovirus family having insect hosts, or from a SV40-derived promoter, retrovirus promoter, metallothionein promoter, human heat shock promoter, cytomegalovirus promoter, SR alpha promoter or the like. The amount and quality of expressed receptor can themselves be tested by well-known methods. For example, the methods described in the references (Nambi, P. et al, *J. Biol. Chem*. 267, 19555-19559, 1992) can be followed.

In the screening method of the present invention, the receptor protein or the like of the present invention may be contained by the receptor protein or the like purified by well-known methods, or cells containing the receptor protein or the like can also be used, as can a cell membrane fraction containing the receptor protein or the like.

When cells containing the receptor protein or the like of the present invention are used in the screening method of the present invention, those cells can be fixed with glutaraldehyde, formalin or the like. Fixing can be according to well-known methods.

The cells containing the receptor protein or the like of the present invention are host cells which express the receptor protein or the like, and *E. coli*, *B. subtilis,* yeast, insect or animal cells or the like are preferred as such host cells.

The cell membrane fraction is a fraction containing many cell membranes obtained by well-known methods after crushing of the cells. Examples of cell crushing methods include pounding of cells with a Potter-Elvehjem homogenizer, crushing with a Waring blender or Polytron (Kinematica), crushing by ultrasound or crushing by extruding the cells through a fine nozzle while applying pressure with a French press or the like. A fractioning method using centrifugal force, such as fraction centrifugation or density gradient centrifugation, is generally used for the cell membrane fraction. For example, crushed cell liquid is centrifuged for a short period of time (normally about 1 to 10 minutes) at a low speed (500 rpm to 3000 rpm), the supernatant is then centrifuged for 30 minutes to 2 hours at a high speed (15000 rpm to 30000 rpm), and the resulting precipitate is the membrane fraction. The expressed receptor protein or the like and membrane components such as cell-derived phospholipids and membrane proteins are abundantly present in the membrane fraction.

The amount of receptor protein in the cells or membrane fraction containing the receptor protein or the like is preferably $10^3$ to $10^8$ molecules per cell and more preferably $10^5$ to $10^7$ molecules per cell. The higher the expressed amount the greater the binding activity (relative activity) of the ligand with respect to the membrane fraction, not only allowing for construction of a highly sensitive screening system, but also for measurement of large volume samples in the same lot.

In order to achieve (1) to (3) above in which compounds are screened which alters the binding properties of a ligand with the receptor protein or the like of the present invention, an appropriate receptor protein fraction and a labeled ligand for example are required.

The receptor protein fraction is desirably either a natural receptor protein fraction or a recombinant receptor protein fraction having activity equivalent thereto or the like. Equivalent activity here signifies equivalent ligand binding activity, signal transmission action or the like.

A labeled ligand, labeled ligand analogue compound or the like can be used as the labeled ligand. For example, a ligand labeled with [$^3$H], [$^{125}$I], [$^{14}$C], [$^{35}$S] or the like can be used.

Specifically, to screen for compounds which alter the binding properties of a ligand with the receptor protein or the like of the present invention, a receptor protein preparation is first prepared by suspending cells or a membrane fraction of cells containing the receptor protein or the like of the present invention in a buffer suitable for screening. The buffer can be any buffer, which does not inhibit binding of the ligand with the receptor protein, such as a Tris-HCl buffer or phosphate buffer of pH 4 to 10 (preferably pH 6 to 8). A surfactant such as CHAPS, Tween-80™ (Kao-Atlas), digitonin, deoxycholate or the like can be added to the buffer for the purpose of reducing non-specific binding. A protease inhibitor such as PMSF, leupeptin E-64 (Peptide Institute), pepstatin or the like can also be added for the purpose of suppressing degradation of the receptor or ligand by proteases. A given amount (5000 cpm to 500000 cpm) of the labeled ligand is added to 0.01 ml to 10 ml of the receptor solution, and $10^{-4}$ M to $10^{-10}$ M of the test compound is included at the same time. A reaction tube filled with a large excess of unlabeled ligand is also prepared for investigating non-specific binding (NSB). The reaction is performed for about 20 minutes to 24 hours or preferably about 30 minutes to 3 hours at about 0°C to 50°C or preferably at about 4°C to 37°C. Following the reaction filtration is performed with fiberglass filter paper or the like, and after washing with a suitable amount of the same buffer radioactivity remaining in the fiberglass filter paper is measured with a liquid scintillation counter or gamma counter. If non-specific binding (NSB) is subtracted from the count with no antagonistic substance ($B_0$) to obtain a count ($B_0$ - NSB) which is given as 10%, a test compound having specific binding (B - NSB) of 50% or less for example can be selected as a candidate substance with competitive inhibition properties.

In order to perform the aforementioned methods of (4) and (5) above for screening compounds which alter the binding properties of a ligand with the receptor protein or the like of the present invention, cell stimulus activity via the aforementioned receptor protein for example can be measured using a well-known method or commercial measurement kit.

Specifically, cells containing the receptor protein or the like of the present invention are first cultured on multiwell plates or the like. For screening purposes fresh medium or a suitable buffer with no toxicity towards the cells is substituted, and the cells are incubated for a fixed time after addition of a test compound or the like, after which the cells are extracted or supernatant collected, and the resulting products are assayed according to the various methods. If production of a substance (for example, arachidonic acid or the like), which is a marker for cell stimulus activity, is difficult to assess due to the presence of a degrading enzyme in the cells, an inhibitor for the degrading enzyme can be added for purposes of the assay. Activity such as cAMP production inhibition or the like can be detected as production inhibition action with respect to cells in which basic production is increased with forskolin or the like.

Cells, which express a suitable receptor protein, are required for screening by measurement of cell stimulus activity. Cell lines having the natural receptor protein or the like of the present invention, or cell lines expressing the aforementioned recombinant receptor protein or the like are desirable as cells which express the receptor protein or the like of the present invention.
A peptide, protein, non-peptide compound, synthetic compound, fermentation product, cell extract, plant extract, animal tissue extract or the like can be used as the test compound, and this compounds may be either a novel compound or a well-known compound.
Compounds designed to bind to the ligand binding pocket based on the atomic coordinates of the active sites of the receptor protein of the present invention and the position of the ligand binding pocket are preferred as test compounds. The atomic coordinates of the active sites of the receptor protein of the present invention and the position of the ligand binding pocket can be measured by well-known methods or methods conforming thereto.
A screening kit for compounds or salts thereof which alter the binding properties of a ligand with the receptor protein or the like of the present invention is one which contains the receptor protein or the like of the present invention, cells containing the receptor protein or the like of the present invention, or a membrane fraction of cells containing the receptor protein or the like of the present invention.
The following are some examples of the screening kit of the present invention.

1. Screening reagents

(1) Measurement buffer and washing buffer
Hanks' Balanced Salt Solution (Gibco) with 0.05% bovine serum albumin (Sigma) added.
Filtered and sterilized with a 0.45 μm filter, stored at 4°C or may be prepared as needed.
(2) G protein-coupled receptor preparation
CHO cells made to express the receptor protein of the present invention, passaged 5 x $10^5$/well on 12-well plates, and incubated for two days at 37°C in 5 % $CO_2$, 95% air.
(3) Labeled ligand
Ligand labeled with commercial [$^3$H], [$^{125}$I], [$^{14}$C], [$^{35}$S] or the like
Stored as an aqueous solution at 4°C or -20°C, diluted for use to 1 μM in measurement buffer.
(4) Ligand standard solution
Ligand dissolved to 1 mM in PBS containing 0.1 % bovine serum albumin (Sigma), stored at -20°C.

2. Measurement methods

(1) CHO cells expressing the receptor protein of the present invention, which have been cultured in 12-well tissue culture plates, are washed twice in 1 ml measurement buffer, and 490 μl of measurement buffer is added to each well.
(2) After addition of 5 μl of a $10^{-3}$ to $10^{-10}$ M test compound solution, 5 μl of labeled ligand is added and reacted for 1 hour at room temperature. 5 μl of $10^{-3}$ M ligand is added in place of the test compound to investigate non-specific binding.
(3) The reaction liquid is removed, and washing performed 3 times with I ml of washing buffer. Labeled ligand bound to the cells is dissolved with 0.2 N NaOH-1% SDS, and mixed with 4 ml of liquid scintillator A (Wako Pure Chemical).
(4) Radioactivity is measured using a liquid scintillation counter (Beckman), and percent maximum binding (PMB) is derived from the following formula:

$$PMB = [(B - NSB)/(B_0 - NSB)] \times 100$$

PMB: percent maximum binding
B: Value when specimen added
NSB: Non-specific binding
$B_0$ Maximum binding

Either (i) or (ii) below can be followed as the specific method of evaluating whether a compound or salt thereof

which alters the binding properties of a ligand with a receptor protein or the like of the present invention is an agonist or antagonist.

(i) After the binding assay shown in the screening method of 1-3 above has been performed to obtain a compound which alters (or particularly which inhibits) the binding properties of a ligand with a receptor protein or the like of the present invention, measurements are performed to determine whether the compound has cell stimulus activity via the aforementioned receptor protein of the present invention. Compounds or salts thereof, which have cell stimulus activity, are agonists, while compounds or salts thereof having no such activity are antagonists.

(ii)

(a) The test compound is brought into contact with cells containing the receptor protein of the present invention, and cell stimulus activity via the receptor protein of the present invention is measured. Compounds or their salts having cell stimulus activity are agonists.

(b) Cell stimulus activity via the receptor protein of the present invention is measured and compared when a compound (for example, a ligand, agonist to the receptor protein of the present invention or the like) which activates the receptor protein of the present invention is brought into contact with cells containing the receptor protein of the present invention, and when a compound which activates the receptor protein of the present invention and a test compound are brought into contact with cells containing the receptor protein of the present invention. Compounds or salts thereof, which can reduce cell stimulus activity due to compounds that activate the receptor protein of the present invention, are antagonists.

Compounds or salts thereof obtained using the screening method or screening kit of the present invention are compounds which act to alter the binding properties of a ligand with the receptor protein or the like of the present invention, and specifically they are (1) compounds having cell stimulus activity via the aforementioned receptor protein of the present invention (so-called agonists to the receptor protein of the present invention), (2) compounds having no such cell stimulus activity (so-called antagonists to the receptor protein of the present invention), (3) compounds which increase the binding force of the ligand with the receptor protein of the present invention and (4) compounds which reduce the binding force of the ligand with the receptor protein of the present invention.

Examples of such compounds include peptides, proteins, non-peptide compounds, synthetic compounds, fermentation products and the like, and these compounds may be either novel compounds or well-known compounds.

Since agonists to the receptor protein or the like of the present invention have action equivalent to the physiological activity of the aforementioned ligand with respect to the receptor protein or the like of the present invention, they are useful as safe, low-toxicity drugs according to the physiological activity.

Since agonists to the receptor protein or the like of the present invention are capable of suppressing the physiological activity of the aforementioned ligand with respect to the receptor protein or the like of the present invention, they are useful as safe, low-toxicity drugs for suppressing such physiological activity.

Compounds, which increase the binding force of the ligand with the receptor protein of the present invention, are useful as safe, low-toxic drugs because they augment the physiological activity of the ligand with respect to the receptor protein of the present invention.

Compounds, which decrease the binding force of the ligand with the receptor protein of the present invention, are useful as safe, low-toxicity drugs because they reduce the physiological activity of the ligand with respect to the receptor protein of the present invention.

Specifically, compounds or salts thereof obtained by the screening method or screening kit of the present invention which act to alter the binding properties of a ligand with a receptor protein of the present invention are useful as birth inducers and as preventative and therapeutic drugs for disorders such as prostate cancer and other cancers (non-small cell carcinoma, ovarian cancer, stomach cancer, bladder cancer, breast cancer, cervical cancer, colon cancer, rectal cancer, large intestinal cancer and the like), prostatomegaly, male gonad dysfunction, infertility, premature birth, endometriosis, cirrhosis of the liver, hepatitis, hepatic insufficiency, calculus and the like.

(7) Preventative and/or therapeutic agent for various disorders containing a compound (agonist, antagonist or the like) which alters the binding properties of the receptor protein of the present invention with a ligand

A compound (agonist, antagonist) which alters the binding properties of the protein of the present invention with a ligand or the aforementioned ligand for the receptor protein of the present invention can be used as a birth inducer or as a preventative and/or therapeutic agent for disorders associated with dysfunction of the receptor protein of the present invention, such as prostate cancer and other cancers (non-small cell carcinoma, ovarian cancer, stomach cancer, bladder cancer, breast cancer, cervical cancer, colon cancer, rectal cancer, large intestinal cancer and the like), prostatomegaly, male gonad dysfunction, infertility, premature birth, endometriosis, cirrhosis of the liver, hepatitis, hepatic insufficiency and calculus and the like.

When such a compound or ligand is used as a preventative and/or therapeutic agent for a disorder associated with dysfunction of the receptor protein of the present invention, it can be formulated according to the ordinary methods described above.

(8) Assay of proteins and the like using antibodies of the present invention, diagnostic drug containing antibodies of the present invention and diagnostic method employing same

Since the antibodies of the present invention can specifically recognize the receptor protein or the like of the present invention, they can be used in assaying the receptor protein or the like of the present invention in a test liquid, and particular in assaying by sandwich immunoassay, competitive methods, immunometric methods, nephrometry and the like.

Special conditions or operations do not need to be devised for applying any of these immunoassay methods to the measurement method of the present invention. It is sufficient to construct a measurement system for the receptor protein or salt thereof of the present invention with the ordinary technical considerations of a person having ordinary skill in the field added to the normal conditions and operations for the respective methods. General texts and manuals can be consulted with regard to the details of these ordinary technical methods [see for example, Irie, Hiroshi ed. Radioimmunoassay (Kodansha, 1974), Irie, Hiroshi ed. Radioimmunoassay Continued (Kodansha, 1979), Ishikawa, Eiji et al ed. Enzyme Immunoassay (Igaku Shoin, 1978), Ishikawa, Eiji et al ed. Enzyme Immunoassay 2nd edition (Igaku Shoin, 1982), Ishikawa, Eiji et al ed. Enzyme Immunoassay 3rd edition (Igaku Shoin, 1987), Methods in Enzymology Vol. 70 Immunochemical Techniques (Part A), Vol. 73 Immunochemical Techniques (Part B), Vol. 74 Immunochemical Techniques (Part C), Vol. 84 Immunochemical Techniques (Part D: Selected Immunoassays), Vol. 92 Immunochemical Techniques (Part E: Monoclonal Antibodies and General Immunoassay Methods), and Vol. 121 Immunochemical Techniques (Part I: Hybridoma Technology and Monoclonal Antibodies) (all Academic Press), WO publication 00/14227 page 39, line 25 through page 42, line 8, EP publication #1111047A2 paragraph [0115] page 19, line 35 through page 20, line 47].

As described above, the antibodies of the present invention can be used to sensitively assay the receptor protein or salt thereof of the present invention.

Moreover, it is also possible to diagnose various diseases associated with dysfunction of the receptor protein of the present invention by using the antibodies of the present invention to assay the receptor protein or salt thereof of the present invention in vivo.

For example, if an increase or decrease in the concentration of the receptor protein is detected in an assay of the concentration of the receptor protein of the present invention using the antibodies of the present invention, it is possible to diagnose a high likelihood of dysfunction or over-expression of the receptor protein for example, particularly prostate cancer, other cancers (non-small cell carcinoma, ovarian cancer, stomach cancer, bladder cancer, breast cancer, cervical cancer, colon cancer, rectal cancer large intestinal cancer), prostatomegaly, male gonad dysfunction, infertility, premature birth, endometriosis, cirrhosis of the liver, hepatitis, hepatic insufficiency or calculus, or a high likelihood of such dysfunction occurring in the future.

The antibodies of the present invention can also be used to specifically detect the receptor protein or the like of the present invention, which is present in a test specimen such as bodily fluid or tissue. They can also be used for preparing an antibody column to be used for purifying the receptor protein or the like of the present invention, for detecting the receptor protein or the like of the present invention in the various fractions during purification, and for analyzing the behavior of the receptor protein of the present invention in test cells.

(9) Method of screening compounds that alter the amount of the receptor protein or partial peptide thereof in cell membranes

Since the antibodies of the present invention can specifically recognize the receptor protein or partial peptide or salt thereof of the present invention, they can be used to screen compounds, which alter the amount of the receptor protein or partial peptide thereof in cell membranes.

Namely, the present invention provides the following screening methods for example:

(i) A method of screening compounds which alter the amount of the receptor protein or partial peptide thereof of the present invention in cell membranes, wherein a) blood, b) specific organs, or c) tissue, cells or the like isolated from specific organs of non-human mammals are ground, the cell membrane fraction is isolated, and the receptor protein or partial peptide thereof of the present invention contained in the cell membrane fraction is assayed;
(ii) A method of screening compounds which alter the amount of the receptor protein or partial peptide thereof of the present invention in cell membranes, wherein a transformant or the like which expresses the receptor protein or partial peptide thereof of the present invention is crushed, the cell membrane fraction is isolated, and the receptor protein or partial peptide thereof of the present invention contained in the cell membrane fraction is assayed;

(iii) A method of screening compounds which alter the amount of the receptor protein or partial peptide thereof of the present invention in cell membranes, wherein a) blood, b) specific organs, or c) tissue, cells or the like isolated from specific organs of non-human mammals are sliced, and the degree of staining of the receptor protein in the cell cortex is quantified by immune staining in order to confirm the protein on the cell membranes; and

(iv) A method of screening compounds which alter the amount of the receptor protein or partial peptide thereof of the present invention in cell membranes, wherein a transformant or the like which expresses the receptor protein or partial peptide thereof of the present invention is sliced, and the degree of staining of the receptor protein in the cell cortex is quantified by immune staining in order to confirm the protein on the cell membranes.

Specifically, the receptor protein or partial peptide thereof of the present invention in a cell membrane fraction is assayed as follows.

(i) A drug (such as an anti-cancer drug) or physical stress (such as immersion stress, electric shock, brightness contrast or low temperature) or the like is applied to normal or disease-model non-human mammals (such as mice, rats, rabbits, sheep, pigs, cows, cats, dogs, monkeys or the like, or more specifically cancer-carrying mice), and after a fixed amount of time blood or specific organs (such as brains, lungs, large intestines, prostate glands or the like) or tissue or cells isolated from organs are obtained. The resulting organs, tissues, cells or the like are suspended for example in suitable buffer (such as a tris-hydrochloric acid buffer, phosphoric acid buffer or Hepes buffer) or the like, the organs, tissues or cells are crushed, and a cell membrane fraction is obtained by a method such as centrifugation, filtration, column fractioning or the like using a surfactant (such as Triton x 100™ or Tween 20™) or the like.

The cell membrane fraction is a fraction containing many cell membranes, which is obtained by well-known methods after crushing of the cells. Examples of cell crushing methods include pounding of cells with a Potter-Elvehjem homogenizer, crushing with a Waring blender or Polytron (Kinematica), crushing by ultrasound or crushing by extruding the cells through a fine nozzle while applying pressure with a french press or the like. A fractioning method using centrifugal force, such as fraction centrifugation or density gradient centrifugation, is generally used for the cell membrane fraction. For example, crushed cell liquid is centrifuged for a short period of time (normally about 1 to 10 minutes) at a low speed (500 rpm to 3000 rpm), the supernatant is then centrifuged for 30 minutes to 2 hours at a high speed (15000 rpm to 30000 rpm), and the resulting precipitate is the membrane fraction. The expressed receptor protein and the like and membrane components such as cell-derived phospholipids and membrane proteins are abundantly present in the membrane fraction.

The receptor protein or partial peptide thereof of the present invention contained in the cell membrane fraction can be assayed for example by Western blotting analysis or sandwich immunoassay using the antibodies of the present invention or the like.

Sandwich immunoassay can be performed by the same methods as those described above, while Western blotting can be performed by well-known methods.

(ii) A transformant expressing the receptor protein or partial peptide thereof of the present invention can be prepared by the methods described above, and the receptor protein or partial peptide thereof of the present invention contained in the cell membrane fraction assayed.

Compounds, which alter the amount of the receptor protein or partial peptide thereof of the present invention in cell membranes can be screened by:

(i) Administering a test compound to normal or disease model non-human mammals a fixed time before (30 minutes to 24 hours before, preferably 30 minutes to 12 hours before, more preferably 1 hour to 6 hours before) or a fixed time after (30 minutes to 3 days after, preferably 1 hour to 2 days after, more preferably 1 hour to 24 hours after) application of a drug, physical stress or the like or simultaneously with the drug or physical stress, and assaying and analyzing the amount of the receptor protein or partial peptide thereof of the present invention in cell membranes a fixed time after administration (30 minutes to 3 days, preferably 1 hour to 2 days, more preferably 1 hour to 24 hours); or

(ii) Mixing a test compound into the medium when culturing the transformant by ordinary methods, and assaying the amount of the receptor protein or partial peptide thereof of the present invention cell membranes after a fixed culture time (1 day to 7 days, preferably 1 day to 3 days, more preferably 2 days to 3 days).

Specifically, the receptor protein or partial peptide thereof of the present invention contained in a cell membrane fraction can be confirmed as follows.

(iii) A drug (such as an anti-dementia drug, blood pressure lowering drug, anti-cancer drug, anti-obesity drug or the like) or physical stress (such as immersion stress, electric shock, brightness contrast or low temperature) or the like is applied to normal or disease-model non-human mammals (such as mice, rats, rabbits, sheep, pigs, cows, cats, dogs, monkeys or the like, or more specifically dementia rats, obese mice, arteriosclerotic rabbits, cancer-carrying mice or the like), and after a fixed amount of time blood or specific organs (such as brains, lungs, large intestines or the like) or tissue or cells isolated from organs are obtained. The resulting organs, tissues, cells or the like are tissue sliced by conventional methods, and immune stained with the antibodies of the present invention. By quantifying the amount of staining of the receptor protein on the cell cortex and confirming the protein on the cell membranes, it is possible to confirm the amount of the receptor protein or partial peptide thereof of the present invention in cell membranes either quantitatively or qualitatively.

(iv) The same can be confirmed by the same methods using a transformant or the like which expresses the receptor protein or partial peptide thereof of the present invention.

A compound or salt thereof obtained by the screening method of the present invention is a compound having the action of altering the amount of the receptor protein or partial peptide thereof of the present invention in cell membranes, and specifically it is (a) a compound which augments cell stimulus activity via the receptor by increasing the amount of the receptor protein or partial peptide thereof of the present invention in cell membranes, or (b) a compound which weakens cell stimulus activity by decreasing the amount of the receptor protein or partial peptide thereof of the present invention in cell membranes.

Examples of this compound include peptides, proteins, non-peptide compounds, synthetic compounds, fermentation products and the like, and these compounds may be either novel compounds or well-known compounds.

A compound, which augments cell stimulus activity is useful as a safe, low-toxic drug for augmenting the physiological activity of the receptor protein or the like of the present invention.

A compound, which weakens cell stimulus activity is useful as a safe, low-toxic drug for decreasing the physiological activity of the receptor protein or the like of the present invention.

(10) Compounds, which alter the amount of the receptor protein or partial peptide thereof of the present invention in cell membranes, and preventative and/or therapeutic agents containing such compounds

As described above, the receptor protein of the present invention is thought to play an important role in vivo for example. Consequently, a compound, which alters the amount of the receptor protein or partial peptide thereof of the present invention in cell membranes, can be used as a preventative and/or therapeutic agent for disorders associated with dysfunction of the receptor protein of the present invention. For example, such a compound can be used as a birth inducer or as a preventative and/or therapeutic drug for disorders caused by over-expression and the like of this receptor protein (such as prostate cancer and other cancers (non-small cell carcinoma, ovarian cancer, stomach cancer, bladder cancer, breast cancer, cervical cancer, colon cancer, rectal cancer, large intestinal cancer and the like), prostatomegaly, male gonad dysfunction, infertility, premature birth, endometriosis, cirrhosis of the liver, hepatitis, hepatic insufficiency, calculus and other disorders).

When this compound is used as a preventative and/or therapeutic agent for a disorder associated with dysfunction of the receptor protein of the present invention, it can be formulated according to the ordinary methods described above.

(11) Drug containing antibodies to the receptor protein or partial peptide or salt thereof of the present invention

The neutralizing activity of antibodies to the receptor protein or partial peptide or salt thereof of the present invention with respect to the receptor protein and the like is activity which inactivates activity associated with the receptor protein, such as signal transmission. Consequently, when such antibodies have neutralizing activity, they can inactivate signal transmission associated with the receptor protein, including cell stimulus activity via the receptor protein (such as activity which promotes or suppresses arachidonic acid release, acetylcholine release, intercellular $Ca^{2+}$ release, intercellular cAMP production, intercellular cAMP suppression, intercellular cGMP production, inositolphosphoric acid production, cell membrane potential fluctuation, phosphorylation of intercellular proteins, activation of c-fos, reduction of pH or the like, particularly promotion activity of intercellular cAMP production). Consequently, such antibodies can be used in the prevention and/or treatment of disorders caused by over-expression and the like of this receptor protein. For example, neutralizing antibodies to the receptor protein of the present invention can be used as a birth inducer or as a preventative and/or therapeutic drug for disorders stemming from over-expression of this receptor protein (such as prostate cancer and other cancers (non-small cell carcinoma, ovarian cancer, stomach cancer, bladder cancer, breast cancer, cervical cancer, colon cancer, rectal cancer, large intestinal cancer and the like), prostatomegaly, male gonad dysfunction, infertility, premature birth, endometriosis, cirrhosis of the liver, hepatitis, hepatic insufficiency, calculus and other disorders).

The formulation, dosage; administration method and the like described above for the drug of the present invention can be used (see "Means of diagnosing diseases and drugs for treating those diseases").

(12) Preparation of animals with introduced DNA of the present invention

The present invention provides non-human mammals having extrinsic DNA of the present invention (abbreviated hereunder as extrinsic DNA of the present invention) or mutant DNA thereof (sometimes abbreviated hereunder as extrinsic mutant DNA of the present invention).

Namely, the present invention provides:

[1] Non-human mammals having extrinsic DNA of the present invention or mutant DNA thereof;
[2] The animals according to [1], wherein the non-human mammals are rodents;
[3] The animals according to [2]; wherein the rodents are mice or rats; and
[4] A recombinant vector containing extrinsic DNA of the present invention or mutant DNA thereof, and capable of expression in mammals.

Non-human mammals having extrinsic DNA of the present invention or mutant DNA thereof (abbreviated hereunder as DNA transposed animals of the present invention) can be created by transposing the target DNA into germinal cells or the like including unfertilized eggs, fertilized eggs, sperm and initial cells thereof, preferably at the embryogenesis stage of non-human mammal development (and more preferably at the single-cell or fertilized egg cell stage, and generally before the 8-cell stage) by the calcium phosphate method, electrical pulse method, lipofection method, agglutination method, micro-injection method, particle gun method, DEAE-dextran method or the like. These DNA transposition methods can also be used to transpose target extrinsic DNA of the present invention into somatic cells, living organs, tissue cells or the like, which are then used for cell cultures or tissue cultures, or animals having the introduced DNA of the present invention can be created by fusing these cells with the aforementioned germinal cells by well-known methods of cell fusion.

Cows, pigs, sheep, goats, rabbits, dogs, cats, guinea pigs, hamsters, mice, rats or the like for example can be used as the non-human mammals. Of these, rodents and particularly mice (such as pure strains C57 BL/6, DBA2 or the like and hybrid strains B6C3F$_1$, BDF$_1$, B6D2F$_1$, BALB/c, ICR and the like) and rats (such as Wistar, SD or the like) are desirable for creating animal disease models because their ontogenesis and life cycles are relatively short and because they are easy to breed.

In addition to the aforementioned non-human mammals, humans and the like are also possible as the "mammals" for the recombinant vector capable of expression in mammals.

The extrinsic DNA of the present invention is not DNA of the present invention originally present in non-human mammals, but DNA of the present invention which has been isolated or extracted from mammals.

DNA or the like wherein a modification (such as a mutation) has occurred in the nucleotide sequence of the original DNA of the present invention, specifically DNA wherein a base addition, deletion or replacement by another base has occurred, can be used as the mutant DNA of the present invention, and abnormal DNA is also included.

Such abnormal DNA signifies DNA which expresses the abnormal receptor protein of the present invention, and for example DNA and the like can be used which expresses a receptor protein which suppresses the function of the normal receptor protein of the present invention.

The extrinsic DNA of the present invention may be derived from either the same species or a different species of mammal than the target animal. For introducing the DNA of the present invention into the target animal, it is normally useful to use a DNA construct having the DNA bound downstream from a promoter capable of promoting expression in animal cells. For example, when introducing human DNA of the present invention, a DNA construct (such as a vector) having the human DNA of the present invention bound downstream from various promoters capable of promoting expression of DNA derived from various mammals (such as rabbits, dogs, cats, guinea pigs, hamsters, rats, mice and the like) having DNA of the present invention with high homology to the human DNA can be microinjected into the fertilized eggs of target mammals (such as fertilized mouse eggs) to create a transgenic mammal which highly expresses the DNA of the present invention.

An *E. coli*-derived plasmid, *B. subtilis*-derived plasmid, yeast-derived plasmid, gamma phage or other bacteriophage, Moloney leukemia virus or other retrovirus or Vaccinia virus or other baculovirus or another animal viruscan be used as the expression vector for the receptor protein of the present invention. Of these, an E. coli-derived plasmid, *B. subtilis*-derived plasmid or yeast-derived plasmid is used by preference.

As the promoter which regulates the aforementioned DNA expression, it is possible to use (1) a DNA promoter derived from a virus (such as a simian virus, cytomegalovirus, Moloney leukemia virus, JC virus, breast cancer virus, polio virus or the like) or (2) a promoter derived from various mammals (such as humans, rabbits, dogs, cats, guinea pigs, hamsters, rats, mice and the like), such as an albumin, insulin II, uroplakin II, elastase, erithropoietin, endothelin, muscle creatine kinase, glial fibrillary acidic protein, glutathione S transferase, platelet-derived growth factor beta, keratin K1, K10 and K14, collagen I and II, cyclic AMP-dependent protein kinase beta I subunit, dystrophin, tartrate-resistant alkali phosphatase, atrial natriuretic factor, endothelial receptor tyrosine kinase (generally abbreviated as Tie2), Na,K-ATPase, neurofilament light chain, metallothionein I and IIA, metalloproteinase 1 tissue inhibitor, MHC class I antibody (H-2L), H-ras, renin, dopamine beta hydroxylase, thyroid peroxidase (TPO), peptide chain elongation factor 1$\alpha$ (EF-1$\alpha$), beta actin, alpha and beta myosin heavy chains, myosin light chains 1 and 2, myelin basic protein, thyroglobulin, Thy-1, immunoglobulin, H chain variable region (VNP), serum amyloid P component, myoglobin, troponin C, smooth muscle alpha actin; preproenkephalin A, vasopressin and other promoters. Of these, the cytomegalovirus promoter, human peptide chain elongation factor 1$\alpha$ (EF-1$\alpha$) promoter, human and chicken beta actin promoters and the like which are capable of high expression throughout the body are preferred.

The aforementioned vector preferably has a sequence (normally called a terminator) which terminates transcription of the target mRNA in the mammal having introduced DNA. For example, the respective DNA sequences derived from viruses or various mammals can be used, and the simian virus SV40 terminator is used by preference.

It is also possible depending on the objective to attach the respective DNA splicing signals, enhancer regions, part of eukaryotic DNA introns or the like on the 5' upstream side of the promoter region, between the promoter region and the translation region or on the 3' downstream side of the translation region in order to induce even higher expression of the extrinsic DNA of the present invention.

The translation region of the normal receptor protein of the present invention may be obtained as DNA derived from livers, kidneys, thyroid glands or fibroblasts from humans and various other mammals (such as rabbits, dogs, cats, guinea pigs, hamsters, rats, mice and the like) and as all or part of genomic DNA from various commercial genome DNA libraries, or else complement DNA prepared by well-known methods from RNA derived from livers, kidneys, thyroid glands or fibroblasts may be obtained as raw material. In the case of extrinsic abnormal DNA, the translation region of the normal receptor protein of the present invention obtained from the aforementioned cells or tissues can be mutated by point mutagenesis to prepare a mutant translation region.

This translation region can be prepared as a DNA construct capable of expression in transgenic animals by ordinary DNA engineering methods of ligation downstream from the promoter and if desired upstream from the transcription termination site.

Introduction of the extrinsic DNA of the present invention at the fertilized egg cell stage ensures that it will be present in all germinal cells and somatic cells of the target mammal. Having extrinsic DNA of the present invention present in germinal cells of the resulting animal after DNA introduction means that all the progeny of the resulting animal will have the extrinsic DNA of the present invention in all their germinal and somatic cells. The descendents of animals of this type which inherit the extrinsic DNA of the present invention will have extrinsic DNA of the present invention in all their germinal and somatic cells.

Non-human mammals having introduced extrinsic normal DNA of the present invention can be bred to confirm the stable presence of the extrinsic DNA, and successive generations can be reared in a normal environment as animals having this DNA.

Introduction of the extrinsic DNA of the present invention at the fertilized egg stage ensures that it is present in excess in all germinal and somatic cells of the target mammals. Having an excess of the extrinsic DNA of the present invention present in germinal cells of the resulting animal after DNA introduction means that all progeny of the resulting animal will have an excess of the extrinsic DNA of the present invention in all their germinal and somatic cells. The descendents of animals of this type which inherit the extrinsic DNA of the present invention will have an excess of the extrinsic DNA of the present invention in all their germinal and somatic cells.

Homozygous animals can be obtained having the introduced DNA in both homologous chromosomes, and male and female animals of this type crossed to breed successive generations in which all progeny have the DNA in excess.

The normal DNA of the present invention is highly expressed in non-human mammals having the normal DNA of the present invention, promoting the function of intrinsic normal DNA and ultimately leading in some cases to hyperfunction of the receptor protein of the present invention, for which they can be used as disease model animals. For example, animals having normal introduced DNA of the present invention can be used to elucidate the pathological mechanisms of hyperfunction of the receptor protein of the present invention and of diseases associated with the receptor protein of the present invention, and to investigate therapy methods for these diseases.

Moreover, since mammals having introduced extrinsic normal DNA of the present invention exhibit symptoms of increased free receptor protein of the present invention, they can also be used in screening tests for therapeutic drugs for diseases associated with the receptor protein of the present invention.

On the other hand, non-human mammals having extrinsic abnormal DNA of the present invention can be bred to confirm the stable presence of the extrinsic DNA, and successive generations reared in a normal environment as animals having this DNA. Moreover, the target extrinsic DNA can be incorporated into the aforementioned plasmid and used as raw material. A DNA construct with a promoter can be prepared by normal DNA engineering techniques.

Introduction of the abnormal DNA of the present invention at the fertilized egg stage ensures that it will be present in all germinal and somatic cells of the target mammal. Having abnormal DNA of the present invention present in germinal cells of the resulting animal after DNA transposition means that all progeny of the resulting animal will having the abnormal DNA of the present invention in all their germinal and somatic cells. The descendents of animals of this type which inherit the extrinsic DNA of the present invention will have abnormal DNA of the present invention in all their germinal and somatic cells. Homozygous animals can be obtained having the introduced DNA in both homologous chromosomes, and these male and female animals crossed to breed successive generations in which all the progeny have this DNA.

The abnormal DNA of the present invention is highly expressed in non-human mammals having the abnormal DNA of the present invention, inhibiting the function of intrinsic normal DNA and ultimately leading in some cases to refractory disease with functionally inactive receptor protein of the present invention, for which such animals can be used as disease model animals. For example, animals having abnormal introduced DNA of the present invention can be used to elucidate the pathological mechanisms of refractory disease with functionally inactive receptor protein of the present invention, and to investigate therapy methods for this condition.

One specific potential use for animals highly expressing the abnormal DNA of the present invention is as a model for elucidating the functional inhibition (dominant negative effect) of the normal receptor protein by the abnormal receptor protein of the present invention in cases of refractory disease with functionally inactive receptor protein of the present invention.

Moreover, since mammals having introduced extrinsic abnormal DNA of the present invention exhibit symptoms of increased free receptor protein of the present invention, they can also be used in screening tests for therapeutic drugs for refractory disease with functionally inactive receptor protein of the present invention.

Other potential uses for the two aforementioned types of animals with introduced DNA of the present invention are for example:

(1) As a cell source for tissue cultures:
(2) For analyzing relatedness with the receptor protein of the present invention specifically expressed or activated by the receptor protein of the present invention, either by directly analyzing DNA or RNA in tissues of DNA transposed animals of the present invention or by analyzing the receptor protein of the present invention expressed by the DNA;
(3) For culturing cells of tissues having DNA according to standard tissue culture techniques, and using them to research the functions of cells from tissues which are ordinarily difficult to culture;
(4) For screening drugs which enhance the functions of cells using the cells according to 3 above; and
(5) For isolating and purifying the mutant receptor protein of the present invention and preparing antibodies thereto.

Moreover, animals having introduced DNA of the present invention can be used to investigate clinical symptoms of diseases associated with the receptor protein of the present invention, including refractory disease with functionally inactive receptor protein of the present invention, and more precise pathological findings can be obtained for various organs of a disease model for a disease associated with the receptor protein of the present invention, contributing to the development of novel therapy methods and to research and therapy for secondary disorders stemming from these disorders.

Also, various organs can also be removed from animals having introduced DNA of the present invention and chopped, and trypsin or another protease can be used to obtain free cells with introduced DNA, which can be cultured or the cultured cells established as a strain. Relatedness with the specification, apoptosis, differentiation or proliferation of cells producing the receptor protein of the present invention can also be investigated as well as the signal transmission mechanisms of these, and abnormalities thereof can also be investigated, providing useful research material for studying the receptor protein of the present invention and its action.

Moreover, in order to develop therapeutic drugs for disorders associated with the receptor protein of the present invention, including refractory disease with functionally inactive receptor protein of the present invention, effective and rapid methods of screening therapeutic drugs for such disorders using the aforementioned testing and assay methods and the like can be provided using animals having introduced DNA of the present invention. Animals having introduced DNA of the present invention or extrinsic DNA-expressing vectors of the present invention can also be used to investigate and develop DNA therapies for disorders associated with the receptor protein of the present invention.

(13) Knockout animals

The present invention provides non-human mammalian embryonic stem cells wherein the DNA of the present invention is inactivated, and non-human mammals having insufficient expression of the DNA of the present invention.

Namely, the present invention provides:

[1] Non-human mammalian embryonic stem cells wherein the DNA of the present invention is inactivated;
[2] The embryonic stem cells according to [1], wherein the DNA is inactivated by introduction of a reporter gene (such as an *E. coli*-derived beta-galactosidase gene);
[3] The embryonic stem cells according to [1], which are neomycin resistant;
[4] The embryonic stem cells according to [1], wherein the non-human mammal is a rodent;
[5] The embryonic stem cells according to [4], wherein the rodent is a mouse;
[6] A non-human mammal expressing the DNA of the present invention wherein the DNA is inactivated;
[7] The non-human mammal according to [6], wherein the DNA is inactivated by introduction of a reporter gene (such as an *E. coli*-derived beta-galactosidase gene), and wherein the reporter gene can be expressed under the control of a promoter for the DNA of the present invention;
[8] The non-human mammal according to [6], wherein the non-human mammal is a rodent;
[9] The non-human mammal according to [8], wherein the rodent is a mouse; and
[10] A method of screening compounds or salts thereof which promote or inhibit promoter activity for the DNA of the present invention, wherein a test compound is administered to the animals according to [7], and expression of the reporter gene is detected.

Non-human mammalian embryonic stem cells wherein the DNA of the present invention is inactivated are the embryonic stem cells (abbreviated below as ES cells) of non-human mammals wherein either DNA expression ability is suppressed by the artificial addition of a mutation into DNA of the present invention present in the non-human mammals, or wherein the DNA has substantially no ability to express the receptor protein of the present invention because the activity of the receptor protein of the present invention encoded by this DNA has been substantially eliminated (sometimes referred to hereunder as knockout DNA of the present invention).
The non-human mammals used may be as described above.
Methods of artificially introducing a mutation into the DNA of the present invention include for example deleting all or part of the DNA sequence by genetic engineering techniques, or inserting or substituting other DNA. With such a mutation, knockout DNA of the present invention can be created for example by shifting the codon reading frame or destroying the function of the promoter or exon.
Specific examples of non-human mammalian embryonic stem cells wherein the DNA of the present invention is inactivated (abbreviated hereunder as DNA inactivated ES cells of the present invention or knockout ES cells of the present invention) can be obtained for example by isolating DNA of the present invention present in the target non-human mammals and either destroying the function of the exon by inserting into the exon a neomycin-resistant gene, hygromycin-resistant gene or other drug-resistant gene or a reporter gene such as lacZ (beta-galactosidase gene) or cat (chloramphenicol acetyltransferase gene), or else preventing synthesis of complete mRNA by inserting a DNA sequence (such as a polyA addition signal or the like) which terminates transcription of the gene into an intron region among the exons. A DNA chain (abbreviated hereunder as the targeting vector) having a DNA sequence constructed with the gene destroyed in this way is introduced into chromosomes of the animal by homologous recombination for example, the resulting ES cells are analyzed either by Southern hybridization using a DNA sequence on or near the DNA of the present invention as a probe or by PCR using the DNA sequence of the targeting vector and the DNA sequence of a neighboring region other than the DNA of the present invention used in preparing the targeting vector as the primers, and knockout ES cells of the present invention are selected.
The original ES cells for inactivating the DNA of the present invention by homologous recombination or the like may be already established cells as described above or may be newly established by the well-known methods of Evans and Kaufman. For example, in the case of mouse ES cells, 129 strains of ES cells are currently in general use, but since the immunological backgrounds are unclear, those established using C57BL/6 mice or $BDF_1$ mice (C57BL/6, DBA/2 and $F_1$) wherein the low egg yield of C57BL/6 mice is improved by cross-breeding with DBA/2 or the like are used instead by preference in order to obtain pure-strain ES cells with a clear immunological background. $BDF_1$ mice offer the advantages of high egg yield and sturdy eggs, and since they are based on C57BL/6 mice, ES cells obtained therefrom can be used advantageously because when disease model mice are created they can be back-crossed with C57BL/6 mice in order to replace the genetic background with that of C57BL/6 mice.
Blastocysts 3.5 days after fertilization are generally used for establishing ES cells, but alternatively many early embryos can be efficiently obtained by collecting 8-celled embryos and culturing them into blastocysts.
Either female or male ES cells may be used, but normally male ES cells are convenient for creating a reproductive series chimera. It is also desirable to distinguish female and male as soon as possible in order to reduce the work of a complicated culture.
One method of distinguishing female and male ES cells is for example to amplify and detect the gene of the sex-determining region of the Y chromosome by PCR. Using this method, only about 1 colony of ES cells (about 50 cells) is required in contrast to the approximately $10^6$ cells required by conventional karyotype analysis, making it possible to perform primary selection of ES cells at the initial culture stage by distinguishing female and male, and therefore greatly reducing the work of the initial culture stage by allowing male cells to be selected early on.

Secondary selection can be performed for example by confirmation of number of chromosomes by G-banding or the like. The number of chromosomes in the resulting ES cells is desirably 100% of the normal number, but when there are difficulties involving physical operations and the like during cell establishment, it is desirable that they are recloned to normal cells (for example, cells with 2n = 40 chromosomes in the case of mice) after knocking out of the ES cell gene.

The resulting embryonic stem cell line is normally highly prolific, but careful subculturing is required because individual developmental potency is easily lost. For example, a method can be adopted such as culturing at about 37°C in a carbon dioxide incubator (preferably with 5% carbon dioxide, 95% air or 5% oxygen, 5% carbon dioxide, 90% air) with LIF (1 to 10000U/ml) on suitable feeder cells such as STO fibroblasts, and for subculture treating with trypsin-EDTA solution (normally 0.001 to 0.5% trypsin/0.1 to 5 mM EDTA, preferably about 0.1 % trypsin/1 mM EDTA) for example to obtain single cells which are inoculated on newly-prepared feeder cells. Such subcultures are normally performed once every 1 to 3 days, at which time the cells are observed and if cells are found with morphological abnormalities such culture cells should desirably be discarded.

ES cells can be differentiated into various types of cells including parietal muscle, visceral muscle, cardiac muscle and the like by monolayer culturing them to high densities under suitable conditions, or float culturing them until they form a cell aggregate (M. J. Evans and M. H. Kaufman, *Nature* 292, p. 154, 1981; G. R. Martin, *Proc. Natl. Acad. Sci. U.S.A.* 78, p. 7634, 1981; T. C. Doetschman et al, *Journal of Embryology and Experimental Morphology* 87, p. 27, 1985), and cells with insufficient expression of the DNA of the present invention obtained by differentiation of ES cells are useful for the receptor protein of the present invention in vitro or for cytobiological study of the receptor protein of the present invention.

Non-human mammals with insufficient expression of the DNA of the present invention can be distinguished from normal animals by measuring the mRNA amounts of the animals by well-known methods and comparing the expressed amounts indirectly.

The non-human mammals used may be as described above.

The non-human mammals with insufficient expression of the DNA of the present invention can for example have the DNA of the present invention knocked out by introducing a targeting vector prepared as described above into mouse embryonic stem cells or mouse eggs cells, and using homologous gene recombination to replace the DNA of the present invention on the chromosomes of the mouse embryonic stem cells or mouse egg cells with a DNA sequence having the DNA of the present invention of the targeting vector inactivated by introduction.

Cells with knocked-out DNA of the present invention can be determined by Southern hybridization analysis using a DNA sequence on or near the DNA of the present invention as the probe, or by PCR analysis using as the primers a DNA sequence on the targeting vector and the DNA sequence of a neighboring region other than the mouse-derived DNA of the present invention used as the targeting vector. When using non-human mammalian embryonic stem cells, a cell strain having the DNA of the present invention inactivated by homologous gene recombination is cloned, the cells are injected at a suitable cell stage such as into 8-cell non-mammalian embryos or blastocysts, and the resulting chimera embryos are transplanted into the uteri of the non-human mammals which have been made pseudopregnant. The resulting animals are chimera animals consisting of both cells with the normal DNA locus of the present invention and cells with the artificially mutated gene locus of the present invention.

When part of the reproductive cells of these chimera animals have the mutated DNA locus of the present invention, individuals in which all tissues are composed of cells having the DNA locus of the present invention with the artificial mutation can be selected by evaluation of coat color or the like from a group of individuals obtained by cross-breeding of such chimera individuals with normal individuals. The resulting individuals are normally individuals with heterozygous insufficient expression of the receptor protein of the present invention, and they can be bred with other individuals having heterozygous insufficient expression of the receptor protein of the present invention and individuals with homozygous insufficient expression of the receptor protein of the present invention obtained from their offspring.

When egg cells are used, for example transgenic non-human mammals can be obtained having a targeting vector introduced into chromosomes by microinjection of DNA solution into the egg cell nuclei, and those with mutations in the DNA locus of the present invention due to homologous gene recombination can be selected by comparison with these transgenic non-human mammals.

Individuals having the DNA of the present invention knocked out in this way, including individual animals obtained by cross breeding, can be successively bred in a normal environment after confirmation that the DNA has been knocked out.

Moreover, normal methods can be followed for obtaining and maintaining a reproductive series. Namely, homozygous animals having inactivated DNA in both homologous chromosomes can be obtained by cross breeding male and female animals having the inactivated DNA. The resulting homozygous animals can be obtained efficiently by breeding to 1 normal individual and several homozygotes per mother. Successive generations of homozygous and heterozygous animals having inactivated DNA can be bred by cross breeding heterozygous males and females.

Non-human mammalian embryonic stem cells wherein the DNA of the present invention is inactivated are extremely useful for creating non-human mammals with insufficient expression of the DNA of the present invention.

Moreover, because they lack various biological activities which are induced by the receptor protein of the present invention, non-human mammals with insufficient expression of the DNA of the present invention can be models for diseases caused by inactivation of the biological activity of the receptor protein of the present invention, and are therefor useful for discovering causes for these diseases and investigating therapy methods.

(14a) Method of screening compounds having therapeutic or preventative effects against diseases stemming from defects or damage of the DNA of the present invention or the like

Non-human animals having insufficient expression of the DNA of the present invention can be used for screening compounds having therapeutic or preventative effects against diseases stemming from defects or damage of the DNA of the present invention.

Namely, the present invention provides a method of screening compounds having therapeutic or preventative effects against diseases stemming from defects or damage of the DNA of the present invention, characterized in that a test compound is administered to non-human mammals having insufficient expression of the DNA of the present invention, and changes in the animals are observed and measured.

The non-human animals having insufficient expression of the DNA of the present invention used in this screening method may be the same as described above.

Examples of test compounds include for example peptides, proteins, non-peptide compounds, synthetic compounds, fermentation products, cell extracts, plant extracts, animal tissue extracts, plasma and the like, and these compounds may be novel compounds or well-known compounds.

Specifically, non-human animals having insufficient expression of the DNA of the present invention can be treated with the test compound and compared with untreated control animals, and changes in the organs, tissues, disease symtpoms and the like of the animals can be used as an indicator for testing the therapeutic and preventative effects of the test compound.

Methods that can be used for treating the test animals with the test compound include oral administration and intravenous injection, and can be selected as appropriate according to the symptoms of the test animal, the nature of the test compound and the like. The dosage of the test compound can be selected as appropriate depending on the method of administration, the nature of the test compound and the like.

In this screening method, if a test compound is administered to a test animal and the test animal's blood sugar level falls by about 10% or more or preferably 30% or more or more preferably 50% or more, the test compound can be selected as a compound having therapeutic or preventative effects for the aforementioned disorder.

Compounds obtained using this screening method are compounds selected from the aforementioned test compounds, and can be used as drugs such as safe, low-toxicity therapeutic or preventative agents for disorders caused by defects or damage of the receptor protein of the present invention or the like (such as therapeutic and/or treatment drugs for prostate cancer and other cancers (non-small cell carcinoma, ovarian cancer, stomach cancer, bladder cancer, breast cancer, cervical cancer, colon cancer, rectal cancer, large intestinal cancer and the like), prostatomegaly, male gonad dysfunction, infertility, premature birth, endometriosis, cirrhosis of the liver, hepatitis, hepatic insufficiency or calculus, or birth inducers or the like). Moreover, compounds derived from compounds obtained by this screening method can be used in the same way.

Compounds obtained by this screening method may also be in the form of salts, and salts of physiologically allowable acids (such as inorganic acids, organic acids and the like) or bases (such as alkaline metals and the like) and the like can be used as salts of these compounds, with physiologically allowable acid-added salts being particularly desirable. Such salts, which can be used, include for example salts of inorganic acids (such as hydrochloric acid, phosphoric acid, hydrobromic acid, sulfuric acid and the like) or organic acids (such as acetic acid, formic acid, propionic acid, fumaric acid, maleic acid, succinic acid, tartaric acid, citric acid, malic acid, oxalic acid, benzoic acid, methanesulfonic acid, benzenesulfonic acid or the like).

A drug containing a compound or salt thereof obtained by this screening method can be manufactured in the same way as the drug described above containing a compound which alters the binding properties of the receptor protein of the present invention with a ligand.

Because a preparation obtained in this way is safe and of low toxicity, it can be administered for example to humans or mammals (such as rats, mice, guinea pigs, rabbits, sheep, pigs, cows, horses, cats, dogs, monkeys and the like).

The dosage of this compound or salt thereof differs depending on the target condition, subject of administration, administration route and the like, but for example in the case of oral administration it is generally about 0.1 to 100 mg or preferably about 1.0 to 50 mg or more preferably about 1.0 to 20 mg per day for example in the case of a prostate cancer patient (weight 60 kg). In the case of parenteral administration the single dose will differ depending on the subject of administration, target organ, symptoms, method of administration and the like, but for example in injection form it is normally desirable to administer about 0.01 to 30 mg or preferably about 0.1 to 20 mg or more preferably about 0.1 to 10 mg a day for example by intravenous injection to a prostate cancer patient (weight 60 kg). In the case of other animals, a dose converted from the 60 kg dose can be administered.

(14b) Method for screening compounds, which promote or inhibit the activity of a promoter for the DNA of the present invention

The present invention provides a method of screening compounds or salts thereof which promote or inhibit the activity of a promoter for the DNA of the present invention, characterized in that a test compound is administered to non-human mammals with insufficient expression of the DNA of the present invention, and the expression of a reporter gene is detected.
Of the non-human mammals with insufficient expression of the DNA of the present invention described above, the non-human mammals with insufficient expression of the DNA of the present invention used in the aforementioned screening method are those in which the DNA of the present invention is inactivated by introduction of a reporter gene, and in which this reporter gene can be expressed under the control of a promoter for the DNA of the present invention.
The test compounds may be as described above.
Reporter genes such as those described above may be used, and the beta-galactosidase gene (lacZ), soluble alkaliphosphatase gene; luciferase gene or the like is desirable.
In non-human mammals with insufficient expression of the DNA of the present invention in which the DNA of the present invention has been replaced by a reporter gene, because the reporter gene is under the control of a promoter for the DNA of the present invention, the activity of the promoter can be detected by tracing the expression of a substance encoded by the reporter gene.
For example, if part of the DNA region encoding the receptor protein of the present invention has been replaced by an *E. coli*-derived beta-galactosidase gene (lacZ), beta-galactosidase is expressed in place of the receptor protein of the present invention in tissues in which the receptor protein of the present invention would originally have been expressed. Consequently, the expression of the receptor protein of the present invention in live animals can easily be observed by staining with a reagent such as 5-bromo-4-chloro-3-indolyl-beta-galactopyranoside (X-gal), which provides a substrate for beta-galactosidase. Specifically, mice lacking the receptor protein of the present invention or tissue slices thereof can be fixed in glutaraldehyde and washed in phosphate buffered saline (PBS), then reacted for about 30 minutes to 1 hour at room temperature or near 37°C with a stain containing X-gal, after which the tissue specimens are washed in a 1 mM EDTA/PBS solution to arrest the beta-galatosidase reaction, and coloration is. observed. The mRNA encoding lacZ can also be detected by ordinary methods.
Compounds or salts thereof obtained by the aforementioned screening method are compounds selected from the aforementioned test compounds, and are compounds, which promote or inhibit promoter activity for the DNA of the present invention.
Compounds obtained by this screening method may form salts, and salts of these compounds, which can be used, include salts with physiologically acceptable acids (such as inorganic acids and the like) and bases (such as organic acids and the like), with physiologically acceptable acid-added salts being particularly desirable. Such salts which can be used include for example salts with inorganic acids (such as hydrochloric acid, phosphoric acid, hydrobromic acid, sulfuric acid and the like) and salts with organic acids (such as acetic acid, formic acid, propionic acid, fumaric acid, maleic acid, succinic acid, tartaric acid, citric acid, malic acid, oxalic acid, benzoic acid, methanesulfonic acid, benzenesulfonic acid or the like).
Since compounds and salts thereof which promote promoter activity for the DNA of the present invention are capable of promoting expression of the receptor protein of the present invention and promoting the function of the receptor protein, they are useful for example as drugs such as preventative and/or therapeutic drugs for disorders associated with dysfunction of the receptor protein of the present invention for example.
Since compounds and salts thereof which inhibit promoter activity for the DNA of the present invention are capable of inhibiting expression of the receptor protein of the present invention and inhibiting the function of the receptor protein, they are useful for example as drugs such as preventative and/or therapeutic drugs for disorders associated with over-expression of the receptor protein of the present invention.
Specifically, compounds which promote or inhibit promoter activity for the DNA of the present invention are useful for example as birth inducers or as preventative and/or therapeutic drugs for prostate cancer and other cancers (non-small cell carcinoma, ovarian cancer, stomach cancer, bladder cancer, breast cancer, cervical cancer, colon cancer, rectal cancer, large intestinal cancer and the like), prostatomegaly, male gonad dysfunction, infertility, premature birth, endometriosis, cirrhosis of the liver, hepatitis, hepatic insufficiency, calculus and the like.

Moreover, compounds derived from compounds obtained by the aforementioned screening method can be used in the same way.

A drug containing a compound or salt thereof obtained by this screening method can be manufactured in the same way as the aforementioned drug containing a compound which alters the binding properties of the receptor protein or salt thereof of the present invention with a ligand.

Because a preparation obtained in this way is safe and of low toxicity, it can be administered for example to humans and other mammals (such as rats, mice, guinea pigs, rabbits, sheep, pigs, cows, horses, cats, dogs, monkeys and the like).

The dosage of this compound or salt thereof differs depending on the target condition, subject of administration, administration route and the like, but for example in the case of oral administration of a compound which promotes or inhibits promoter activity for the DNA of the present invention, it is generally about 0.1 to 100 mg or preferably about 1.0 to 50 mg or more preferably about 1.0 to 20 mg per day for example in the case of a prostate cancer patient (weight 60 kg). In the case of parenteral administration the single dose will differ depending on the subject of administration, target organ, symptoms, method of administration and the like, but for example in injection form it is normally desirable to administer about 0.01 to 30 mg or preferably about 0.1 to 20 mg or more preferably about 0.1 to 10 mg a day by intravenous injection to a prostate cancer patient (weight 60 kg). In the case of other animals, a dose converted from the 60 kg dose can be administered.

In this way, non-human mammals having insufficient expression of the DNA of the present invention are extremely useful for screening compounds or salts thereof which promote or inhibit the activity of a promoter for the DNA of the present invention, and can contribute greatly to discovering the causes of various disorders stemming from insufficient expression of the DNA of the present invention, or to the development of preventative and/or therapeutic drugs.

Moreover, by using DNA containing a promoter region for the receptor protein of the present invention, attaching genes encoding various proteins downstream therefrom and injecting it into egg cells of animals to create so-called transgenic animals, it is possible to specifically induce synthesis of the receptor protein and study its effects in vivo. Moreover, if a suitable reporter gene is bound to the aforementioned promoter part and a cell line such as that expressed thereby is established, it can be used as a search system for low molecular weight compounds which act to specifically promote or inhibit the productive ability of the receptor protein of the present invention itself in vivo.

It was also discovered using the aforementioned method for analyzing gene expression of the present invention that the EDG-1 receptor is highly expressed in vascular endothelial cells and the EDG-2 receptor is highly expressed in vascular smooth muscle cells. Consequently, vascular endothelial cells are useful for screening EDG-1 receptor agonists or antagonists, particularly EDG-1 receptor agonists which can be preventative and/or therapeutic drugs for arteriosclerosis, myocardial infarction, cerebral infarction or ischemic disease, while vascular smooth muscle cells are useful for screening EDG-2 receptor agonists or antagonists, particularly EDG-2 receptor antagonists which can be preventative and/or therapeutic drugs for arteriosclerosis, myocardial infarction, cerebral infarction or ischemic disease.

A screening method for an EDG-1 receptor agonist or antagonist or an EDG-2 receptor agonist or antagonist of the present invention is explained below.

The EDG-1 receptor used in the present invention is a receptor comprising an amino acid sequence identical to or substantially identical to the amino acid sequence represented by SEQ ID NO: 38. The EDG-1 receptor having the amino acid sequence represented by SEQ ID NO: 38 has the same amino acid sequence as the protein described under Genbank Accession Number AAA52336.

The EDG-2 receptor used in the present invention is a receptor comprising an amino acid sequence identical to or substantially identical to the amino acid sequence represented by SEQ ID NO: 40. The EDG-2 receptor having the amino acid sequence represented by SEQ ID NO: 40 has the same amino acid sequence as the protein described under Genbank Accession Number U80811.

A "substantially identical amino acid sequence" signifies an amino acid sequence having for example about 50% or greater or preferably about 60% or greater or more preferably about 70% or greater or even more preferably about 80% or greater or still more preferably about 90% or greater or most preferably about 95% or greater homology with the compared amino acid sequence.

A protein comprising an amino acid sequence substantially identical to the amino acid sequence represented by SEQ ID NO: 38 and having substantially the same activity as an EDG-1 receptor comprising the amino acid sequence represented by SEQ ID NO: 38 or the like for example is preferred as the protein comprising an amino acid sequence substantially identical to the amino acid sequence represented by SEQ ID NO: 38.

A protein comprising an amino acid sequence substantially identical to the amino acid sequence represented by SEQ ID NO: 40 and having substantially the same activity as an EDG-2 receptor comprising the amino acid sequence represented by SEQ ID NO: 40 or the like for example is preferred as the protein comprising an amino acid sequence substantially identical to the amino acid sequence represented by SEQ ID NO: 40.

Examples of substantially the same activity include for example ligand binding activity, signal transmission and the like. Substantially the same means that the activities are the same in character. Consequently, activity such as ligand binding activity, signal transmission and the like is preferably equivalent (such as about 0.01 to 100 times or more preferably 0.5 to 20 times or more preferably 0.5 to 2 times), but quantitative factors such as the degree of activity and the molecular weight of the protein may be different.

Measurement of activity such as ligand binding activity and signal transmission can be accomplished by well-known methods, and for example they can be measured according to the ligand determination method or screening method described below.

A protein comprising (1) an amino acid sequence having one or two or more (preferably about 1 to 30 or more preferably about 1 to 10 or still more preferably several (1 to 5)) amino acids deleted from the amino acid sequence represented by SEQ ID NO: 38, (2) an amino acid sequence having one or two or more (preferably about 1 to 30 or more preferably about 1 to 10 or still more preferably several (1 to 5)) amino acids added to the amino acid sequence represented by SEQ ID NO: 38, (3) an amino acid sequence having one or two or more (preferably about 1 to 30 or more preferably about 1 to 10 or still more preferably several (1 to 5)) amino acids replaced by other amino acids in the amino acid sequence represented by SEQ ID NO: 38, or (4) an amino acid sequence which is a combination of these or the like can be used as the EDG-1 receptor.

A protein comprising (1) an amino acid sequence having one or two or more (preferably about 1 to 30 or more preferably about 1 to 10 or still more preferably several (1 to 5)) amino acids deleted from the amino acid sequence represented by SEQ ID NO: 40, (2) an amino acid sequence having one or two or more (preferably about 1 to 30 or more preferably about 1 to 10 or still more preferably several (1 to 5)) amino acids added to the amino acid sequence represented by SEQ ID NO: 40, (3) an amino acid sequence having one or two or more (preferably about 1 to 30 or more preferably about 1 to 10 or still more preferably several (1 to 5)) amino acids replaced by other amino acids in the amino acid sequence represented by SEQ ID NO: 40, or (4) an amino acid sequence which is a combination of these can be used as the EDG-2 receptor.

Following peptide marking conventions, the left end of the EDG-1 receptor and EDG-2 receptor is the N terminus (amino terminus) and the right end is the C terminus (carboxyl terminus). The C terminus of the receptor protein of the present invention may be a carboxyl group (-COOH), carboxylate ($-COO^-$), amide ($-CONH_2$) or ester (-COOR).

A methyl, ethyl, n-propyl, isopropyl or n-butyl or other $C_{1-6}$ alkyl group for example, a cyclopentyl, cyclohexyl or other $C_{3-8}$ cycloalkyl group for example, a phenyl, alpha-naphthyl or other $C_{6-12}$ aryl group for example, a benzyl, phenethyl or other phenyl-$C_{1-2}$ alkyl group for example or an alpha-naphthylmethyl or other alpha-naphthyl-$C_{1-2}$ alkyl group or other $C_{7-14}$ aralkyl group or else a pivaloyl oxymethyl group or the like widely used as an ester for oral use can be used as the R in the ester here.

When the EDG-1 receptor and EDG-2 receptor have carboxyl groups (or carboxylates) somewhere other than the C terminus, they are included as EDG-1 and EDG-2 receptors if the carboxyl group is amidified or esterified. The aforementioned ester of the C terminus for example can be used as the ester in this case.

The aforementioned EDG-1 receptor and EDG-2 receptor in which the amino group of the methionine residue of the N terminus is protected by a protective group (such as a formyl group, acetyl or other $C_{2-6}$ alkanoyl or other $C_{1-6}$ acyl group or the like), in which a glutamyl group produced by nicking of the N end in vivo is pyroglutaminated, or in which a substitutional group (such as an -OH, -SH, amino group, imidazole group, indole group, guanidino group or the like) on a side chain of an amino acid in the molecule is protected by a suitable protective group (such as a formyl group, acetyl or other $C_{2-6}$ alkanoyl or other $C_{1-6}$ acyl group or the like) are included as the EDG-1 and EDG-2 receptor, as are composite proteins such as so-called glycoproteins having bound sugar chains.

Specific examples of the EDG-1 receptor include for example a receptor protein consisting of the amino acid sequence represented by SEQ ID NO: 38 and the like.

Specific examples of the EDG-2 receptor include for example a receptor protein consisting of the amino acid sequence represented by SEQ ID NO: 40 and the like.

The method for screening EDG-1 receptor agonists and antagonists of the present invention is characterized by the use of vascular endothelial cells in which expression of the EDG-1 receptor is extremely high in comparison with other cells or tissues.

Similarly, the method of screening EDG-2 receptor agonists and antagonists of the present invention is characterized by the use of vascular smooth muscle cells in which expression of the EDG-2 receptor is extremely high in comparison with other cells or tissues.

There are no particular limits on ligands for the EDG-1 receptor as long as they are compounds which bind to the EDG-1 receptor, but for example Sphingosine-1-phosphate (S1P) and the like can be used. A compound, which binds to the EDG-1 receptor, is abbreviated simply as EDG-1.

There are no particular limits on ligands for the EDG-2 receptor as long as they are compounds, which bind to the EDG-2 receptor, but for example lysophosphatidic acid (LPA) and the like can be used. A compound, which binds to the EDG-2 receptor, is abbreviated simply as EDG-2.

Specifically, the screening method of the present invention provides:

(1) Method A for screening EDG-1 receptor agonists or antagonists, characterized by a comparison of (i) bringing EDG-1 into contact with vascular endothelial cells and (ii) bringing EDG-1 and a test compound into contact with vascular endothelial cells; and

(2) Method B for screening EDG-2 receptor agonists or antagonists, characterized by a comparison of (i) bringing EDG-2 into contact with vascular smooth muscle cells and (ii) bringing EDG-2 and a test compound into contact with vascular smooth muscle cells.

Screening method A of the present invention is characterized in that activity which promotes or activity which suppresses arachidonic acid release, acetylcholine release, intercellular $Ca^{2+}$ release, intercellular cAMP production, intercellular cAMP suppression, intercellular cGMP production, inositolphosphoric acid production, cell membrane potential fluctuation, phosphorylation of intercellular proteins, activation of c-fos, cell proliferation, carbon monoxide production, wandering activity, low molecular weight G protein Rho and Rac activation, phosphatidyl inositol (PI) 3 kinase activity, pH reduction or the like for example in vascular endothelial cells is measured and compared in cases (i) and (ii).

Screening method B of the present invention is characterized in that activity which promotes or activity which suppresses arachidonic acid release, acetylcholine release, intercellular $Ca^{2+}$ release, intercellular cAMP production, intercellular cAMP suppression, intercellular cGMP production, inositolphosphoric acid production, cell membrane potential fluctuation, phosphorylation of intercellular proteins, activation of c-fos, cell proliferation, carbon monoxide production, wandering activity, low molecular weight G protein Rho and Rac activation, phosphatidyl inositol (PI) 3 kinase activity, pH reduction or the like for example in vascular smooth muscle cells is measured and compared in cases (i) and (ii).

More specifically, screening method A of the present invention is:

(1a) A method of screening EDG-1 receptor agonists wherein the intercellular cAMP suppression, intercellular cGMP production, inositolphosphoric acid production, cell membrane potential fluctuation, phosphorylation of intercellular proteins, activation of e-fos, cell proliferation, carbon monoxide production, wandering activity, low molecular weight G protein Rho and Rac activation, phosphatidyl inositol (PI) 3 kinase activity and pH reduction of vascular endothelial cells are measured when a test compound is brought into contact with the vascular endothelial cells, and the test compound is selected if the aforementioned activity rises by for example 10% or more or preferably 30% or more or particularly preferably 50% or more;

(1b) A method of screening EDG-1 receptor agonists wherein the intercellular cAMP suppression, intercellular cGMP production, inositolphosphoric acid production, cell membrane potential fluctuation, phosphorylation of intercellular proteins, activation of c-fos, cell proliferation, carbon monoxide production, wandering activity, low molecular weight G protein Rho and Rac activation, phosphatidyl inositol (PI) 3 kinase activity and pH reduction of vascular endothelial cells are measured in a case in which EDG-1 and a test compound are brought into contact with the vascular endothelial cells in comparison with a case in which EDG-1 is brought into contact with the vascular endothelial cells, and the test compound is selected if the aforementioned activity rises by for example 10% or more or preferably 30% or more or particularly preferably 50% or more; or

(1c) A method of screening EDG-1 receptor antagonists wherein the intercellular cAMP suppression, intercellular cGMP production, inositolphosphoric acid production, cell membrane potential fluctuation, phosphorylation of intercellular proteins, activation of c-fos, cell proliferation, carbon monoxide production, wandering activity, low molecular weight G protein Rho and Rac activation, phosphatidyl inositol (PI) 3 kinase activity and pH reduction of vascular endothelial cells are measured in a case in which EDG-1 and a test compound are brought into contact with the vascular endothelial cells in comparison with a case in which EDG-1 is brought into contact with the vascular endothelial cells, and the test compound is selected if the aforementioned activity decreases by 10% or more or preferably 30% or more or particularly preferably 50% or more.

Similarly, screening method B of the present invention is:

(2a) A method of screening EDG-2 receptor agonists wherein the intercellular cAMP suppression, intercellular cGMP production, inositolphosphoric acid production, cell membrane potential fluctuation, phosphorylation of intercellular proteins, activation of c-fos, cell proliferation, carbon monoxide production, wandering activity, low molecular weight G protein Rho and Rac activation, phosphatidyl inositol (PI) 3 kinase activity and pH reduction of vascular smooth muscle cells are measured when a test compound is brought into contact with the vascular smooth muscle cells, and the test compound is selected if the aforementioned activity rises by for example 10% or more or preferably 30% or more or particularly preferably 50% or more;

(2b) A method of screening EDG-2 receptor agonists wherein the intercellular cAMP suppression, intercellular cGMP production, inositolphosphoric acid production, cell membrane potential fluctuation, phosphorylation of intercellular proteins, activation of c-fos, cell proliferation, carbon monoxide production, wandering activity, low molecular weight G protein Rho and Rac activation, phosphatidyl inositol (PI) 3 kinase activity and pH reduction of vascular smooth muscle cells are measured in a case in which EDG-1 and a test compound are brought into contact with the vascular smooth muscle cells in comparison with a case in which EDG-1 is brought into contact with the vascular smooth muscle cells, and the test compound is selected if the aforementioned activity rises by for example 10% or more or preferably 30% or more or particularly preferably 50% or more; or

(2c) A method of screening EDG-2 receptor antagonists wherein the intercellular cAMP suppression, intercellular cGMP production, inositolphosphoric acid production, cell membrane potential fluctuation, phosphorylation of intercellular proteins, activation of c-fos, cell proliferation, carbon monoxide production, wandering activity, low molecular weight G protein Rho and Rac activation, phosphatidyl inositol (PI) 3 kinase activity and pH reduction of vascular smooth muscle cells are measured in a case in which EDG-2 and a test compound are brought into contact with the vascular smooth muscle cells in comparison with a case in which EDG-2 is brought into contact with the vascular smooth muscle cells, and the test compound is selected if the aforementioned activity decreases by 10% or more or preferably 30% or more or particularly preferably 50% or more.

Test compounds which can be used include for example peptides, proteins, non-peptide compounds, synthetic compounds, fermentation products, cell extracts, plant extracts, animal tissue extracts and the like, and these compounds may be either novel compounds or well-known compounds.

It is also preferable that a compound which alters the binding properties of EDG-1 with EDG-1 receptors be used as the test compound in screening method A, and that screening method A be used as secondary evaluation system for this compound.

It is also preferable that a compound, which alters the binding properties of EDG-2 with EDG-2 receptors be used as the test compound in screening method B, and that screening method B be used as secondary evaluation system.

Screening of compounds, which alter the binding properties of EDG-1 with EDG-1 receptors and compounds, which alter the binding properties of EDG-2 with EDG-2 receptors can be accomplished in accordance with the methods described above for screening compounds which alter the binding properties of the dJ287G14.2 receptor protein with ligands.

The screening method of the present invention is explained in detail below.

Vascular endothelial cells or vascular smooth muscle cells can be prepared from the blood vessels of humans and other mammals (such as guinea pigs, rats, mice, rabbits, pigs, sheep, cows, monkeys and the like) in accordance with well-known methods.

Culture of vascular endothelial cells can be accomplished for example according to the methods described in *Cell* 1999 Oct. 29; 99(3): 301-12, or methods conforming thereto.

Culture of vascular smooth muscle cells can be accomplished for example according to the methods described in *Cardiovasc. Res.* 1996 Sep; 32(3): 516-23, or methods conforming thereto.

Specifically, to implement the screening method of the present invention, activity which promotes or activity which suppresses the arachidonic acid release, acetylcholine release, intercellular $Ca^{2+}$ release, intercellular cAMP production, intercellular cAMP suppression, intercellular cGMP production, inositolphosphoric acid production, cell membrane potential fluctuation, phosphorylation of intercellular proteins, c-fos activation, pH reduction and the like of vascular endothelial cells or vascular smooth muscle cells can be measured using well-known methods or a commercial measurement kit.

Specifically, vascular endothelial cells or vascular smooth muscle cells are first cultured on multi-well plates or the like. Fresh medium or a suitable buffer exhibiting no toxicity towards the cells is substituted ahead of time in preparation for screening, a test compound or the like is added, and after a fixed incubation time the cells are extracted or supernatant is collected and the resulting products are assayed according to the respective methods therefor. If production of substances, which are markers for cell stimulus activity (for example, arachidonic acid and the like), is difficult to test because of degradation enzymes contained in the cells, an inhibitor for these degradation enzymes may be added for purposes of the assay. Activity such as cAMP production inhibition or the like can be detected as production inhibition action with respect to cells in which basic production is increased with forskolin or the like.

A screening kit for EDG-1 receptor agonists or antagonists or EDG-2 receptor agonists or antagonists, is one, which contains vascular endothelial cells or vascular smooth muscle cells.

The following can be given as an example of a screening kit of the present invention.

1. Screening reagents

(1) Measurement buffer and washing buffer

0.05% bovine serum albumin (Sigma) added to Hanks' Balanced Salt Solution (Gibco).

May be filtered and sterilized with an 0.45 µm filter and stored at 4°C, or prepared as needed.

(2) Vascular endothelial cells or vascular smooth muscle cells

Vascular endothelial cells or vascular smooth muscle cells are passaged 5 x $10^5$/well on 12-well plates, and cultured for 2 days at 37°C in 5% $CO_2$, 95% air.

(3) Ligand standard solution

EDG-1 or EDG-2 is dissolved to 1 mM in PBS containing 0.1 % bovine serum albumin (Sigma), and stored at -20°C.

Because an EDG-1 receptor agonist obtained using screening method A or a screening kit of the present invention has an action which is the same as the physiological activity of EDG-1, it is useful as a safe, low toxicity drug corresponding to this physiological activity.

Because an EDG-1 receptor antagonist obtained using screening method A or a screening kit of the present invention can suppress the physiological activity of EDG-1, it is useful as a safe, low toxicity drug which suppresses this physiological activity.

Because an EDG-2 receptor agonist obtained using screening method A or a screening kit of the present invention has an action which is the same as the physiological activity of EDG-2, it is useful as a safe, low toxicity drug corresponding to this physiological activity.

Because an EDG-2 receptor antagonist obtained using screening method B or a screening kit of the present invention can suppress the physiological activity of EDG-2, it is useful as a safe, low toxic drug, which suppresses this physiological activity.

Specifically, EDG-1 receptor agonists and EDG-2 receptor antagonists obtained using a screening method or screening kit of the present invention are useful as preventative and/or therapeutic drugs for disorders such as arteriosclerosis, cardiac infarction, cerebral infarction, ischemic disease and the like.

When an EDG-1 receptor agonist or antagonist or EDG-2 receptor agonist or antagonist is used as a preventative and/or therapeutic agent for the aforementioned disorders, it can be formulated in the same way as the aforementioned drug containing a compound which alters the binding properties of the dJ287G 14.2 receptor protein with a ligand.

Because a preparation obtained in this way is safe and of low toxicity, it can be administered for example to humans or mammals (such as rats, mice, guinea pigs, rabbits, sheep, pigs, cows, horses, cats, dogs, monkeys and the like).

The dosage of the EDG-1 receptor agonist or EDG-2 receptor antagonist differs depending on the target disease, subject of administration, administration route and the like, but for example in the case of oral administration of an EDG-1 receptor agonist or EDG-2 receptor antagonist it is generally about 0.1 to 100 mg or preferably about 1.0 to 50 mg or more preferably about 1.0 to 20 mg a day for example in the case of an arteriosclerosis patient (weight 60 kg). In the case of parenteral administration, the single dose will differ depending on the subject of administration, target organ, symptoms and administration method and the like, but for example in injection form it is normally desirable to administer about 0.01 to 30 mg or preferably about 0.1 to 20 mg or more preferably about 0.1 to 10 mg a day by intravenous injection to an arteriosclerosis patient (weight 60 kg) for example. In the case of other animals, a dose converted from the 60 kg dose can be administered.

(Identification of abbreviations)

When bases, amino acids, compounds and the like are represented by abbreviations in these specifications, representation is based on the abbreviations of the IUPAC-IUB Commission on Biochemical Nomenclature or on conventional abbreviations used in the field, and some examples are given below. When an amino acid may have optical isomers, the L form is indicated unless otherwise specified.

| | |
|---|---|
| DNA | Deoxyribonucleic acid |
| cDNA | Complementary deoxyribonucleic acid |
| a or A | Adenine |
| t or T | Thymine |
| g or G | Guanine |

| | |
|---|---|
| c or C | Cytosine |
| u or U | Uracil |
| RNA | Ribonucleic acid |
| mRNA | Messenger ribonucleic acid |
| dATP | Deoxyadenosine triphosphate |
| dTTP | Deoxythymidine triphosphate |
| dGTP | Deoxyguanosine triphosphate |
| dCTP | Deoxycytidine triphosphate |
| ATP | Adenosine triphosphate |
| Gly | Glycine |
| Ala | Alanine |
| Val | Valine |
| Leu | Leucine |
| Ile | Isoleucine |
| Ser | Serine |
| Thr | Threonine |
| Cys | Cysteine |
| Met | Methionine |
| Glu | Glutamic acid |
| Asp | Aspartic acid |
| Lys | Lysine |
| Arg | Arginine |
| His | Histidine |
| Phe | Phenylalanine |
| Tyr | Tyrosine |
| Trp | Tryptophan |
| Pro | Proline |
| Asn | Asparagine |
| Gln | Glutamine |
| pGlu | Pyroglutamic acid |

The sequences described in the sequence listing of these specifications are as follows.

[SEQ ID NO: 1] This represents the nucleotide sequence of hORL1.
[SEQ ID NO: 2] This represents the nucleotide sequence of the upstream primer M-572F used in Example 1.
[SEQ ID NO: 3] This represents the nucleotide sequence of the downstream primer M-714R used in Example 1.
[SEQ ID NO: 4] This represents the nucleotide sequence of the probe M-658T used in Example 1.
[SEQ ID NO: 5] This represents the nucleotide sequence of a primer used in Examples 2 and 3.
[SEQ ID NO: 6] This represents the nucleotide sequence of a primer used in Examples 2 and 3.
[SEQ ID NO: 7] This represents the nucleotide sequence of the probe used in Examples 2 and 3.
[SEQ ID NO: 8] This represents the nucleotide sequence of DNA encoding the human dJ287G14.2 receptor.
[SEQ ID NO: 9] This represents the amino acid sequence of the human dJ287G14.2 receptor.
[SEQ ID NO: 10] This represents the nucleotide sequence of cDNA obtained in Example 2.
[SEQ ID NO: 11] This represents the nucleotide sequence of a primer used in PCR in Reference Example 1.
[SEQ ID NO: 12] This represents the nucleotide sequence of a primer used in PCR in Reference Example 1.
[SEQ ID NO: 13] This represents the amino acid sequence of mouse dJ287G14.2 receptor A.
[SEQ ID NO: 14] This represents the nucleotide sequence of DNA encoding mouse dJ287G14.2 receptor A.
[SEQ ID NO: 15] This represents the amino acid sequence of mouse dJ287G14.2 receptor B.
[SEQ ID NO: 16] This represents the nucleotide sequence of DNA encoding mouse dJ287G14.2 receptor B.
[SEQ ID NO: 17] This represents the amino acid sequence of mouse dJ287G14.2 receptor C.
[SEQ ID NO: 18] This represents the nucleotide sequence of DNA encoding mouse dJ287G14.2 receptor C.
[SEQ ID NO: 19] This represents the amino acid sequence of mouse dJ287G14.2 receptor D.
[SEQ ID NO: 20] This represents the nucleotide sequence of DNA encoding mouse dJ287G14.2 receptor D.
[SEQ ID NO: 21] This represents the amino acid sequence of mouse dJ287G14.2 receptor E.
[SEQ ID NO: 22] This represents the nucleotide sequence of DNA encoding mouse dJ287G14.2 receptor E.
[SEQ ID NO: 23] This represents the amino acid sequence of mouse dJ287G14.2 receptor F.
[SEQ ID NO: 24] This represents the nucleotide sequence of DNA encoding mouse dJ287G14.2 receptor F.
[SEQ ID NO: 25] This represents the amino acid sequence of mouse dJ287G14.2 receptor G.

[SEQ ID NO: 26] This represents the nucleotide sequence of DNA encoding mouse dJ287G14.2 receptor G.
[SEQ ID NO: 27] This represents the amino acid sequence of mouse dJ287G14.2 receptor H.
[SEQ ID NO: 28] This represents the nucleotide sequence of DNA encoding mouse dJ287G14.2 receptor H.
[SEQ ID NO: 29] This represents the nucleotide sequence of a primer used in Example 6.
[SEQ ID NO: 30] This represents the nucleotide sequence of a primer used in Example 6.
[SEQ ID NO: 31] This represents the nucleotide sequence of a primer used in Example 6.
[SEQ ID NO: 32] This represents the nucleotide sequence of a primer used in Example 6.
[SEQ ID NO: 33] This represents the nucleotide sequence of a primer used in Example 6.
[SEQ ID NO: 34] This represents the nucleotide sequence of a primer used in Example 6.
[SEQ ID NO: 35] This represents the nucleotide sequence of a primer used in Example 6.
[SEQ ID NO: 36] This represents the nucleotide sequence of a primer used in Example 6.
[SEQ ID NO: 37] This represents the nucleotide sequence of a primer used in Example 6.
[SEQ ID NO: 38] This represents the amino acid sequence of the human EDG-1 receptor.
[SEQ ID NO: 39] This represents the nucleotide sequence of cDNA encoding the amino acid sequence of the human EDG-1 receptor.
[SEQ ID NO: 40] This represents the amino acid sequence of the human EDG-2 receptor.
[SEQ ID NO: 41] This represents the nucleotide sequence of cDNA encoding the amino acid sequence of the human EDG-2 receptor.
[SEQ ID NO: 42] This represents the amino acid sequence of the human EDG-3 receptor.
[SEQ ID NO: 43] This represents the nucleotide sequence of cDNA encoding the amino acid sequence of the human EDG-3 receptor.
[SEQ ID NO: 44] This represents the amino acid sequence of the human EDG-4 receptor.
[SEQ ID NO: 45] This represents the nucleotide sequence of cDNA encoding the amino acid sequence of the human EDG-4 receptor.
[SEQ ID NO: 46] This represents the amino acid sequence of the human EDG-5 receptor.
[SEQ ID NO: 47] This represents the nucleotide sequence of cDNA encoding the amino acid sequence of the human EDG-5 receptor.
[SEQ ID NO: 48] This represents the amino acid sequence of the human EDG-6 receptor.
[SEQ ID NO: 49] This represents the nucleotide sequence of cDNA encoding the amino acid sequence of the human EDG-6 receptor.
[SEQ ID NO: 50] This represents the amino acid sequence of the human EDG-7 receptor.
[SEQ ID NO: 51] This represents the nucleotide sequence of cDNA encoding the amino acid sequence of the human EDG-7 receptor.
[SEQ ID NO: 52] This represents the amino acid sequence of the human EDG-8 receptor.
[SEQ ID NO: 53] This represents the nucleotide sequence of cDNA encoding the amino acid sequence of the human EDG-8 receptor.
[SEQ ID NO: 54] This represents the nucleotide sequence of a primer for the human EDG-1 receptor used in Example 7.
[SEQ ID NO: 55] This represents the nucleotide sequence of a primer for the human EDG-1 receptor used in Example 7.
[SEQ ID NO: 56] This represents the nucleotide sequence of a probe for the human EDG-1 receptor used in Example 7.
[SEQ ID NO: 57] This represents the nucleotide sequence of a primer for the human EDG-2 receptor used in Example 7.
[SEQ ID NO: 58] This represents the nucleotide sequence of a primer for the human EDG-2 receptor used in Example 7.
[SEQ ID NO: 59] This represents the nucleotide sequence of a probe for the human EDG-2 receptor used in Example 7.
[SEQ ID NO: 60] This represents the nucleotide sequence of a primer for the human EDG-3 receptor used in Example 7.
[SEQ ID NO: 61] This represents the nucleotide sequence of a primer for the human EDG-3 receptor used in Example 7.
[SEQ ID NO: 62] This represents the nucleotide sequence of a probe for the human EDG-3 receptor used in Example 7.
[SEQ ID NO: 63] This represents the nucleotide sequence of a primer for the human EDG-4 receptor used in Example 7.
[SEQ ID NO: 64] This represents the nucleotide sequence of a primer for the human EDG-4 receptor used in Example 7.

[SEQ ID NO: 65] This represents the nucleotide sequence of a probe for the human EDG-4 receptor used in Example 7.
[SEQ ID NO: 66] This represents the nucleotide sequence of a primer for the human EDG-5 receptor used in Example 7.
[SEQ ID NO: 67] This represents the nucleotide sequence of a primer for the human EDG-5 receptor used in Example 7.
[SEQ ID NO: 68] This represents the nucleotide sequence of a probe for the human EDG-5 receptor used in Example 7.
[SEQ ID NO: 69] This represents the nucleotide sequence of a primer for the human EDG-6 receptor used in Example 7.
[SEQ ID NO: 70] This represents the nucleotide sequence of a primer for the human EDG-6 receptor used in Example 7.
[SEQ ID NO: 71] This represents the nucleotide sequence of a probe for the human EDG-6 receptor used in Example 7.
[SEQ ID NO: 72] This represents the nucleotide sequence of a primer for the human EDG-7 receptor used in Example 7.
[SEQ ID NO: 73] This represents the nucleotide sequence of a primer for the human EDG-7 receptor used in Example 7.
[SEQ ID NO: 74] This represents the nucleotide sequence of a probe for the human EDG-7 receptor used in Example 7.
[SEQ ID NO: 75] This represents the nucleotide sequence of a primer for the human EDG-8 receptor used in Example 7.
[SEQ ID NO: 76] This represents the nucleotide sequence of a primer for the human EDG-8 receptor used in Example 7.
[SEQ ID NO: 77] This represents the nucleotide sequence of a probe for the human EDG-8 receptor used in Example 7.

Examples

The present invention is explained in more detail below with reference to examples, but these do not limit the scope of the present invention.

Example 1

In this example, an mRNA sample derived from normal adult human brain is used, the presence or absence and produced amounts of mRNA derived from genes belonging to the target G protein-coupled receptor, tyrosine kinase receptor, ion channel and other gene families in the sample are assayed by TaqMan, and the expressed levels of genes belonging to the target G protein-coupled receptor, tyrosine kinase receptor, ion channel and other families are analyzed. In this example, a 384-well plate is used, and the expression of 192 genes belonging to the target G protein-coupled receptor, tyrosine kinase receptor, ion channel and other families is analyzed. Genes are selected for example from the target GPCR genes ORL; $M_1$; $M_2$; $M_3$; $M_4$; $M_5$; $A_1$; $A_{2A}$, $A_{2B}$, $A_3$; α1A; α1B; α1D; α2A; α2B; α2C; β1; β2; β3; AT1; AT2; BB1; BB2; bb3; $B_1$; $B_2$; CB1; CB2; CCR1; CCR2; CCR3; CCR4; CCR5; CCR6; CCR7; CCR8; CCR9; CCR10; CXCR1; CXCR2; CXCR3; CXCR4; CXCR5; $CX_3CR1$; XCR1; C3a; C5a; fMLP; $CCK_1$; $CCK_2$; $CRF_1$; $CRF_2$; D1; D2; D3; D4; D5; $ET_A$; $ET_B$; GAL1; GAL2; GAL3; $mglu_1$; $mglu_2$; $mglu_3$; $mglu_4$; $mglu_5$; $mglu_6$; $mglu_7$; $mglu_8$; FSH; LSH; TSH; $H_1$; $H_2$; $H_3$; $H_4$; 5-$HT_{1A}$; 5-$HT_{1B}$; 5-$HT_{1D}$; 5-$ht_{1B}$; 5-$ht_{1F}$; 5-$HT_{2A}$; 5-$HT_{2F}$; $5HT_{2C}$; 5-$HT_3$; 5-$HT_4$; 5-$ht_{5A}$; 5-$ht_{5B}$; 5-$HT_6$; 5-$HT_7$; BLT, $CysLT_1$, $CysLT_2$; edg1; edg2; edg3; edg4; $MC_1$; $MC_2$; $MC_3$; $MC_4$; $MC_5$; $MT_1$; $MT_2$; $MT_3$; $Y_1$; $Y_2$; $Y_4$; $Y_5$; $Y_6$; NTS1; NTS2; DOP; KOP; MOP; NOP; $P2Y_1$; $P2Y_2$; $P2Y_4$; $P2Y_6$; $P2Y_{11}$; $P2Y_{12}$; PPAR-α; PPAR-β; PPAR-γ; DP; FP; IP; TP; $EP_1$; $EP_2$; $EP_3$; $EP_4$; PAR1; PAR2; PAR3; PAR4; $sst_1$; $sst_2$; $sst_3$; $sst_4$; $sst_5$; $NK_1$; $NK_2$; $NK_3$; $TRH_1$; $TRH_2$; $VPAC_1$; $VPAC_2$; $PAC_1$; $V_{1a}$; $V_{1b}$; $V_2$; and OT; $Na^+$ channels (Type I; Type II/Type IIA; Type III; SCL11/NaG; PN1; NaCh6; NaDRG; SkM1/μ1, SkM2); $K^+$ channels (Kv; EAG; KQT; IRK; ROMK; GIRK; $K_{ATP}$ and the like); $Ca^{2+}$ channels (α1G; α1E; α1S; α1C; α1D; α1B; α1A; IP3; ryanodine receptor and the like); $Cl^-$ channels ($GABA_A$; $GABA_C$; glycine receptor; C1C0; C1C1; CFTR and the like); and non-selective cation channels (nAChR; 5-$HT_3$; NMDA; AMPA; $P_{2X}$ATP; CNG and the like) and others.

(1) Samples

Amplification is performed using the following samples.
First, preparation of samples for hORL1 mRNA (SEQ ID NO: 1, one type of hGPCR mRNA, GenBank Accession No. X77130) is described.

(Sample for hORL1 mRNA assay)

Serial dilution solution of hORL1 positive control cDNA.
25 ng of total RNA derived from normal adult human brain.
The normal human adult brain in this case is known to express hORL1 mRNA.

(Amplification primers and detection probe for hORL1 mRNA assay)

Amplification of the hORL1 mRNA region was performed using upstream primer M-572F (SEQ ID NO: 2) and downstream primer M-714R (SEQ ID NO: 3). Upstream primer M572F hybridizes with the complement sequence of sites 749 through 771 in the hORL1 mRNA, while downstream primer M-714R hybridizes with the sequence of sites 869 through 891 in the hORL1 mRNA. Using these primers, a product of 143 base pairs is amplified which contains part of the full-length hORL1 mRNA sequence.
Detection is performed using M-658T (SEQ ID NO: 4). This probe hybridizes with the complement sequence of sites 835 through 860 in the hORL1 mRNA.
In order to permit TaqMan-type detection, the probe M-658T is labeled at the 5'-terminal with a fluorescein fluorescent dye (FAM: reporter), and at the 3'-terminal with a rhodamine fluorescent dye (TAMRA: quencher).
When the labeled probe is in a non-hybridizing state, the fluorescence of the reporter is suppressed by the movement phenomenon of fluorescent resonance energy. To prevent elongation of the probe by DNA polymerase during amplification, the 3'-terminus of the probe is synthesized by a phosphate block.

(Specimens for assay of target mRNA other than hORL1 mRNA)

The remaining 191 types of target mRNA assay specimens are prepared in the same way as the hORL1 mRNA above.

(2) Amplification

The respective PCR amplifications are performed using a TaqMan (TM) EZ RT-PCR Kit (Applied Biosystems Japan K.K.), with a total reaction liquid volume of 20 µl. The final reagent concentrations are as follows: Sample gene, 50 mM Bicine, pH 8.2, 115 mM KOAc, 0.01 mM EDTA, 60 nM ROX, 8% (W/V) glycerol, 3 mM Mn $(OAc)_2$, 300 µM dATP, dGTP, dCTP, 600 µM dUTP, 900 nM of each primer, 250 nM of probe, 0.5 units AmpErase UNG, 5 units rTth DNA polymerase.
The amplification reaction is performed using an ABI PRIZM (TM) 7900 HT sequence detection system (Applied Biosystems Japan K.K.), with the following thermal cycle profile.
Thermal cycling times and temperatures

Incubation before reaction: 2 minutes at 50°C
Reverse transcription: 30 minutes at 60°C
Inactivation: 5 minutes at 95°C
Denaturing & annealing/elongation: 40 cycles of 20 seconds at 94°C, 1 minute at 62°C

(3) Quantitative TaqMan analysis

In the TaqMan reaction, the probe, which hybridizes with the aforementioned target sequence, is hydrolyzed from the 5'-terminal by the 5'-->3' exonuclease activity of the DNA polymerase during amplification. As a result, the reporter fluorescent dye is dissociated, and fluorescent strength increases.
Accumulation of the amplification product is measured by measuring the increase in fluorescent strength of the reporter fluorescent dye in the reaction liquid. At the same time, the fluorescent strength of a fluorescent reference (fluorescent dye: ROX) used to correct experimental errors in the reaction liquid is measured. During each amplification cycle, the aforementioned reporter fluorescent dye and reference fluorescent dye are excited by light near their wavelengths of maximum excitation, and the luminescence of the reporter fluorescent dye and reference fluorescent dye is measured near the maximum of luminescence. These frequencies are determined in advance by an ABI PRIZM (TM) 7900 HT sequence detection system, and if another detector is used appropriate frequencies should be selected.
The fluorescence measurement values are analyzed by 7900 HT SDS Software (Applied Biosystems Japan K.K.). First, the fluorescent strength of the reporter fluorescent dye is standardized by the fluorescent strength of the reference fluorescent dye, and a standardized reporter signal (Rn) is calculated. In addition, the relatively constant mean Rn value (baseline) during the cycles of the initial PCR cycles is subtracted from Rn to give ΔRn. ΔRn is plotted against number of cycles on an amplification curve, and the number of cycles at which the analysis algorithm first detects an increase in the fluorescence signal (ΔRn) corresponding to exponential amplification of the amplification product is given as the threshold cycle ($C_T$). Specifically, in order to determine this $C_T$, the initial PCR cycles (3-15 cycles) during which the amplification product is assumed not to have reached exponential amplification were taken as the baseline for calculating the standard deviation of mean ΔRn within this cycle. Next, a value 10 times this standard deviation is defined as the threshold, and the number of cycles corresponding to this threshold value on each amplification curve is given as $C_T$.

During the exponential amplification period of the amplification product, $C_T$ is proportional to the logarithm of the initial number of target copies. The accumulation of amplification product during later cycles inhibits the reaction and causes it to plateau.

(5) Results

$C_T$ values such as the following are obtained for example from each sample for hORL1 mRNA assay. Each $C_T$ value represents a mean value obtained from multiple (such as four) reactions.

| (Control samples) | ($C_T$ value) |
|---|---|
| $10^6$ copies of hORL1 positive control | 21.1, |
| $10^5$ copies of hORL1 positive control | 24.4, |
| $10^4$ copies of hORL1 positive control | 27.5, |
| $10^3$ copies of hORL1 positive control | 30.8, |
| $10^2$ copies of hORL1 positive control | 33.9, |
| 0 copies of hORL1 positive control | 40.0. |
| (Normal adult human brain sample) | ($C_T$ value) |
| 25 ng of total RNA derived from normal adult human brain | 27.2 |

The calibration curve is derived from $C_T$ values obtained from amplification of a template (standard sample) of a known quantity of hORL1 cDNA positive control. Specifically, $C_T$ is plotted against the initial known volume (logarithm) of the standard sample to prepare the calibration curve. The linear equation $C_T$ = (Log $[DNA]_T$ - Log $[DNA]_0$)/ Log (1 + e) (where $[DNA]_0$ is the initial concentration of target template, $[DNA]_T$ is the concentration of amplification product during the $C_T$ cycle, e is the mean amplification efficiency, and Log(X) is a logarithm representing the base when X is 10) is used for the approximation curve, and the parameters are set by 7900 HT SDS software.

The following calibration curve is obtained from the $C_T$ values obtained from the aforementioned control samples:

$$C_T = 40.83 - 3.307 * \text{Log } [DNA]_0$$

The number of copies when the aforementioned normal adult human brain-derived sample is processed is calculated using a calibration curve prepared from the template of a known control. The mRNA concentration is obtained by dividing the calculated number of copies by the size of the sample.

When the standard curve described above is used to calculate the hORL1 mRNA concentration (number of hORL1 mRNA copies per ng of total RNA) for the aforementioned tissue sample, the results are as follows:

| Calculated hORL1 concentration (number of copies/ng) | |
|---|---|
| (Sample) | hORL1 concentration |
| 25 ng of total RNA derived from normal adult human brains | $1.04 * 10^3$ |

The produced amount of gene mRNA belonging to other target G protein-coupled receptor, tyrosine kinase receptor, ion channel and other gene families is assayed in the same way below. The expressed amounts of genes belonging to all target G protein-coupled receptor, tyrosine kinase receptor, ion channel and other gene families in normal adult human brain are analyzed by comparing the produced amounts of gene mRNA belonging to the various target G protein-coupled receptor, tyrosine kinase receptor, ion channel and other gene families.

Example 2 Expression analysis of dJ287G14.2 in prostate cancer cells

(Cells and media)

PrEC was purchased from Takara Shuzo. LNCaP-FGC was purchased from Dainippon Pharmaceutical. PC-3 and Du145 cells were purchased from ATCC. PrEC was cultured using a prostate epithelial cell medium kit (Takara Shuzo). LNCaP-FGC was cultured in RPMI-1640 with 10% FCS (Invitrogen), PC-3 cells in Hams F12K with 10% FCS (Invitrogen), and Du145 cells in Minimum essential medium Eagle with 10% FCS, 2 mM L-glutamine, Eagle BSS and non-essential amino acids (all Invitrogen) added.

(RNA extraction and cDNA synthesis)

The respective cells were cultured to the pre-confluent stage. After being stripped with 0.25% trypsin-1 mM EDTA (Invitrogen) the cells were counted, and total RNA was extracted and purified according to the Rneasy mini Kit (Qiagen) manual. First strand cDNA was synthesized from the extracted RNA according to the SuperScript II (Invitrogen) manual, dissolved as described below after ethanol sedimentation and used.

(Assay using TaqMan)

The synthesized cDNA was dissolved in TE so as to correspond to 10 mg/ml RNA, then diluted to 6.67 ng/µl in TE containing 50 µg/ml of yeast tRNA. The amplification reaction reagent was prepared to a total reaction liquid volume of 15 µl per 3.75 µl (corresponding to 25 ng of RNA) of diluted cDNA solution, using TaqMan (TM) Universal PCR Master Mix (Applied Biosystems Japan K.K.) and a TaqMan (TM) Probe Kit (Applied Biosystems Japan K.K.). The respective primers and probe used were those represented by SEQ ID NOS:5-7, which were designed using Primer Express software (Applied Biosystems) from the nucleotide sequence of the human dJ287G14.2 receptor. The final concentrations were according to the manual.

TaqMan (TM) PCR was performed with an ABI PRISM (TM) 7900HT sequence detection system (Applied Biosystems Japan), and the thermal cycles were according to the TaqMan (TM) Universal PCR Master Mix (Applied Biosystems Japan) manual.

Quantitative TaqMan analysis of the amplification product was performed using 7900HT SDS Software (Applied Biosystems Japan). The number of copies was derived using as the standard the cDNA represented by SEQ ID NO: 10, which comprises a primer amplification region designed based on the nucleotide sequence (SEQ ID NO: 8) of the human dJ287G14.2 receptor.

Expression of dJ287G14.2 in the respective cell lines is shown in Figure 1.

Example 3 Expressed amounts of dJ287G14.2 receptor mRNA in human tissues

An ABI PRISM 7900HT (Applied Biosystems) was used for assaying expressed amounts of mRNA. The primers and probe from the human dJ287G14.2 receptor nucleotide sequence used in the assay were the same as those used in Example 2. The cDNA used as the samples was reverse transcripted using random primers from 1 µg of polyA+ RNA (Clontech) derived from various human tissues. Reactions were performed using reverse transcriptase SuperScript II (GIBCO BRL) according to the attached protocols, followed by ethanol sedimentation and dissolution in 100 µl of TE. The reaction liquid for assay was prepared 20 µl per well according to the protocols for the TaqMan Universal PCR Master Mix (Applied Biosystems), with primer (0.9 µM), probe (0.25 µM) and 0.5 µl of sample cDNA added to the reaction liquid. 40 reaction cycles of 2 minutes at 50°C and 10 minutes at 95°C followed by 15 seconds at 95°C and 1 minute at 60°C were performed in the ABI PRISM 7900HT.

The distribution of dJ287G14.2 receptor mRNA expression in various human tissues is shown in Figure 2. High expression was observed in the placenta, liver and the like.

Reference Example 1 Obtaining dJ287G14.2 receptor gene by PCR from cDNA of human prostate cancer-derived cell line LNCAP

Amplification by PCR was performed using the following two types of synthetic DNA with human prostate cancer-derived cell line LNCAP cDNA as the template.

F1: 5'-CTCGAGATGATGTTTCGCTCAGATCGAATGTGG-3' (SEQ ID NO: 11)

R1: 5'-GCTAGCTCAGCATGGGCCAGTTTTGACAAGGAC-3' (SEQ ID NO: 12)

For the PCR reaction liquid, 1 μl cDNA solution, 0.5 μl F1 (10 μM), 0.5 μl R1 (10 μM), 2.5 μl enclosed 10x reaction liquid, 2.5 μl dNTP (10 mM), 0.5 μl ExTaq (Takara) and 17.5 μl Ootsuka distilled water were added for a total of 25 μl. The reaction liquid was PCR reacted using a ThermalCycler 9600. The PCR conditions were denaturing for 2 minutes at 95°C followed by 35 cycles of 10 seconds at 98°C, 20 seconds at 63°C and 180 seconds at 72°C. After amplification of about 3700 bp of PCR product was confirmed by electrophoresis using part of the PCR product, PCR product was purified using a QIAGEN PCR purification kit and subcloned to a TA vector (pCR2. I TOPO), and sequencing was performed to obtain the sequence (SEQ ID NO: 8) shown in Figures 3 and 4. The amino acid sequence anticipated from the DNA sequence of Figures 3 and 4 is shown in Figures 5 through 8 (SEQ ID NO: 9).

Example 4 cAMP production increase activity caused by plant lectins in dJ/GFP-expressing CHO cells

The CHO cell line used in the experiment was CHO-dJ287G14.2-GFP, which stably expresses a fused protein of GFP and the dJ287G14.2 obtained in Example 1. CHO-dJ287G14.2-GFP cells or mock CHO cells transfected with pAKKO-111H were seeded to a concentration of 2 x $10^4$/well on 96-well plates cultured overnight, and used to measure cAMP production. HBSS (Hank's balanced salt solution, Gibco) with 0.1% bovine serum albumin and 0.2 mM 3-Isobutyl-1-methylxanthine (IBMX, Sigma) added was used as the assay buffer. The cells were washed twice in assay buffer, and preincubated for 30 minutes at 37°C. The cells were washed twice, and samples of lectins and the like diluted with assay buffer were added and incubated for 30 minutes. The culture supernatant was discarded, and cAMP production was measured by a cAMP Screen System (ABI). As shown in Figure 9, the results show concentration-dependent increases in cAMP production due to addition of ConA (Concanavalin A, Wako) Lentil Lectin (Wako) and Pea Lectin (Wako) in CHO-dJ287G14.2-GFP. As shown in Figure 10, however, there was no increase in cAMP production due to addition of the aforementioned lectins in mock CHO cells.

Example 5 Movement inside the cell of dJ287G14.2-GFP protein made to be expressed in CHO cells due to addition of Concanavalin A

An expression plasmid was constructed for expressing a fused protein consisting of Green Fluorescent Protein (GFP) cDNA isolated together with the translation frame from *Aequoria victoria* attached to the C terminus of dJ287G14.2. A fragment cut from GFP expression vector pQB125 (Takara Shuzo) was used as the GFP cDNA. The termination codon of dJ287G14.2 was modified by PCR to a recognition sequence for reductase XbaI, the GFP fragment was ligated thereto, and the expression vector pAKKO-111H described in Example 1 was inserted. As a result, a plasmid of a dJ287G14.2 and GFP fused protein (hereunder, dJ287G14.2-GFP) expression vector was constructed. Next, this dJ287G14.2-GFP expression vector plasmid was introduced by gene transfer (Wako Pure Chemical) into CHOdhfr$^-$ cells. Two days later selection medium was substituted, cells were selected from the proliferated transformants using GFP expression as the marker, and a CHO cell line CHO-dJ287G14.2-GFP expressing the dJ287G14.2-GFP fused protein was established.

Expression of the dJ287G14.2-GFP fused protein in the cells was located by sowing CHO-dJ287G14.2-GFP in a Lab-Tek II cover glass chamber (Nalgen Nunc), culturing them overnight under conditions of 37°C, 5% $CO_2$, substituting a medium for confocal microscopy (Hanks' Balanced Salt Solution (GIBCO BRL)), and observing GFP fluorescence with a confocal microscope (Leica). The medium for confocal microscopy was replaced with medium having sword bean-derived lectin and concanavalin A (ConA, Wako Pure Chemical) added at concentrations of 60 μg/ml, and the cells were observed after reacting for 2 hours at 37°C. GFP excitation was performed at 488 nM.

As a result, dJ287G14.2-GFP fused protein was seen to be expressed in cell membranes (Figure 11). When these cells were cultured for two hours in medium with ConA added, GFP fluorescence was found to be moving not into the cell membranes but into the cytoplasm (Figure 12). This indicated not only that dJ287G14.2 is a G protein-coupled receptor which is expressed in cell membranes, but also that dJ287G14.2 moves into the cytoplasm in response to ConA, or in other words that it is internalized by ConA.

Example 6 Cloning of mouse dJ287G14.2 counterpart

An exon encoding mouse dJ287G14.2 was searched using the Celera mouse genome database, and based on this sequence translation region amplification primers, 5' RACE primers and 3' RACE primers were prepared. Amplification of the translation region was performed by PCR using primers (SEQ ID NOS: 29, 30, 31, 32), mouse spleen, lung, and 14-day fetal cDNA (Marathon-ready cDNA, Clontech) as the templates and PyroBEST DNA polymerase (Takara). Amplification of 3' RACE was performed using gene-specific primers (SEQ ID NOS: 33, 34), mouse spleen cDNA (Marathon-ready cDNA) as the template and Advantage-2 polymerase mix (Clontech). Amplification of 5' RACE was performed by the oligo-cap method using gene-specific primers (SEQ ID NOS: 35, 36, 37), mouse placental cDNA (Cap site cDNA dT Mouse Placenta, NIPPON GENE) as the template and Gene TaqNT (NIPPON GENE). From these results it is shown that mouse dJ287G14.2 exists in two types wherein the sequence of the N terminal differs depending on the connections from the donor site of the first exon to the acceptor site of the second exon, and that it exists with the sixth exon skipped and with the $26^{th}$ exon skipped and the amino acid sequence of the C terminal altered, and ORFs (SEQ ID NOS: 14, 16, 18, 20, 22, 24, 26, 28) were obtained which encode mouse dJ287G14.2 (SEQ ID NOS: 13, 15, 17, 19, 21, 23, 25, 27) consisting of a total of 1165-1258 amino acid residues.

Example 7 Expression of G protein-coupled receptor EDG family mRNA in vascular cells

Total RNA was prepared according to the Isogen (Nippon Gene) manual from fixed quantities of normal human coronary vascular endothelial cells, normal human coronary vascular smooth muscle cells, normal human aortal vascular endothelial cells and normal human aortal vascular smooth muscle cells (purchased from Asahi Technoglass). A reaction was performed from 1 μg of RNA at 42° C using SuperScript II reverse transcriptase (GIBCO BRL) as the reverse transcriptase, according to the attached manual, followed after completion of the reaction by ethanol sedimentation and dissolution in TE (corresponding to 100 ng/μl RNA). Expression of mRNA of the EDG family was assayed using a Sequence Detection System Prism 7900HT system (Applied Biosystems). In order to assay the expressed amounts of the various receptors, TaqMan probes and primers, which specifically recognized the various receptors, were designed and synthesized using Primer Express (PE Applied Biosystems software). 5'-CCACCGACCCATGTACTATTTT-3' (SEQ ID NO: 54), 5'-TGTAGGCTACTCCTGCCAACAG-3' (SEQ ID NO: 55) and 5'-(Fam)-TTGGCAATCTGGCCCTCTCAGA-(Tamra)-3' (SEQ ID NO: 56) as the TaqMan probe were used for detecting EDG-1,5'-ACTGTCAGCACATGGCTCCTT-3' (SEQ ID NO: 57), 5'-ACCGTAATGTGCCTCTCGATT-3' (SEQ ID NO: 58) and 5'-(Fam)-ATTGACACCAGCCTGACGGCAT-(Tamra)-3' (SEQ ID NO: 59) as the TaqMan probe for detecting EDG-2, 5'-CCGTGCTCTTCTTGGTCAT-3' (SEQ ID NO: 60), 5'-CCAGATGGCAATCAAAACC-3' (SEQ ID NO: 61) and 5'-(Fam)-TGCAGCTTCATCGTCTTGGAGAACCT-(Tamra)-3' (SEQ ID NO: 62) as the TaqMan probe for detecting EDG-3, 5'-CCTGGTCAAGACTGTTGTCATC-3' (SEQ ID NO: 63), 5'-CAGGACATTGCAGGACTCA-3' (SEQ ID NO: 64) and 5'-(Fam)-TGGTACTGCTCCTGGATGGTTTAGGCT-(Tamra)-3' (SEQ ID NO: 65) as the TaqMan probe for detecting EDG-4, 5'-CCAACAAGGTCCAGGAACA-3' (SEQ ID NO: 66), 5'-AGGTTTTCCACCACAATGG-3' (SEQ ID NO: 67) and 5'-(Fam) -AATTATACCAAGGAGACGCTGGAAACGC-(Tamra)-3' (SEQ ID NO: 68) as the TaqMan probe for detecting EDG-5, 5'-GAACTGCCTGTGCGCCTTT-3' (SEQ ID NO: 69), 5'-CCATAGAGGCCCATGATGGT-3' (SEQ ID NO: 70) and 5'-(Fam)-TCTGCCCCTCTACTCCAAGCGCTACATC-(Tamra)-3' (SEQ ID NO: 71) as the TaqMan probe for detecting EDG-6, 5'-TGACTGCTTCCCTCACCAA-3' (SEQ ID NO: 72), 5'-GCATCCTCATGATTGACATGTG-3' (SEQ ID NO: 73) and 5'-(Fam)-TTGCTGGTTATCGCCGTGGAGA-(Tamra)-3' (SEQ ID NO: 74) as the TaqMan probe for detecting EDG-7, or 5'-CTTGCTCCACTGTCTTGCC-3' (SEQ ID NO: 75), 5'-TAGAGTGCACAGATCGCGG-3' (SEQ ID NO: 76) and 5'-(Fam)-CTCTACGCCAAGGCCTACGTGCTCTTCT-(Tamra)-3' (SEQ ID NO: 77) as the TaqMan probe for detecting EDG-8.

The reaction liquid for assay was prepared by adding the respective G protein-coupled receptor primers (0.9 μM), probe (0.25 μM) and cDNA corresponding to 25 ng total RNA according to the manual for the TaqMan Universal PCR Master Mix (Applied Biosystems). The PCR reaction was performed in 40 cycles of 2 minutes at 50°C and 10 minutes at 95°C followed by 15 seconds at 95°C and 1 minute at 60°C. In normal human coronary vascular endothelial cells and normal human aortal vascular endothelial cells, EDG-1 exhibited the highest expression of all of the 354 G protein-coupled receptor assayed--1,676,305 copies/25 ng total RNA and 1,017,396 copies/25 ng total RNA, respectively. Likewise, in normal human coronary vascular smooth muscle cells and normal human aortal vascular smooth muscle cells, EDG-2 exhibited the highest expression of all the 354 G protein-coupled receptors assayed--148,922 copies/25 ng total RNA and 310,544 copies/25 ng total RNA, respectively. EDG-3, 4, 5, 6, 7 and 8 exhibited much less expression than EDG-1 and EDG-2. These results indicate that vascular endothelial cells are suitable for screening agonists and antagonists to EDG-1, and vascular smooth muscle cells for screening agonists and antagonists to EDG-2.

## INDUSTRIAL APPLICABILITY

With the analysis method and assay kit of the present invention, a system is provided which can detect the presence or absence of target mRNA and the transcripted amount thereof in a sample containing multiple types of mRNA with high sensitivity in one operation. Consequently, with the present invention it is possible to provide a system, which can rapidly perform expression analysis of target genes with high sensitivity.

Moreover, by selecting as the target mRNA gene mRNA belonging to multiple G protein-coupled receptor, tyrosine kinase receptor, ion channel and other families, it is possible to rapidly and with high sensitivity specify disease genes associated with genes and the like belonging to the G protein-coupled receptor, tyrosine kinase receptor, ion channel and other families.

With the diagnostic method of the present invention, it is possible to diagnose with high precision a disease from which a patient suffers using an mRNA sample collected from that patient.

A receptor protein or partial peptide or salt thereof of the present invention or DNA encoding a receptor protein or partial peptide or salt thereof of the present invention is useful as a preventative and/or therapeutic drug for prostate cancer and other cancers (non-small cell carcinoma, ovarian cancer, stomach cancer, bladder cancer, breast cancer, cervical cancer, colon cancer, rectal cancer, large intestinal cancer and the like), prostatomegaly, male gonad dysfunction, infertility, premature birth, endometriosis, cirrhosis of the liver, hepatitis, hepatic insufficiency, calculus and the like.

Also, by using a receptor protein or partial peptide or salt thereof together with a protein (such as a lectin) having an affinity for a sugar chain which is one ligand, it is possible to efficiently screen compounds which alter the binding properties of the ligand with the dJ287G14.2 receptor or partial peptide or salt thereof of the present invention.

Moreover, EDG1 receptor agonists and antagonists can be efficiently screened using vascular endothelial cells, and EDG2 receptor agonists and antagonists using vascular smooth muscle cells.

## Claims

1. A method for analyzing gene expression wherein genes whose expression is characteristically promoted or inhibited in certain cells or tissue, are identified by quantitatively analyzing the individual expressed amounts of multiple genes collectively.

2. The method according to Claim 1, wherein expression analysis is performed collectively on a plurality of genes belonging to a specific gene family so that the genes in that family whose expression is characteristically promoted or inhibited in certain cells or tissues are identified by computing expressed amounts as absolute values.

3. The method according to Claim 1, wherein an amplification reaction is performed by bringing mRNA sample which may contain a plurality of mRNA targets into contact at a plurality of reaction sites with individual amplification reagents each of which comprises a primer pair corresponding to a particular mRNA target, and gene expression analysis is performed by measuring the amounts produced in the resulting amplification product.

4. The method according to Claim 1, wherein an amplification reaction is performed by bringing mRNA sample which may contain multiple mRNA targets into contact, at the reaction sites of a reaction device having multiple reaction sites, with individual amplification reagents each of which comprises a primer pair corresponding to a particular mRNA target, and gene expression analysis is performed by measuring the amounts produced in the resulting amplification product.

5. The method according to Claim 2, wherein the specific gene family is the G protein-coupled receptor gene family.

6. The method according to Claim 2, wherein the specific gene family is the tyrosine kinase receptor gene family.

7. The method according to Claim 2, wherein the specific gene family is the ion channel gene family.

8. The method according to Claim 2, wherein the specific gene family is a gene family associated with any of transcription factors, transporters, protein kinases, protein phosphatases, proteases, heat shock proteins, ATPases and DNA-binding proteins.

9. A drug comprising a gene or product of a gene, which is specified by a method described in any of Claims 1 through 8, and the expression of which is characteristically promoted or inhibited in certain cells or tissue.

10. The method according to Claim 3 or 4, wherein the reaction device is a plate having a plurality of wells as reaction sites.

11. The method according to Claim 10, wherein the plate is a 96-well or 384-well plate.

12. The method according to Claim 3 or 4, wherein 10 to 800 primer pairs are used.

**13.** The method according to Claim 3 or 4, wherein 10 to 300 primer pairs are used.

**14.** The method according to Claim 3, 4, 12 or 13, wherein the amplification reaction is a polymerase chain reaction.

**15.** The method according to Claim 14, wherein SNP analysis is performed.

**16.** The method according to Claim 3 or 4, wherein the produced amounts of amplification products are measured using probes which are complementary or substantially complementary to said amplification products.

**17.** The method according to Claim 16, wherein the probes are probes which hybridize with mRNA.

**18.** The method according to Claim 17, wherein the probes are fluorescence labeled probes.

**19.** The method according to Claim 3 or 4, wherein normal human-derived mRNA sample and mRNA sample derived from a patient with a specific disease are used as the mRNA samples.

**20.** The method according to Claim 19, wherein mRNA whose expression is promoted or inhibited in mRNA sample derived from a disease patient is specified, and a gene encoding said mRNA is designated as a disease-associated gene for that disease.

**21.** The method according to Claim 20, wherein the specific gene family is the G protein-coupled receptor protein gene family, and wherein a cancer-related gene is specified by using mRNA samples derived from a cancer patient.

**22.** A primer pair kit comprising two or more pairs of primers each consisting of a first primer which is complementary or substantially complementary to one chain of an exon region of a target gene sequence and a second primer which is complementary or substantially complementary to the other chain of the exon region of the target gene sequence.

**23.** The primer pair kit according to Claim 22, wherein the target gene is a human G protein-coupled receptor protein gene, tyrosine kinase receptor gene or ion channel gene.

**24.** The primer pair kit according to Claim 23, composed of 10 to 800 primer pairs.

**25.** The primer pair kit according to Claim 23, composed of 10 to 300 primer pairs.

**26.** A usage of the primer pair kit according to Claim 23, for purposes of specifying disease-associated genes.

**27.** An mRNA assay kit having each reaction site in a reaction device with a plurality of reaction sites filled with a respective amplification reagent comprising a primer pair corresponding to a particular mRNA target.

**28.** The kit according to Claim 27, also comprising a fluorescent probe.

**29.** The kit according to Claim 28, also comprising Tth DNA polymerase,

**30.** A method of diagnosing a patient's disease using the method according to any of Claims 1 through 4 or the assay kit according to Claim 27 by assaying the mRNA of a plurality of target disease genes which may be contained in an mRNA sample collected from the patient, or by measuring the mutated amount of said mRNA.

**31.** The diagnostic method according to Claim 30, wherein cancer is diagnosed by identifying cancer-associated human G protein-coupled receptor protein genes.

**32.** A drug comprising an agonist, antagonist or antibodies to the gene product of a gene identified by the diagnostic method of Claim 30, or DNA encoding said gene product.

**33.** The drug according to Claim 32, which is a cancer therapy drug.

**34.** A method of treating diseases involving a gene identified by the diagnostic method according to Claim 30 by administering an agonist, antagonist or antibodies to the gene product of said gene or DNA encoding said gene product.

**35.** The treatment method according to Claim 34, wherein the disease is cancer.

**36.** A drug containing a receptor protein which contains an amino acid sequence identical to or substantially identical to the amino acid sequence represented by SEQ ID NO:9, or a partial peptide or salt thereof.

**37.** The drug according to Claim 36 which is a birth inducer or a preventative and/or therapeutic drug for cancer, prostatomegaly, male gonad dysfunction, infertility, premature birth, endometriosis, cirrhosis of the liver, hepatitis, hepatic insufficiency or calculus.

**38.** The drug according to Claim 37 wherein the cancer is prostate cancer, non-small cell carcinoma, ovarian cancer, stomach cancer, bladder cancer, breast cancer, cervical cancer, colon cancer, rectal cancer or large intestinal cancer.

**39.** A diagnostic drug containing antibodies to a protein which contains an amino acid sequence identical to or substantially identical to the amino acid sequence represented by SEQ ID NO:9, or to a partial peptide or salt thereof.

**40.** The diagnostic drug according to Claim 39 which is a diagnostic drug for cancer, prostatomegaly, male gonad dysfunction, infertility, premature birth, endometriosis, cirrhosis of the liver, hepatitis, hepatic insufficiency or calculus.

**41.** A drug containing antibodies to a protein which contains an amino acid sequence identical to or substantially identical to the amino acid sequence represented by SEQ ID NO:9, or to a partial peptide or salt thereof.

**42.** The drug according to Claim 41 which is a birth inducer or a preventative and/or therapeutic drug for cancer, prostatomegaly, male gonad dysfunction, infertility, premature birth, endometriosis, cirrhosis of the liver, hepatitis, hepatic insufficiency or calculus.

**43.** A method of screening compounds or salts of compounds which alter the binding properties of a G protein-coupled receptor protein containing an amino acid sequence identical to or substantially identical to the amino acid sequence represented by SEQ ID NO:9 or of a salt thereof with a protein showing affinity for a sugar chain, **characterized by** the use of (1) said G protein-coupled receptor protein, or a partial peptide or salt thereof, and (2) the protein showing affinity for a sugar chain.

**44.** The screening method according to Claim 43, wherein the protein showing affinity for a sugar chain is a protein showing affinity for an asparagine-linked sugar chain or a serine/threonine-linked sugar chain.

**45.** The screening method according to Claim 43, wherein the protein showing affinity for a sugar chain is a lectin.

**46.** The screening method according to Claim 43, wherein the protein showing affinity for a sugar chain is concanavalin A, lentil lectin, pea lectin, datura stramonium lectin, sophora japonica lectin or phytohemagglutinin.

**47.** A screening kit for compounds or salts of compounds which alter the binding properties of a G protein-coupled receptor protein containing an amino acid sequence identical to or substantially identical to the amino acid sequence represented by SEQ ID NO:9 or of a salt thereof with a protein showing affinity for a sugar chain, **characterized in** containing (1) the aforementioned G protein-coupled receptor protein, or a partial peptide or salt thereof, and (2) the protein showing affinity for a sugar chain.

**48.** A compound or salt of a compound which alters the binding properties of a protein showing affinity for a sugar chain with a G protein-coupled receptor protein containing an amino acid sequence identical to or substantially identical to the amino acid sequence represented by SEQ ID NO: 9 or with a salt thereof, obtained using the screening method according to Claim 46 or the screening kit according to Claim 47.

**49.** A drug containing a compound or salt thereof according to Claim 48.

**50.** The drug according to Claim 49 which is a birth inducer or a preventative and/or therapeutic drug for cancer, prostatomegaly, male gonad dysfunction, infertility, premature birth, endometriosis, cirrhosis of the liver, hepatitis,

hepatic insufficiency or calculus.

**51.** The drug according to Claim 50 wherein the cancer is prostate cancer, non-small cell carcinoma, ovarian cancer, stomach cancer, bladder cancer, breast cancer, cervical cancer, colon cancer, rectal cancer or large intestinal cancer.

**52.** A method of screening birth inducers or compounds or salts of compounds for preventing and/or treating cancer, prostatomegaly, male gonad dysfunction, infertility, premature birth, endometriosis, cirrhosis of the liver, hepatitis, hepatic insufficiency or calculus by altering the expressed amount of a G protein-coupled receptor protein containing an amino acid sequence identical to or substantially identical to the amino acid sequence represented by SEQ ID NO: 9, **characterized by** the use of a polynucleotide containing a polynucleotide encoding said G protein-coupled receptor protein or a partial peptide thereof.

**53.** A screening kit for screening birth inducers or compounds or salts of compounds for preventing and/or treating cancer, prostatomegaly, male gonad dysfunction, infertility, premature birth, endometriosis, cirrhosis of the liver, hepatitis, hepatic insufficiency or calculus by altering the expressed amount of a G protein-coupled receptor protein containing an amino acid sequence identical to or substantially identical to the amino acid sequence represented by SEQ ID NO: 9, said screening kit containing a polynucleotide containing a polynucleotide encoding said G protein-coupled receptor protein or a partial peptide thereof.

**54.** A birth inducer or compound or salt of a compound for preventing and/or treating cancer, prostatomegaly, male gonad dysfunction, infertility, premature birth, endometriosis, cirrhosis of the liver, hepatitis, hepatic insufficiency or calculus by altering the expressed amount of a G protein-coupled receptor protein containing an amino acid sequence identical to or substantially identical to the amino acid sequence represented by SEQ ID NO: 9 or of a partial peptide thereof, that can be obtained using the screening method according to Claim 52 or the screening kit according to Claim 53,

**55.** A birth inducer or preventative and/or therapeutic agent for cancer, prostatomegaly, male gonad dysfunction, infertility, premature birth, endometriosis, cirrhosis of the liver, hepatitis; hepatic insufficiency or calculus, containing a compound or salt thereof according to Claim 54.

**56.** The agent according to Claim 55 wherein the cancer is prostate cancer, non-small cell carcinoma, ovarian cancer, stomach cancer, bladder cancer, breast cancer, cervical cancer, colon cancer, rectal cancer or large intestinal cancer.

**57.** A signal transmission enhancing agent for a G protein-coupled receptor protein containing an amino acid sequence identical to or substantially identical to the amino acid sequence represented SEQ ID NO: 9, containing a protein showing affinity for a sugar chain.

**58.** The agent according to Claim 57, which is a birth inducer or preventative and/or therapeutic agent for cancer, prostatomegaly, male gonad dysfunction, infertility, premature birth, endometriosis, cirrhosis of the liver, hepatitis, hepatic insufficiency or calculus.

**59.** The agent according to Claim 58, wherein the cancer is prostate cancer, non-small cell carcinoma, ovarian cancer, stomach cancer, bladder cancer, breast cancer, cervical cancer, colon cancer, rectal cancer or large intestinal cancer.

**60.** A birth inducer or preventative and/or therapeutic agent for cancer, prostatomegaly, male gonad dysfunction, infertility, premature birth, endometriosis, cirrhosis of the liver, hepatitis, hepatic insufficiency or calculus, containing a polynucleotide which contains a polynucleotide encoding a G protein-coupled receptor protein containing an amino acid sequence identical to or substantially identical to the amino acid sequence represented by SEQ ID NO: 9, or a partial peptide thereof.

**61.** The agent according to Claim 60, wherein the cancer is prostate cancer, non-small cell carcinoma, ovarian cancer, stomach cancer, bladder cancer, breast cancer, cervical cancer, colon cancer, rectal cancer or large intestinal cancer.

**62.** A diagnostic drug for cancer, prostatomegaly, male gonad dysfunction, infertility, premature birth, endometriosis, cirrhosis of the liver, hepatitis, hepatic insufficiency or calculus, containing a polynucleotide which contains a polynucleotide encoding a G protein-coupled receptor protein containing an amino acid sequence identical to or substantially identical to the amino acid sequence represented by SEQ ID NO: 9, or a partial peptide thereof.

**63.** The diagnostic drug according to Claim 62, wherein the cancer is prostate cancer, non-small cell carcinoma, ovarian cancer, stomach cancer, bladder cancer, breast cancer, cervical cancer, colon cancer, rectal cancer or large intestinal cancer.

**64.** A birth inducer or preventative and/or therapeutic agent for cancer, prostatomegaly, male gonad dysfunction, infertility, premature birth, endometriosis, cirrhosis of the liver, hepatitis, hepatic insufficiency or calculus, containing an antisense polynucleotide which contains a nucleotide sequence or part of a nucleotide sequence complementary to a polynucleotide containing a polynucleotide which encodes a G protein-coupled receptor protein containing an amino acid sequence identical to or substantially identical to the amino acid sequence represented by SEQ ID NO: 9, or a partial peptide thereof.

**65.** The agent according to Claim 64, wherein the cancer is prostate cancer, non-small cell carcinoma, ovarian cancer, stomach cancer, bladder cancer, breast cancer, cervical cancer, colon cancer, rectal cancer or large intestinal cancer.

**66.** A diagnostic drug for cancer, prostatomegaly, male gonad dysfunction, infertility, premature birth, endometriosis, cirrhosis of the liver, hepatitis, hepatic insufficiency or calculus, containing an antisense polynucleotide which contains a nucleotide sequence or part of a nucleotide sequence complementary to a polynucleotide containing a polynucleotide which encodes a G protein-coupled receptor protein containing an amino acid sequence identical to or substantially identical to the amino acid sequence of SEQ ID NO: 9, or a partial peptide thereof.

**67.** The diagnostic drug according to Claim 66, which is a diagnostic drug for cancer.

**68.** The diagnostic drug according to Claim 67, wherein the cancer is prostate cancer, non-small cell carcinoma, ovarian cancer, stomach cancer, bladder cancer, breast cancer, cervical cancer, colon cancer, rectal cancer or large intestinal cancer.

**69.** A method of preventing and/or treating cancer, prostatomegaly, male gonad dysfunction, infertility, premature birth, endometriosis, cirrhosis of the liver, hepatitis, hepatic insufficiency or calculus, or a method of inducing birth, **characterized by** administering, to mammals, an effective dose of a receptor protein containing an amino acid sequence identical to or substantially identical to the amino acid sequence represented by SEQ ID NO: 9, or a partial peptide or salt thereof.

**70.** A method of preventing and/or treating cancer, prostatomegaly, male gonad dysfunction, infertility, premature birth, endometriosis, cirrhosis of the liver, hepatitis, hepatic insufficiency or calculus, or a method of inducing birth, **characterized by** administering, to mammals, an effective dose of antibodies to a protein containing an amino acid sequence identical to or substantially identical to the amino acid sequence represented by SEQ ID NO:9, or to a partial peptide or salt thereof.

**71.** A method of preventing and/or treating cancer, prostatomegaly, male gonad dysfunction, infertility, premature birth, endometriosis, cirrhosis of the liver, hepatitis, hepatic insufficiency or calculus, or a method of inducing birth, **characterized by** administering, to mammals, an effective dose of a compound or salt thereof which alters the binding properties of a G protein-coupled receptor protein containing an amino acid sequence identical to or substantially identical to the amino acid sequence represented by SEQ ID NO: 9, or of a partial peptide or salt thereof, with a protein showing affinity for a sugar chains.

**72.** A method of preventing and/or treating cancer, prostatomegaly, male gonad dysfunction, infertility, premature birth, endometriosis, cirrhosis of the liver, hepatitis, hepatic insufficiency or calculus, or a method of inducing birth, **characterized by** administering, to mammals, an effective dose of a compound or salt thereof which alters the expressed amount of a G protein-coupled receptor protein containing an amino acid sequence identical to or substantially identical to the amino acid sequence represented by SEQ ID NO: 9.

**73.** A method of preventing and/or treating cancer, prostatomegaly, male gonad dysfunction, infertility, premature birth, endometriosis, cirrhosis of the liver, hepatitis, hepatic insufficiency or calculus, or a method of inducing birth, **char-**

**acterized by** administering, to mammals, an effective dose of a protein or salt thereof showing affinity for a sugar chain.

**74.** A method of preventing and/or treating cancer, prostatomegaly, male gonad dysfunction, infertility, premature birth, endometriosis, cirrhosis of the liver, hepatitis, hepatic insufficiency or calculus, or a method of inducing birth, **characterized by** administering, to mammals, an effective dose of an antisense polynucleotide containing a nucleotide sequence or part of a nucleotide sequence complementary to a polynucleotide encoding a protein or partial peptide of a protein containing an amino acid sequence identical to or substantially identical to the amino acid sequence represented SEQ ID NO: 9.

**75.** A method of preventing and/or treating cancer, prostatomegaly, male gonad dysfunction, infertility, premature birth, endometriosis, cirrhosis of the liver, hepatitis, hepatic insufficiency or calculus, or a method of inducing birth, **characterized by** administering, to mammals, an effective dose of a polynucleotide containing a polynucleotide which encodes a G protein-coupled receptor protein containing an amino acid sequence identical to or substantially identical to the amino acid sequence represented by SEQ ID NO: 9, or a partial peptide thereof.

**76.** A usage of a receptor protein containing an amino acid sequence identical to or substantially identical to the amino acid sequence represented by SEQ ID NO: 9, or a partial peptide or salt thereof, for manufacturing a birth inducer or a preventative and/or therapeutic agent for cancer, prostatomegaly, male gonad dysfunction, infertility, premature birth, endometriosis, cirrhosis of the liver, hepatitis, hepatic insufficiency or calculus.

**77.** A usage of antibodies to a receptor protein containing an amino acid sequence identical to or substantially identical to the amino acid sequence represented by SEQ ID NO: 9, or to a partial peptide or salt thereof, for manufacturing a birth inducer or a preventative and/or therapeutic agent for cancer, prostatomegaly, male gonad dysfunction, infertility, premature birth, endometriosis, cirrhosis of the liver, hepatitis, hepatic insufficiency or calculus.

**78.** A usage of a compound or salt thereof which alters the binding properties of a protein showing affinity for a sugar chain with a G protein-coupled receptor protein containing an amino acid sequence identical to or substantially identical to the amino acid sequence represented by SEQ ID NO: 9, or with a partial peptide or salt thereof, for manufacturing a birth inducer or a preventative and/or therapeutic agent for cancer, prostatomegaly, male gonad dysfunction, infertility, premature birth, endometriosis, cirrhosis of the liver, hepatitis, hepatic insufficiency or calculus.

**79.** A usage of a compound or salt thereof which alters the expressed amount of a G protein-coupled receptor protein containing an amino acid sequence identical to or substantially identical to the amino acid sequence represented by SEQ ID NO: 9, for manufacturing a birth inducer or a preventative and/or therapeutic agent for cancer, prostatomegaly, male gonad dysfunction, infertility, premature birth, endometriosis, cirrhosis of the liver, hepatitis, hepatic insufficiency or calculus.

**80.** A usage of a protein or salt thereof showing affinity for a sugar chain for manufacturing a birth inducer or a preventative and/or therapeutic agent for cancer, prostatomegaly, male gonad dysfunction, infertility, premature birth, endometriosis, cirrhosis of the liver, hepatitis, hepatic insufficiency or calculus.

**81.** A usage of an antisense polynucleotide containing a nucleotide sequence or part of a nucleotide sequence complementary to a polynucleotide which encodes a protein containing an amino acid sequence identical to or substantially identical to the amino acid sequence represented by SEQ ID NO: 9 or a partial peptide thereof, for manufacturing a birth inducer or a preventative and/or therapeutic agent for cancer, prostatomegaly, male gonad dysfunction, infertility, premature birth, endometriosis, cirrhosis of the liver, hepatitis, hepatic insufficiency or calculus.

**82.** A usage of a polynucleotide containing a polynucleotide encoding a G protein-coupled receptor protein containing an amino acid sequence identical to or substantially identical to the amino acid sequence represented by SEQ ID NO: 9 or a partial peptide thereof, for manufacturing a birth inducer or a preventative and/or therapeutic agent for cancer, prostatomegaly, male gonad dysfunction, infertility, premature birth, endometriosis, cirrhosis of the liver, hepatitis, hepatic insufficiency or calculus.

**83.** A G protein-coupled receptor protein containing an amino acid sequence identical to or substantially identical to the amino acid sequence represented by SEQ ID NO: 13, SEQ ID NO: 15, SEQ ID NO: 17, SEQ ID NO: 19, SEQ ID NO: 21, SEQ ID NO: 23, SEQ ID NO: 25 or SEQ ID NO: 27, or a salt thereof.

**84.** A G protein-coupled receptor protein consisting of an amino acid sequence represented by SEQ ID NO: 13, SEQ ID NO: 15, SEQ ID NO: 17, SEQ ID NO: 9, SEQ ID NO: 21, SEQ ID NO: 23, SEQ ID NO: 25 or SEQ ID NO: 27, or a salt thereof.

**85.** A polynucleotide containing the polynucleotide encoding the G protein-coupled receptor protein according to Claim 81.

**86.** DNA consisting of a nucleotide sequence represented by SEQ ID NO: 14, SEQ ID NO: 16, SEQ ID NO: 18, SEQ ID NO: 20, SEQ ID NO: 22, SEQ ID NO: 24, SEQ ID NO: 26 or SEQ ID NO: 28.

**87.** A recombinant vector containing the polynucleotide according to Claim 83.

**88.** A transformant transformed by the recombinant vector according to Claim 87.

**89.** A method of manufacturing the G protein-coupled receptor protein or salt thereof according to Claim 83, wherein the transformant according to Claim 88 is cultured in order to produce the G protein-coupled receptor protein or salt thereof according to Claim 83.

**90.** Antibodies to the G protein-coupled receptor protein or salt thereof according to Claim 83.

**91.** DNA which hybridizes under highly stringent conditions with DNA consisting of a nucleotide sequence represented by SEQ ID NO: 14, SEQ ID NO: 16, SEQ ID NO: 18, SEQ ID NO: 20, SEQ ID NO: 22, SEQ ID NO: 24, SEQ ID NO: 26 or SEQ ID NO: 28.

**92.** A polynucleotide containing a nucleotide sequence or part of a nucleotide sequence complementary to the polynucleotide according to Claim 85.

**93.** A method for screening EDG-1 receptor agonists or antagonists, **characterized by** the use of vascular endothelial cells.

**94.** The screening method according to Claim 93, wherein the EDG-1 receptor is a G protein-coupled receptor protein containing an amino acid sequence identical to or substantially identical to the amino acid sequence represented by SEQ ID NO: 38, or a partial peptide or salt thereof.

**95.** A screening kit for EDG-1 receptor agonists or antagonists, **characterized by** containing vascular endothelial cells.

**96.** An EDG-1 receptor agonist or antagonist obtained using the screening method according to Claim 93 or the screening kit according to Claim 95.

**97.** A preventative and/or therapeutic agent for arteriosclerosis, myocardial infarction; cerebral infarction or ischemic disease, containing an EDG-1 receptor agonist obtained using the screening method according to Claim 93 or the screening kit according to Claim 85.

**98.** A method of screening EDG-2 receptor agonists or antagonists, **characterized by** the use of vascular smooth muscle cells.

**99.** The screening method according to Claim 98 wherein the EDG-2 receptor is a G protein-coupled receptor protein containing an amino acid sequence identical to or substantially identical to the amino acid sequence represented by SEQ ID NO: 40, or a partial peptide or salt thereof.

**100.** A kit for screening EDG-2 receptor agonists or antagonists, **characterized by** containing vascular smooth muscle cells.

**101.** An EDG-2 receptor agonist or antagonist obtained using the screening method according to Claim 98 or the screening kit according to Claim 100.

**102.** A preventative and/or therapeutic agent for arteriosclerosis, myocardial infarction, cerebral infarction or ischemic disease, containing an EDG-2 receptor agonist or antagonist obtained using the screening method according to Claim 98 or the screening kit according to Claim 100.

**103.** A method of preventing and/or treating arteriosclerosis, myocardial infarction, cerebral infarction or ischemic disease, **characterized by** administering, to mammals, an effective dose f an EDG-1 receptor agonist obtained using the screening method according to Claim 93 or the screening kit according to Claim 95, or an EDG-2 receptor antagonist obtained using the screening method according to Claim 98 or the screening kit according to Claim 100.

**104.** A usage of an EDG-1 receptor agonist obtained using the screening method according to Claim 93 or the screening kit according to Claim 95 or an EDG-2 receptor antagonist obtained using the screening method according to Claim 98 or the screening kit according to Claim 100, for manufacturing a preventative and/or therapeutic agent for arteriosclerosis, myocardial infarction, cerebral infarction or ischemic disease.

Fig. 1

dJ287G14.2

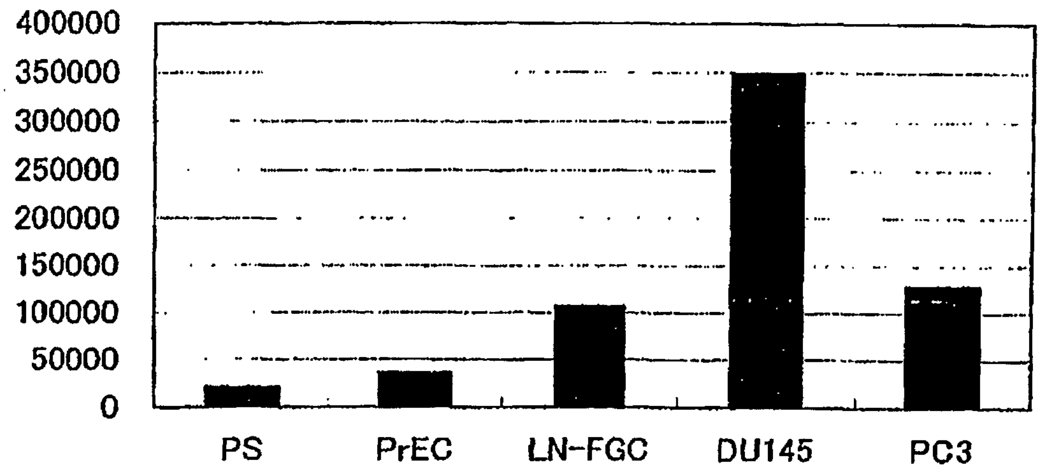

Fig. 2
dJ287G14.2

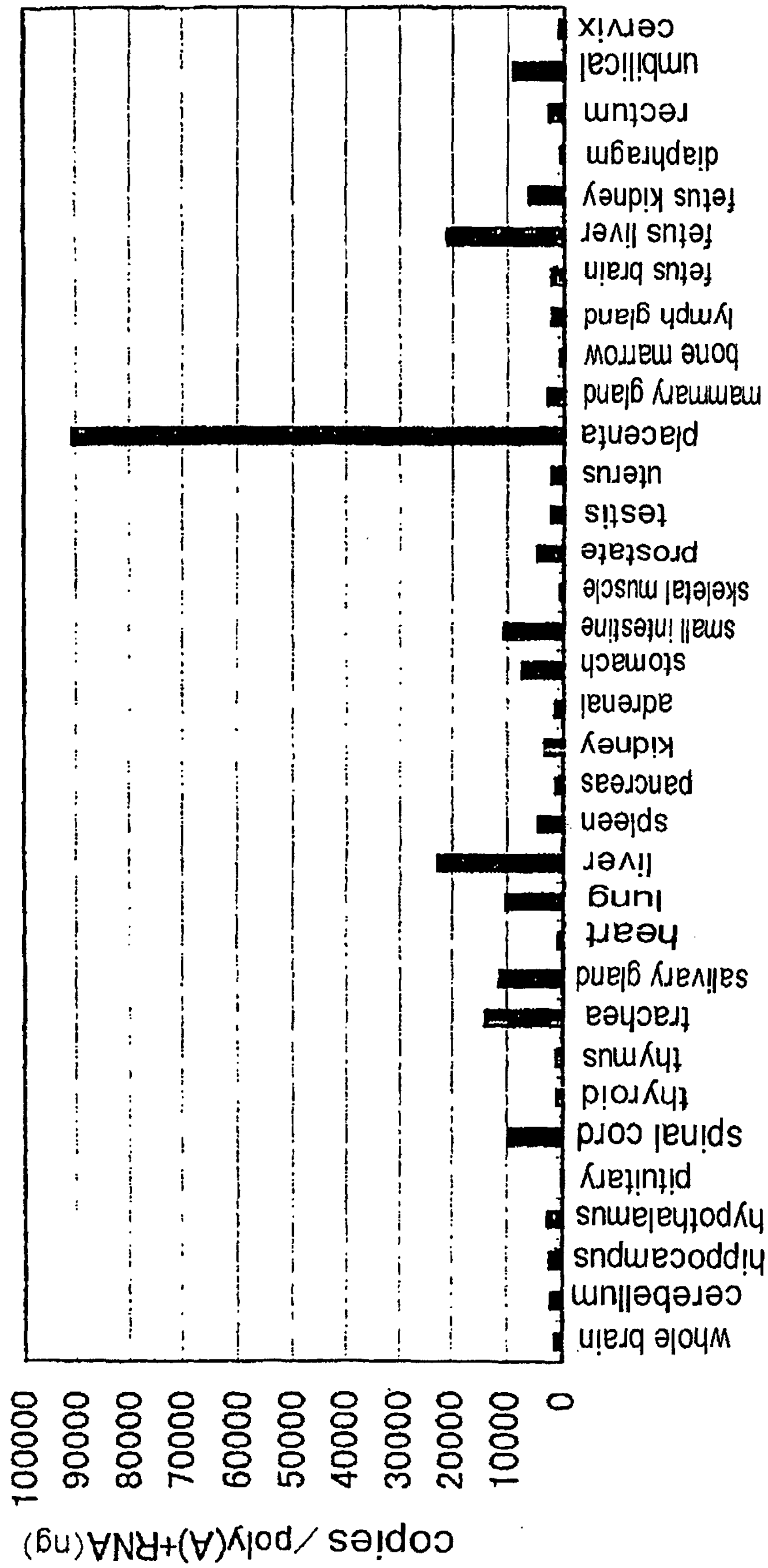

# Fig. 3

## DNA sequence

ATGATGTTTC GCTCAGATCG AATGTGGAGC TGCCATTGGA AATGGAAGCC CAGTCCTCTC 60

CTGTTCTTAT TTGCTTTATA TATCATGTGT GTTCCTCACT CAGTGTGGGG ATGTGCCAAC 120

TGCCGAGTGG TTTTGTCCAA CCCTTCTGGG ACCTTTACTT CTCCATGCTA CCCTAACGAC 180

TACCCAAACA GCCAGGCTTG CATGTGGACG CTCCGAGCCC CCACCGGTTA TATCATTCAG 240

ATAACATTTA ACGACTTTGA CATTGAAGAA GCTCCCAATT GCATTTATGA CTCATTATCC 300

CTTGATAATG GAGAGAGCCA GACTAAATTT TGTGGAGCAA CTGCCAAAGG CCTATCATTT 360

AACTCAAGTG CGAATGAGAT GCATGTGTCC TTTTCAAGTG ACTTTAGCAT CCAGAAGAAA 420

GGTTTCAATG CCAGCTACAT CAGAGTTGCC GTGTCCTTAA GGAATCAAAA GGTCATTTTA 480

CCCCAGACAT CAGATGCTTA CCAGGTATCT GTTGCAAAAA GCATCTCTAT TCCAGAGCTC 540

AGTGCTTTCA CACTCTGCTT TGAAGCAACC AAAGTTGGCC ATGAAGACAG TGATTGGACA 600

GCTTTCTCCT ACTCAAATGC ATCCTTCACA CAATTGCTCA GTTTTGGAAA GGCCAAGAGT 660

GGCTACTTTC TATCCATTTC TGATTCAAAA TGTTTGTTGA ATAATGCATT ACCTGTCAAA 720

GAAAAAGAAG ACATTTTTGC AGAAAGCTTT GAACAGCTCT GCCTTGTTTG GAATAATTCT 780

TTGGGCTCTA TTGGTGTAAA TTTCAAAAGA AACTATGAAA CAGTTCCATG TGATTCTACC 840

ATTAGTAAAG TTATTCCTGG GAATGGGAAA TTGTTGTTGG GCTCCAATCA AAATGAAATT 900

GTCTCTCTAA AAGGGGACAT TTATAACTTT CGACTTTGGA ATTTTACCAT GAATGCCAAA 960

ATCCTCTCCA ACCTCAGCTG TAATGTGAAA GGGAATGTAG TCGACTGGCA AAATGACTTC 1020

TGGAATATCC CAAACCTAGC TCTGAAAGCT GAAAGCAACC TAAGCTGTGG TTCCTACCTG 1080

ATCCCGCTCC CAGCAGCAGA ACTGGCCAGC TGTGCAGACC TGGGGACCCT CTGTCAAGCT 1140

ACTGTAAACT CTCCTAGTAC TACACCACCC ACTGTCACCA CTAACATGCC TGTTACTAAC 1200

AGAATCGATA AACAAAGGAA TGATGGAATT ATCTATAGAA TATCCGTAGT GATTCAGAAC 1260

ATCCTTCGTC ACCCTGAGGT AAAAGTACAG AGCAAGGTGG CAGAATGGCT CAATTCAACC 1320

TTCCAAAATT GGAACTACAC GGTTTATGTC GTTAATATCA GTTTTCACCT GAGTGCTGGA 1380

GAGGACAAGA TTAAAGTCAA GAGAAGCCTT GAGGATGAGC CAAGGTTGGT GCTTTGGGCC 1440

CTTCTAGTTT ACAATGCTAC CAACAATACT AATTTAGAAG GAAAAATCAT TCAGCAGAAG 1500

CTCCTAAAAA ATAATGAGTC CTTGGATGAA GGCTTGAGGC TACATACAGT GAATGTGAGA 1560

CAACTGGGTC ATTGTCTTGC CATGGAGGAA CCCAAAGGCT ACTACTGGCC ATCTATCCAA 1620

CCTTCTGAAT ACGTTCTTCC TTGTCCAGAC AAGCCTGGCT TTTCTGCTTC TCGGATATGT 1680

TTTTACAATG CTACCAACCC ATTGGTAACC TACTGGGGAC CTGTTGATAT CTCCAACTGT 1740

TTAAAAGAAG CAAATGAAGT TGCTAACCAG ATTTTAAATT TAACTGCTGA TGGGCAGAAC 1800

# Fig. 4

TTAACCTCAG CCAATATTAC CAACATTGTG GAACAGGTCA AAAGAATTGT GAATAAAGAA 1860
GAAAACATTG ATATAACACT TGGCTCAACT CTAATGAATA TATTTTCTAA TATCTTAAGC 1920
AGTTCAGACA GTGACTTGCT TGAGTCATCT TCTGAAGCTT TAAAAACAAT TGATGAATTG 1980
GCCTTCAAGA TAGACCTAAA TAGCACATCA CATGTGAATA TTACAACTCG GAACTTGGCT 2040
CTCAGCGTAT CATCCCTGTT ACCAGGGACA AATGCAATTT CAAATTTTAG CATTGGTCTT 2100
CCAAGCAATA ATGAATCGTA TTTCCAGATG GATTTTGAGA GTGGACAAGT GGATCCACTG 2160
GCATCTGTAA TTTTGCCTCC AAACTTACTT GAGAATTTAA GTCCAGAAGA TTCTGTATTA 2220
GTTAGAAGAG CACAGTTTAC TTTCTTCAAC AAAACTGGAC TTTTCCAGGA TGTAGGACCC 2280
CAAAGAAAAA CTTTAGTGAG TTATGTGATG GCGTGCAGTA TTGGAAACAT TACTATCCAG 2340
AATCTGAAGG ATCCTGTTCA AATAAAAATC AAACATACAA GAACTCAGGA AGTGCATCAT 2400
CCCATCTGTG CCTTCTGGGA TCTGAACAAA AACAAAAGTT TTGGAGGATG GAACACGTCA 2460
GGATGTGTTG CACACAGAGA TTCAGATGCA AGTGAGACAG TCTGCCTGTG TAACCACTTC 2520
ACACACTTTG GAGTTCTGAT GGACCTTCCA AGAAGTGCCT CACAGTTAGA TGCAAGAAAC 2580
ACTAAAGTCC TCACTTTCAT CAGCTATATT GGGTGTGGAA TATCTGCTAT TTTTTCAGCA 2640
GCAACTCTCC TGACATATGT TGCTTTTGAG AAATTGCGAA GGGATTATCC CTCCAAAATC 2700
TTGATGAACC TGAGCACAGC CCTGCTGTTC CTGAATCTCC TCTTCCTCCT AGATGGCTGG 2760
ATCACCTCCT TCAATGTGGA TGGACTTTGC ATTGCTGTTG CAGTCCTGTT GCATTTCTTC 2820
CTTCTGGCAA CCTTTACCTG GATGGGGCTA GAAGCAATTC ACATGTACAT TGCTCTAGTT 2880
AAAGTATTTA ACACTTACAT TCGCCGATAC ATTCTAAAAT TCTGCATCAT TGGCTGGGGT 2940
TTGCCTGCCT TAGTGGTGTC AGTTGTTCTA GCGAGCAGAA ACAACAATGA AGTCTATGGA 3000
AAAGAAAGTT ATGGGAAAGA AAAAGGTGAT GAATTCTGTT GGATTCAAGA TCCAGTCATA 3060
TTTTATGTGA CCTGTGCTGG GTATTTTGGA GTCATGTTTT TTCTGAACAT TGCCATGTTC 3120
ATTGTGGTAA TGGTGCAGAT CTGTGGGAGG AATGGCAAGA GAAGCAACCG GACCCTGAGA 3180
GAAGAAGTGT TAAGGAACCT GCGCAGTGTG GTTAGCTTGA CCTTTCTGTT GGGCATGACA 3240
TGGGGTTTTG CATTCTTTGC CTGGGGACCC TTAAATATCC CCTTCATGTA CCTCTTCTCC 3300
ATCTTCAATT CATTACAAGG CTTATTTATA TTCATCTTCC ACTGTGCTAT GAAGGAGAAT 3360
GTTCAGAAAC AGTGGCGGCG GCATCTCTGC TGTGGTAGAT TTCGGTTAGC AGATAACTCA 3420
GATTGGAGTA AGACAGCTAC CAATATCATC AAGAAAAGTT CTGATAATCT AGGAAAATCT 3480
TTGTCTTCAA GCTCCATTGG TTCCAACTCA ACCTATCTTA CATCCAAATC TAAATCCAGC 3540
TCTACCACCT ATTTCAAAAG GAATAGCCAC ACAGATAATG TCTCCTATGA GCATTCCTTC 3600
AACAAAAGTG GATCACTCAG ACAGTGCTTC CATGGACAAG TCCTTGTCAA AACTGGCCCA 3660
TGCTGA 3666

# Fig. 5

## Amino acid sequence

Met Met Phe Arg Ser Asp Arg Met Trp Ser Cys His Trp Lys Trp Lys
5 10 15
Pro Ser Pro Leu Leu Phe Leu Phe Ala Leu Tyr Ile Met Cys Val Pro
20 25 30
His Ser Val Trp Gly Cys Ala Asn Cys Arg Val Val Leu Ser Asn Pro
35 40 45
Ser Gly Thr Phe Thr Ser Pro Cys Tyr Pro Asn Asp Tyr Pro Asn Ser
50 55 60
Gln Ala Cys Met Trp Thr Leu Arg Ala Pro Thr Gly Tyr Ile Ile Gln
65 70 75 80
Ile Thr Phe Asn Asp Phe Asp Ile Glu Glu Ala Pro Asn Cys Ile Tyr
85 90 95
Asp Ser Leu Ser Leu Asp Asn Gly Glu Ser Gln Thr Lys Phe Cys Gly
100 105 110
Ala Thr Ala Lys Gly Leu Ser Phe Asn Ser Ser Ala Asn Glu Met His
115 120 125
Val Ser Phe Ser Ser Asp Phe Ser Ile Gln Lys Lys Gly Phe Asn Ala
130 135 140
Ser Tyr Ile Arg Val Ala Val Ser Leu Arg Asn Gln Lys Val Ile Leu
145 150 155 160
Pro Gln Thr Ser Asp Ala Tyr Gln Val Ser Val Ala Lys Ser Ile Ser
165 170 175
Ile Pro Glu Leu Ser Ala Phe Thr Leu Cys Phe Glu Ala Thr Lys Val
180 185 190
Gly His Glu Asp Ser Asp Trp Thr Ala Phe Ser Tyr Ser Asn Ala Ser
195 200 205
Phe Thr Gln Leu Leu Ser Phe Gly Lys Ala Lys Ser Gly Tyr Phe Leu
210 215 220
Ser Ile Ser Asp Ser Lys Cys Leu Leu Asn Asn Ala Leu Pro Val Lys
225 230 235 240
Glu Lys Glu Asp Ile Phe Ala Glu Ser Phe Glu Gln Leu Cys Leu Val
245 250 255
Trp Asn Asn Ser Leu Gly Ser Ile Gly Val Asn Phe Lys Arg Asn Tyr
260 265 270
Glu Thr Val Pro Cys Asp Ser Thr Ile Ser Lys Val Ile Pro Gly Asn
275 280 285
Gly Lys Leu Leu Leu Gly Ser Asn Gln Asn Glu Ile Val Ser Leu Lys
290 295 300
Gly Asp Ile Tyr Asn Phe Arg Leu Trp Asn Phe Thr Met Asn Ala Lys

# Fig. 6

305 310 315 320
Ile Leu Ser Asn Leu Ser Cys Asn Val Lys Gly Asn Val Val Asp Trp
325 330 335
Gln Asn Asp Phe Trp Asn Ile Pro Asn Leu Ala Leu Lys Ala Glu Ser
340 345 350
Asn Leu Ser Cys Gly Ser Tyr Leu Ile Pro Leu Pro Ala Ala Glu Leu
355 360 365
Ala Ser Cys Ala Asp Leu Gly Thr Leu Cys Gln Ala Thr Val Asn Ser
370 375 380
Pro Ser Thr Thr Pro Pro Thr Val Thr Thr Asn Met Pro Val Thr Asn
385 390 395 400
Arg Ile Asp Lys Gln Arg Asn Asp Gly Ile Ile Tyr Arg Ile Ser Val
405 410 415
Val Ile Gln Asn Ile Leu Arg His Pro Glu Val Lys Val Gln Ser Lys
420 425 430
Val Ala Glu Trp Leu Asn Ser Thr Phe Gln Asn Trp Asn Tyr Thr Val
435 440 445
Tyr Val Val Asn Ile Ser Phe His Leu Ser Ala Gly Glu Asp Lys Ile
450 455 460
Lys Val Lys Arg Ser Leu Glu Asp Glu Pro Arg Leu Val Leu Trp Ala
465 470 475 480
Leu Leu Val Tyr Asn Ala Thr Asn Asn Thr Asn Leu Glu Gly Lys Ile
485 490 495
Ile Gln Gln Lys Leu Leu Lys Asn Asn Glu Ser Leu Asp Glu Gly Leu
500 505 510
Arg Leu His Thr Val Asn Val Arg Gln Leu Gly His Cys Leu Ala Met
515 520 525
Glu Glu Pro Lys Gly Tyr Tyr Trp Pro Ser Ile Gln Pro Ser Glu Tyr
530 535 540
Val Leu Pro Cys Pro Asp Lys Pro Gly Phe Ser Ala Ser Arg Ile Cys
545 550 555 560
Phe Tyr Asn Ala Thr Asn Pro Leu Val Thr Tyr Trp Gly Pro Val Asp
565 570 575
Ile Ser Asn Cys Leu Lys Glu Ala Asn Glu Val Ala Asn Gln Ile Leu
580 585 590
Asn Leu Thr Ala Asp Gly Gln Asn Leu Thr Ser Ala Asn Ile Thr Asn
595 600 605
Ile Val Glu Gln Val Lys Arg Ile Val Asn Lys Glu Glu Asn Ile Asp
610 615 620
Ile Thr Leu Gly Ser Thr Leu Met Asn Ile Phe Ser Asn Ile Leu Ser
625 630 635 640

# Fig. 7

Ser Ser Asp Ser Asp Leu Leu Glu Ser Ser Ser Glu Ala Leu Lys Thr
645 650 655

Ile Asp Glu Leu Ala Phe Lys Ile Asp Leu Asn Ser Thr Ser His Val
660 665 670

Asn Ile Thr Thr Arg Asn Leu Ala Leu Ser Val Ser Ser Leu Leu Pro
675 680 685

Gly Thr Asn Ala Ile Ser Asn Phe Ser Ile Gly Leu Pro Ser Asn Asn
690 695 700

Glu Ser Tyr Phe Gln Met Asp Phe Glu Ser Gly Gln Val Asp Pro Leu
705 710 715 720

Ala Ser Val Ile Leu Pro Pro Asn Leu Leu Glu Asn Leu Ser Pro Glu
725 730 735

Asp Ser Val Leu Val Arg Arg Ala Gln Phe Thr Phe Phe Asn Lys Thr
740 745 750

Gly Leu Phe Gln Asp Val Gly Pro Gln Arg Lys Thr Leu Val Ser Tyr
755 760 765

Val Met Ala Cys Ser Ile Gly Asn Ile Thr Ile Gln Asn Leu Lys Asp
770 775 780

Pro Val Gln Ile Lys Ile Lys His Thr Arg Thr Gln Glu Val His His
785 790 795 800

Pro Ile Cys Ala Phe Trp Asp Leu Asn Lys Asn Lys Ser Phe Gly Gly
805 810 815

Trp Asn Thr Ser Gly Cys Val Ala His Arg Asp Ser Asp Ala Ser Glu
820 825 830

Thr Val Cys Leu Cys Asn His Phe Thr His Phe Gly Val Leu Met Asp
835 840 845

Leu Pro Arg Ser Ala Ser Gln Leu Asp Ala Arg Asn Thr Lys Val Leu
850 855 860

Thr Phe Ile Ser Tyr Ile Gly Cys Gly Ile Ser Ala Ile Phe Ser Ala
865 870 875 880

Ala Thr Leu Leu Thr Tyr Val Ala Phe Glu Lys Leu Arg Arg Asp Tyr
885 890 895

Pro Ser Lys Ile Leu Met Asn Leu Ser Thr Ala Leu Leu Phe Leu Asn
900 905 910

Leu Leu Phe Leu Leu Asp Gly Trp Ile Thr Ser Phe Asn Val Asp Gly
915 920 925

Leu Cys Ile Ala Val Ala Val Leu Leu His Phe Phe Leu Leu Ala Thr
930 935 940

Phe Thr Trp Met Gly Leu Glu Ala Ile His Met Tyr Ile Ala Leu Val
945 950 955 960

# Fig. 8

Lys Val Phe Asn Thr Tyr Ile Arg Arg Tyr Ile Leu Lys Phe Cys Ile
965 970 975

Ile Gly Trp Gly Leu Pro Ala Leu Val Val Ser Val Val Leu Ala Ser
980 985 990

Arg Asn Asn Asn Glu Val Tyr Gly Lys Glu Ser Tyr Gly Lys Glu Lys
995 1000 1005

Gly Asp Glu Phe Cys Trp Ile Gln Asp Pro Val Ile Phe Tyr Val Thr
1010 1015 1020

Cys Ala Gly Tyr Phe Gly Val Met Phe Phe Leu Asn Ile Ala Met Phe
1025 1030 1035 1040

Ile Val Val Met Val Gln Ile Cys Gly Arg Asn Gly Lys Arg Ser Asn
1045 1050 1055

Arg Thr Leu Arg Glu Glu Val Leu Arg Asn Leu Arg Ser Val Val Ser
1060 1065 1070

Leu Thr Phe Leu Leu Gly Met Thr Trp Gly Phe Ala Phe Phe Ala Trp
1075 1080 1085

Gly Pro Leu Asn Ile Pro Phe Met Tyr Leu Phe Ser Ile Phe Asn Ser
1090 1095 1100

Leu Gln Gly Leu Phe Ile Phe Ile Phe His Cys Ala Met Lys Glu Asn
1105 1110 1115 1120

Val Gln Lys Gln Trp Arg Arg His Leu Cys Cys Gly Arg Phe Arg Leu
1125 1130 1135

Ala Asp Asn Ser Asp Trp Ser Lys Thr Ala Thr Asn Ile Ile Lys Lys
1140 1145 1150

Ser Ser Asp Asn Leu Gly Lys Ser Leu Ser Ser Ser Ser Ile Gly Ser
1155 1160 1165

Asn Ser Thr Tyr Leu Thr Ser Lys Ser Lys Ser Ser Ser Thr Thr Tyr
1170 1175 1180

Phe Lys Arg Asn Ser His Thr Asp Asn Val Ser Tyr Glu His Ser Phe
1185 1190 1195 1200

Asn Lys Ser Gly Ser Leu Arg Gln Cys Phe His Gly Gln Val Leu Val
1205 1210 1215

Lys Thr Gly Pro Cys End
1220

Fig. 9

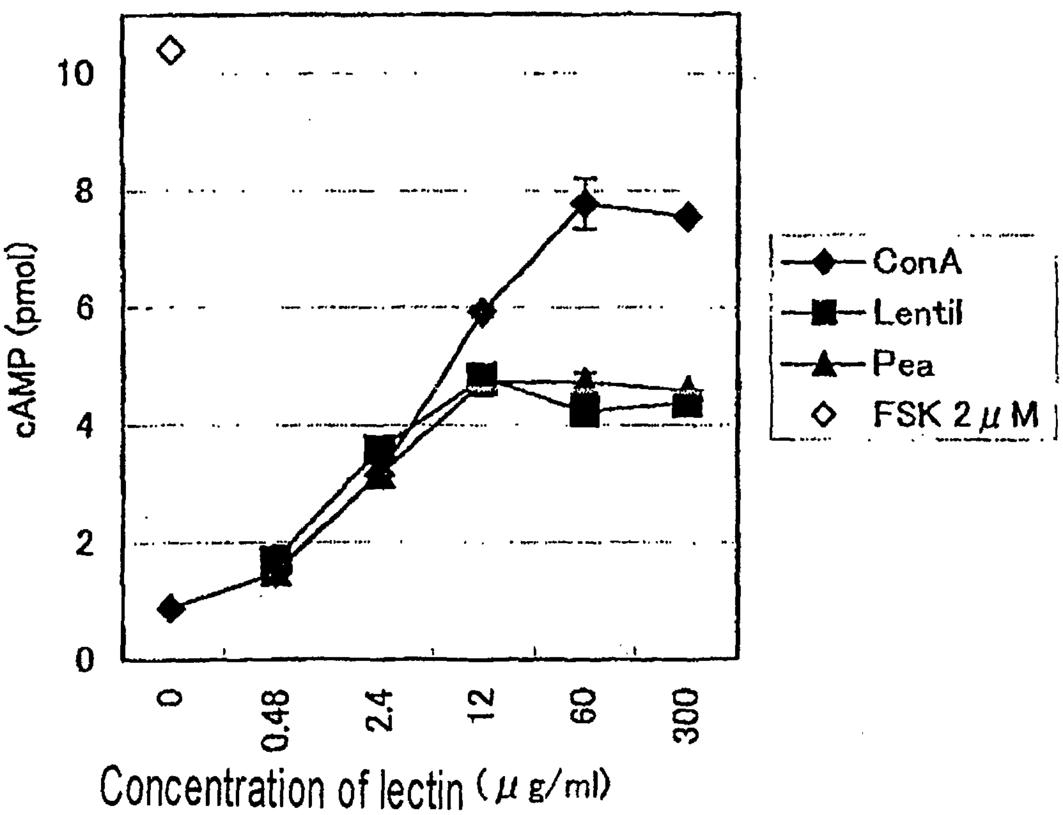
10
8
6
4
2
0
cAMP (pmol)
0
0.48
2.4
12
60
300
Concentration of lectin (μg/ml)
ConA
Lentil
Pea
FSK 2μM

Fig. 10

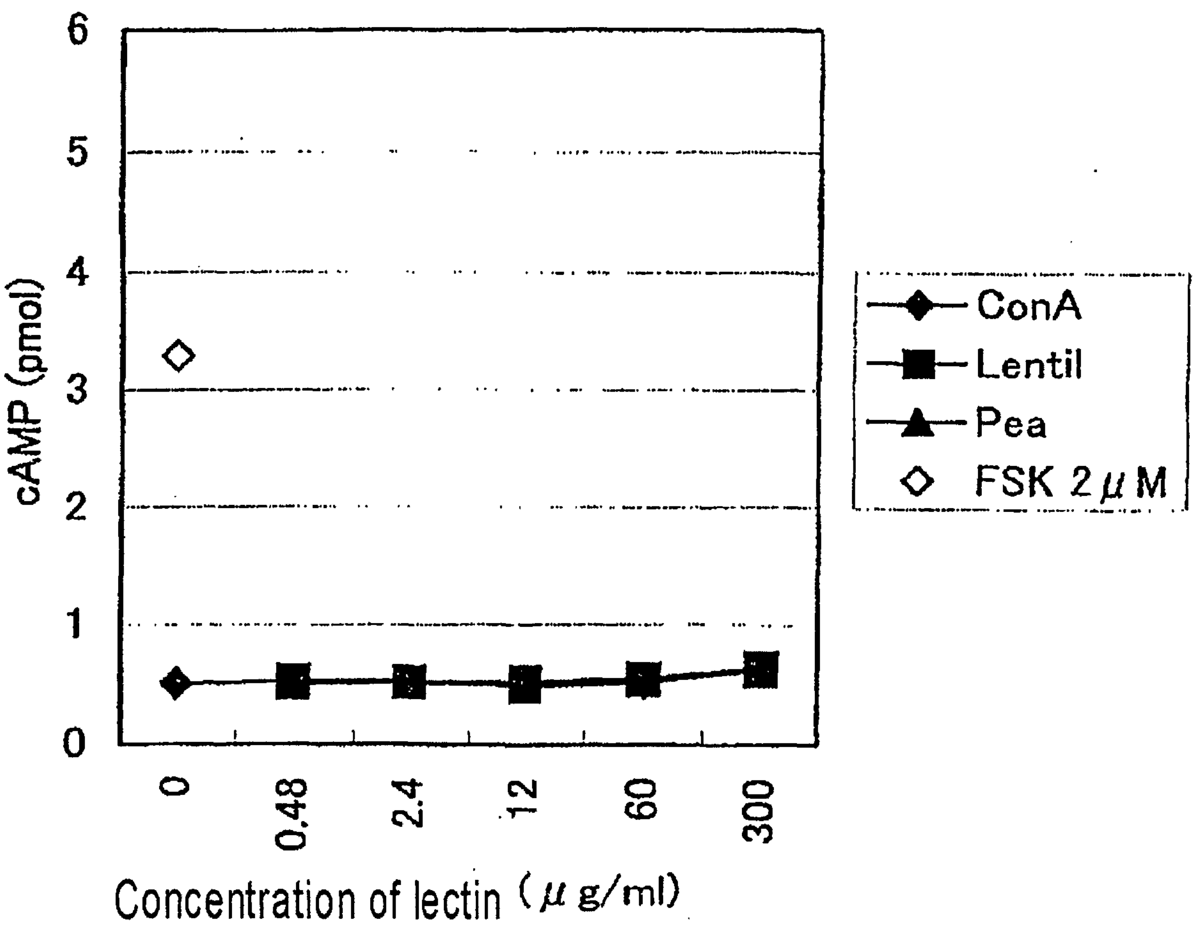
6
5
4
3
2
1
0
cAMP (pmol)
0
0.48
2.4
12
60
300
Concentration of lectin (μg/ml)
ConA
Lentil
Pea
FSK 2μM

Fig. 11

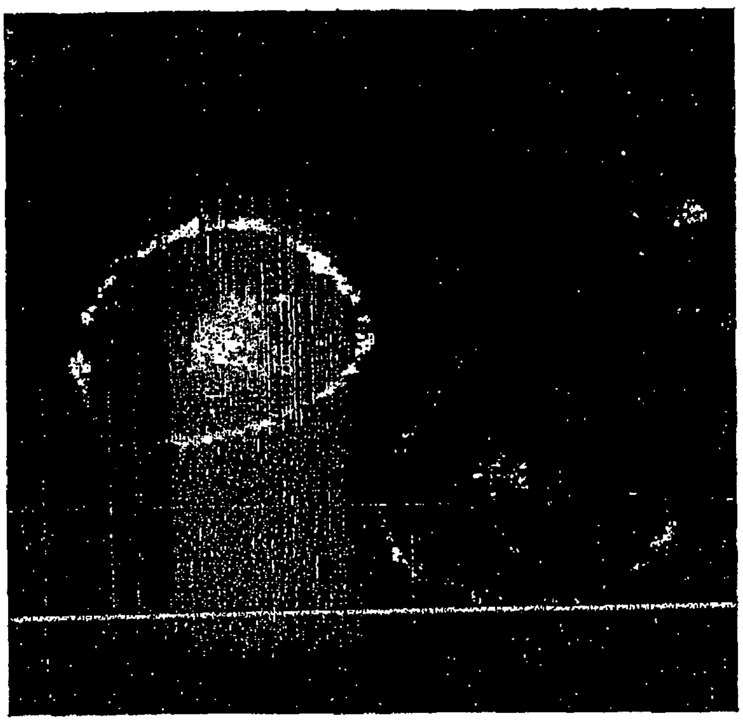

Fig. 12

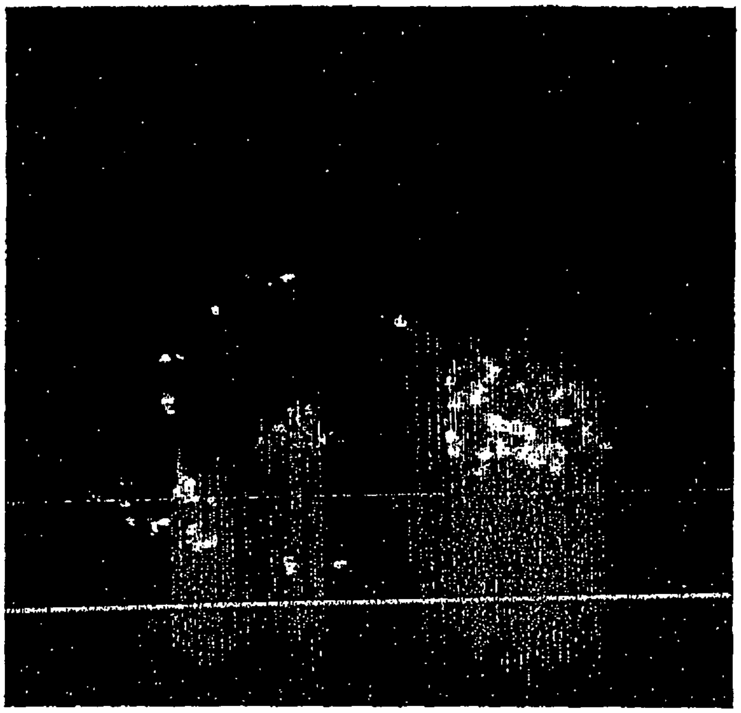

# INTERNATIONAL SEARCH REPORT

International application No.
PCT/JP02/13097

A. CLASSIFICATION OF SUBJECT MATTER
Int.Cl[7] C12N15/00, C12Q1/68, A61K38/17, A61P35/00, C07K14/47, C12P21/02, C07K16/18, G01N33/566, G01N33/50, G01N33/15

According to International Patent Classification (IPC) or to both national classification and IPC

B. FIELDS SEARCHED

Minimum documentation searched (classification system followed by classification symbols)
Int.Cl[7] C12N15/00, C12Q1/68, A61K38/17, A61P35/00, C07K14/47, C12P21/02, C07K16/18, G01N33/566, G01N33/50, G01N33/15

Documentation searched other than minimum documentation to the extent that such documents are included in the fields searched

Electronic data base consulted during the international search (name of data base and, where practicable, search terms used)
JICST file(JOIS), BIOSIS/WPI(DIALOG)

C. DOCUMENTS CONSIDERED TO BE RELEVANT

| Category* | Citation of document, with indication, where appropriate, of the relevant passages | Relevant to claim No. |
|---|---|---|
| X | Takayuki MATSUMOTO, "IBD no Byohen Keisei ni okeru Cytokine mRNA Teiryo no Igi", Nanjisei Enshosei Chokan Shogai ni Kansuru Chosa Kenkyuhan Buntan Kenkyu Hokokusho, 2000, pages 73 to 74 | 1-8,10-21 |
| X | Manami NAGANO et al., "TaqMan Chemistry ni yoru Teiryoteki PCR to SNP no Kaiseki", Cell Technology, 2000, separate volume, pages 113 to 123 | 1-8,10-21 |
| A | EP 1108789 A2 (HOFFMANN-LA ROCHE AG.), 20 June, 2001 (20.06.01), & JP 2001-204483 A | 1-8,10-21 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 March, 2003 (14.03.03) | 01 April, 2003 (01.04.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/13097

C (Continuation). DOCUMENTS CONSIDERED TO BE RELEVANT

| Category* | Citation of document, with indication, where appropriate, of the relevant passages | Relevant to claim No. |
|---|---|---|
| A | Tao WANG et al., "Influence of $\beta_1$ -adrenoceptor blockade on the gene expression of adenylate cyclase subtypes and β-adrenoceptor kinase in human atrium", Clinical Science, 2001 September, Vol.101, pages 211 to 217 | 1-8,10-21 |
| A | BIOSIS No.: 200200151652<br>& Blood, 2001 November, 98(11part2), p.78b | 1-8,10-21 |
| A | BIOSIS No.: 200100311314<br>& Blood, 2001 November, 96(11part2), p.39b | 1-8,10-21 |

Form PCT/ISA/210 (continuation of second sheet) (July 1998)

## INTERNATIONAL SEARCH REPORT

International application No.
PCT/JP02/13097

**Box I Observations where certain claims were found unsearchable (Continuation of item 2 of first sheet)**

This international search report has not been established in respect of certain claims under Article 17(2)(a) for the following reasons:

1. [X] Claims Nos.: see below
because they relate to subject matter not required to be searched by this Authority, namely:
Claims 30, 31, 34, 35, 69 to 75 and 103 involve therapeutic methods and diagnostic methods to be practiced on the human body.

2. [X] Claims Nos.: see extra sheet
because they relate to parts of the international application that do not comply with the prescribed requirements to such an extent that no meaningful international search can be carried out, specifically:
Although claim 9 relates to a drug containing a gene or the like specified by a method, it is completely unknown what specific substances are involved in the scope of the gene and what are not. Thus, this claim is described in an extremely unclear manner. For the same reason, (continued to extra sheet)

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:
(See extra sheet)

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [X] No required additional search fees were timely paid by the applicant. Consequently, this international search report is restricted to the invention first mentioned in the claims; it is covered by claims Nos.: 1-8, 10-21

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP02/13097

Continuation of Box No.I-2 of continuation of first sheet(1)

claims 32, 33, 48 to 51, 54 to 56, 96, 97, 101, 102 and 104 are extremely unclear. Concerning claims 78 and 79, the "compound showing a change in the binding properties (expression dose)" is unknown in particular and, therefore, extremely unclear.

The claims described in Box No.I-2 are claims 9, 32 to 33, 48 to 51, 54 to 56, 78, 79, 96, 97, 101, 102 and 104.

Continuation of Box No.II of continuation of first sheet(1)

Claim 1 relates to "a method of analyzing gene expression characterized by comprising qualitatively analyzing the individual expression doses of a plural number of genes and thus identifying a gene showing characteristic promotion or inhibition in its expression in specific cells or tissue" wherein the kit of a pair of primers as set forth in claim 22 or the mRNA quantification kit as set forth in claim 27 is not always employed. Thus, there is no relationship between these groups of inventions involving one or more of the same or corresponding special technical features.

Also, there is no technical relationship involving any special technical features among claim 1 and the claims relating to SEQ ID NO:9 or SEQ ID NOS:13 to 28.

Furthermore, there is no technical relationship involving any special technical features among claim 1 and the "use of a protein showing an affinity for a sugar chain" as set forth in claim 80, the "method of screening an agonist or an antagonist of EDG-1 receptor" as set forth in claim 93 and the "method of screening an agonist or an antagonist of EDG-2 receptor" as set forth in claim 98.

Such being the case, these groups of inventions are not considered as relating to a group of inventions so linked as to form a single general inventive concept. Thus the claims are classified into the following 8 groups of inventions.

- Claims 1 to 8 and 10 to 21.
- Claims 22 to 26.
- Claims 27 to 29.
- Claims 36 to 47, 52, 53, 57 to 68, 76, 77, 81, 82, 85 and 92.
- Claim 80.
- Claims 83, 84 and 86 to 91.
- Claims 93 to 95.
- Claims 98 to 100.

Form PCT/ISA/210 (extra sheet) (July 1998)

[Sequence Listing]

<110> Takeda Chemical Industries, Ltd.

<120> Method of Analyzing Gene Expression

<130> P02-0155PCT

<150> JP 2001-382053
<151> 2001-12-14

<150> JP 2002-45104
<151> 2002-02-21

<150> JP 2002-140111
<151> 2002-05-15

<150> JP 2002-333769
<151> 2002-11-12

<160> 77

<210> 1
<211> 1973
<212> DNA
<213> Homo sapiens

<400> 1
```
ctgccggctc actcggctgc tgcgtctggt ctggcgtctg ctgagaagat cctcttctac    60
cctgctctgc acctgtgctc gactgccagc cggctgaggg cgggggtctc cacggtggtc   120
ccagctccca aggaggttgc agaagtaccg tacagagtgg atttgcaggg cagtggcatg   180
gagcccctct tccccgcgcc gttctgggag gttatctacg gcagccacct tcagggcaac   240
ctgtccctcc tgagccccaa ccacagtctg ctgcccccgc atctgctgct caatgccagc   300
cacggcgcct tcctgcccct cgggctcaag gtcaccatcg tggggctcta cctggccgtg   360
tgtgtcggag ggctcctggg gaactgcctt gtcatgtacg tcatcctcag gcacaccaaa   420
atgaagacag ccaccaatat ttacatcttt aacctggccc tggccgacac tctggtcctg   480
ctgacgctgc ccttccaggg cacggacatc ctcctgggct tctggccgtt tgggaatgcg   540
ctgtgcaaga cagtcattgc cattgactac tacaacatgt tcaccagcac cttcacccta   600
actgccatga gtgtggatcg ctatgtagcc atctgccacc ccatccgtgc cctcgacgtc   660
cgcacgtcca gcaaagccca ggctgtcaat gtggccatct gggccctggc ctctgttgtc   720
ggtgttcccg ttgccatcat gggctcggca caggtcgagg atgaagagat cgagtgcctg   780
gtggagatcc ctacccctca ggattactgg ggcccggtgt ttgccatctg catcttcctc   840
ttctccttca tcgtccccgt gctcgtcatc tctgtctgct acagcctcat gatccggcgg   900
ctccgtggag tccgcctgct ctcgggctcc cgagagaagg accggaacct gcggcgcatc   960
actcggctgg tgctggtggt agtggctgtg ttcgtgggct gctggacgcc tgtccaggtc  1020
ttcgtgctgg cccaagggct gggggttcag ccgagcagcg agactgccgt ggccattctg  1080
cgcttctgca cggccctggg ctacgtcaac agctgcctca accccatcct ctacgccttc  1140
ctggatgaga acttcaaggc ctgcttccgc aagttctgct gtgcatctgc cctgcgccgg  1200
gacgtgcagg tgtctgaccg cgtgcgcagc attgccaagg acgtggccct ggcctgcaag  1260
acctctgaga cggtaccgcg gcccgcatga ctaggcgtgg acctgcccat ggtgcctgtc  1320
agcccgcaga gcccatctac gcccaacaca gagctcacac aggtcactgc tctctaggcg  1380
gacacaccct gggccctgag catccagagc ctgggatggg cttttccctg tgggccaggg  1440
atgctcggtc ccagaggagg acctagtgac atcatgggac aggtcaaagc attagggcca  1500
cctccatggc cccagacaga ctaaagctgc cctcctggtg cagggccgag ggacacaag  1560
gacctacctg gaagcagctg acatgctggt ggacggccgt tactggagcc cgtgcccctc  1620
cctccccgtg cttcatgtga ctcttggcct ctctgctgct gcgttggcag aaccctgggt  1680
gggcaggcac ccggaggagg agcagcagct gtgtcatcct gtgcccccca tgtgctgtgt  1740
gctgtttgca tggcagggct ccagctgcct tcagccctgt gacgtctcct cagggcagct  1800
ggacaggctt ggcacggccc gggaagtgca gcaggcagct tttctttggg gtgggacttg  1860
ccctgagctt ggagctgcca cctggaggac ttgcctgttc cgactccacc tgtgcagccg  1920
gggccacccc aggagaaagt gtccaggtgg gggctggcag tccctggctg cag          1973
```

<210> 2
<211> 23
<212> DNA
<213> Artificial Sequence

<220>

<223> Primer M-572F

<400> 2
cacaggtcga ggatgaagag atc 23

<210> 3
<211> 23
<212> DNA
<213> Artificial Sequence

<220>
<223> Primer M-714R

<400> 3
catgaggctg tagcagacag aga 23

<210> 4
<211> 26
<212> DNA
<213> Artificial Sequence

<220>
<221> misc_binding
<223> Probe M-658T, labeled 5'-terminal with FAM and 3'-terminal with TAMR/

<400> 4
ttcctcttct ccttcatcgt ccccgt 26

<210> 5
<211> 24
<212> DNA
<213> Artificial Sequence

<220>
<223> Forward Primer

<400> 5
ccctgagaga agaagtgtta agga 24

<210> 6
<211> 18
<212> DNA
<213> Artificial Sequence

<220>
<223> Reverse Primer

<400> 6
gcaaaacccc atgtcatg 18

<210> 7
<211> 28
<212> DNA
<213> Artificial Sequence

<220>
<223> Probe

<400> 7
cgcagtgtgg ttagcttgac ctttctgt 28

<210> 8
<211> 3663
<212> DNA
<213> Homo sapiens

<400> 8
atgatgtttc gctcagatcg aatgtggagc tgccattgga aatggaagcc cagtcctctc 60

```
ctgttcttat ttgctttata tatcatgtgt gttcctcact cagtgtgggg atgtgccaac   120
tgccgagtgg ttttgtccaa cccttctggg acctttactt ctccatgcta ccctaacgac   180
tacccaaaca gccaggcttg catgtggacg ctccgagccc ccaccggtta tatcattcag   240
ataacattta acgactttga cattgaagaa gctcccaatt gcatttatga ctcattatcc   300
cttgataatg gagagagcca gactaaattt tgtggagcaa ctgccaaagg cctatcattt   360
aactcaagtg cgaatgagat gcatgtgtcc ttttcaagtg actttagcat ccagaagaaa   420
ggtttcaatg ccagctacat cagagttgcc gtgtccttaa ggaatcaaaa ggtcatttta   480
ccccagacat cagatgctta ccaggtatct gttgcaaaaa gcatctctat tccagagctc   540
agtgctttca cactctgctt tgaagcaacc aaagttggcc atgaagacag tgattggaca   600
gctttctcct actcaaatgc atccttcaca caattgctca gttttggaaa ggccaagagt   660
ggctactttc tatccatttc tgattcaaaa tgtttgttga ataatgcatt acctgtcaaa   720
gaaaaagaag acatttttgc agaaagcttt gaacagctct gccttgtttg gaataattct   780
ttgggctcta ttggtgtaaa tttcaaaaga aactatgaaa cagttccatg tgattctacc   840
attagtaaag ttattcctgg gaatgggaaa ttgttgttgg gctccaatca aaatgaaatt   900
gtctctctaa aaggggacat ttataacttt cgactttgga attttaccat gaatgccaaa   960
atcctctcca acctcagctg taatgtgaaa gggaatgtag tcgactggca aaatgacttc  1020
tggaatatcc caaacctagc tctgaaagct gaaagcaacc taagctgtgg ttcctacctg  1080
atcccgctcc cagcagcaga actggccagc tgtgcagacc tggggaccct ctgtcaagct  1140
actgtaaact ctcctagtac tacaccaccc actgtcacca ctaacatgcc tgttactaac  1200
agaatcgata aacaaaggaa tgatggaatt atctatagaa tatccgtagt gattcagaac  1260
atccttcgtc accctgaggt aaaagtacag agcaaggtgg cagaatggct caattcaacc  1320
ttccaaaatt ggaactacac ggtttatgtc gttaatatca gttttcacct gagtgctgga  1380
gaggacaaga ttaaagtcaa gagaagcctt gaggatgagc caaggttggt gctttgggcc  1440
cttctagttt acaatgctac caacaatact aatttagaag gaaaaatcat tcagcagaag  1500
ctcctaaaaa ataatgagtc cttggatgaa ggcttgaggc tacatacagt gaatgtgaga  1560
caactgggtc attgtcttgc catggaggaa cccaaaggct actactggcc atctatccaa  1620
ccttctgaat acgttcttcc ttgtccagac aagcctggct ttctgcttc tcggatatgt   1680
ttttacaatg ctaccaaccc attggtaacc tactggggac ctgttgatat ctccaactgt  1740
ttaaaagaag caaatgaagt tgctaaccag atttaaatt taactgctga tgggcagaac   1800
ttaacctcag ccaatattac caacattgtg gaacaggtca aagaattgt gaataaagaa    1860
gaaaacattg atataacact tggctcaact ctaatgaata tattttctaa tatcttaagc  1920
agttcagaca gtgacttgct tgagtcatct tctgaagctt taaaaacaat tgatgaattg  1980
gccttcaaga tagacctaaa tagcacatca catgtgaata ttacaactcg gaacttggct  2040
ctcagcgtat catccctgtt accagggaca aatgcaattt caaattttag cattggtctt  2100
ccaagcaata atgaatcgta tttccagatg gattttgaga gtggacaagt ggatccactg  2160
gcatctgtaa ttttgcctcc aaacttactt gagaatttaa gtccagaaga ttctgtatta  2220
gttagaagag cacagtttac tttcttcaac aaaactggac ttttccagga tgtaggaccc  2280
caaagaaaaa ctttagtgag ttatgtgatg gcgtgcagta ttggaaacat tactatccag  2340
aatctgaagg atcctgttca aataaaaatc aaacatacaa gaactcagga agtgcatcat  2400
cccatctgtg ccttctggga tctgaacaaa aacaaaagtt ttggaggatg gaacacgtca  2460
ggatgtgttg cacacagaga ttcagatgca agtgagacag tctgcctgtg taaccacttc  2520
acacactttg gagttctgat ggaccttcca agaagtgcct cacagttaga tgcaagaaac  2580
actaaagtcc tcactttcat cagctatatt gggtgtggaa tatctgctat tttttcagca  2640
gcaactctcc tgacatatgt tgcttttgag aaattgcgaa gggattatcc ctccaaaatc  2700
ttgatgaacc tgagcacagc cctgctgttc ctgaatctcc tcttcctcct agatggctgg  2760
atcacctcct tcaatgtgga tggactttgc attgctgttg cagtcctgtt gcatttcttc  2820
cttctggcaa cctttacctg gatggggcta gaagcaattc acatgtacat tgctctagtt  2880
aaagtattta acacttacat tcgccgatac attctaaaat tctgcatcat tggctggggt  2940
ttgcctgcct tagtggtgtc agttgttcta gcgagcagaa acaacaatga agtctatgga  3000
aaagaaagtt atgggaaaga aaaaggtgat gaattctgtt ggattcaaga tccagtcata  3060
ttttatgtga cctgtgctgg gtattttgga gtcatgtttt ttctgaacat tgccatgttc  3120
attgtggtaa tggtgcagat ctgtgggagg aatggcaaga gaagcaaccg gaccctgaga  3180
gaagaagtgt taaggaacct gcgcagtgtg gttagcttga cctttctgtt gggcatgaca  3240
tggggttttg cattctttgc ctggggaccc ttaaatatcc ccttcatgta cctcttctcc  3300
atcttcaatt cattacaagg cttatttata ttcatcttcc actgtgctat gaaggagaat  3360
gttcagaaac agtggcggcg gcatctctgc tgtggtagat ttcggttagc agataactca  3420
gattggagta agacagctac caatatcatc aagaaaagtt ctgataatct aggaaaatct  3480
ttgtcttcaa gctccattgg ttccaactca acctatctta catccaaatc taaatccagc  3540
tctaccacct atttcaaaag gaatagccac acagataatg tctcctatga gcattccttc  3600
aacaaaagtg gatcactcag acagtgcttc catggacaag tccttgtcaa aactggccca  3660
tgc                                                                3663
```

<210> 9
<211> 1221
<212> PRT
<213> Homo sapiens

<400> 9

```
Met Met Phe Arg Ser Asp Arg Met Trp Ser Cys His Trp Lys Trp Lys
                5                   10                  15
Pro Ser Pro Leu Leu Phe Leu Phe Ala Leu Tyr Ile Met Cys Val Pro
            20                  25                  30
His Ser Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Ala Thr Val Asn Ser
    370                 375                 380
Pro Ser Thr Thr Pro Pro Thr Val Thr Thr Asn Met Pro Val Thr Asn
385                 390                 395                 400
Arg Ile Asp Lys Gln Arg Asn Asp Gly Ile Ile Tyr Arg Ile Ser Val
                405                 410                 415
Val Ile Gln Asn Ile Leu Arg His Pro Glu Val Lys Val Gln Ser Lys
            420                 425                 430
Val Ala Glu Trp Leu Asn Ser Thr Phe Gln Asn Trp Asn Tyr Thr Val
        435                 440                 445
Tyr Val Val Asn Ile Ser Phe His Leu Ser Ala Gly Glu Asp Lys Ile
    450                 455                 460
Lys Val Lys Arg Ser Leu Glu Asp Glu Pro Arg Leu Val Leu Trp Ala
465                 470                 475                 480
Leu Leu Val Tyr Asn Ala Thr Asn Asn Thr Asn Leu Glu Gly Lys Ile
                485                 490                 495
Ile Gln Gln Lys Leu Leu Lys Asn Asn Glu Ser Leu Asp Glu Gly Leu
            500                 505                 510
Arg Leu His Thr Val Asn Val Arg Gln Leu Gly His Cys Leu Ala Met
        515                 520                 525
Glu Glu Pro Lys Gly Tyr Tyr Trp Pro Ser Ile Gln Pro Ser Glu Tyr
    530                 535                 540
```

```
Val Leu Pro Cys Pro Asp Lys Pro Gly Phe Ser Ala Ser Arg Ile Cys
545                 550                 555                 560
Phe Tyr Asn Ala Thr Asn Pro Leu Val Thr Tyr Trp Gly Pro Val Asp
                565                 570                 575
Ile Ser Asn Cys Leu Lys Glu Ala Asn Glu Val Ala Asn Gln Ile Leu
            580                 585                 590
Asn Leu Thr Ala Asp Gly Gln Asn Leu Thr Ser Ala Asn Ile Thr Asn
        595                 600                 605
Ile Val Glu Gln Val Lys Arg Ile Val Asn Lys Glu Glu Asn Ile Asp
    610                 615                 620
Ile Thr Leu Gly Ser Thr Leu Met Asn Ile Phe Ser Asn Ile Leu Ser
625                 630                 635                 640
Ser Ser Asp Ser Asp Leu Leu Glu Ser Ser Ser Glu Ala Leu Lys Thr
                645                 650                 655
Ile Asp Glu Leu Ala Phe Lys Ile Asp Leu Asn Ser Thr Ser His Val
            660                 665                 670
Asn Ile Thr Thr Arg Asn Leu Ala Leu Ser Val Ser Ser Leu Leu Pro
        675                 680                 685
Gly Thr Asn Ala Ile Ser Asn Phe Ser Ile Gly Leu Pro Ser Asn Asn
    690                 695                 700
Glu Ser Tyr Phe Gln Met Asp Phe Glu Ser Gly Gln Val Asp Pro Leu
705                 710                 715                 720
Ala Ser Val Ile Leu Pro Pro Asn Leu Leu Glu Asn Leu Ser Pro Glu
                725                 730                 735
Asp Ser Val Leu Val Arg Arg Ala Gln Phe Thr Phe Phe Asn Lys Thr
            740                 745                 750
Gly Leu Phe Gln Asp Val Gly Pro Gln Arg Lys Thr Leu Val Ser Tyr
        755                 760                 765
Val Met Ala Cys Ser Ile Gly Asn Ile Thr Ile Gln Asn Leu Lys Asp
    770                 775                 780
Pro Val Gln Ile Lys Ile Lys His Thr Arg Thr Gln Glu Val His His
785                 790                 795                 800
Pro Ile Cys Ala Phe Trp Asp Leu Asn Lys Asn Lys Ser Phe Gly Gly
                805                 810                 815
Trp Asn Thr Ser Gly Cys Val Ala His Arg Asp Ser Asp Ala Ser Glu
            820                 825                 830
Thr Val Cys Leu Cys Asn His Phe Thr His Phe Gly Val Leu Met Asp
        835                 840                 845
Leu Pro Arg Ser Ala Ser Gln Leu Asp Ala Arg Asn Thr Lys Val Leu
    850                 855                 860
Thr Phe Ile Ser Tyr Ile Gly Cys Gly Ile Ser Ala Ile Phe Ser Ala
865                 870                 875                 880
Ala Thr Leu Leu Thr Tyr Val Ala Phe Glu Lys Leu Arg Arg Asp Tyr
                885                 890                 895
Pro Ser Lys Ile Leu Met Asn Leu Ser Thr Ala Leu Leu Phe Leu Asn
            900                 905                 910
Leu Leu Phe Leu Leu Asp Gly Trp Ile Thr Ser Phe Asn Val Asp Gly
        915                 920                 925
Leu Cys Ile Ala Val Ala Val Leu Leu His Phe Phe Leu Leu Ala Thr
    930                 935                 940
Phe Thr Trp Met Gly Leu Glu Ala Ile His Met Tyr Ile Ala Leu Val
945                 950                 955                 960
Lys Val Phe Asn Thr Tyr Ile Arg Arg Tyr Ile Leu Lys Phe Cys Ile
                965                 970                 975
Ile Gly Trp Gly Leu Pro Ala Leu Val Val Ser Val Val Leu Ala Ser
            980                 985                 990
Arg Asn Asn Asn Glu Val Tyr Gly Lys Glu Ser Tyr Gly Lys Glu Lys
        995                 1000                1005
Gly Asp Glu Phe Cys Trp Ile Gln Asp Pro Val Ile Phe Tyr Val Thr
    1010                1015                1020
Cys Ala Gly Tyr Phe Gly Val Met Phe Phe Leu Asn Ile Ala Met Phe
1025                1030                1035                1040
Ile Val Val Met Val Gln Ile Cys Gly Arg Asn Gly Lys Arg Ser Asn
                1045                1050                1055
Arg Thr Leu Arg Glu Glu Val Leu Arg Asn Leu Arg Ser Val Val Ser
            1060                1065                1070
Leu Thr Phe Leu Leu Gly Met Thr Trp Gly Phe Ala Phe Phe Ala Trp
        1075                1080                1085
```

```
Gly Pro Leu Asn Ile Pro Phe Met Tyr Leu Phe Ser Ile Phe Asn Ser
   1090                1095                1100
Leu Gln Gly Leu Phe Ile Phe Ile Phe His Cys Ala Met Lys Glu Asn
1105                1110                1115                1120
Val Gln Lys Gln Trp Arg Arg His Leu Cys Cys Gly Arg Phe Arg Leu
                1125                1130                1135
Ala Asp Asn Ser Asp Trp Ser Lys Thr Ala Thr Asn Ile Ile Lys Lys
            1140                1145                1150
Ser Ser Asp Asn Leu Gly Lys Ser Leu Ser Ser Ser Ser Ile Gly Ser
        1155                1160                1165
Asn Ser Thr Tyr Leu Thr Ser Lys Ser Lys Ser Ser Ser Thr Thr Tyr
   1170                1175                1180
Phe Lys Arg Asn Ser His Thr Asp Asn Val Ser Tyr Glu His Ser Phe
1185                1190                1195                1200
Asn Lys Ser Gly Ser Leu Arg Gln Cys Phe His Gly Gln Val Leu Val
                1205                1210                1215
Lys Thr Gly Pro Cys
            1220
```

<210> 10
<211> 79
<212> DNA
<213> Homo sapiens

<400> 10
```
ccctgagaga agaagtgtta aggaacctgc gcagtgtggt tagcttgacc tttctgttgg     60
gcatgacatg ggttttgc                                                   79
```

<210> 11
<211> 33
<212> DNA
<213> Artificial Sequence

<220>
<223> Designed oligonucleotide primer to amplify DNA encoding dj287G14.2

<400> 11
```
ctcgagatga tgtttcgctc agatcgaatg tgg                    33
```

<210> 12
<211> 33
<212> DNA
<213> Artificial Sequence

<220>
<223> Designed oligonucleotide primer to amplify DNA encoding dj287G14.2

<400> 12
```
gctagctcag catgggccag ttttgacaag gac                    33
```

<210> 13
<211> 1193
<212> PRT
<213> Mus musculus

<400> 13
```
Met Met Phe Asp Thr Leu Gly Lys Arg Cys Cys Pro Trp Arg Leu Lys
                5                   10                  15
Pro Ser Ala Leu Leu Phe Leu Phe Val Leu Cys Val Thr Cys Val Pro
            20                  25                  30
Leu Ser Val Cys Gly Cys Gly Ser Cys Arg Leu Val Leu Ser Asn Pro
        35                  40                  45
Ser Gly Thr Phe Thr Ser Pro Cys Tyr Pro Asn Asp Tyr Pro Asn Thr
    50                  55                  60
Gln Ser Cys Ser Trp Thr Leu Arg Ala Pro Ala Gly Tyr Ile Ile Gln
65                  70                  75                  80
Ile Thr Phe Asn Asp Phe Asp Ile Glu Glu Ala Pro Asn Cys Ile Tyr
                85                  90                  95
```

```
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Val Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Leu Asp Ala Tyr Gln Val Ser Val Ala Lys Ser Ile Ser
                165                 170                 175
Ile Pro Glu Leu Lys Ala Phe Thr Leu Cys Phe Glu Ala Ser Lys Val
            180                 185                 190
Gly Asn Glu Gly Gly Asp Trp Thr Ala Phe Ser Tyr Ser Asp Glu Ser
        195                 200                 205
Leu Thr Gln Leu Leu Ser Leu Glu Lys Ala Ser Asn Gly Tyr Phe Leu
    210                 215                 220
Ser Ile Ser Gly Ser Arg Cys Leu Leu Asn Asn Ala Leu Pro Val Lys
225                 230                 235                 240
Asp Lys Glu Asp Ile Phe Thr Glu Asn Leu Glu Gln Leu Cys Leu Val
                245                 250                 255
Trp Asn Asn Ser Trp Gly Ser Val Gly Ile Asn Phe Lys Lys Asn Tyr
            260                 265                 270
Glu Thr Val Pro Cys Asp Ser Thr Ile Ser Ala Val Val Pro Gly Asp
        275                 280                 285
Gly Thr Leu Leu Leu Gly Ser Asp Arg Asp Glu Ile Ala Ser Leu Arg
    290                 295                 300
Gly Ser Ile Tyr Asn Phe Arg Leu Trp Asn Phe Thr Met Asp Leu Lys
305                 310                 315                 320
Ala Leu Ser Asn Leu Ser Cys Ser Val Ser Gly Asn Val Ile Asp Trp
                325                 330                 335
His Asn Asp Phe Trp Ser Ile Ser Thr Gln Ala Leu Lys Ala Glu Gly
            340                 345                 350
Asn Leu Ser Cys Gly Ser Tyr Leu Ile Gln Leu Pro Ala Ala Glu Leu
        355                 360                 365
Thr Asn Cys Ser Glu Leu Gly Thr Leu Cys Gln Ala Thr Val Ser Pro
    370                 375                 380
Pro Ser Thr Pro Pro Pro Thr Val Thr Thr Asn Ile Pro Val Thr Asn
385                 390                 395                 400
Arg Val His Lys Gln Lys Asn Asp Gly Ile Met Tyr Arg Ile Ser Val
                405                 410                 415
Val Ile His Asn Asp Phe Asn His Pro Glu Val Lys Val Gln Thr Lys
            420                 425                 430
Val Ala Glu Trp Leu Asn Ser Thr Phe Gln Asn Trp Asn Tyr Thr Val
        435                 440                 445
Tyr Val Val Asn Ile Ser Phe His Gln Lys Val Gly Glu Asp Arg Met
    450                 455                 460
Lys Val Lys Arg Asp Ile Met Asp Asp Asp Lys Arg Leu Val Leu Trp
465                 470                 475                 480
Ala Leu Leu Val Tyr Asn Ala Thr Asn Asn Val Ser Leu Asn Glu Glu
                485                 490                 495
Lys Ile Lys Gln Lys Leu Met Thr Asn Asn Ala Ser Leu Glu Asp Gly
            500                 505                 510
Leu Arg Leu Cys Glu Val Asp Val Asn Gln Leu Gly Met Cys Ser Ala
        515                 520                 525
Leu Glu Asp Pro Asp Gly Phe Ser Trp Pro Ala Thr Leu Pro Ser Val
    530                 535                 540
Tyr Lys Gln Pro Cys Pro Asn Lys Pro Gly Phe Phe Met Thr Arg Ala
545                 550                 555                 560
Cys Leu Ser Asn Gly Thr Ser Thr Phe Trp Gly Pro Val Asp Thr Ser
                565                 570                 575
Asn Cys Ser Arg Gln Ser Asn Glu Val Ala Asn Glu Ile Leu Asn Gln
            580                 585                 590
Thr Gly Asp Gly Gln Asn Leu Thr Ser Ala Asn Ile Asn Ser Ile Val
        595                 600                 605
Glu Lys Val Lys Arg Ile Val Asn Lys Glu Glu Asn Ile Asp Ile Thr
    610                 615                 620
Leu Gly Ser Thr Leu Met Asn Ile Phe Ser Asn Ile Leu Ser Ser Ser
625                 630                 635                 640
```

Asp Ser Asp Leu Leu Glu Ser Ser Thr Glu Ala Leu Lys Thr Ile Asp
645 650 655
Glu Leu Ala Phe Lys Ile Asp Leu Asn Ser Thr Pro His Val Asn Ile
660 665 670
Glu Thr Gln Asn Leu Ala Leu Gly Val Ser Ser Leu Ile Pro Gly Thr
675 680 685
Asn Ala Pro Ser Asn Phe Ser Ile Gly Leu Pro Ser Asn Asn Glu Ser
690 695 700
Tyr Phe Gln Met Asp Phe Gly Asn Gly Gln Thr Asp Pro Leu Ala Ser
705 710 715 720
Val Ile Leu Pro Pro Asn Leu Leu Glu Asn Leu Ser Pro Glu Asp Ser
725 730 735
Val Leu Val Arg Arg Ala Gln Phe Thr Phe Phe Asn Lys Thr Gly Leu
740 745 750
Phe Gln Asp Val Gly Ser Gln Arg Lys Val Leu Val Ser Tyr Val Met
755 760 765
Ala Cys Ser Ile Gly Asn Ile Thr Ile Gln Asn Leu Lys Asp Pro Val
770 775 780
Gln Ile Lys Ile Lys His Thr Arg Thr Gln Glu Val His His Pro Ile
785 790 795 800
Cys Ala Phe Trp Asp Met Asn Lys Asn Lys Ser Phe Gly Gly Trp Asn
805 810 815
Thr Ser Gly Cys Val Ala His Ser Asp Leu Asp Ala Gly Glu Thr Ile
820 825 830
Cys Leu Cys Ser His Phe Thr His Phe Gly Val Leu Met Asp Leu Pro
835 840 845
Arg Ser Ala Ser Gln Ile Asp Gly Arg Asn Thr Lys Val Leu Thr Phe
850 855 860
Ile Thr Tyr Ile Gly Cys Gly Ile Ser Ala Ile Phe Ser Ala Ala Thr
865 870 875 880
Leu Leu Thr Tyr Val Ala Phe Glu Lys Leu Arg Arg Asp Tyr Pro Ser
885 890 895
Lys Ile Leu Met Asn Leu Ser Ser Ala Leu Leu Phe Leu Asn Leu Ile
900 905 910
Phe Leu Leu Asp Gly Trp Val Thr Ser Phe Gly Val Ala Gly Leu Cys
915 920 925
Thr Ala Val Ala Ala Leu Leu His Phe Phe Leu Leu Ala Thr Phe Thr
930 935 940
Trp Met Gly Leu Glu Ala Ile His Met Tyr Ile Ala Leu Val Lys Val
945 950 955 960
Phe Asn Thr Tyr Ile His Arg Tyr Ile Leu Lys Phe Cys Ile Ile Gly
965 970 975
Trp Gly Leu Pro Ala Leu Val Val Ser Ile Ile Leu Val Ser Arg Arg
980 985 990
Gln Asn Glu Val Tyr Gly Lys Glu Ser Tyr Gly Lys Asp Gln Asp Asp
995 1000 1005
Glu Phe Cys Trp Ile Gln Asp Pro Val Val Phe Tyr Val Ser Cys Ala
1010 1015 1020
Gly Tyr Phe Gly Val Met Phe Phe Leu Asn Val Ala Met Phe Ile Val
1025 1030 1035 1040
Val Met Val Gln Ile Cys Gly Arg Asn Gly Lys Arg Ser Asn Arg Thr
1045 1050 1055
Leu Arg Glu Glu Val Leu Arg Asn Leu Arg Ser Val Val Ser Leu Thr
1060 1065 1070
Phe Leu Leu Gly Met Thr Trp Gly Phe Ala Phe Phe Ala Trp Gly Pro
1075 1080 1085
Leu Asn Ile Pro Phe Met Tyr Leu Phe Ser Ile Phe Asn Ser Leu Gln
1090 1095 1100
Gly Leu Phe Ile Phe Ile Phe His Cys Ala Met Lys Glu Asn Val Gln
1105 1110 1115 1120
Lys Gln Trp Arg Arg His Leu Cys Cys Gly Arg Phe Arg Leu Ala Asp
1125 1130 1135
Asn Ser Asp Trp Ser Lys Thr Ala Thr Asn Ile Ile Lys Lys Ser Ser
1140 1145 1150
Asp Asn Leu Gly Lys Ser Leu Ser Ser Ser Ser Ile Gly Ser Asn Ser
1155 1160 1165
Thr Tyr Leu Thr Ser Lys Ser Lys Ser Ser Ser Thr Thr Tyr Phe Lys
1170 1175 1180

Arg Asn Ser His Ser Asp Asn Phe Ser
1185 1190

<210> 14
<211> 3579
<212> DNA
<213> Mus musculus

<400> 14
atgatgtttg acactctcgg gaagaggtgc tgcccttgga gactgaagcc aagcgccctg 60
ctgttcctgt ttgttttatg tgttacctgt gttcctctct cagtgtgcgg atgtggcagc 120
tgcagacttg tcctgtccaa tccttccggt acctttacgt ctccgtgtta ccctaatgac 180
taccctaata cccagtcttg ttcgtggacc ctccgagccc ctgccggcta catcattcag 240
ataacgttca atgacttcga cattgaagaa gctcccaact gtatctatga ctcattgtcc 300
ctcgataatg gagagagcca gacaaaattc tgtggagcga ctgccaaggg cctgtcattt 360
aactccagcg tgaatgagat gcatgtgtcc ttttcaagtg actttagtat ccagaagaaa 420
ggtttcaacg ccagctacat cagagttgct gtgtccttga ggaatcaaaa ggtcattttg 480
ccccagacat tagatgctta ccaggtatca gttgcaaaaa gcatctccat tcctgaactc 540
aaagctttca cgctctgttt tgaagcctcc aaagttggca atgaaggtgg tgactggaca 600
gctttctcct actcagacga gtcccttaca cagctgctca gtcttgaaaa ggccagtaat 660
ggctacttcc tgtccatctc tggctcaaga tgcttgttga acaatgcgtt acctgtgaag 720
gacaaagagg acatcttcac agaaaacttg gagcagctct gtcttgtgtg gaataattct 780
tggggctccg ttggtataaa tttcaaaaag aactatgaaa cagttccatg cgattccacc 840
atcagtgctg tcgtacccgg ggatgggaca ttgctgttgg gctccgacag agatgagatc 900
gcctctctaa ggggcagcat ctataacttt cgactttgga attttaccat ggatctgaaa 960
gccctctcca acctcagctg tagtgtgtct gggaatgtca tagactggca caatgacttt 1020
tggagcatct caacccaagc tctgaaagcc gagggcaacc tgagctgtgg ttcctacctg 1080
atccagcttc ctgcagcaga gctgacaaac tgttcagaac tggggactct ctgtcaagct 1140
actgtaagcc ctccttctac tccaccaccc actgtcacca ctaatatccc cgttactaac 1200
agagtccata aacaaaagaa tgacggaata atgtatcgaa tatctgttgt gattcacaat 1260
gactttaatc accctgaagt aaaagtgcag accaaagtag cagaatggct caattcaacc 1320
tttcagaatt ggaactacac tgtttatgtg gttaatataa gttttcatca aaaagtagga 1380
gaggacagga tgaaagtcaa gagagacatc atggacgatg acaaaaggtt ggtgctctgg 1440
gcccttctag tctacaatgc taccaataac gtcagcctga atgaagagaa gattaaacaa 1500
aagcttatga caaataatgc atcactagag gatggactga ggctgtgtga agtcgacgtg 1560
aaccagctgg gtatgtgtag cgccttggag gatccagacg gctttagttg gccagccacc 1620
ttaccctctg tctacaaaca accatgtcca aacaagcctg gctttttat gactcgagcg 1680
tgcctttcta atggaacatc aaccttctgg ggccctgttg acacttccaa ctgttcaaga 1740
caatcaaatg aagtggccaa tgagatttta aaccaaactg gtgatgggca gaacctcacc 1800
tcggctaata tcaacagcat tgtagaaaag gtcaaacgga tcgtgaacaa agaagaaaac 1860
attgacatca ccctcggctc cactctaatg aatatatttt ctaatatctt aagcagttca 1920
gatagcgatt tgcttgagtc ttctactgaa gctttaaaaa caattgacga gctagccttc 1980
aaaatagacc tgaatagcac cccacatgtg aacattgaga cacagaattt ggcccttgga 2040
gtctcatccc taattccagg aacaaatgca ccttcaaatt ttagcattgg ccttccaagc 2100
aataatgaat cgtacttcca gatggacttc gggaacggac agacagatcc actggcatct 2160
gtgattttgc ctccaaattt gcttgagaat ttaagccccg aagattctgt attggtcagg 2220
agagcacagt tcactttctt caacaaaacc ggacttttcc aggatgttgg atctcaaaga 2280
aaagtcctcg tgagttatgt gatggcgtgc agcattggaa acattactat ccagaatctg 2340
aaagatccgg ttcaaatcaa aatcaaacac accagaacac aggaagtgca tcatcctatc 2400
tgtgccttct gggatatgaa caaaaacaaa agtttcgggg ggtggaacac ctcaggatgt 2460
gttgcccact ctgatttgga cgctggtgag accatttgtc tgtgcagcca cttcactcac 2520
tttggagttc tgatggatct tccaaggagt gcctcacaaa tagatggaag aaacacaaaa 2580
gtcctcacgt tcattaccta tattgggtgc ggaatatctg ccatttttctc agctgcaact 2640
ctcctgacat atgttgcttt tgagaagctg cgcagggatt atccctccaa aatcctgatg 2700
aatctgagct cggccttgct cttcctgaat ctcatcttcc tcctggatgg ctgggtcact 2760
tcctttggcg tggctggact ctgcacggct gtggctgccc tgttgcactt cttcctcctg 2820
gctaccttca cctggatggg gctggaagcc atccacatgt acattgctct tgtgaaagtg 2880
tttaacactt acatccaccg ctatattcta aaattctgca tcataggctg ggtctgcca 2940
gccttggtgg tgtcaattat tctagtgagc agaagacaaa atgaagtata tggaaaagaa 3000
agttatggga aagatcagga tgatgaattc tgctggattc aggatcctgt ggtgttttat 3060
gtgagctgtg ccgggtactt cggagtcatg ttcttcctga atgtcgccat gttcattgtg 3120
gtcatggtgc agatctgtgg gaggaatgga aagagaagca accggaccct gagagaagag 3180
gttttaagaa acctgcgcag tgtggtcagc ctgaccttcc tgcttggcat gacgtggggg 3240
tttgctttct ttgcctgggg accccttaaat attcctttca tgtacctctt ctccatcttc 3300
aattcattac aaggtttatt tatattcatc ttccactgtg cgatgaagga gaatgttcag 3360
aaacagtgga ggcgtcacct ctgctgtggc aggtttcggc tagcagacaa ctcagattgg 3420
agtaagacag ctaccaatat catcaagaag agctccgata acctggggaa atctttgtct 3480
tcaagctcca ttggctccaa ttcaacatat ctcacatcca aatcaaagtc cagctccact 3540

acctatttca aaagaaacag ccactcggat aatttctcc 3579

<210> 15
<211> 1248
<212> PRT
<213> Mus musculus

<400> 15
```
Met Met Phe Asp Thr Leu Gly Lys Arg Cys Cys Pro Trp Arg Leu Lys
                5                   10                  15
Pro Ser Ala Leu Leu Phe Leu Phe Val Leu Cys Val Thr Cys Val Pro
            20                  25                  30
Leu Ser Val Cys Gly Cys Gly Ser Cys Arg Leu Val Leu Ser Asn Pro
        35                  40                  45
Ser Gly Thr Phe Thr Ser Pro Cys Tyr Pro Asn Asp Tyr Pro Asn Thr
    50                  55                  60
Gln Ser Cys Ser Trp Thr Leu Arg Ala Pro Ala Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Val Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Leu Asp Ala Tyr Gln Val Ser Val Ala Lys Ser Ile Ser
                165                 170                 175
Ile Pro Glu Leu Lys Ala Phe Thr Leu Cys Phe Glu Ala Ser Lys Val
            180                 185                 190
Gly Asn Glu Gly Gly Asp Trp Thr Ala Phe Ser Tyr Ser Asp Glu Ser
        195                 200                 205
Leu Thr Gln Leu Leu Ser Leu Glu Lys Ala Ser Asn Gly Tyr Phe Leu
    210                 215                 220
Ser Ile Ser Gly Ser Arg Cys Leu Leu Asn Asn Ala Leu Pro Val Lys
225                 230                 235                 240
Asp Lys Glu Asp Ile Phe Thr Glu Asn Leu Glu Gln Leu Cys Leu Val
                245                 250                 255
Trp Asn Asn Ser Trp Gly Ser Val Gly Ile Asn Phe Lys Lys Asn Tyr
            260                 265                 270
Glu Thr Val Pro Cys Asp Ser Thr Ile Ser Ala Val Val Pro Gly Asp
        275                 280                 285
Gly Thr Leu Leu Leu Gly Ser Asp Arg Asp Glu Ile Ala Ser Leu Arg
    290                 295                 300
Gly Ser Ile Tyr Asn Phe Arg Leu Trp Asn Phe Thr Met Asp Leu Lys
305                 310                 315                 320
Ala Leu Ser Asn Leu Ser Cys Ser Val Ser Gly Asn Val Ile Asp Trp
                325                 330                 335
His Asn Asp Phe Trp Ser Ile Ser Thr Gln Ala Leu Lys Ala Glu Gly
            340                 345                 350
Asn Leu Ser Cys Gly Ser Tyr Leu Ile Gln Leu Pro Ala Ala Glu Leu
        355                 360                 365
Thr Asn Cys Ser Glu Leu Gly Thr Leu Cys Gln Ala Thr Val Ser Pro
    370                 375                 380
Pro Ser Thr Pro Pro Pro Thr Val Thr Thr Asn Ile Pro Val Thr Asn
385                 390                 395                 400
Arg Val His Lys Gln Lys Asn Asp Gly Ile Met Tyr Arg Ile Ser Val
                405                 410                 415
Val Ile His Asn Asp Phe Asn His Pro Glu Val Lys Val Gln Thr Lys
            420                 425                 430
Val Ala Glu Trp Leu Asn Ser Thr Phe Gln Asn Trp Asn Tyr Thr Val
        435                 440                 445
Tyr Val Val Asn Ile Ser Phe His Gln Lys Val Gly Glu Asp Arg Met
    450                 455                 460
Lys Val Lys Arg Asp Ile Met Asp Asp Asp Lys Arg Leu Val Leu Trp
465                 470                 475                 480
```

```
Ala Leu Leu Val Tyr Asn Ala Thr Asn Asn Val Ser Leu Asn Glu Glu
                485                 490                 495
Lys Ile Lys Gln Lys Leu Met Thr Asn Asn Ala Ser Leu Glu Asp Gly
            500                 505                 510
Leu Arg Leu Cys Glu Val Asp Val Asn Gln Leu Gly Met Cys Ser Ala
        515                 520                 525
Leu Glu Asp Pro Asp Gly Phe Ser Trp Pro Ala Thr Leu Pro Ser Val
    530                 535                 540
Tyr Lys Gln Pro Cys Pro Asn Lys Pro Gly Phe Phe Met Thr Arg Ala
545                 550                 555                 560
Cys Leu Ser Asn Gly Thr Ser Thr Phe Trp Gly Pro Val Asp Thr Ser
                565                 570                 575
Asn Cys Ser Arg Gln Ser Asn Glu Val Ala Asn Glu Ile Leu Asn Gln
            580                 585                 590
Thr Gly Asp Gly Gln Asn Leu Thr Ser Ala Asn Ile Asn Ser Ile Val
        595                 600                 605
Glu Lys Val Lys Arg Ile Val Asn Lys Glu Glu Asn Ile Asp Ile Thr
    610                 615                 620
Leu Gly Ser Thr Leu Met Asn Ile Phe Ser Asn Ile Leu Ser Ser Ser
625                 630                 635                 640
Asp Ser Asp Leu Leu Glu Ser Ser Thr Glu Ala Leu Lys Thr Ile Asp
                645                 650                 655
Glu Leu Ala Phe Lys Ile Asp Leu Asn Ser Thr Pro His Val Asn Ile
            660                 665                 670
Glu Thr Gln Asn Leu Ala Leu Gly Val Ser Ser Leu Ile Pro Gly Thr
        675                 680                 685
Asn Ala Pro Ser Asn Phe Ser Ile Gly Leu Pro Ser Asn Asn Glu Ser
    690                 695                 700
Tyr Phe Gln Met Asp Phe Gly Asn Gly Gln Thr Asp Pro Leu Ala Ser
705                 710                 715                 720
Val Ile Leu Pro Pro Asn Leu Leu Glu Asn Leu Ser Pro Glu Asp Ser
                725                 730                 735
Val Leu Val Arg Arg Ala Gln Phe Thr Phe Phe Asn Lys Thr Gly Leu
            740                 745                 750
Phe Gln Asp Val Gly Ser Gln Arg Lys Val Leu Val Ser Tyr Val Met
        755                 760                 765
Ala Cys Ser Ile Gly Asn Ile Thr Ile Gln Asn Leu Lys Asp Pro Val
    770                 775                 780
Gln Ile Lys Ile Lys His Thr Arg Thr Gln Glu Val His His Pro Ile
785                 790                 795                 800
Cys Ala Phe Trp Asp Met Asn Lys Asn Lys Ser Phe Gly Gly Trp Asn
                805                 810                 815
Thr Ser Gly Cys Val Ala His Ser Asp Leu Asp Ala Gly Glu Thr Ile
            820                 825                 830
Cys Leu Cys Ser His Phe Thr His Phe Gly Val Leu Met Asp Leu Pro
        835                 840                 845
Arg Ser Ala Ser Gln Ile Asp Gly Arg Asn Thr Lys Val Leu Thr Phe
    850                 855                 860
Ile Thr Tyr Ile Gly Cys Gly Ile Ser Ala Ile Phe Ser Ala Ala Thr
865                 870                 875                 880
Leu Leu Thr Tyr Val Ala Phe Glu Lys Leu Arg Arg Asp Tyr Pro Ser
                885                 890                 895
Lys Ile Leu Met Asn Leu Ser Ser Ala Leu Leu Phe Leu Asn Leu Ile
            900                 905                 910
Phe Leu Leu Asp Gly Trp Val Thr Ser Phe Gly Val Ala Gly Leu Cys
        915                 920                 925
Thr Ala Val Ala Ala Leu Leu His Phe Phe Leu Leu Ala Thr Phe Thr
    930                 935                 940
Trp Met Gly Leu Glu Ala Ile His Met Tyr Ile Ala Leu Val Lys Val
945                 950                 955                 960
Phe Asn Thr Tyr Ile His Arg Tyr Ile Leu Lys Phe Cys Ile Ile Gly
                965                 970                 975
Trp Gly Leu Pro Ala Leu Val Val Ser Ile Ile Leu Val Ser Arg Arg
            980                 985                 990
Gln Asn Glu Val Tyr Gly Lys Glu Ser Tyr Gly Lys Asp Gln Asp Asp
        995                 1000                1005
Glu Phe Cys Trp Ile Gln Asp Pro Val Val Phe Tyr Val Ser Cys Ala
    1010                1015                1020
```

Gly Tyr Phe Gly Val Met Phe Phe Leu Asn Val Ala Met Phe Ile Val
1025 1030 1035 1040
Val Met Val Gln Ile Cys Gly Arg Asn Gly Lys Arg Ser Asn Arg Thr
1045 1050 1055
Leu Arg Glu Glu Val Leu Arg Asn Leu Arg Ser Val Val Ser Leu Thr
1060 1065 1070
Phe Leu Leu Gly Met Thr Trp Gly Phe Ala Phe Phe Ala Trp Gly Pro
1075 1080 1085
Leu Asn Ile Pro Phe Met Tyr Leu Phe Ser Ile Phe Asn Ser Leu Gln
1090 1095 1100
Gly Leu Phe Ile Phe Ile Phe His Cys Ala Met Lys Glu Asn Val Gln
1105 1110 1115 1120
Lys Gln Trp Arg Arg His Leu Cys Cys Gly Arg Phe Arg Leu Ala Asp
1125 1130 1135
Asn Ser Asp Trp Ser Lys Thr Ala Thr Asn Ile Ile Lys Lys Ser Ser
1140 1145 1150
Asp Asn Leu Gly Lys Ser Leu Ser Ser Ser Ser Ile Gly Ser Asn Ser
1155 1160 1165
Thr Tyr Leu Thr Ser Lys Ser Lys Ser Ser Ser Thr Thr Tyr Phe Lys
1170 1175 1180
Arg Asn Ser His Ser Asp Ser Thr Ser Thr Asp Lys Ser Leu Ser Lys
1185 1190 1195 1200
Leu Thr His Ala Gly Gly Glu Gln Thr Ser Ile Ile Pro Val His Gln
1205 1210 1215
Val Ile Asp Lys Val Lys Gly Tyr Cys Asn Ala His Ser Asp Asn Phe
1220 1225 1230
Tyr Lys Asn Ile Ile Leu Ser Asp Ala Phe Ser His Ser Thr Lys Phe
1235 1240 1245

<210> 16
<211> 3744
<212> DNA
<213> Mus musculus

<400> 16
atgatgtttg acactctcgg gaagaggtgc tgcccttgga gactgaagcc aagcgccctg 60
ctgttcctgt ttgttttatg tgttacctgt gttcctctct cagtgtgcgg atgtggcagc 120
tgcagacttg tcctgtccaa tccttccggt acctttacgt ctccgtgtta ccctaatgac 180
taccctaata cccagtcttg ttcgtggacc ctccgagccc ctgccggcta catcattcag 240
ataacgttca atgacttcga cattgaagaa gctcccaact gtatctatga ctcattgtcc 300
ctcgataatg gagagagcca gacaaaattc tgtggagcga ctgccaaggg cctgtcattt 360
aactccagcg tgaatgagat gcatgtgtcc ttttcaagtg actttagtat ccagaagaaa 420
ggtttcaacg ccagctacat cagagttgct gtgtccttga ggaatcaaaa ggtcattttg 480
ccccagacat tagatgctta ccaggtatca gttgcaaaaa gcatctccat tcctgaactc 540
aaagctttca cgctctgttt tgaagcctcc aaagttggca atgaaggtgg tgactggaca 600
gctttctcct actcagacga gtcccttaca cagctgctca gtcttgaaaa ggccagtaat 660
ggctacttcc tgtccatctc tggctcaaga tgcttgttga acaatgcgtt acctgtgaag 720
gacaaagagg acatcttcac agaaaacttg gagcagctct gtcttgtgtg gaataattct 780
tggggctccg ttggtataaa tttcaaaaag aactatgaaa cagttccatg cgattccacc 840
atcagtgctg tcgtacccgg ggatgggaca ttgctgttgg gctccgacag agatgagatc 900
gcctctctaa ggggcagcat ctataacttt cgactttgga attttaccat ggatctgaaa 960
gccctctcca acctcagctg tagtgtgtct gggaatgtca tagactggca caatgacttt 1020
tggagcatct caacccaagc tctgaaagcc gagggcaacc tgagctgtgg ttcctacctg 1080
atccagcttc ctgcagcaga gctgacaaac tgttcagaac tggggactct ctgtcaagct 1140
actgtaagcc ctccttctac tccaccaccc actgtcacca ctaatatccc cgttactaac 1200
agagtccata aacaaaagaa tgacggaata atgtatcgaa tatctgttgt gattcacaat 1260
gactttaatc accctgaagt aaaagtgcag accaaagtag cagaatggct caattcaacc 1320
tttcagaatt ggaactacac tgtttatgtg gttaatataa gttttcatca aaaagtagga 1380
gaggacagga tgaaagtcaa gagagacatc atggacgatg acaaaaggtt ggtgctctgg 1440
gcccttctag tctacaatgc taccaataac gtcagcctga atgaagagaa gattaaacaa 1500
aagcttatga caaataatgc atcactagag gatggactga ggctgtgtga agtcgacgtg 1560
aaccagctgg gtatgtgtag cgccttggag gatccagacg gctttagttg gccagccacc 1620
ttaccctctg tctacaaaca accatgtcca aacaagcctg gcttttttat gactcgagcg 1680
tgcctttcta atggaacatc aaccttctgg ggccctgttg acacttccaa ctgttcaaga 1740
caatcaaatg aagtggccaa tgagatttta aaccaaactg gtgatgggca gaacctcacc 1800
tcggctaata tcaacagcat tgtagaaaag gtcaaacgga tcgtgaacaa agaagaaaac 1860
attgacatca ccctcggctc cactctaatg aatatatttt ctaatatctt aagcagttca 1920
gatagcgatt tgcttgagtc ttctactgaa gctttaaaaa caattgacga gctagccttc 1980

```
aaaatagacc tgaatagcac cccacatgtg aacattgaga cacagaattt ggcccttgga 2040
gtctcatccc taattccagg aacaaatgca ccttcaaatt ttagcattgg ccttccaagc 2100
aataatgaat cgtacttcca gatggacttc gggaacggac agacagatcc actggcatct 2160
gtgattttgc ctccaaattt gcttgagaat ttaagccccg aagattctgt attggtcagg 2220
agagcacagt tcactttctt caacaaaacc ggacttttcc aggatgttgg atctcaaaga 2280
aaagtcctcg tgagttatgt gatggcgtgc agcattggaa acattactat ccagaatctg 2340
aaagatccgg ttcaaatcaa aatcaaacac accagaacac aggaagtgca tcatcctatc 2400
tgtgccttct gggatatgaa caaaaacaaa agtttcgggg ggtggaacac ctcaggatgt 2460
gttgcccact ctgatttgga cgctggtgag accatttgtc tgtgcagcca cttcactcac 2520
tttggagttc tgatggatct tccaaggagt gcctcacaaa tagatggaag aaacacaaaa 2580
gtcctcacgt tcattaccta tattgggtgc ggaatatctg ccattttctc agctgcaact 2640
ctcctgacat atgttgcttt tgagaagctg cgcagggatt atccctccaa aatcctgatg 2700
aatctgagct cggccttgct cttcctgaat ctcatcttcc tcctggatgg ctgggtcact 2760
tcctttggcg tggctggact ctgcacggct gtggctgccc tgttgcactt cttcctcctg 2820
gctaccttca cctggatggg gctggaagcc atccacatgt acattgctct tgtgaaagtg 2880
tttaacactt acatccaccg ctatattcta aaattctgca tcataggctg ggtctgcca 2940
gccttggtgg tgtcaattat tctagtgagc agaagacaaa atgaagtata tggaaaagaa 3000
agttatggga aagatcagga tgatgaattc tgctggattc aggatcctgt ggtgttttat 3060
gtgagctgtg ccgggtactt cggagtcatg ttcttcctga atgtcgccat gttcattgtg 3120
gtcatggtgc agatctgtgg gaggaatgga aagagaagca accggaccct gagagaagag 3180
gttttaagaa acctgcgcag tgtggtcagc ctgaccttcc tgcttggcat gacgtggggg 3240
tttgctttct ttgcctgggg acccttaaat attcctttca tgtacctctt ctccatcttc 3300
aattcattac aaggtttatt tatattcatc ttccactgtg cgatgaagga gaatgttcag 3360
aaacagtgga ggcgtcacct ctgctgtggc aggtttcggc tagcagacaa ctcagattgg 3420
agtaagacag ctaccaatat catcaagaag agctccgata acctggggaa atctttgtct 3480
tcaagctcca ttggctccaa ttcaacatat ctcacatcca aatcaaagtc cagctccact 3540
acctatttca aaagaaacag ccactcggac agcacttcca cggacaagtc cctgtcaaaa 3600
ctgacccatg ctggtggaga acagacgtca atcattcccg tccatcaggt tattgataag 3660
gtcaagggtt actgtaatgc ccattccgat aacttctata aaaatatcat cctgtcagat 3720
gccttcagcc acagcacaaa gttt                                        3744
```

<210> 17
<211> 1203
<212> PRT
<213> Mus musculus

<400> 17

```
Met Ser Val Arg Pro Leu Gly Ile Ser Thr Arg Met Phe Asp Thr Leu
                5                   10                  15
Gly Lys Arg Cys Cys Pro Trp Arg Leu Lys Pro Ser Ala Leu Leu Phe
            20                  25                  30
Leu Phe Val Leu Cys Val Thr Cys Val Pro Leu Ser Val Cys Gly Cys
        35                  40                  45
Gly Ser Cys Arg Leu Val Leu Ser Asn Pro Ser Gly Thr Phe Thr Ser
    50                  55                  60
Pro Cys Tyr Pro Asn Asp Tyr Pro Asn Thr Gln Ser Cys Ser Trp Thr
65                  70                  75                  80
Leu Arg Ala Pro Ala Gly Tyr Ile Ile Gln Ile Thr Phe Asn Asp Phe
                85                  90                  95
Asp Ile Glu Glu Ala Pro Asn Cys Ile Tyr Asp Ser Leu Ser Leu Asp
            100                 105                 110
Asn Gly Glu Ser Gln Thr Lys Phe Cys Gly Ala Thr Ala Lys Gly Leu
        115                 120                 125
Ser Phe Asn Ser Ser Val Asn Glu Met His Val Ser Phe Ser Ser Asp
    130                 135                 140
Phe Ser Ile Gln Lys Lys Gly Phe Asn Ala Ser Tyr Ile Arg Val Ala
145                 150                 155                 160
Val Ser Leu Arg Asn Gln Lys Val Ile Leu Pro Gln Thr Leu Asp Ala
                165                 170                 175
Tyr Gln Val Ser Val Ala Lys Ser Ile Ser Ile Pro Glu Leu Lys Ala
            180                 185                 190
Phe Thr Leu Cys Phe Glu Ala Ser Lys Val Gly Asn Glu Gly Gly Asp
        195                 200                 205
Trp Thr Ala Phe Ser Tyr Ser Asp Glu Ser Leu Thr Gln Leu Leu Ser
    210                 215                 220
Leu Glu Lys Ala Ser Asn Gly Tyr Phe Leu Ser Ile Ser Gly Ser Arg
225                 230                 235                 240
Cys Leu Leu Asn Asn Ala Leu Pro Val Lys Asp Lys Glu Asp Ile Phe
```

245 250 255
Thr Glu Asn Leu Glu Gln Leu Cys Leu Val Trp Asn Asn Ser Trp Gly
260 265 270
Ser Val Gly Ile Asn Phe Lys Lys Asn Tyr Glu Thr Val Pro Cys Asp
275 280 285
Ser Thr Ile Ser Ala Val Val Pro Gly Asp Gly Thr Leu Leu Leu Gly
290 295 300
Ser Asp Arg Asp Glu Ile Ala Ser Leu Arg Gly Ser Ile Tyr Asn Phe
305 310 315 320
Arg Leu Trp Asn Phe Thr Met Asp Leu Lys Ala Leu Ser Asn Leu Ser
325 330 335
Cys Ser Val Ser Gly Asn Val Ile Asp Trp His Asn Asp Phe Trp Ser
340 345 350
Ile Ser Thr Gln Ala Leu Lys Ala Glu Gly Asn Leu Ser Cys Gly Ser
355 360 365
Tyr Leu Ile Gln Leu Pro Ala Ala Glu Leu Thr Asn Cys Ser Glu Leu
370 375 380
Gly Thr Leu Cys Gln Ala Thr Val Ser Pro Pro Ser Thr Pro Pro Pro
385 390 395 400
Thr Val Thr Thr Asn Ile Pro Val Thr Asn Arg Val His Lys Gln Lys
405 410 415
Asn Asp Gly Ile Met Tyr Arg Ile Ser Val Val Ile His Asn Asp Phe
420 425 430
Asn His Pro Glu Val Lys Val Gln Thr Lys Val Ala Glu Trp Leu Asn
435 440 445
Ser Thr Phe Gln Asn Trp Asn Tyr Thr Val Tyr Val Val Asn Ile Ser
450 455 460
Phe His Gln Lys Val Gly Glu Asp Arg Met Lys Val Lys Arg Asp Ile
465 470 475 480
Met Asp Asp Asp Lys Arg Leu Val Leu Trp Ala Leu Leu Val Tyr Asn
485 490 495
Ala Thr Asn Asn Val Ser Leu Asn Glu Glu Lys Ile Lys Gln Lys Leu
500 505 510
Met Thr Asn Asn Ala Ser Leu Glu Asp Gly Leu Arg Leu Cys Glu Val
515 520 525
Asp Val Asn Gln Leu Gly Met Cys Ser Ala Leu Glu Asp Pro Asp Gly
530 535 540
Phe Ser Trp Pro Ala Thr Leu Pro Ser Val Tyr Lys Gln Pro Cys Pro
545 550 555 560
Asn Lys Pro Gly Phe Phe Met Thr Arg Ala Cys Leu Ser Asn Gly Thr
565 570 575
Ser Thr Phe Trp Gly Pro Val Asp Thr Ser Asn Cys Ser Arg Gln Ser
580 585 590
Asn Glu Val Ala Asn Glu Ile Leu Asn Gln Thr Gly Asp Gly Gln Asn
595 600 605
Leu Thr Ser Ala Asn Ile Asn Ser Ile Val Glu Lys Val Lys Arg Ile
610 615 620
Val Asn Lys Glu Glu Asn Ile Asp Ile Thr Leu Gly Ser Thr Leu Met
625 630 635 640
Asn Ile Phe Ser Asn Ile Leu Ser Ser Ser Asp Ser Asp Leu Leu Glu
645 650 655
Ser Ser Thr Glu Ala Leu Lys Thr Ile Asp Glu Leu Ala Phe Lys Ile
660 665 670
Asp Leu Asn Ser Thr Pro His Val Asn Ile Glu Thr Gln Asn Leu Ala
675 680 685
Leu Gly Val Ser Ser Leu Ile Pro Gly Thr Asn Ala Pro Ser Asn Phe
690 695 700
Ser Ile Gly Leu Pro Ser Asn Asn Glu Ser Tyr Phe Gln Met Asp Phe
705 710 715 720
Gly Asn Gly Gln Thr Asp Pro Leu Ala Ser Val Ile Leu Pro Pro Asn
725 730 735
Leu Leu Glu Asn Leu Ser Pro Glu Asp Ser Val Leu Val Arg Arg Ala
740 745 750
Gln Phe Thr Phe Phe Asn Lys Thr Gly Leu Phe Gln Asp Val Gly Ser
755 760 765
Gln Arg Lys Val Leu Val Ser Tyr Val Met Ala Cys Ser Ile Gly Asn
770 775 780
Ile Thr Ile Gln Asn Leu Lys Asp Pro Val Gln Ile Lys Ile Lys His

```
785                 790                 795                 800
Thr Arg Thr Gln Glu Val His His Pro Ile Cys Ala Phe Trp Asp Met
                805                 810                 815
Asn Lys Asn Lys Ser Phe Gly Gly Trp Asn Thr Ser Gly Cys Val Ala
            820                 825                 830
His Ser Asp Leu Asp Ala Gly Glu Thr Ile Cys Leu Cys Ser His Phe
        835                 840                 845
Thr His Phe Gly Val Leu Met Asp Leu Pro Arg Ser Ala Ser Gln Ile
    850                 855                 860
Asp Gly Arg Asn Thr Lys Val Leu Thr Phe Ile Thr Tyr Ile Gly Cys
865                 870                 875                 880
Gly Ile Ser Ala Ile Phe Ser Ala Ala Thr Leu Leu Thr Tyr Val Ala
                885                 890                 895
Phe Glu Lys Leu Arg Arg Asp Tyr Pro Ser Lys Ile Leu Met Asn Leu
            900                 905                 910
Ser Ser Ala Leu Leu Phe Leu Asn Leu Ile Phe Leu Leu Asp Gly Trp
        915                 920                 925
Val Thr Ser Phe Gly Val Ala Gly Leu Cys Thr Ala Val Ala Ala Leu
    930                 935                 940
Leu His Phe Phe Leu Leu Ala Thr Phe Thr Trp Met Gly Leu Glu Ala
945                 950                 955                 960
Ile His Met Tyr Ile Ala Leu Val Lys Val Phe Asn Thr Tyr Ile His
                965                 970                 975
Arg Tyr Ile Leu Lys Phe Cys Ile Ile Gly Trp Gly Leu Pro Ala Leu
            980                 985                 990
Val Val Ser Ile Ile Leu Val Ser Arg Arg Gln Asn Glu Val Tyr Gly
        995                 1000                1005
Lys Glu Ser Tyr Gly Lys Asp Gln Asp Asp Glu Phe Cys Trp Ile Gln
    1010                1015                1020
Asp Pro Val Val Phe Tyr Val Ser Cys Ala Gly Tyr Phe Gly Val Met
1025                1030                1035                1040
Phe Phe Leu Asn Val Ala Met Phe Ile Val Val Met Val Gln Ile Cys
                1045                1050                1055
Gly Arg Asn Gly Lys Arg Ser Asn Arg Thr Leu Arg Glu Glu Val Leu
            1060                1065                1070
Arg Asn Leu Arg Ser Val Val Ser Leu Thr Phe Leu Leu Gly Met Thr
        1075                1080                1085
Trp Gly Phe Ala Phe Phe Ala Trp Gly Pro Leu Asn Ile Pro Phe Met
    1090                1095                1100
Tyr Leu Phe Ser Ile Phe Asn Ser Leu Gln Gly Leu Phe Ile Phe Ile
1105                1110                1115                1120
Phe His Cys Ala Met Lys Glu Asn Val Gln Lys Gln Trp Arg Arg His
                1125                1130                1135
Leu Cys Cys Gly Arg Phe Arg Leu Ala Asp Asn Ser Asp Trp Ser Lys
            1140                1145                1150
Thr Ala Thr Asn Ile Ile Lys Lys Ser Ser Asp Asn Leu Gly Lys Ser
        1155                1160                1165
Leu Ser Ser Ser Ser Ile Gly Ser Asn Ser Thr Tyr Leu Thr Ser Lys
    1170                1175                1180
Ser Lys Ser Ser Ser Thr Thr Tyr Phe Lys Arg Asn Ser His Ser Asp
1185                1190                1195                1200
Asn Phe Ser
```

<210> 18
<211> 3609
<212> DNA
<213> Mus musculus

<400> 18

```
atgagtgtgc ggcctctggg aatctccaca cggatgtttg acactctcgg gaagaggtgc    60
tgcccttgga gactgaagcc aagcgccctg ctgttcctgt ttgttttatg tgttacctgt   120
gttcctctct cagtgtgcgg atgtggcagc tgcagacttg tcctgtccaa tccttccggt   180
acctttacgt ctccgtgtta ccctaatgac taccctaata cccagtcttg ttcgtggacc   240
ctccgagccc ctgccggcta catcattcag ataacgttca atgacttcga cattgaagaa   300
gctcccaact gtatctatga ctcattgtcc ctcgataatg gagagagcca gacaaaattc   360
tgtggagcga ctgccaaggg cctgtcattt aactccagcg tgaatgagat gcatgtgtcc   420
ttttcaagtg actttagtat ccagaagaaa ggtttcaacg ccagctacat cagagttgct   480
```

gtgtccttga ggaatcaaaa ggtcattttg ccccagacat tagatgctta ccaggtatca 540
gttgcaaaaa gcatctccat tcctgaactc aaagctttca cgctctgttt tgaagcctcc 600
aaagttggca atgaaggtgg tgactggaca gctttctcct actcagacga gtcccttaca 660
cagctgctca gtcttgaaaa ggccagtaat ggctacttcc tgtccatctc tggctcaaga 720
tgcttgttga acaatgcgtt acctgtgaag gacaaagagg acatcttcac agaaaacttg 780
gagcagctct gtcttgtgtg gaataattct tggggctccg ttggtataaa tttcaaaaag 840
aactatgaaa cagttccatg cgattccacc atcagtgctg tcgtacccgg ggatgggaca 900
ttgctgttgg gctccgacag agatgagatc gcctctctaa ggggcagcat ctataacttt 960
cgactttgga attttaccat ggatctgaaa gccctctcca acctcagctg tagtgtgtct 1020
gggaatgtca tagactggca caatgacttt tggagcatct caacccaagc tctgaaagcc 1080
gagggcaacc tgagctgtgg ttcctacctg atccagcttc ctgcagcaga gctgacaaac 1140
tgttcagaac tggggactct ctgtcaagct actgtaagcc ctccttctac tccaccaccc 1200
actgtcacca ctaatatccc cgttactaac agagtccata aacaaaagaa tgacggaata 1260
atgtatcgaa tatctgttgt gattcacaat gactttaatc accctgaagt aaaagtgcag 1320
accaaagtag cagaatggct caattcaacc tttcagaatt ggaactacac tgtttatgtg 1380
gttaatataa gttttcatca aaaagtagga gaggacagga tgaaagtcaa gagagacatc 1440
atggacgatg acaaaaggtt ggtgctctgg gcccttctag tctacaatgc taccaataac 1500
gtcagcctga atgaagagaa gattaaacaa aagcttatga caaataatgc atcactagag 1560
gatggactga ggctgtgtga agtcgacgtg aaccagctgg gtatgtgtag cgccttggag 1620
gatccagacg gctttagttg gccagccacc ttaccctctg tctacaaaca accatgtcca 1680
aacaagcctg gctttttat gactcgagcg tgcctttcta atggaacatc aaccttctgg 1740
ggccctgttg acacttccaa ctgttcaaga caatcaaatg aagtggccaa tgagatttta 1800
aaccaaactg gtgatgggca gaacctcacc tcggctaata tcaacagcat tgtagaaaag 1860
gtcaaacgga tcgtgaacaa agaagaaaac attgacatca ccctcggctc cactctaatg 1920
aatatatttt ctaatatctt aagcagttca gatagcgatt tgcttgagtc ttctactgaa 1980
gctttaaaaa caattgacga gctagccttc aaaatagacc tgaatagcac cccacatgtg 2040
aacattgaga cacagaattt ggcccttgga gtctcatccc taattccagg aacaaatgca 2100
ccttcaaatt ttagcattgg ccttccaagc aataatgaat cgtacttcca gatggacttc 2160
gggaacggac agacagatcc actggcatct gtgattttgc ctccaaattt gcttgagaat 2220
ttaagccccg aagattctgt attggtcagg agagcacagt tcactttctt caacaaaacc 2280
ggacttttcc aggatgttgg atctcaaaga aaagtcctcg tgagttatgt gatggcgtgc 2340
agcattggaa acattactat ccagaatctg aaagatccgg ttcaaatcaa aatcaaacac 2400
accagaacac aggaagtgca tcatcctatc tgtgccttct gggatatgaa caaaaacaaa 2460
agtttcgggg ggtggaacac ctcaggatgt gttgcccact ctgatttgga cgctggtgag 2520
accatttgtc tgtgcagcca cttcactcac tttggagttc tgatggatct tccaaggagt 2580
gcctcacaaa tagatggaag aaacacaaaa gtcctcacgt tcattaccta tattgggtgc 2640
ggaatatctg ccattttctc agctgcaact ctcctgacat atgttgcttt tgagaagctg 2700
cgcagggatt atccctccaa aatcctgatg aatctgagct cggccttgct cttcctgaat 2760
ctcatcttcc tcctggatgg ctgggtcact tcctttggcg tggctggact ctgcacggct 2820
gtggctgccc tgttgcactt cttcctcctg gctaccttca cctggatggg gctggaagcc 2880
atccacatgt acattgctct tgtgaaagtg tttaacactt acatccaccg ctatattcta 2940
aaattctgca tcataggctg gggtctgcca gccttggtgg tgtcaattat tctagtgagc 3000
agaagacaaa atgaagtata tggaaaagaa agttatggga aagatcagga tgatgaattc 3060
tgctggattc aggatcctgt ggtgttttat gtgagctgtg ccgggtactt cggagtcatg 3120
ttcttcctga atgtcgccat gttcattgtg gtcatggtgc agatctgtgg gaggaatgga 3180
aagagaagca accggaccct gagagaagag gttttaagaa acctgcgcag tgtggtcagc 3240
ctgaccttcc tgcttggcat gacgtggggg tttgctttct ttgcctgggg accccttaaat 3300
attcctttca tgtacctctt ctccatcttc aattcattac aaggtttatt tatattcatc 3360
ttccactgtg cgatgaagga gaatgttcag aaacagtgga ggcgtcacct ctgctgtggc 3420
aggtttcggc tagcagacaa ctcagattgg agtaagacag ctaccaatat catcaagaag 3480
agctccgata acctggggaa atctttgtct tcaagctcca ttggctccaa ttcaacatat 3540
ctcacatcca aatcaaagtc cagctccact acctatttca aaagaaacag ccactcggat 3600
aatttctcc 3609

<210> 19
<211> 1258
<212> PRT
<213> Mus musculus

<400> 19
Met Ser Val Arg Pro Leu Gly Ile Ser Thr Arg Met Phe Asp Thr Leu
5 10 15
Gly Lys Arg Cys Cys Pro Trp Arg Leu Lys Pro Ser Ala Leu Leu Phe
20 25 30
Leu Phe Val Leu Cys Val Thr Cys Val Pro Leu Ser Val Cys Gly Cys
35 40 45
Gly Ser Cys Arg Leu Val Leu Ser Asn Pro Ser Gly Thr Phe Thr Ser
50 55 60

```
Pro Cys Tyr Pro Asn Asp Tyr Pro Asn Thr Gln Ser Cys Ser Trp Thr
65                  70                  75                  80
Leu Arg Ala Pro Ala Gly Tyr Ile Ile Gln Ile Thr Phe Asn Asp Phe
                85                  90                  95
Asp Ile Glu Glu Ala Pro Asn Cys Ile Tyr Asp Ser Leu Ser Leu Asp
            100                 105                 110
Asn Gly Glu Ser Gln Thr Lys Phe Cys Gly Ala Thr Ala Lys Gly Leu
        115                 120                 125
Ser Phe Asn Ser Ser Val Asn Glu Met His Val Ser Phe Ser Ser Asp
    130                 135                 140
Phe Ser Ile Gln Lys Lys Gly Phe Asn Ala Ser Tyr Ile Arg Val Ala
145                 150                 155                 160
Val Ser Leu Arg Asn Gln Lys Val Ile Leu Pro Gln Thr Leu Asp Ala
                165                 170                 175
Tyr Gln Val Ser Val Ala Lys Ser Ile Ser Ile Pro Glu Leu Lys Ala
            180                 185                 190
Phe Thr Leu Cys Phe Glu Ala Ser Lys Val Gly Asn Glu Gly Gly Asp
        195                 200                 205
Trp Thr Ala Phe Ser Tyr Ser Asp Glu Ser Leu Thr Gln Leu Leu Ser
    210                 215                 220
Leu Glu Lys Ala Ser Asn Gly Tyr Phe Leu Ser Ile Ser Gly Ser Arg
225                 230                 235                 240
Cys Leu Leu Asn Asn Ala Leu Pro Val Lys Asp Lys Glu Asp Ile Phe
                245                 250                 255
Thr Glu Asn Leu Glu Gln Leu Cys Leu Val Trp Asn Asn Ser Trp Gly
            260                 265                 270
Ser Val Gly Ile Asn Phe Lys Lys Asn Tyr Glu Thr Val Pro Cys Asp
        275                 280                 285
Ser Thr Ile Ser Ala Val Val Pro Gly Asp Gly Thr Leu Leu Leu Gly
    290                 295                 300
Ser Asp Arg Asp Glu Ile Ala Ser Leu Arg Gly Ser Ile Tyr Asn Phe
305                 310                 315                 320
Arg Leu Trp Asn Phe Thr Met Asp Leu Lys Ala Leu Ser Asn Leu Ser
                325                 330                 335
Cys Ser Val Ser Gly Asn Val Ile Asp Trp His Asn Asp Phe Trp Ser
            340                 345                 350
Ile Ser Thr Gln Ala Leu Lys Ala Glu Gly Asn Leu Ser Cys Gly Ser
        355                 360                 365
Tyr Leu Ile Gln Leu Pro Ala Ala Glu Leu Thr Asn Cys Ser Glu Leu
    370                 375                 380
Gly Thr Leu Cys Gln Ala Thr Val Ser Pro Pro Ser Thr Pro Pro Pro
385                 390                 395                 400
Thr Val Thr Thr Asn Ile Pro Val Thr Asn Arg Val His Lys Gln Lys
                405                 410                 415
Asn Asp Gly Ile Met Tyr Arg Ile Ser Val Val Ile His Asn Asp Phe
            420                 425                 430
Asn His Pro Glu Val Lys Val Gln Thr Lys Val Ala Glu Trp Leu Asn
        435                 440                 445
Ser Thr Phe Gln Asn Trp Asn Tyr Thr Val Tyr Val Val Asn Ile Ser
    450                 455                 460
Phe His Gln Lys Val Gly Glu Asp Arg Met Lys Val Lys Arg Asp Ile
465                 470                 475                 480
Met Asp Asp Asp Lys Arg Leu Val Leu Trp Ala Leu Leu Val Tyr Asn
                485                 490                 495
Ala Thr Asn Asn Val Ser Leu Asn Glu Glu Lys Ile Lys Gln Lys Leu
            500                 505                 510
Met Thr Asn Asn Ala Ser Leu Glu Asp Gly Leu Arg Leu Cys Glu Val
        515                 520                 525
Asp Val Asn Gln Leu Gly Met Cys Ser Ala Leu Glu Asp Pro Asp Gly
    530                 535                 540
Phe Ser Trp Pro Ala Thr Leu Pro Ser Val Tyr Lys Gln Pro Cys Pro
545                 550                 555                 560
Asn Lys Pro Gly Phe Phe Met Thr Arg Ala Cys Leu Ser Asn Gly Thr
                565                 570                 575
Ser Thr Phe Trp Gly Pro Val Asp Thr Ser Asn Cys Ser Arg Gln Ser
            580                 585                 590
Asn Glu Val Ala Asn Glu Ile Leu Asn Gln Thr Gly Asp Gly Gln Asn
        595                 600                 605
```

Leu Thr Ser Ala Asn Ile Asn Ser Ile Val Glu Lys Val Lys Arg Ile
610 615 620
Val Asn Lys Glu Glu Asn Ile Asp Ile Thr Leu Gly Ser Thr Leu Met
625 630 635 640
Asn Ile Phe Ser Asn Ile Leu Ser Ser Ser Asp Ser Asp Leu Leu Glu
645 650 655
Ser Ser Thr Glu Ala Leu Lys Thr Ile Asp Glu Leu Ala Phe Lys Ile
660 665 670
Asp Leu Asn Ser Thr Pro His Val Asn Ile Glu Thr Gln Asn Leu Ala
675 680 685
Leu Gly Val Ser Ser Leu Ile Pro Gly Thr Asn Ala Pro Ser Asn Phe
690 695 700
Ser Ile Gly Leu Pro Ser Asn Asn Glu Ser Tyr Phe Gln Met Asp Phe
705 710 715 720
Gly Asn Gly Gln Thr Asp Pro Leu Ala Ser Val Ile Leu Pro Pro Asn
725 730 735
Leu Leu Glu Asn Leu Ser Pro Glu Asp Ser Val Leu Val Arg Arg Ala
740 745 750
Gln Phe Thr Phe Phe Asn Lys Thr Gly Leu Phe Gln Asp Val Gly Ser
755 760 765
Gln Arg Lys Val Leu Val Ser Tyr Val Met Ala Cys Ser Ile Gly Asn
770 775 780
Ile Thr Ile Gln Asn Leu Lys Asp Pro Val Gln Ile Lys Ile Lys His
785 790 795 800
Thr Arg Thr Gln Glu Val His His Pro Ile Cys Ala Phe Trp Asp Met
805 810 815
Asn Lys Asn Lys Ser Phe Gly Gly Trp Asn Thr Ser Gly Cys Val Ala
820 825 830
His Ser Asp Leu Asp Ala Gly Glu Thr Ile Cys Leu Cys Ser His Phe
835 840 845
Thr His Phe Gly Val Leu Met Asp Leu Pro Arg Ser Ala Ser Gln Ile
850 855 860
Asp Gly Arg Asn Thr Lys Val Leu Thr Phe Ile Thr Tyr Ile Gly Cys
865 870 875 880
Gly Ile Ser Ala Ile Phe Ser Ala Ala Thr Leu Leu Thr Tyr Val Ala
885 890 895
Phe Glu Lys Leu Arg Arg Asp Tyr Pro Ser Lys Ile Leu Met Asn Leu
900 905 910
Ser Ser Ala Leu Leu Phe Leu Asn Leu Ile Phe Leu Leu Asp Gly Trp
915 920 925
Val Thr Ser Phe Gly Val Ala Gly Leu Cys Thr Ala Val Ala Ala Leu
930 935 940
Leu His Phe Phe Leu Leu Ala Thr Phe Thr Trp Met Gly Leu Glu Ala
945 950 955 960
Ile His Met Tyr Ile Ala Leu Val Lys Val Phe Asn Thr Tyr Ile His
965 970 975
Arg Tyr Ile Leu Lys Phe Cys Ile Ile Gly Trp Gly Leu Pro Ala Leu
980 985 990
Val Val Ser Ile Ile Leu Val Ser Arg Arg Gln Asn Glu Val Tyr Gly
995 1000 1005
Lys Glu Ser Tyr Gly Lys Asp Gln Asp Asp Glu Phe Cys Trp Ile Gln
1010 1015 1020
Asp Pro Val Val Phe Tyr Val Ser Cys Ala Gly Tyr Phe Gly Val Met
1025 1030 1035 1040
Phe Phe Leu Asn Val Ala Met Phe Ile Val Val Met Val Gln Ile Cys
1045 1050 1055
Gly Arg Asn Gly Lys Arg Ser Asn Arg Thr Leu Arg Glu Glu Val Leu
1060 1065 1070
Arg Asn Leu Arg Ser Val Val Ser Leu Thr Phe Leu Leu Gly Met Thr
1075 1080 1085
Trp Gly Phe Ala Phe Phe Ala Trp Gly Pro Leu Asn Ile Pro Phe Met
1090 1095 1100
Tyr Leu Phe Ser Ile Phe Asn Ser Leu Gln Gly Leu Phe Ile Phe Ile
1105 1110 1115 1120
Phe His Cys Ala Met Lys Glu Asn Val Gln Lys Gln Trp Arg Arg His
1125 1130 1135
Leu Cys Cys Gly Arg Phe Arg Leu Ala Asp Asn Ser Asp Trp Ser Lys
1140 1145 1150

Thr Ala Thr Asn Ile Ile Lys Lys Ser Ser Asp Asn Leu Gly Lys Ser
1155 1160 1165
Leu Ser Ser Ser Ser Ile Gly Ser Asn Ser Thr Tyr Leu Thr Ser Lys
1170 1175 1180
Ser Lys Ser Ser Ser Thr Thr Tyr Phe Lys Arg Asn Ser His Ser Asp
1185 1190 1195 1200
Ser Thr Ser Thr Asp Lys Ser Leu Ser Lys Leu Thr His Ala Gly Gly
1205 1210 1215
Glu Gln Thr Ser Ile Ile Pro Val His Gln Val Ile Asp Lys Val Lys
1220 1225 1230
Gly Tyr Cys Asn Ala His Ser Asp Asn Phe Tyr Lys Asn Ile Ile Leu
1235 1240 1245
Ser Asp Ala Phe Ser His Ser Thr Lys Phe
1250 1255

<210> 20
<211> 3774
<212> DNA
<213> Mus musculus

<400> 20
atgagtgtgc ggcctctggg aatctccaca cggatgtttg acactctcgg gaagaggtgc 60
tgcccttgga gactgaagcc aagcgccctg ctgttcctgt ttgttttatg tgttacctgt 120
gttcctctct cagtgtgcgg atgtggcagc tgcagacttg tcctgtccaa tccttccggt 180
acctttacgt ctccgtgtta ccctaatgac taccctaata cccagtcttg ttcgtggacc 240
ctccgagccc ctgccggcta catcattcag ataacgttca atgacttcga cattgaagaa 300
gctcccaact gtatctatga ctcattgtcc ctcgataatg gagagagcca gacaaaattc 360
tgtggagcga ctgccaaggg cctgtcattt aactccagcg tgaatgagat gcatgtgtcc 420
ttttcaagtg actttagtat ccagaagaaa ggtttcaacg ccagctacat cagagttgct 480
gtgtccttga ggaatcaaaa ggtcattttg ccccagacat tagatgctta ccaggtatca 540
gttgcaaaaa gcatctccat tcctgaactc aaagctttca cgctctgttt tgaagcctcc 600
aaagttggca atgaaggtgg tgactggaca gctttctcct actcagacga gtcccttaca 660
cagctgctca gtcttgaaaa ggccagtaat ggctacttcc tgtccatctc tggctcaaga 720
tgcttgttga acaatgcgtt acctgtgaag gacaaagagg acatcttcac agaaaacttg 780
gagcagctct gtcttgtgtg gaataattct tggggctccg ttggtataaa tttcaaaaag 840
aactatgaaa cagttccatg cgattccacc atcagtgctg tcgtacccgg ggatgggaca 900
ttgctgttgg gctccgacag agatgagatc gcctctctaa ggggcagcat ctataacttt 960
cgactttgga attttaccat ggatctgaaa gccctctcca acctcagctg tagtgtgtct 1020
gggaatgtca tagactggca caatgacttt tggagcatct caacccaagc tctgaaagcc 1080
gagggcaacc tgagctgtgg ttcctacctg atccagcttc ctgcagcaga gctgacaaac 1140
tgttcagaac tggggactct ctgtcaagct actgtaagcc ctccttctac tccaccaccc 1200
actgtcacca ctaatatccc cgttactaac agagtccata aacaaaagaa tgacggaata 1260
atgtatcgaa tatctgttgt gattcacaat gactttaatc accctgaagt aaaagtgcag 1320
accaaagtag cagaatggct caattcaacc tttcagaatt ggaactacac tgtttatgtg 1380
gttaatataa gttttcatca aaaagtagga gaggacagga tgaaagtcaa gagagacatc 1440
atggacgatg acaaaaggtt ggtgctctgg gcccttctag tctacaatgc taccaataac 1500
gtcagcctga atgaagagaa gattaaacaa aagcttatga caaataatgc atcactagag 1560
gatggactga ggctgtgtga agtcgacgtg aaccagctgg gtatgtgtag cgccttggag 1620
gatccagacg gctttagttg gccagccacc ttaccctctg tctacaaaca accatgtcca 1680
aacaagcctg gctttttat gactcgagcg tgcctttcta atggaacatc aaccttctgg 1740
ggccctgttg acacttccaa ctgttcaaga caatcaaatg aagtggccaa tgagatttta 1800
aaccaaactg gtgatgggca gaacctcacc tcggctaata tcaacagcat tgtagaaaag 1860
gtcaaacgga tcgtgaacaa agaagaaaac attgacatca ccctcggctc cactctaatg 1920
aatatatttt ctaatatctt aagcagttca gatagcgatt tgcttgagtc ttctactgaa 1980
gctttaaaaa caattgacga gctagccttc aaaatagacc tgaatagcac cccacatgtg 2040
aacattgaga cacagaattt ggcccttgga gtctcatccc taattccagg aacaaatgca 2100
ccttcaaatt ttagcattgg ccttccaagc aataatgaat cgtacttcca gatggacttc 2160
gggaacggac agacagatcc actggcatct gtgattttgc ctccaaattt gcttgagaat 2220
ttaagccccg aagattctgt attggtcagg agagcacagt tcactttctt caacaaaacc 2280
ggacttttcc aggatgttgg atctcaaaga aaagtcctcg tgagttatgt gatggcgtgc 2340
agcattggaa acattactat ccagaatctg aaagatccgg ttcaaatcaa aatcaaacac 2400
accagaacac aggaagtgca tcatcctatc tgtgccttct gggatatgaa caaaaacaaa 2460
agtttcgggg ggtggaacac ctcaggatgt gttgcccact ctgatttgga cgctggtgag 2520
accatttgtc tgtgcagcca cttcactcac tttggagttc tgatggatct tccaaggagt 2580
gcctcacaaa tagatggaag aaacacaaaa gtcctcacgt tcattaccta tattgggtgc 2640
ggaatatctg ccatttctc agctgcaact ctcctgacat atgttgcttt tgagaagctg 2700
cgcagggatt atccctccaa aatcctgatg aatctgagct cggccttgct cttcctgaat 2760
ctcatcttcc tcctggatgg ctgggtcact tcctttggcg tggctggact ctgcacggct 2820

```
gtggctgccc tgttgcactt cttcctcctg gctaccttca cctggatggg gctggaagcc 2880
atccacatgt acattgctct tgtgaaagtg tttaacactt acatccaccg ctatattcta 2940
aaattctgca tcataggctg gggtctgcca gccttggtgg tgtcaattat tctagtgagc 3000
agaagacaaa atgaagtata tggaaaagaa agttatggga aagatcagga tgatgaattc 3060
tgctggattc aggatcctgt ggtgttttat gtgagctgtg ccgggtactt cggagtcatg 3120
ttcttcctga atgtcgccat gttcattgtg gtcatggtgc agatctgtgg gaggaatgga 3180
aagagaagca accggaccct gagagaagag gttttaagaa acctgcgcag tgtggtcagc 3240
ctgaccttcc tgcttggcat gacgtggggg tttgctttct ttgcctgggg acccttaaat 3300
attcctttca tgtacctctt ctccatcttc aattcattac aaggtttatt tatattcatc 3360
ttccactgtg cgatgaagga gaatgttcag aaacagtgga ggcgtcacct ctgctgtggc 3420
aggtttcggc tagcagacaa ctcagattgg agtaagacag ctaccaatat catcaagaag 3480
agctccgata acctggggaa atctttgtct tcaagctcca ttggctccaa ttcaacatat 3540
ctcacatcca aatcaaagtc cagctccact acctatttca aaagaaacag ccactcggac 3600
agcacttcca cggacaagtc cctgtcaaaa ctgacccatg ctggtggaga acagacgtca 3660
atcattcccg tccatcaggt tattgataag gtcaagggtt actgtaatgc ccattccgat 3720
aacttctata aaaatatcat cctgtcagat gccttcagcc acagcacaaa gttt       3774
```

<210> 21
<211> 1165
<212> PRT
<213> Mus musculus

<400> 21

```
Met Met Phe Asp Thr Leu Gly Lys Arg Cys Cys Pro Trp Arg Leu Lys
                5                   10                  15
Pro Ser Ala Leu Leu Phe Leu Phe Val Leu Cys Val Thr Cys Val Pro
            20                  25                  30
Leu Ser Val Cys Gly Cys Gly Ser Cys Arg Leu Val Leu Ser Asn Pro
        35                  40                  45
Ser Gly Thr Phe Thr Ser Pro Cys Tyr Pro Asn Asp Tyr Pro Asn Thr
    50                  55                  60
Gln Ser Cys Ser Trp Thr Leu Arg Ala Pro Ala Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Val Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Leu Asp Ala Tyr Gln Val Ser Val Ala Lys Ser Ile Ser
                165                 170                 175
Ile Pro Glu Leu Lys Ala Phe Thr Leu Cys Phe Glu Ala Ser Lys Val
            180                 185                 190
Gly Asn Glu Gly Gly Asp Trp Thr Ala Phe Ser Tyr Ser Asp Glu Ser
        195                 200                 205
Leu Thr Gln Leu Leu Ser Leu Glu Lys Ala Ser Asn Gly Tyr Phe Leu
    210                 215                 220
Ser Ile Ser Gly Ser Arg Cys Leu Leu Asn Asn Ala Leu Pro Val Lys
225                 230                 235                 240
Asp Lys Glu Asp Ile Phe Thr Glu Asn Leu Glu Gln Leu Cys Leu Val
                245                 250                 255
Trp Asn Asn Ser Trp Gly Ser Val Gly Ile Asn Phe Lys Lys Asn Tyr
            260                 265                 270
Glu Thr Val Pro Cys Asp Ser Thr Ile Ser Ala Val Val Pro Gly Asp
        275                 280                 285
Gly Thr Leu Leu Leu Gly Ser Asp Arg Asp Glu Ile Ala Ser Leu Arg
    290                 295                 300
Gly Ser Ile Tyr Asn Phe Arg Leu Trp Asn Phe Thr Met Asp Leu Lys
305                 310                 315                 320
Ala Leu Ser Asn Leu Ser Cys Ser Val Ser Gly Asn Val Ile Asp Trp
                325                 330                 335
His Asn Asp Phe Trp Ser Ile Ser Thr Gln Ala Leu Lys Ala Glu Gly
            340                 345                 350
Asn Leu Ser Cys Gly Ser Tyr Leu Ile Gln Leu Pro Ala Ala Glu Leu
```

```
                355                 360                 365
Thr Asn Cys Ser Glu Leu Gly Thr Leu Cys Gln Asp Gly Ile Met Tyr
    370                 375                 380
Arg Ile Ser Val Val Ile His Asn Asp Phe Asn His Pro Glu Val Lys
385                 390                 395                 400
Val Gln Thr Lys Val Ala Glu Trp Leu Asn Ser Thr Phe Gln Asn Trp
                405                 410                 415
Asn Tyr Thr Val Tyr Val Val Asn Ile Ser Phe His Gln Lys Val Gly
            420                 425                 430
Glu Asp Arg Met Lys Val Lys Arg Asp Ile Met Asp Asp Asp Lys Arg
        435                 440                 445
Leu Val Leu Trp Ala Leu Leu Val Tyr Asn Ala Thr Asn Asn Val Ser
    450                 455                 460
Leu Asn Glu Glu Lys Ile Lys Gln Lys Leu Met Thr Asn Asn Ala Ser
465                 470                 475                 480
Leu Glu Asp Gly Leu Arg Leu Cys Glu Val Asp Val Asn Gln Leu Gly
                485                 490                 495
Met Cys Ser Ala Leu Glu Asp Pro Asp Gly Phe Ser Trp Pro Ala Thr
            500                 505                 510
Leu Pro Ser Val Tyr Lys Gln Pro Cys Pro Asn Lys Pro Gly Phe Phe
        515                 520                 525
Met Thr Arg Ala Cys Leu Ser Asn Gly Thr Ser Thr Phe Trp Gly Pro
    530                 535                 540
Val Asp Thr Ser Asn Cys Ser Arg Gln Ser Asn Glu Val Ala Asn Glu
545                 550                 555                 560
Ile Leu Asn Gln Thr Gly Asp Gly Gln Asn Leu Thr Ser Ala Asn Ile
                565                 570                 575
Asn Ser Ile Val Glu Lys Val Lys Arg Ile Val Asn Lys Glu Glu Asn
            580                 585                 590
Ile Asp Ile Thr Leu Gly Ser Thr Leu Met Asn Ile Phe Ser Asn Ile
        595                 600                 605
Leu Ser Ser Ser Asp Ser Asp Leu Leu Glu Ser Ser Thr Glu Ala Leu
    610                 615                 620
Lys Thr Ile Asp Glu Leu Ala Phe Lys Ile Asp Leu Asn Ser Thr Pro
625                 630                 635                 640
His Val Asn Ile Glu Thr Gln Asn Leu Ala Leu Gly Val Ser Ser Leu
                645                 650                 655
Ile Pro Gly Thr Asn Ala Pro Ser Asn Phe Ser Ile Gly Leu Pro Ser
            660                 665                 670
Asn Asn Glu Ser Tyr Phe Gln Met Asp Phe Gly Asn Gly Gln Thr Asp
        675                 680                 685
Pro Leu Ala Ser Val Ile Leu Pro Pro Asn Leu Leu Glu Asn Leu Ser
    690                 695                 700
Pro Glu Asp Ser Val Leu Val Arg Arg Ala Gln Phe Thr Phe Phe Asn
705                 710                 715                 720
Lys Thr Gly Leu Phe Gln Asp Val Gly Ser Gln Arg Lys Val Leu Val
                725                 730                 735
Ser Tyr Val Met Ala Cys Ser Ile Gly Asn Ile Thr Ile Gln Asn Leu
            740                 745                 750
Lys Asp Pro Val Gln Ile Lys Ile Lys His Thr Arg Thr Gln Glu Val
        755                 760                 765
His His Pro Ile Cys Ala Phe Trp Asp Met Asn Lys Asn Lys Ser Phe
    770                 775                 780
Gly Gly Trp Asn Thr Ser Gly Cys Val Ala His Ser Asp Leu Asp Ala
785                 790                 795                 800
Gly Glu Thr Ile Cys Leu Cys Ser His Phe Thr His Phe Gly Val Leu
                805                 810                 815
Met Asp Leu Pro Arg Ser Ala Ser Gln Ile Asp Gly Arg Asn Thr Lys
            820                 825                 830
Val Leu Thr Phe Ile Thr Tyr Ile Gly Cys Gly Ile Ser Ala Ile Phe
        835                 840                 845
Ser Ala Ala Thr Leu Leu Thr Tyr Val Ala Phe Glu Lys Leu Arg Arg
    850                 855                 860
Asp Tyr Pro Ser Lys Ile Leu Met Asn Leu Ser Ser Ala Leu Leu Phe
865                 870                 875                 880
Leu Asn Leu Ile Phe Leu Leu Asp Gly Trp Val Thr Ser Phe Gly Val
                885                 890                 895
Ala Gly Leu Cys Thr Ala Val Ala Ala Leu Leu His Phe Phe Leu Leu
```

```
                900                     905                     910
Ala Thr Phe Thr Trp Met Gly Leu Glu Ala Ile His Met Tyr Ile Ala
            915                     920                     925
Leu Val Lys Val Phe Asn Thr Tyr Ile His Arg Tyr Ile Leu Lys Phe
        930                     935                     940
Cys Ile Ile Gly Trp Gly Leu Pro Ala Leu Val Val Ser Ile Ile Leu
945                     950                     955                     960
Val Ser Arg Arg Gln Asn Glu Val Tyr Gly Lys Glu Ser Tyr Gly Lys
                965                     970                     975
Asp Gln Asp Asp Glu Phe Cys Trp Ile Gln Asp Pro Val Val Phe Tyr
            980                     985                     990
Val Ser Cys Ala Gly Tyr Phe Gly Val Met Phe Phe Leu Asn Val Ala
        995                     1000                    1005
Met Phe Ile Val Val Met Val Gln Ile Cys Gly Arg Asn Gly Lys Arg
    1010                    1015                    1020
Ser Asn Arg Thr Leu Arg Glu Glu Val Leu Arg Asn Leu Arg Ser Val
1025                    1030                    1035                    1040
Val Ser Leu Thr Phe Leu Leu Gly Met Thr Trp Gly Phe Ala Phe Phe
                1045                    1050                    1055
Ala Trp Gly Pro Leu Asn Ile Pro Phe Met Tyr Leu Phe Ser Ile Phe
            1060                    1065                    1070
Asn Ser Leu Gln Gly Leu Phe Ile Phe Ile Phe His Cys Ala Met Lys
        1075                    1080                    1085
Glu Asn Val Gln Lys Gln Trp Arg Arg His Leu Cys Cys Gly Arg Phe
    1090                    1095                    1100
Arg Leu Ala Asp Asn Ser Asp Trp Ser Lys Thr Ala Thr Asn Ile Ile
1105                    1110                    1115                    1120
Lys Lys Ser Ser Asp Asn Leu Gly Lys Ser Leu Ser Ser Ser Ser Ile
                1125                    1130                    1135
Gly Ser Asn Ser Thr Tyr Leu Thr Ser Lys Ser Lys Ser Ser Ser Thr
            1140                    1145                    1150
Thr Tyr Phe Lys Arg Asn Ser His Ser Asp Asn Phe Ser
        1155                    1160                    1165
```

<210> 22
<211> 3495
<212> DNA
<213> Mus musculus

<400> 22

```
atgatgtttg acactctcgg gaagaggtgc tgcccttgga gactgaagcc aagcgccctg     60
ctgttcctgt ttgttttatg tgttacctgt gttcctctct cagtgtgcgg atgtggcagc    120
tgcagacttg tcctgtccaa tccttccggt acctttacgt ctccgtgtta ccctaatgac    180
taccctaata cccagtcttg ttcgtggacc ctccgagccc ctgccggcta catcattcag    240
ataacgttca atgacttcga cattgaagaa gctcccaact gtatctatga ctcattgtcc    300
ctcgataatg gagagagcca gacaaaattc tgtggagcga ctgccaaggg cctgtcattt    360
aactccagcg tgaatgagat gcatgtgtcc ttttcaagtg actttagtat ccagaagaaa    420
ggtttcaacg ccagctacat cagagttgct gtgtccttga ggaatcaaaa ggtcattttg    480
ccccagacat tagatgctta ccaggtatca gttgcaaaaa gcatctccat tcctgaactc    540
aaagctttca cgctctgttt tgaagcctcc aaagttggca atgaaggtgg tgactggaca    600
gctttctcct actcagacga gtcccttaca cagctgctca gtcttgaaaa ggccagtaat    660
ggctacttcc tgtccatctc tggctcaaga tgcttgttga acaatgcgtt acctgtgaag    720
gacaaagagg acatcttcac agaaaacttg gagcagctct gtcttgtgtg gaataattct    780
tggggctccg ttggtataaa tttcaaaaag aactatgaaa cagttccatg cgattccacc    840
atcagtgctg tcgtacccgg ggatgggaca ttgctgttgg gctccgacag agatgagatc    900
gcctctctaa ggggcagcat ctataacttt cgactttgga attttaccat ggatctgaaa    960
gccctctcca acctcagctg tagtgtgtct gggaatgtca tagactggca caatgacttt   1020
tggagcatct caacccaagc tctgaaagcc gagggcaacc tgagctgtgg ttcctacctg   1080
atccagcttc ctgcagcaga gctgacaaac tgttcagaac tggggactct ctgtcaagac   1140
ggaataatgt atcgaatatc tgttgtgatt cacaatgact ttaatcaccc tgaagtaaaa   1200
gtgcagacca aagtagcaga atggctcaat tcaacctttc agaattggaa ctacactgtt   1260
tatgtggtta atataagttt tcatcaaaaa gtaggagagg acaggatgaa agtcaagaga   1320
gacatcatgg acgatgacaa aaggttggtg ctctgggccc ttctagtcta caatgctacc   1380
aataacgtca gcctgaatga agagaagatt aaacaaaagc ttatgacaaa taatgcatca   1440
ctagaggatg gactgaggct gtgtgaagtc gacgtgaacc agctgggtat gtgtagcgcc   1500
ttggaggatc cagacggctt tagttggcca gccaccttac cctctgtcta caaacaacca   1560
tgtccaaaca agcctggctt ttttatgact cgagcgtgcc tttctaatgg aacatcaacc   1620
ttctggggcc ctgttgacac ttccaactgt tcaagacaat caaatgaagt ggccaatgag   1680
```

attttaaacc aaactggtga tgggcagaac ctcacctcgg ctaatatcaa cagcattgta 1740
gaaaaggtca aacggatcgt gaacaaagaa gaaaacattg acatcaccct cggctccact 1800
ctaatgaata tattttctaa tatcttaagc agttcagata gcgatttgct tgagtcttct 1860
actgaagctt taaaaacaat tgacgagcta gccttcaaaa tagacctgaa tagcacccca 1920
catgtgaaca ttgagacaca gaatttggcc cttggagtct catccctaat tccaggaaca 1980
aatgcacctt caaattttag cattggcctt ccaagcaata atgaatcgta cttccagatg 2040
gacttcggga acggacagac agatccactg gcatctgtga ttttgcctcc aaatttgctt 2100
gagaatttaa gccccgaaga ttctgtattg gtcaggagag cacagttcac tttcttcaac 2160
aaaaccggac ttttccagga tgttggatct caaagaaaag tcctcgtgag ttatgtgatg 2220
gcgtgcagca ttggaaacat tactatccag aatctgaaag atccggttca aatcaaaatc 2280
aaacacacca gaacacagga agtgcatcat cctatctgtg ccttctggga tatgaacaaa 2340
aacaaaagtt tcgggggtg gaacacctca ggatgtgttg cccactctga tttggacgct 2400
ggtgagacca tttgtctgtg cagccacttc actcactttg gagttctgat ggatcttcca 2460
aggagtgcct cacaaataga tggaagaaac acaaaagtcc tcacgttcat tacctatatt 2520
gggtgcggaa tatctgccat ttttctcagct gcaactctcc tgacatatgt tgcttttgag 2580
aagctgcgca gggattatcc ctccaaaatc ctgatgaatc tgagctcggc cttgctcttc 2640
ctgaatctca tcttcctcct ggatggctgg gtcacttcct ttggcgtggc tggactctgc 2700
acggctgtgg ctgccctgtt gcacttcttc ctcctggcta ccttcacctg gatggggctg 2760
gaagccatcc acatgtacat tgctcttgtg aaagtgttta acacttacat ccaccgctat 2820
attctaaaat tctgcatcat aggctggggt ctgccagcct tggtggtgtc aattattcta 2880
gtgagcagaa gacaaaatga agtatatgga aaagaaagtt atgggaaaga tcaggatgat 2940
gaattctgct ggattcagga tcctgtggtg ttttatgtga gctgtgccgg gtacttcgga 3000
gtcatgttct tcctgaatgt cgccatgttc attgtggtca tggtgcagat ctgtgggagg 3060
aatggaaaga gaagcaaccg gaccctgaga gaagaggttt taagaaacct gcgcagtgtg 3120
gtcagcctga ccttcctgct tggcatgacg tgggggtttg ctttctttgc ctggggaccc 3180
ttaaatattc ctttcatgta cctcttctcc atcttcaatt cattacaagg tttatttata 3240
ttcatcttcc actgtgcgat gaaggagaat gttcagaaac agtggaggcg tcacctctgc 3300
tgtggcaggt ttcggctagc agacaactca gattggagta agacagctac caatatcatc 3360
aagaagagct ccgataacct ggggaaatct ttgtcttcaa gctccattgg ctccaattca 3420
acatatctca catccaaatc aaagtccagc tccactacct atttcaaaag aaacagccac 3480
tcggataatt tctcc 3495

<210> 23
<211> 1220
<212> PRT
<213> Mus musculus

<400> 23
Met Met Phe Asp Thr Leu Gly Lys Arg Cys Cys Pro Trp Arg Leu Lys
1 5 10 15
Pro Ser Ala Leu Leu Phe Leu Phe Val Leu Cys Val Thr Cys Val Pro
20 25 30
Leu Ser Val Cys Gly Cys Gly Ser Cys Arg Leu Val Leu Ser Asn Pro
35 40 45
Ser Gly Thr Phe Thr Ser Pro Cys Tyr Pro Asn Asp Tyr Pro Asn Thr
50 55 60
Gln Ser Cys Ser Trp Thr Leu Arg Ala Pro Ala Gly Tyr Ile Ile Gln
65 70 75 80
Ile Thr Phe Asn Asp Phe Asp Ile Glu Glu Ala Pro Asn Cys Ile Tyr
85 90 95
Asp Ser Leu Ser Leu Asp Asn Gly Glu Ser Gln Thr Lys Phe Cys Gly
100 105 110
Ala Thr Ala Lys Gly Leu Ser Phe Asn Ser Ser Val Asn Glu Met His
115 120 125
Val Ser Phe Ser Ser Asp Phe Ser Ile Gln Lys Lys Gly Phe Asn Ala
130 135 140
Ser Tyr Ile Arg Val Ala Val Ser Leu Arg Asn Gln Lys Val Ile Leu
145 150 155 160
Pro Gln Thr Leu Asp Ala Tyr Gln Val Ser Val Ala Lys Ser Ile Ser
165 170 175
Ile Pro Glu Leu Lys Ala Phe Thr Leu Cys Phe Glu Ala Ser Lys Val
180 185 190
Gly Asn Glu Gly Gly Asp Trp Thr Ala Phe Ser Tyr Ser Asp Glu Ser
195 200 205
Leu Thr Gln Leu Leu Ser Leu Glu Lys Ala Ser Asn Gly Tyr Phe Leu
210 215 220
Ser Ile Ser Gly Ser Arg Cys Leu Leu Asn Asn Ala Leu Pro Val Lys
225 230 235 240

```
Asp Lys Glu Asp Ile Phe Thr Glu Asn Leu Glu Gln Leu Cys Leu Val
                245                 250                 255
Trp Asn Asn Ser Trp Gly Ser Val Gly Ile Asn Phe Lys Lys Asn Tyr
            260                 265                 270
Glu Thr Val Pro Cys Asp Ser Thr Ile Ser Ala Val Val Pro Gly Asp
        275                 280                 285
Gly Thr Leu Leu Leu Gly Ser Asp Arg Asp Glu Ile Ala Ser Leu Arg
    290                 295                 300
Gly Ser Ile Tyr Asn Phe Arg Leu Trp Asn Phe Thr Met Asp Leu Lys
305                 310                 315                 320
Ala Leu Ser Asn Leu Ser Cys Ser Val Ser Gly Asn Val Ile Asp Trp
                325                 330                 335
His Asn Asp Phe Trp Ser Ile Ser Thr Gln Ala Leu Lys Ala Glu Gly
            340                 345                 350
Asn Leu Ser Cys Gly Ser Tyr Leu Ile Gln Leu Pro Ala Ala Glu Leu
        355                 360                 365
Thr Asn Cys Ser Glu Leu Gly Thr Leu Cys Gln Asp Gly Ile Met Tyr
    370                 375                 380
Arg Ile Ser Val Val Ile His Asn Asp Phe Asn His Pro Glu Val Lys
385                 390                 395                 400
Val Gln Thr Lys Val Ala Glu Trp Leu Asn Ser Thr Phe Gln Asn Trp
                405                 410                 415
Asn Tyr Thr Val Tyr Val Val Asn Ile Ser Phe His Gln Lys Val Gly
            420                 425                 430
Glu Asp Arg Met Lys Val Lys Arg Asp Ile Met Asp Asp Asp Lys Arg
        435                 440                 445
Leu Val Leu Trp Ala Leu Leu Val Tyr Asn Ala Thr Asn Asn Val Ser
    450                 455                 460
Leu Asn Glu Glu Lys Ile Lys Gln Lys Leu Met Thr Asn Asn Ala Ser
465                 470                 475                 480
Leu Glu Asp Gly Leu Arg Leu Cys Glu Val Asp Val Asn Gln Leu Gly
                485                 490                 495
Met Cys Ser Ala Leu Glu Asp Pro Asp Gly Phe Ser Trp Pro Ala Thr
            500                 505                 510
Leu Pro Ser Val Tyr Lys Gln Pro Cys Pro Asn Lys Pro Gly Phe Phe
        515                 520                 525
Met Thr Arg Ala Cys Leu Ser Asn Gly Thr Ser Thr Phe Trp Gly Pro
    530                 535                 540
Val Asp Thr Ser Asn Cys Ser Arg Gln Ser Asn Glu Val Ala Asn Glu
545                 550                 555                 560
Ile Leu Asn Gln Thr Gly Asp Gly Gln Asn Leu Thr Ser Ala Asn Ile
                565                 570                 575
Asn Ser Ile Val Glu Lys Val Lys Arg Ile Val Asn Lys Glu Glu Asn
            580                 585                 590
Ile Asp Ile Thr Leu Gly Ser Thr Leu Met Asn Ile Phe Ser Asn Ile
        595                 600                 605
Leu Ser Ser Ser Asp Ser Asp Leu Leu Glu Ser Ser Thr Glu Ala Leu
    610                 615                 620
Lys Thr Ile Asp Glu Leu Ala Phe Lys Ile Asp Leu Asn Ser Thr Pro
625                 630                 635                 640
His Val Asn Ile Glu Thr Gln Asn Leu Ala Leu Gly Val Ser Ser Leu
                645                 650                 655
Ile Pro Gly Thr Asn Ala Pro Ser Asn Phe Ser Ile Gly Leu Pro Ser
            660                 665                 670
Asn Asn Glu Ser Tyr Phe Gln Met Asp Phe Gly Asn Gly Gln Thr Asp
        675                 680                 685
Pro Leu Ala Ser Val Ile Leu Pro Pro Asn Leu Leu Glu Asn Leu Ser
    690                 695                 700
Pro Glu Asp Ser Val Leu Val Arg Arg Ala Gln Phe Thr Phe Phe Asn
705                 710                 715                 720
Lys Thr Gly Leu Phe Gln Asp Val Gly Ser Gln Arg Lys Val Leu Val
                725                 730                 735
Ser Tyr Val Met Ala Cys Ser Ile Gly Asn Ile Thr Ile Gln Asn Leu
            740                 745                 750
Lys Asp Pro Val Gln Ile Lys Ile Lys His Thr Arg Thr Gln Glu Val
        755                 760                 765
His His Pro Ile Cys Ala Phe Trp Asp Met Asn Lys Asn Lys Ser Phe
    770                 775                 780
```

Gly Gly Trp Asn Thr Ser Gly Cys Val Ala His Ser Asp Leu Asp Ala
785 790 795 800
Gly Glu Thr Ile Cys Leu Cys Ser His Phe Thr His Phe Gly Val Leu
805 810 815
Met Asp Leu Pro Arg Ser Ala Ser Gln Ile Asp Gly Arg Asn Thr Lys
820 825 830
Val Leu Thr Phe Ile Thr Tyr Ile Gly Cys Gly Ile Ser Ala Ile Phe
835 840 845
Ser Ala Ala Thr Leu Leu Thr Tyr Val Ala Phe Glu Lys Leu Arg Arg
850 855 860
Asp Tyr Pro Ser Lys Ile Leu Met Asn Leu Ser Ser Ala Leu Leu Phe
865 870 875 880
Leu Asn Leu Ile Phe Leu Leu Asp Gly Trp Val Thr Ser Phe Gly Val
885 890 895
Ala Gly Leu Cys Thr Ala Val Ala Ala Leu Leu His Phe Phe Leu Leu
900 905 910
Ala Thr Phe Thr Trp Met Gly Leu Glu Ala Ile His Met Tyr Ile Ala
915 920 925
Leu Val Lys Val Phe Asn Thr Tyr Ile His Arg Tyr Ile Leu Lys Phe
930 935 940
Cys Ile Ile Gly Trp Gly Leu Pro Ala Leu Val Val Ser Ile Ile Leu
945 950 955 960
Val Ser Arg Arg Gln Asn Glu Val Tyr Gly Lys Glu Ser Tyr Gly Lys
965 970 975
Asp Gln Asp Asp Glu Phe Cys Trp Ile Gln Asp Pro Val Val Phe Tyr
980 985 990
Val Ser Cys Ala Gly Tyr Phe Gly Val Met Phe Phe Leu Asn Val Ala
995 1000 1005
Met Phe Ile Val Val Met Val Gln Ile Cys Gly Arg Asn Gly Lys Arg
1010 1015 1020
Ser Asn Arg Thr Leu Arg Glu Glu Val Leu Arg Asn Leu Arg Ser Val
1025 1030 1035 1040
Val Ser Leu Thr Phe Leu Leu Gly Met Thr Trp Gly Phe Ala Phe Phe
1045 1050 1055
Ala Trp Gly Pro Leu Asn Ile Pro Phe Met Tyr Leu Phe Ser Ile Phe
1060 1065 1070
Asn Ser Leu Gln Gly Leu Phe Ile Phe Ile Phe His Cys Ala Met Lys
1075 1080 1085
Glu Asn Val Gln Lys Gln Trp Arg Arg His Leu Cys Cys Gly Arg Phe
1090 1095 1100
Arg Leu Ala Asp Asn Ser Asp Trp Ser Lys Thr Ala Thr Asn Ile Ile
1105 1110 1115 1120
Lys Lys Ser Ser Asp Asn Leu Gly Lys Ser Leu Ser Ser Ser Ser Ile
1125 1130 1135
Gly Ser Asn Ser Thr Tyr Leu Thr Ser Lys Ser Lys Ser Ser Ser Thr
1140 1145 1150
Thr Tyr Phe Lys Arg Asn Ser His Ser Asp Ser Thr Ser Thr Asp Lys
1155 1160 1165
Ser Leu Ser Lys Leu Thr His Ala Gly Gly Glu Gln Thr Ser Ile Ile
1170 1175 1180
Pro Val His Gln Val Ile Asp Lys Val Lys Gly Tyr Cys Asn Ala His
1185 1190 1195 1200
Ser Asp Asn Phe Tyr Lys Asn Ile Ile Leu Ser Asp Ala Phe Ser His
1205 1210 1215
Ser Thr Lys Phe
1220

<210> 24
<211> 3660
<212> DNA
<213> Mus musculus

<400> 24
atgatgtttg acactctcgg gaagaggtgc tgcccttgga gactgaagcc aagcgccctg 60
ctgttcctgt ttgttttatg tgttacctgt gttcctctct cagtgtgcgg atgtggcagc 120
tgcagacttg tcctgtccaa tccttccggt acctttacgt ctccgtgtta ccctaatgac 180
taccctaata cccagtcttg ttcgtggacc ctccgagccc ctgccggcta catcattcag 240
ataacgttca atgacttcga cattgaagaa gctcccaact gtatctatga ctcattgtcc 300

```
ctcgataatg gagagagcca gacaaaattc tgtggagcga ctgccaaggg cctgtcattt   360
aactccagcg tgaatgagat gcatgtgtcc ttttcaagtg actttagtat ccagaagaaa   420
ggtttcaacg ccagctacat cagagttgct gtgtccttga ggaatcaaaa ggtcattttg   480
ccccagacat tagatgctta ccaggtatca gttgcaaaaa gcatctccat tcctgaactc   540
aaagctttca cgctctgttt tgaagcctcc aaagttggca atgaaggtgg tgactggaca   600
gctttctcct actcagacga gtcccttaca cagctgctca gtcttgaaaa ggccagtaat   660
ggctacttcc tgtccatctc tggctcaaga tgcttgttga acaatgcgtt acctgtgaag   720
gacaaagagg acatcttcac agaaaacttg gagcagctct gtcttgtgtg gaataattct   780
tggggctccg ttggtataaa tttcaaaaag aactatgaaa cagttccatg cgattccacc   840
atcagtgctg tcgtacccgg ggatgggaca ttgctgttgg gctccgacag agatgagatc   900
gcctctctaa ggggcagcat ctataacttt cgactttgga attttaccat ggatctgaaa   960
gccctctcca acctcagctg tagtgtgtct gggaatgtca tagactggca caatgacttt  1020
tggagcatct caacccaagc tctgaaagcc gagggcaacc tgagctgtgg ttcctacctg  1080
atccagcttc ctgcagcaga gctgacaaac tgttcagaac tggggactct ctgtcaagac  1140
ggaataatgt atcgaatatc tgttgtgatt cacaatgact ttaatcaccc tgaagtaaaa  1200
gtgcagacca aagtagcaga atggctcaat tcaacctttc agaattggaa ctacactgtt  1260
tatgtggtta atataagttt tcatcaaaaa gtaggagagg acaggatgaa agtcaagaga  1320
gacatcatgg acgatgacaa aaggttggtg ctctgggccc ttctagtcta caatgctacc  1380
aataacgtca gcctgaatga agagaagatt aaacaaaagc ttatgacaaa taatgcatca  1440
ctagaggatg gactgaggct gtgtgaagtc gacgtgaacc agctgggtat gtgtagcgcc  1500
ttggaggatc cagacggctt tagttggcca gccaccttac cctctgtcta caaacaacca  1560
tgtccaaaca agcctggctt ttttatgact cgagcgtgcc tttctaatgg aacatcaacc  1620
ttctggggcc ctgttgacac ttccaactgt tcaagacaat caaatgaagt ggccaatgag  1680
attttaaacc aaactggtga tgggcagaac ctcacctcgg ctaatatcaa cagcattgta  1740
gaaaaggtca aacggatcgt gaacaaagaa gaaaacattg acatcaccct cggctccact  1800
ctaatgaata tattttctaa tatcttaagc agttcagata gcgatttgct tgagtcttct  1860
actgaagctt taaaaacaat tgacgagcta gccttcaaaa tagacctgaa tagcacccca  1920
catgtgaaca ttgagacaca gaatttggcc cttggagtct catccctaat tccaggaaca  1980
aatgcacctt caaattttag cattggcctt ccaagcaata atgaatcgta cttccagatg  2040
gacttcggga acggacagac agatccactg gcatctgtga ttttgcctcc aaatttgctt  2100
gagaatttaa gccccgaaga ttctgtattg gtcaggagag cacagttcac tttcttcaac  2160
aaaaccggac ttttccagga tgttggatct caaagaaaag tcctcgtgag ttatgtgatg  2220
gcgtgcagca ttggaaacat tactatccag aatctgaaag atccggttca aatcaaaatc  2280
aaacacacca gaacacagga agtgcatcat cctatctgtg ccttctggga tatgaacaaa  2340
aacaaaagtt tcgggggtg gaacacctca ggatgtgttg cccactctga tttggacgct  2400
ggtgagacca tttgtctgtg cagccacttc actcactttg gagttctgat ggatcttcca  2460
aggagtgcct cacaaataga tggaagaaac acaaaagtcc tcacgttcat tacctatatt  2520
gggtgcggaa tatctgccat ttctcagct gcaactctcc tgacatatgt tgcttttgag  2580
aagctgcgca gggattatcc ctccaaaatc ctgatgaatc tgagctcggc cttgctcttc  2640
ctgaatctca tcttcctcct ggatggctgg gtcacttcct ttggcgtggc tggactctgc  2700
acggctgtgg ctgccctgtt gcacttcttc ctcctggcta ccttcacctg gatggggctg  2760
gaagccatcc acatgtacat tgctcttgtg aaagtgttta acacttacat ccaccgctat  2820
attctaaaat tctgcatcat aggctggggt ctgccagcct tggtggtgtc aattattcta  2880
gtgagcagaa gacaaaatga agtatatgga aaagaaagtt atgggaaaga tcaggatgat  2940
gaattctgct ggattcagga tcctgtggtg ttttatgtga gctgtgccgg gtacttcgga  3000
gtcatgttct tcctgaatgt cgccatgttc attgtggtca tggtgcagat ctgtgggagg  3060
aatggaaaga gaagcaaccg gaccctgaga gaagaggttt taagaaacct gcgcagtgtg  3120
gtcagcctga ccttcctgct tggcatgacg tgggggtttg ctttctttgc ctggggaccc  3180
ttaaatattc ctttcatgta cctcttctcc atcttcaatt cattacaagg tttatttata  3240
ttcatcttcc actgtgcgat gaaggagaat gttcagaaac agtggaggcg tcacctctgc  3300
tgtggcaggt ttcggctagc agacaactca gattggagta agacagctac caatatcatc  3360
aagaagagct ccgataacct ggggaaatct ttgtcttcaa gctccattgg ctccaattca  3420
acatatctca catccaaatc aaagtccagc tccactacct atttcaaaag aaacagccac  3480
tcggacagca cttccacgga caagtccctg tcaaaactga cccatgctgg tggagaacag  3540
acgtcaatca ttcccgtcca tcaggttatt gataaggtca agggttactg taatgcccat  3600
tccgataact tctataaaaa tatcatcctg tcagatgcct tcagccacag cacaaagttt  3660
```

<210> 25
<211> 1175
<212> PRT
<213> Mus musculus

<400> 25

```
Met Ser Val Arg Pro Leu Gly Ile Ser Thr Arg Met Phe Asp Thr Leu
1               5                   10                  15
Gly Lys Arg Cys Cys Pro Trp Arg Leu Lys Pro Ser Ala Leu Leu Phe
            20                  25                  30
Leu Phe Val Leu Cys Val Thr Cys Val Pro Leu Ser Val Cys Gly Cys
```

```
        35                  40                  45
Gly Ser Cys Arg Leu Val Leu Ser Asn Pro Ser Gly Thr Phe Thr Ser
    50                  55                  60
Pro Cys Tyr Pro Asn Asp Tyr Pro Asn Thr Gln Ser Cys Ser Trp Thr
65                  70                  75                  80
Leu Arg Ala Pro Ala Gly Tyr Ile Ile Gln Ile Thr Phe Asn Asp Phe
                85                  90                  95
Asp Ile Glu Glu Ala Pro Asn Cys Ile Tyr Asp Ser Leu Ser Leu Asp
            100                 105                 110
Asn Gly Glu Ser Gln Thr Lys Phe Cys Gly Ala Thr Ala Lys Gly Leu
        115                 120                 125
Ser Phe Asn Ser Ser Val Asn Glu Met His Val Ser Phe Ser Ser Asp
    130                 135                 140
Phe Ser Ile Gln Lys Lys Gly Phe Asn Ala Ser Tyr Ile Arg Val Ala
145                 150                 155                 160
Val Ser Leu Arg Asn Gln Lys Val Ile Leu Pro Gln Thr Leu Asp Ala
                165                 170                 175
Tyr Gln Val Ser Val Ala Lys Ser Ile Ser Ile Pro Glu Leu Lys Ala
            180                 185                 190
Phe Thr Leu Cys Phe Glu Ala Ser Lys Val Gly Asn Glu Gly Gly Asp
        195                 200                 205
Trp Thr Ala Phe Ser Tyr Ser Asp Glu Ser Leu Thr Gln Leu Leu Ser
    210                 215                 220
Leu Glu Lys Ala Ser Asn Gly Tyr Phe Leu Ser Ile Ser Gly Ser Arg
225                 230                 235                 240
Cys Leu Leu Asn Asn Ala Leu Pro Val Lys Asp Lys Glu Asp Ile Phe
                245                 250                 255
Thr Glu Asn Leu Glu Gln Leu Cys Leu Val Trp Asn Asn Ser Trp Gly
            260                 265                 270
Ser Val Gly Ile Asn Phe Lys Lys Asn Tyr Glu Thr Val Pro Cys Asp
        275                 280                 285
Ser Thr Ile Ser Ala Val Val Pro Gly Asp Gly Thr Leu Leu Leu Gly
    290                 295                 300
Ser Asp Arg Asp Glu Ile Ala Ser Leu Arg Gly Ser Ile Tyr Asn Phe
305                 310                 315                 320
Arg Leu Trp Asn Phe Thr Met Asp Leu Lys Ala Leu Ser Asn Leu Ser
                325                 330                 335
Cys Ser Val Ser Gly Asn Val Ile Asp Trp His Asn Asp Phe Trp Ser
            340                 345                 350
Ile Ser Thr Gln Ala Leu Lys Ala Glu Gly Asn Leu Ser Cys Gly Ser
        355                 360                 365
Tyr Leu Ile Gln Leu Pro Ala Ala Glu Leu Thr Asn Cys Ser Glu Leu
    370                 375                 380
Gly Thr Leu Cys Gln Asp Gly Ile Met Tyr Arg Ile Ser Val Val Ile
385                 390                 395                 400
His Asn Asp Phe Asn His Pro Glu Val Lys Val Gln Thr Lys Val Ala
                405                 410                 415
Glu Trp Leu Asn Ser Thr Phe Gln Asn Trp Asn Tyr Thr Val Tyr Val
            420                 425                 430
Val Asn Ile Ser Phe His Gln Lys Val Gly Glu Asp Arg Met Lys Val
        435                 440                 445
Lys Arg Asp Ile Met Asp Asp Lys Arg Leu Val Leu Trp Ala Leu
    450                 455                 460
Leu Val Tyr Asn Ala Thr Asn Asn Val Ser Leu Asn Glu Glu Lys Ile
465                 470                 475                 480
Lys Gln Lys Leu Met Thr Asn Asn Ala Ser Leu Glu Asp Gly Leu Arg
                485                 490                 495
Leu Cys Glu Val Asp Val Asn Gln Leu Gly Met Cys Ser Ala Leu Glu
            500                 505                 510
Asp Pro Asp Gly Phe Ser Trp Pro Ala Thr Leu Pro Ser Val Tyr Lys
        515                 520                 525
Gln Pro Cys Pro Asn Lys Pro Gly Phe Phe Met Thr Arg Ala Cys Leu
    530                 535                 540
Ser Asn Gly Thr Ser Thr Phe Trp Gly Pro Val Asp Thr Ser Asn Cys
545                 550                 555                 560
Ser Arg Gln Ser Asn Glu Val Ala Asn Glu Ile Leu Asn Gln Thr Gly
                565                 570                 575
Asp Gly Gln Asn Leu Thr Ser Ala Asn Ile Asn Ser Ile Val Glu Lys
```

```
                580                       585                       590
Val Lys Arg Ile Val Asn Lys Glu Glu Asn Ile Asp Ile Thr Leu Gly
            595                 600                 605
Ser Thr Leu Met Asn Ile Phe Ser Asn Ile Leu Ser Ser Ser Asp Ser
        610                 615                 620
Asp Leu Leu Glu Ser Ser Thr Glu Ala Leu Lys Thr Ile Asp Glu Leu
625                 630                 635                 640
Ala Phe Lys Ile Asp Leu Asn Ser Thr Pro His Val Asn Ile Glu Thr
                645                 650                 655
Gln Asn Leu Ala Leu Gly Val Ser Ser Leu Ile Pro Gly Thr Asn Ala
            660                 665                 670
Pro Ser Asn Phe Ser Ile Gly Leu Pro Ser Asn Asn Glu Ser Tyr Phe
        675                 680                 685
Gln Met Asp Phe Gly Asn Gly Gln Thr Asp Pro Leu Ala Ser Val Ile
    690                 695                 700
Leu Pro Pro Asn Leu Leu Glu Asn Leu Ser Pro Glu Asp Ser Val Leu
705                 710                 715                 720
Val Arg Arg Ala Gln Phe Thr Phe Phe Asn Lys Thr Gly Leu Phe Gln
                725                 730                 735
Asp Val Gly Ser Gln Arg Lys Val Leu Val Ser Tyr Val Met Ala Cys
            740                 745                 750
Ser Ile Gly Asn Ile Thr Ile Gln Asn Leu Lys Asp Pro Val Gln Ile
        755                 760                 765
Lys Ile Lys His Thr Arg Thr Gln Glu Val His His Pro Ile Cys Ala
    770                 775                 780
Phe Trp Asp Met Asn Lys Asn Lys Ser Phe Gly Gly Trp Asn Thr Ser
785                 790                 795                 800
Gly Cys Val Ala His Ser Asp Leu Asp Ala Gly Glu Thr Ile Cys Leu
                805                 810                 815
Cys Ser His Phe Thr His Phe Gly Val Leu Met Asp Leu Pro Arg Ser
            820                 825                 830
Ala Ser Gln Ile Asp Gly Arg Asn Thr Lys Val Leu Thr Phe Ile Thr
        835                 840                 845
Tyr Ile Gly Cys Gly Ile Ser Ala Ile Phe Ser Ala Ala Thr Leu Leu
    850                 855                 860
Thr Tyr Val Ala Phe Glu Lys Leu Arg Arg Asp Tyr Pro Ser Lys Ile
865                 870                 875                 880
Leu Met Asn Leu Ser Ser Ala Leu Leu Phe Leu Asn Leu Ile Phe Leu
                885                 890                 895
Leu Asp Gly Trp Val Thr Ser Phe Gly Val Ala Gly Leu Cys Thr Ala
            900                 905                 910
Val Ala Ala Leu Leu His Phe Phe Leu Leu Ala Thr Phe Thr Trp Met
        915                 920                 925
Gly Leu Glu Ala Ile His Met Tyr Ile Ala Leu Val Lys Val Phe Asn
    930                 935                 940
Thr Tyr Ile His Arg Tyr Ile Leu Lys Phe Cys Ile Ile Gly Trp Gly
945                 950                 955                 960
Leu Pro Ala Leu Val Val Ser Ile Ile Leu Val Ser Arg Arg Gln Asn
                965                 970                 975
Glu Val Tyr Gly Lys Glu Ser Tyr Gly Lys Asp Gln Asp Asp Glu Phe
            980                 985                 990
Cys Trp Ile Gln Asp Pro Val Val Phe Tyr Val Ser Cys Ala Gly Tyr
        995                 1000                1005
Phe Gly Val Met Phe Phe Leu Asn Val Ala Met Phe Ile Val Val Met
    1010                1015                1020
Val Gln Ile Cys Gly Arg Asn Gly Lys Arg Ser Asn Arg Thr Leu Arg
1025                1030                1035                1040
Glu Glu Val Leu Arg Asn Leu Arg Ser Val Val Ser Leu Thr Phe Leu
                1045                1050                1055
Leu Gly Met Thr Trp Gly Phe Ala Phe Phe Ala Trp Gly Pro Leu Asn
            1060                1065                1070
Ile Pro Phe Met Tyr Leu Phe Ser Ile Phe Asn Ser Leu Gln Gly Leu
        1075                1080                1085
Phe Ile Phe Ile Phe His Cys Ala Met Lys Glu Asn Val Gln Lys Gln
    1090                1095                1100
Trp Arg Arg His Leu Cys Cys Gly Arg Phe Arg Leu Ala Asp Asn Ser
1105                1110                1115                1120
Asp Trp Ser Lys Thr Ala Thr Asn Ile Ile Lys Lys Ser Ser Asp Asn
```

```
            1125                1130                1135
Leu Gly Lys Ser Leu Ser Ser Ser Ser Ile Gly Ser Asn Ser Thr Tyr
        1140                1145                1150
Leu Thr Ser Lys Ser Lys Ser Ser Ser Thr Thr Tyr Phe Lys Arg Asn
    1155                1160                1165
Ser His Ser Asp Asn Phe Ser
1170                1175
```

<210> 26
<211> 3525
<212> DNA
<213> Mus musculus

<400> 26

```
atgagtgtgc ggcctctggg aatctccaca cggatgtttg acactctcgg gaagaggtgc     60
tgcccttgga gactgaagcc aagcgccctg ctgttcctgt ttgttttatg tgttacctgt    120
gttcctctct cagtgtgcgg atgtggcagc tgcagacttg tcctgtccaa tccttccggt    180
acctttacgt ctccgtgtta ccctaatgac taccctaata cccagtcttg ttcgtggacc    240
ctccgagccc ctgccggcta catcattcag ataacgttca atgacttcga cattgaagaa    300
gctcccaact gtatctatga ctcattgtcc ctcgataatg gagagagcca gacaaaattc    360
tgtggagcga ctgccaaggg cctgtcattt aactccagcg tgaatgagat gcatgtgtcc    420
ttttcaagtg actttagtat ccagaagaaa ggtttcaacg ccagctacat cagagttgct    480
gtgtccttga ggaatcaaaa ggtcattttg ccccagacat tagatgctta ccaggtatca    540
gttgcaaaaa gcatctccat tcctgaactc aaagctttca cgctctgttt tgaagcctcc    600
aaagttggca atgaaggtgg tgactggaca gctttctcct actcagacga gtcccttaca    660
cagctgctca gtcttgaaaa ggccagtaat ggctacttcc tgtccatctc tggctcaaga    720
tgcttgttga acaatgcgtt acctgtgaag gacaaagagg acatcttcac agaaaacttg    780
gagcagctct gtcttgtgtg gaataattct tggggctccg ttggtataaa tttcaaaaag    840
aactatgaaa cagttccatg cgattccacc atcagtgctg tcgtacccgg ggatgggaca    900
ttgctgttgg gctccgacag agatgagatc gcctctctaa ggggcagcat ctataacttt    960
cgactttgga attttaccat ggatctgaaa gccctctcca acctcagctg tagtgtgtct   1020
gggaatgtca tagactggca caatgacttt tggagcatct caacccaagc tctgaaagcc   1080
gagggcaacc tgagctgtgg ttcctacctg atccagcttc ctgcagcaga gctgacaaac   1140
tgttcagaac tggggactct ctgtcaagac ggaataatgt atcgaatatc tgttgtgatt   1200
cacaatgact ttaatcaccc tgaagtaaaa gtgcagacca aagtagcaga atggctcaat   1260
tcaacctttc agaattggaa ctacactgtt tatgtggtta atataagttt tcatcaaaaa   1320
gtaggagagg acaggatgaa agtcaagaga gacatcatgg acgatgacaa aaggttggtg   1380
ctctgggccc ttctagtcta caatgctacc aataacgtca gcctgaatga agagaagatt   1440
aaacaaaagc ttatgacaaa taatgcatca ctagaggatg gactgaggct gtgtgaagtc   1500
gacgtgaacc agctgggtat gtgtagcgcc ttggaggatc cagacggctt tagttggcca   1560
gccaccttac cctctgtcta caaacaacca tgtccaaaca agcctggctt ttttatgact   1620
cgagcgtgcc tttctaatgg aacatcaacc ttctggggcc ctgttgacac ttccaactgt   1680
tcaagacaat caaatgaagt ggccaatgag attttaaacc aaactggtga tgggcagaac   1740
ctcacctcgg ctaatatcaa cagcattgta gaaaaggtca aacggatcgt gaacaaagaa   1800
gaaaacattg acatcaccct cggctccact ctaatgaata tattttctaa tatcttaagc   1860
agttcagata gcgatttgct tgagtcttct actgaagctt taaaaacaat tgacgagcta   1920
gccttcaaaa tagacctgaa tagcacccca catgtgaaca ttgagacaca gaatttggcc   1980
cttggagtct catccctaat tccaggaaca aatgcacctt caaattttag cattggcctt   2040
ccaagcaata atgaatcgta cttccagatg gacttcggga acggacagac agatccactg   2100
gcatctgtga ttttgcctcc aaatttgctt gagaatttaa gccccgaaga ttctgtattg   2160
gtcaggagag cacagttcac tttcttcaac aaaaccggac ttttccagga tgttggatct   2220
caaagaaaag tcctcgtgag ttatgtgatg gcgtgcagca ttggaaacat tactatccag   2280
aatctgaaag atccggttca aatcaaaatc aaacacacca gaacacagga agtgcatcat   2340
cctatctgtg ccttctggga tatgaacaaa aacaaaagtt tcgggggtg gaacacctca   2400
ggatgtgttg cccactctga tttggacgct ggtgagacca tttgtctgtg cagccacttc   2460
actcactttg gagttctgat ggatcttcca aggagtgcct cacaaataga tggaagaaac   2520
acaaaagtcc tcacgttcat tacctatatt gggtgcggaa tatctgccat ttttctcagct   2580
gcaactctcc tgacatatgt tgcttttgag aagctgcgca gggattatcc ctccaaaatc   2640
ctgatgaatc tgagctcggc cttgctcttc ctgaatctca tcttcctcct ggatggctgg   2700
gtcacttcct ttggcgtggc tggactctgc acggctgtgg ctgccctgtt gcacttcttc   2760
ctcctggcta ccttcacctg gatggggctg gaagccatcc acatgtacat tgctcttgtg   2820
aaagtgttta acacttacat ccaccgctat attctaaaat tctgcatcat aggctggggt   2880
ctgccagcct tggtggtgtc aattattcta gtgagcagaa gacaaaatga agtatatgga   2940
aaagaaagtt atgggaaaga tcaggatgat gaattctgct ggattcagga tcctgtggtg   3000
ttttatgtga gctgtgccgg gtacttcgga gtcatgttct tcctgaatgt cgccatgttc   3060
attgtggtca tggtgcagat ctgtgggagg aatggaaaga gaagcaaccg gaccctgaga   3120
gaagaggttt taagaaacct gcgcagtgtg gtcagcctga ccttcctgct tggcatgacg   3180
tgggggtttg ctttctttgc ctggggaccc ttaaatattc ctttcatgta cctcttctcc   3240
```

```
atcttcaatt cattacaagg tttatttata ttcatcttcc actgtgcgat gaaggagaat   3300
gttcagaaac agtggaggcg tcacctctgc tgtggcaggt ttcggctagc agacaactca   3360
gattggagta agacagctac caatatcatc aagaagagct ccgataacct ggggaaatct   3420
ttgtcttcaa gctccattgg ctccaattca acatatctca catccaaatc aaagtccagc   3480
tccactacct atttcaaaag aaacagccac tcggataatt tctcc                   3525

<210> 27
<211> 1230
<212> PRT
<213> Mus musculus

<400> 27
Met Ser Val Arg Pro Leu Gly Ile Ser Thr Arg Met Phe Asp Thr Leu
                5                   10                  15
Gly Lys Arg Cys Cys Pro Trp Arg Leu Lys Pro Ser Ala Leu Leu Phe
            20                  25                  30
Leu Phe Val Leu Cys Val Thr Cys Val Pro Leu Ser Val Cys Gly Cys
        35                  40                  45
Gly Ser Cys Arg Leu Val Leu Ser Asn Pro Ser Gly Thr Phe Thr Ser
    50                  55                  60
Pro Cys Tyr Pro Asn Asp Tyr Pro Asn Thr Gln Ser Cys Ser Trp Thr
65                  70                  75                  80
Leu Arg Ala Pro Ala Gly Tyr Ile Ile Gln Ile Thr Phe Asn Asp Phe
                85                  90                  95
Asp Ile Glu Glu Ala Pro Asn Cys Ile Tyr Asp Ser Leu Ser Leu Asp
            100                 105                 110
Asn Gly Glu Ser Gln Thr Lys Phe Cys Gly Ala Thr Ala Lys Gly Leu
        115                 120                 125
Ser Phe Asn Ser Ser Val Asn Glu Met His Val Ser Phe Ser Ser Asp
    130                 135                 140
Phe Ser Ile Gln Lys Lys Gly Phe Asn Ala Ser Tyr Ile Arg Val Ala
145                 150                 155                 160
Val Ser Leu Arg Asn Gln Lys Val Ile Leu Pro Gln Thr Leu Asp Ala
                165                 170                 175
Tyr Gln Val Ser Val Ala Lys Ser Ile Ser Ile Pro Glu Leu Lys Ala
            180                 185                 190
Phe Thr Leu Cys Phe Glu Ala Ser Lys Val Gly Asn Glu Gly Gly Asp
        195                 200                 205
Trp Thr Ala Phe Ser Tyr Ser Asp Glu Ser Leu Thr Gln Leu Leu Ser
    210                 215                 220
Leu Glu Lys Ala Ser Asn Gly Tyr Phe Leu Ser Ile Ser Gly Ser Arg
225                 230                 235                 240
Cys Leu Leu Asn Asn Ala Leu Pro Val Lys Asp Lys Glu Asp Ile Phe
                245                 250                 255
Thr Glu Asn Leu Glu Gln Leu Cys Leu Val Trp Asn Asn Ser Trp Gly
            260                 265                 270
Ser Val Gly Ile Asn Phe Lys Lys Asn Tyr Glu Thr Val Pro Cys Asp
        275                 280                 285
Ser Thr Ile Ser Ala Val Val Pro Gly Asp Gly Thr Leu Leu Leu Gly
    290                 295                 300
Ser Asp Arg Asp Glu Ile Ala Ser Leu Arg Gly Ser Ile Tyr Asn Phe
305                 310                 315                 320
Arg Leu Trp Asn Phe Thr Met Asp Leu Lys Ala Leu Ser Asn Leu Ser
                325                 330                 335
Cys Ser Val Ser Gly Asn Val Ile Asp Trp His Asn Asp Phe Trp Ser
            340                 345                 350
Ile Ser Thr Gln Ala Leu Lys Ala Glu Gly Asn Leu Ser Cys Gly Ser
        355                 360                 365
Tyr Leu Ile Gln Leu Pro Ala Ala Glu Leu Thr Asn Cys Ser Glu Leu
    370                 375                 380
Gly Thr Leu Cys Gln Asp Gly Ile Met Tyr Arg Ile Ser Val Val Ile
385                 390                 395                 400
His Asn Asp Phe Asn His Pro Glu Val Lys Val Gln Thr Lys Val Ala
                405                 410                 415
Glu Trp Leu Asn Ser Thr Phe Gln Asn Trp Asn Tyr Thr Val Tyr Val
            420                 425                 430
Val Asn Ile Ser Phe His Gln Lys Val Gly Glu Asp Arg Met Lys Val
        435                 440                 445
```

Lys Arg Asp Ile Met Asp Asp Asp Lys Arg Leu Val Leu Trp Ala Leu
450 455 460
Leu Val Tyr Asn Ala Thr Asn Asn Val Ser Leu Asn Glu Glu Lys Ile
465 470 475 480
Lys Gln Lys Leu Met Thr Asn Asn Ala Ser Leu Glu Asp Gly Leu Arg
485 490 495
Leu Cys Glu Val Asp Val Asn Gln Leu Gly Met Cys Ser Ala Leu Glu
500 505 510
Asp Pro Asp Gly Phe Ser Trp Pro Ala Thr Leu Pro Ser Val Tyr Lys
515 520 525
Gln Pro Cys Pro Asn Lys Pro Gly Phe Phe Met Thr Arg Ala Cys Leu
530 535 540
Ser Asn Gly Thr Ser Thr Phe Trp Gly Pro Val Asp Thr Ser Asn Cys
545 550 555 560
Ser Arg Gln Ser Asn Glu Val Ala Asn Glu Ile Leu Asn Gln Thr Gly
565 570 575
Asp Gly Gln Asn Leu Thr Ser Ala Asn Ile Asn Ser Ile Val Glu Lys
580 585 590
Val Lys Arg Ile Val Asn Lys Glu Glu Asn Ile Asp Ile Thr Leu Gly
595 600 605
Ser Thr Leu Met Asn Ile Phe Ser Asn Ile Leu Ser Ser Ser Asp Ser
610 615 620
Asp Leu Leu Glu Ser Ser Thr Glu Ala Leu Lys Thr Ile Asp Glu Leu
625 630 635 640
Ala Phe Lys Ile Asp Leu Asn Ser Thr Pro His Val Asn Ile Glu Thr
645 650 655
Gln Asn Leu Ala Leu Gly Val Ser Ser Leu Ile Pro Gly Thr Asn Ala
660 665 670
Pro Ser Asn Phe Ser Ile Gly Leu Pro Ser Asn Asn Glu Ser Tyr Phe
675 680 685
Gln Met Asp Phe Gly Asn Gly Gln Thr Asp Pro Leu Ala Ser Val Ile
690 695 700
Leu Pro Pro Asn Leu Leu Glu Asn Leu Ser Pro Glu Asp Ser Val Leu
705 710 715 720
Val Arg Arg Ala Gln Phe Thr Phe Phe Asn Lys Thr Gly Leu Phe Gln
725 730 735
Asp Val Gly Ser Gln Arg Lys Val Leu Val Ser Tyr Val Met Ala Cys
740 745 750
Ser Ile Gly Asn Ile Thr Ile Gln Asn Leu Lys Asp Pro Val Gln Ile
755 760 765
Lys Ile Lys His Thr Arg Thr Gln Glu Val His His Pro Ile Cys Ala
770 775 780
Phe Trp Asp Met Asn Lys Asn Lys Ser Phe Gly Gly Trp Asn Thr Ser
785 790 795 800
Gly Cys Val Ala His Ser Asp Leu Asp Ala Gly Glu Thr Ile Cys Leu
805 810 815
Cys Ser His Phe Thr His Phe Gly Val Leu Met Asp Leu Pro Arg Ser
820 825 830
Ala Ser Gln Ile Asp Gly Arg Asn Thr Lys Val Leu Thr Phe Ile Thr
835 840 845
Tyr Ile Gly Cys Gly Ile Ser Ala Ile Phe Ser Ala Ala Thr Leu Leu
850 855 860
Thr Tyr Val Ala Phe Glu Lys Leu Arg Arg Asp Tyr Pro Ser Lys Ile
865 870 875 880
Leu Met Asn Leu Ser Ser Ala Leu Leu Phe Leu Asn Leu Ile Phe Leu
885 890 895
Leu Asp Gly Trp Val Thr Ser Phe Gly Val Ala Gly Leu Cys Thr Ala
900 905 910
Val Ala Ala Leu Leu His Phe Phe Leu Leu Ala Thr Phe Thr Trp Met
915 920 925
Gly Leu Glu Ala Ile His Met Tyr Ile Ala Leu Val Lys Val Phe Asn
930 935 940
Thr Tyr Ile His Arg Tyr Ile Leu Lys Phe Cys Ile Ile Gly Trp Gly
945 950 955 960
Leu Pro Ala Leu Val Val Ser Ile Ile Leu Val Ser Arg Arg Gln Asn
965 970 975
Glu Val Tyr Gly Lys Glu Ser Tyr Gly Lys Asp Gln Asp Asp Glu Phe
980 985 990

```
Cys Trp Ile Gln Asp Pro Val Val Phe Tyr Val Ser Cys Ala Gly Tyr
        995                 1000                1005
Phe Gly Val Met Phe Phe Leu Asn Val Ala Met Phe Ile Val Val Met
    1010                1015                1020
Val Gln Ile Cys Gly Arg Asn Gly Lys Arg Ser Asn Arg Thr Leu Arg
1025                1030                1035                1040
Glu Glu Val Leu Arg Asn Leu Arg Ser Val Val Ser Leu Thr Phe Leu
                1045                1050                1055
Leu Gly Met Thr Trp Gly Phe Ala Phe Phe Ala Trp Gly Pro Leu Asn
            1060                1065                1070
Ile Pro Phe Met Tyr Leu Phe Ser Ile Phe Asn Ser Leu Gln Gly Leu
        1075                1080                1085
Phe Ile Phe Ile Phe His Cys Ala Met Lys Glu Asn Val Gln Lys Gln
    1090                1095                1100
Trp Arg Arg His Leu Cys Cys Gly Arg Phe Arg Leu Ala Asp Asn Ser
1105                1110                1115                1120
Asp Trp Ser Lys Thr Ala Thr Asn Ile Ile Lys Lys Ser Ser Asp Asn
                1125                1130                1135
Leu Gly Lys Ser Leu Ser Ser Ser Ser Ile Gly Ser Asn Ser Thr Tyr
            1140                1145                1150
Leu Thr Ser Lys Ser Lys Ser Ser Ser Thr Thr Tyr Phe Lys Arg Asn
        1155                1160                1165
Ser His Ser Asp Ser Thr Ser Thr Asp Lys Ser Leu Ser Lys Leu Thr
    1170                1175                1180
His Ala Gly Gly Glu Gln Thr Ser Ile Ile Pro Val His Gln Val Ile
1185                1190                1195                1200
Asp Lys Val Lys Gly Tyr Cys Asn Ala His Ser Asp Asn Phe Tyr Lys
                1205                1210                1215
Asn Ile Ile Leu Ser Asp Ala Phe Ser His Ser Thr Lys Phe
            1220                1225                1230
```

<210> 28
<211> 3690
<212> DNA
<213> Mus musculus

<400> 28

```
atgagtgtgc ggcctctggg aatctccaca cggatgtttg acactctcgg gaagaggtgc     60
tgcccttgga gactgaagcc aagcgccctg ctgttcctgt ttgttttatg tgttacctgt    120
gttcctctct cagtgtgcgg atgtggcagc tgcagacttg tcctgtccaa tccttccggt    180
acctttacgt ctccgtgtta ccctaatgac taccctaata cccagtcttg ttcgtggacc    240
ctccgagccc ctgccggcta catcattcag ataacgttca atgacttcga cattgaagaa    300
gctcccaact gtatctatga ctcattgtcc ctcgataatg gagagagcca gacaaaattc    360
tgtggagcga ctgccaaggg cctgtcattt aactccagcg tgaatgagat gcatgtgtcc    420
ttttcaagtg actttagtat ccagaagaaa ggtttcaacg ccagctacat cagagttgct    480
gtgtccttga ggaatcaaaa ggtcattttg ccccagacat tagatgctta ccaggtatca    540
gttgcaaaaa gcatctccat tcctgaactc aaagctttca cgctctgttt tgaagcctcc    600
aaagttggca atgaaggtgg tgactggaca gctttctcct actcagacga gtcccttaca    660
cagctgctca gtcttgaaaa ggccagtaat ggctacttcc tgtccatctc tggctcaaga    720
tgcttgttga acaatgcgtt acctgtgaag gacaaagagg acatcttcac agaaaacttg    780
gagcagctct gtcttgtgtg gaataattct tggggctccg ttggtataaa tttcaaaaag    840
aactatgaaa cagttccatg cgattccacc atcagtgctg tcgtacccgg ggatgggaca    900
ttgctgttgg gctccgacag agatgagatc gcctctctaa ggggcagcat ctataacttt    960
cgactttgga attttaccat ggatctgaaa gccctctcca acctcagctg tagtgtgtct   1020
gggaatgtca tagactggca caatgacttt tggagcatct caacccaagc tctgaaagcc   1080
gagggcaacc tgagctgtgg ttcctacctg atccagcttc ctgcagcaga gctgacaaac   1140
tgttcagaac tggggactct ctgtcaagac ggaataatgt atcgaatatc tgttgtgatt   1200
cacaatgact ttaatcaccc tgaagtaaaa gtgcagacca aagtagcaga atggctcaat   1260
tcaacctttc agaattggaa ctacactgtt tatgtggtta atataagttt tcatcaaaaa   1320
gtaggagagg acaggatgaa agtcaagaga gacatcatgg acgatgacaa aaggttggtg   1380
ctctgggccc ttctagtcta caatgctacc aataacgtca gcctgaatga agagaagatt   1440
aaacaaaagc ttatgacaaa taatgcatca ctagaggatg gactgaggct gtgtgaagtc   1500
gacgtgaacc agctgggtat gtgtagcgcc ttggaggatc cagacggctt tagttggcca   1560
gccaccttac cctctgtcta caaacaacca tgtccaaaca agcctggctt ttttatgact   1620
cgagcgtgcc tttctaatgg aacatcaacc ttctggggcc ctgttgacac ttccaactgt   1680
tcaagacaat caaatgaagt ggccaatgag attttaaacc aaactggtga tgggcagaac   1740
ctcacctcgg ctaatatcaa cagcattgta gaaaaggtca aacggatcgt gaacaaagaa   1800
gaaaacattg acatcaccct cggctccact ctaatgaata tattttctaa tatcttaagc   1860
```

```
agttcagata gcgatttgct tgagtcttct actgaagctt taaaaacaat tgacgagcta 1920
gccttcaaaa tagacctgaa tagcacccca catgtgaaca ttgagacaca gaatttggcc 1980
cttggagtct catccctaat tccaggaaca aatgcacctt caaattttag cattggcctt 2040
ccaagcaata atgaatcgta cttccagatg gacttcggga acggacagac agatccactg 2100
gcatctgtga ttttgcctcc aaatttgctt gagaatttaa gccccgaaga ttctgtattg 2160
gtcaggagag cacagttcac tttcttcaac aaaaccggac ttttccagga tgttggatct 2220
caaagaaaag tcctcgtgag ttatgtgatg gcgtgcagca ttggaaacat tactatccag 2280
aatctgaaag atccggttca aatcaaaatc aaacacacca gaacacagga agtgcatcat 2340
cctatctgtg ccttctggga tatgaacaaa aacaaaagtt tcgggggggtg gaacacctca 2400
ggatgtgttg cccactctga tttggacgct ggtgagacca tttgtctgtg cagccacttc 2460
actcactttg gagttctgat ggatcttcca aggagtgcct cacaaataga tggaagaaac 2520
acaaaaagtcc tcacgttcat tacctatatt gggtgcggaa tatctgccat tttctcagct 2580
gcaactctcc tgacatatgt tgcttttgag aagctgcgca gggattatcc ctccaaaatc 2640
ctgatgaatc tgagctcggc cttgctcttc ctgaatctca tcttcctcct ggatggctgg 2700
gtcacttcct ttggcgtggc tggactctgc acggctgtgg ctgccctgtt gcacttcttc 2760
ctcctggcta ccttcacctg gatggggctg gaagccatcc acatgtacat tgctcttgtg 2820
aaagtgttta acacttacat ccaccgctat attctaaaat tctgcatcat aggctggggt 2880
ctgccagcct tggtggtgtc aattattcta gtgagcagaa gacaaaatga agtatatgga 2940
aaagaaagtt atgggaaaga tcaggatgat gaattctgct ggattcagga tcctgtggtg 3000
ttttatgtga gctgtgccgg gtacttcgga gtcatgttct tcctgaatgt cgccatgttc 3060
attgtggtca tggtgcagat ctgtgggagg aatggaaaga gaagcaaccg gaccctgaga 3120
gaagaggttt taagaaacct gcgcagtgtg gtcagcctga ccttcctgct tggcatgacg 3180
tggggggtttg ctttctttgc ctggggaccc ttaaatattc ctttcatgta cctcttctcc 3240
atcttcaatt cattacaagg tttatttata ttcatcttcc actgtgcgat gaaggagaat 3300
gttcagaaac agtggaggcg tcacctctgc tgtggcaggt ttcggctagc agacaactca 3360
gattggagta agacagctac caatatcatc aagaagagct ccgataacct ggggaaatct 3420
ttgtcttcaa gctccattgg ctccaattca acatatctca catccaaatc aaagtccagc 3480
tccactacct atttcaaaag aaacagccac tcggacagca cttccacgga caagtccctg 3540
tcaaaactga cccatgctgg tggagaacag acgtcaatca ttcccgtcca tcaggttatt 3600
gataaggtca agggttactg taatgcccat tccgataact tctataaaaa tatcatcctg 3660
tcagatgcct tcagccacag cacaaagttt 3690
```

<210> 29
<211> 27
<212> DNA
<213> Artificial Sequence

<220>
<223> Primer

<400> 29
gaagaggtgc tgcccttgga gactgaa 27

<210> 30
<211> 27
<212> DNA
<213> Artificial Sequence

<220>
<223> Primer

<400> 30
ctcttaggag aaattatccg agtggct 27

<210> 31
<211> 24
<212> DNA
<213> Artificial Sequence

<220>
<223> Primer

<400> 31
ggacattgct gttgggctcc gaca 24

<210> 32
<211> 24
<212> DNA

<213> Artificial Sequence

<220>
<223> Primer

<400> 32
tgcacgccat cacataactc acga 24

<210> 33
<211> 24
<212> DNA
<213> Artificial Sequence

<220>
<223> Primer

<400> 33
gcaggtttcg gctagcagac aact 24

<210> 34
<211> 26
<212> DNA
<213> Artificial Sequence

<220>
<223> Primer

<400> 34
ccaaatcaaa gtccagctcc actacc 26

<210> 35
<211> 21
<212> DNA
<213> Artificial Sequence

<220>
<223> Primer

<400> 35
aaggtaccgg aaggattgga c 21

<210> 36
<211> 21
<212> DNA
<213> Artificial Sequence

<220>
<223> Primer

<400> 36
cattagggta acacggagac g 21

<210> 37
<211> 21
<212> DNA
<213> Artificial Sequence

<220>
<223> Primer

<400> 37
cggagggtcc acgaacaaga c 21

<210> 38
<211> 382
<212> PRT
<213> Homo sapiens

<400> 38
Met Gly Pro Thr Ser Val Pro Leu Val Lys Ala His Arg Ser Ser Val
1               5                   10                  15
Ser Asp Tyr Val Asn Tyr Asp Ile Ile Val Arg His Tyr Asn Tyr Thr
            20                  25                  30
Gly Lys Leu Asn Ile Ser Ala Asp Lys Glu Asn Ser Ile Lys Leu Thr
        35                  40                  45
Ser Val Val Phe Ile Leu Ile Cys Cys Phe Ile Ile Leu Glu Asn Ile
    50                  55                  60
Phe Val Leu Leu Thr Ile Trp Lys Thr Lys Lys Phe His Arg Pro Met
65                  70                  75                  80
Tyr Tyr Phe Ile Gly Asn Leu Ala Leu Ser Asp Leu Leu Ala Gly Val
                85                  90                  95
Ala Tyr Thr Ala Asn Leu Leu Leu Ser Gly Ala Thr Thr Tyr Lys Leu
            100                 105                 110
Thr Pro Ala Gln Trp Phe Leu Arg Glu Gly Ser Met Phe Val Ala Leu
        115                 120                 125
Ser Ala Ser Val Phe Ser Leu Leu Ala Ile Ala Ile Glu Arg Tyr Ile
    130                 135                 140
Thr Met Leu Lys Met Lys Leu His Asn Gly Ser Asn Asn Phe Arg Leu
145                 150                 155                 160
Phe Leu Leu Ile Ser Ala Cys Trp Val Ile Ser Leu Ile Leu Gly Gly
                165                 170                 175
Leu Pro Ile Met Gly Trp Asn Cys Ile Ser Ala Leu Ser Ser Cys Ser
            180                 185                 190
Thr Val Leu Pro Leu Tyr His Lys His Tyr Ile Leu Phe Cys Thr Thr
        195                 200                 205
Val Phe Thr Leu Leu Leu Leu Ser Ile Val Ile Leu Tyr Cys Arg Ile
    210                 215                 220
Tyr Ser Leu Val Arg Thr Arg Ser Arg Arg Leu Thr Phe Arg Lys Asn
225                 230                 235                 240
Ile Ser Lys Ala Ser Arg Ser Ser Glu Lys Ser Leu Ala Leu Leu Lys
                245                 250                 255
Thr Val Ile Ile Val Leu Ser Val Phe Ile Ala Cys Trp Ala Pro Leu
            260                 265                 270
Phe Ile Leu Leu Leu Leu Asp Val Gly Cys Lys Val Lys Thr Cys Asp
        275                 280                 285
Ile Leu Phe Arg Ala Glu Tyr Phe Leu Val Leu Ala Val Leu Asn Ser
    290                 295                 300
Gly Thr Asn Pro Ile Ile Tyr Thr Leu Thr Asn Lys Glu Met Arg Arg
305                 310                 315                 320
Ala Phe Ile Arg Ile Met Ser Cys Cys Lys Cys Pro Ser Gly Asp Ser
                325                 330                 335
Ala Gly Lys Phe Lys Arg Pro Ile Ile Ala Gly Met Glu Phe Ser Arg
            340                 345                 350
Ser Lys Ser Asp Asn Ser Ser His Pro Gln Lys Asp Glu Gly Asp Asn
        355                 360                 365
Pro Glu Thr Ile Met Ser Ser Gly Asn Val Asn Ser Ser Ser
    370                 375                 380

<210> 39
<211> 1146
<212> DNA
<213> Homo sapiens

<400> 39
atggggccca ccagcgtccc gctggtcaag gcccaccgca gctcggtctc tgactacgtc 60
aactatgata tcatcgtccg gcattacaac tacacgggaa agctgaatat cagcgcggac 120
aaggagaaca gcattaaact gacctcggtg gtgttcattc tcatctgctg ctttatcatc 180
ctggagaaca tctttgtctt gctgaccatt tggaaaacca agaaattcca ccgacccatg 240
tactatttta ttggcaatct ggccctctca gacctgttgg caggagtagc ctacacagct 300
aacctgctct tgtctggggc caccacctac aagctcactc ccgcccagtg gtttctgcgg 360
gaagggagta tgtttgtggc cctgtcagcc tccgtgttca gtctcctcgc catcgccatt 420
gagcgctata tcacaatgct gaaaatgaaa ctccacaacg ggagcaataa cttccgcctc 480
ttcctgctaa tcagcgcctg ctgggtcatc tccctcatcc tgggtggcct gcctatcatg 540
ggctggaact gcatcagtgc gctgtccagc tgctccaccg tgctgccgct ctaccacaag 600
cactatatcc tcttctgcac cacggtcttc actctgcttc tgctctccat cgtcattctg 660
tactgcagaa tctactcctt ggtcaggact cggagccgcc gcctgacgtt ccgcaagaac 720

```
atttccaagg ccagccgcag ctctgagaag tcgctggcgc tgctcaagac cgtaattatc    780
gtcctgagcg tcttcatcgc ctgctgggca ccgctcttca tcctgctcct gctggatgtg    840
ggctgcaagg tgaagacctg tgacatcctc ttcagagcgg agtacttcct ggtgttagct    900
gtgctcaact ccggcaccaa ccccatcatt tacactctga ccaacaagga gatgcgtcgg    960
gccttcatcc ggatcatgtc ctgctgcaag tgcccgagcg gagactctgc tggcaaattc   1020
aagcgaccca tcatcgccgg catggaattc agccgcagca aatcggacaa ttcctcccac   1080
ccccagaaag acgaagggga caacccagag accattatgt cttctggaaa cgtcaactct   1140
tcttcc                                                              1146
```

<210> 40
<211> 364
<212> PRT
<213> Homo sapiens

<400> 40

```
Met Ala Ala Ile Ser Thr Ser Ile Pro Val Ile Ser Gln Pro Gln Phe
                5                   10                  15
Thr Ala Met Asn Glu Pro Gln Cys Phe Tyr Asn Glu Ser Ile Ala Phe
            20                  25                  30
Phe Tyr Asn Arg Ser Gly Lys His Leu Ala Thr Glu Trp Asn Thr Val
        35                  40                  45
Ser Lys Leu Val Met Gly Leu Gly Ile Thr Val Cys Ile Phe Ile Met
    50                  55                  60
Leu Ala Asn Leu Leu Val Met Val Ala Ile Tyr Val Asn Arg Arg Phe
65                  70                  75                  80
His Phe Pro Ile Tyr Tyr Leu Met Ala Asn Leu Ala Ala Ala Asp Phe
                85                  90                  95
Phe Ala Gly Leu Ala Tyr Phe Tyr Leu Met Phe Asn Thr Gly Pro Asn
            100                 105                 110
Thr Arg Arg Leu Thr Val Ser Thr Trp Leu Leu Arg Gln Gly Leu Ile
        115                 120                 125
Asp Thr Ser Leu Thr Ala Ser Val Ala Asn Leu Leu Ala Ile Ala Ile
    130                 135                 140
Glu Arg His Ile Thr Val Phe Arg Met Gln Leu His Thr Arg Met Ser
145                 150                 155                 160
Asn Arg Arg Val Val Val Val Ile Val Val Ile Trp Thr Met Ala Ile
                165                 170                 175
Val Met Gly Ala Ile Pro Ser Val Gly Trp Asn Cys Ile Cys Asp Ile
            180                 185                 190
Glu Asn Cys Ser Asn Met Ala Pro Leu Tyr Ser Asp Ser Tyr Leu Val
        195                 200                 205
Phe Trp Ala Ile Phe Asn Leu Val Thr Phe Val Val Met Val Val Leu
    210                 215                 220
Tyr Ala His Ile Phe Gly Tyr Val Arg Gln Arg Thr Met Arg Met Ser
225                 230                 235                 240
Arg His Ser Ser Gly Pro Arg Arg Asn Arg Asp Thr Met Met Ser Leu
                245                 250                 255
Leu Lys Thr Val Val Ile Val Leu Gly Ala Phe Ile Ile Cys Trp Thr
            260                 265                 270
Pro Gly Leu Val Leu Leu Leu Leu Asp Val Cys Cys Pro Gln Cys Asp
        275                 280                 285
Val Leu Ala Tyr Glu Lys Phe Phe Leu Leu Leu Ala Glu Phe Asn Ser
    290                 295                 300
Ala Met Asn Pro Ile Ile Tyr Ser Tyr Arg Asp Lys Glu Met Ser Ala
305                 310                 315                 320
Thr Phe Arg Gln Ile Leu Cys Cys Gln Arg Ser Glu Asn Pro Thr Gly
                325                 330                 335
Pro Thr Glu Gly Ser Asp Arg Ser Ala Ser Ser Leu Asn His Thr Ile
            340                 345                 350
Leu Ala Gly Val His Ser Asn Asp His Ser Val Val
        355                 360
```

<210> 41
<211> 1092
<212> DNA
<213> Homo sapiens

<400> 41

```
atggctgcca tctctacttc catccctgta atttcacagc cccagttcac agccatgaat     60
gaaccacagt gcttctacaa cgagtccatt gccttctttt ataaccgaag tggaaagcat    120
cttgccacag aatggaacac agtcagcaag ctggtgatgg gacttggaat cactgtttgt    180
atcttcatca tgttggccaa cctattggtc atggtggcaa tctatgtcaa ccgccgcttc    240
cattttccta tttattacct aatggctaat ctggctgctg cagacttctt tgctgggttg    300
gcctacttct atctcatgtt caacacagga cccaatactc ggagactgac tgttagcaca    360
tggctcctgc gtcagggcct cattgacacc agcctgacgg catctgtggc caacttactg    420
gctattgcaa tcgagaggca cattacggtt ttccgcatgc agctccacac acggatgagc    480
aaccggcggg tagtggtggt cattgtggtc atctggacta tggccatcgt tatgggtgct    540
atacccagtg tgggctggaa ctgtatctgt gatattgaaa attgttccaa catggcaccc    600
ctctacagtg actcttactt agtcttctgg gccattttca acttggtgac ctttgtggta    660
atggtggttc tctatgctca catctttggc tatgttcgcc agaggactat gagaatgtct    720
cggcatagtt ctggaccccg gcggaatcgg gataccatga tgagtcttct gaagactgtg    780
gtcattgtgc ttggggcctt tatcatctgc tggactcctg gattggtttt gttacttcta    840
gacgtgtgct gtccacagtg cgacgtgctg gcctatgaga aattcttcct tctccttgct    900
gaattcaact ctgccatgaa ccccatcatt tactcctacc gcgacaaaga aatgagcgcc    960
acctttaggc agatcctctg ctgccagcgc agtgagaacc ccaccggccc cacagaaggc   1020
tcagaccgct cggcttcctc cctcaaccac accatcttgg ctggagttca cagcaatgac   1080
cactctgtgg tt                                                       1092
```

<210> 42
<211> 378
<212> PRT
<213> Homo sapiens

<400> 42

```
Met Ala Thr Ala Leu Pro Pro Arg Leu Gln Pro Val Arg Gly Asn Glu
                5                   10                  15
Thr Leu Arg Glu His Tyr Gln Tyr Val Gly Lys Leu Ala Gly Arg Leu
            20                  25                  30
Lys Glu Ala Ser Glu Gly Ser Thr Leu Thr Thr Val Leu Phe Leu Val
        35                  40                  45
Ile Cys Ser Phe Ile Val Leu Glu Asn Leu Met Val Leu Ile Ala Ile
    50                  55                  60
Trp Lys Asn Asn Lys Phe His Asn Arg Met Tyr Phe Phe Ile Gly Asn
65                  70                  75                  80
Leu Ala Leu Cys Asp Leu Leu Ala Gly Ile Ala Tyr Lys Val Asn Ile
                85                  90                  95
Leu Met Ser Gly Lys Lys Thr Phe Ser Leu Ser Pro Thr Val Trp Phe
            100                 105                 110
Leu Arg Glu Gly Ser Met Phe Val Ala Leu Gly Ala Ser Thr Cys Ser
        115                 120                 125
Leu Leu Ala Ile Ala Ile Glu Arg His Leu Thr Met Ile Lys Met Arg
    130                 135                 140
Pro Tyr Asp Ala Asn Lys Arg His Arg Val Phe Leu Leu Ile Gly Met
145                 150                 155                 160
Cys Trp Leu Ile Ala Phe Thr Leu Gly Ala Leu Pro Ile Leu Gly Trp
                165                 170                 175
Asn Cys Leu His Asn Leu Pro Asp Cys Ser Thr Ile Leu Pro Leu Tyr
            180                 185                 190
Ser Lys Lys Tyr Ile Ala Phe Cys Ile Ser Ile Phe Thr Ala Ile Leu
        195                 200                 205
Val Thr Ile Val Ile Leu Tyr Ala Arg Ile Tyr Phe Leu Val Lys Ser
    210                 215                 220
Ser Ser Arg Lys Val Ala Asn His Asn Asn Ser Glu Arg Ser Met Ala
225                 230                 235                 240
Leu Leu Arg Thr Val Val Ile Val Val Ser Val Phe Ile Ala Cys Trp
                245                 250                 255
Ser Pro Leu Phe Ile Leu Phe Leu Ile Asp Val Ala Cys Arg Val Gln
            260                 265                 270
Ala Cys Pro Ile Leu Phe Lys Ala Gln Trp Phe Ile Val Leu Ala Val
        275                 280                 285
Leu Asn Ser Ala Met Asn Pro Val Ile Tyr Thr Leu Ala Ser Lys Glu
    290                 295                 300
Met Arg Arg Ala Phe Phe Arg Leu Val Cys Asn Cys Leu Val Arg Gly
305                 310                 315                 320
Arg Gly Ala Arg Ala Ser Pro Ile Gln Pro Ala Leu Asp Pro Ser Arg
                325                 330                 335
```

Ser Lys Ser Ser Ser Ser Asn Asn Ser Ser His Ser Pro Lys Val Lys
340 345 350
Glu Asp Leu Pro His Thr Asp Pro Ser Ser Cys Ile Met Asp Lys Asn
355 360 365
Ala Ala Leu Gln Asn Gly Ile Phe Cys Asn
370 375

<210> 43
<211> 1134
<212> DNA
<213> Homo sapiens

<400> 43
atggcaactg ccctcccgcc gcgtctccag ccggtgcggg ggaacgagac cctgcgggag 60
cattaccagt acgtggggaa gttggcgggc aggctgaagg aggcctccga gggcagcacg 120
ctcaccaccg tgctcttctt ggtcatctgc agcttcatcg tcttggagaa cctgatggtt 180
ttgattgcca tctggaaaaa caataaattt cacaaccgca tgtacttttt cattggcaac 240
ctggctctct gcgacctgct ggccggcatc gcttacaagg tcaacattct gatgtctggc 300
aagaagacgt tcagcctgtc tcccacggtc tggttcctca gggagggcag tatgttcgtg 360
gcccttgggg cgtccacctg cagcttactg gccatcgcca tcgagcggca cttgacaatg 420
atcaaaatga ggccttacga cgccaacaag aggcaccgcg tcttcctcct gatcgggatg 480
tgctggctca ttgccttcac gctgggcgcc ctgcccattc tgggctggaa ctgcctgcac 540
aatctccctg actgctctac catcctgccc ctctactcca agaagtacat tgccttctgc 600
atcagcatct tcacggccat cctggtgacc atcgtgatcc tctacgcacg catctacttc 660
ctggtgaagt ccagcagccg taaggtggcc aaccacaaca actcggagcg gtccatggca 720
ctgctgcgga ccgtggtgat tgtggtgagc gtgttcatcg cctgctggtc cccactcttc 780
atcctcttcc tcattgatgt ggcctgcagg gtgcaggcgt gccccatcct cttcaaggct 840
cagtggttca tcgtgttggc tgtgctcaac tccgccatga acccggtcat ctacacgctg 900
gccagcaagg agatgcggcg ggccttcttc cgtctggtct gcaactgcct ggtcagggga 960
cggggggccc gcgcctcacc catccagcct gcgctcgacc caagcagaag taaatcaagc 1020
agcagcaaca atagcagcca ctctccgaag gtcaaggaag acctgcccca cacagacccc 1080
tcatcctgca tcatggacaa gaacgcagca cttcagaatg ggatcttctg caac 1134

<210> 44
<211> 351
<212> PRT
<213> Homo sapiens

<400> 44
Met Val Ile Met Gly Gln Cys Tyr Tyr Asn Glu Thr Ile Gly Phe Phe
5 10 15
Tyr Asn Asn Ser Gly Lys Glu Leu Ser Ser His Trp Arg Pro Lys Asp
20 25 30
Val Val Val Val Ala Leu Gly Leu Thr Val Ser Val Leu Val Leu Leu
35 40 45
Thr Asn Leu Leu Val Ile Ala Ala Ile Ala Ser Asn Arg Arg Phe His
50 55 60
Gln Pro Ile Tyr Tyr Leu Leu Gly Asn Leu Ala Ala Ala Asp Leu Phe
65 70 75 80
Ala Gly Val Ala Tyr Leu Phe Leu Met Phe His Thr Gly Pro Arg Thr
85 90 95
Ala Arg Leu Ser Leu Glu Gly Trp Phe Leu Arg Gln Gly Leu Leu Asp
100 105 110
Thr Ser Leu Thr Ala Ser Val Ala Thr Leu Leu Ala Ile Ala Val Glu
115 120 125
Arg His Arg Ser Val Met Ala Val Gln Leu His Ser Arg Leu Pro Arg
130 135 140
Gly Arg Val Val Met Leu Ile Val Gly Val Trp Val Ala Ala Leu Gly
145 150 155 160
Leu Gly Leu Leu Pro Ala His Ser Trp His Cys Leu Cys Ala Leu Asp
165 170 175
Arg Cys Ser Arg Met Ala Pro Leu Leu Ser Arg Ser Tyr Leu Ala Val
180 185 190
Trp Ala Leu Ser Ser Leu Leu Val Phe Leu Leu Met Val Ala Val Tyr
195 200 205
Thr Arg Ile Phe Phe Tyr Val Arg Arg Arg Val Gln Arg Met Ala Glu
210 215 220
His Val Ser Cys His Pro Arg Tyr Arg Glu Thr Thr Leu Ser Leu Val

```
225                 230                 235                 240
Lys Thr Val Val Ile Ile Leu Gly Ala Phe Val Val Cys Trp Thr Pro
                245                 250                 255
Gly Gln Val Val Leu Leu Leu Asp Gly Leu Gly Cys Glu Ser Cys Asn
            260                 265                 270
Val Leu Ala Val Glu Lys Tyr Phe Leu Leu Leu Ala Glu Ala Asn Ser
        275                 280                 285
Leu Val Asn Ala Ala Val Tyr Ser Cys Arg Asp Ala Glu Met Arg Arg
    290                 295                 300
Thr Phe Arg Arg Leu Leu Cys Cys Ala Cys Leu Arg Gln Ser Thr Arg
305                 310                 315                 320
Glu Ser Val His Tyr Thr Ser Ser Ala Gln Gly Gly Ala Ser Thr Arg
                325                 330                 335
Ile Met Leu Pro Glu Asn Gly His Pro Leu Met Asp Ser Thr Leu
            340                 345                 350
```

<210> 45
<211> 1056
<212> DNA
<213> Homo sapiens

<400> 45

```
atggtcatca tgggccagtg ctactacaac gagaccatcg gcttcttcta taacaacagt     60
ggcaaagagc tcagctccca ctggcggccc aaggatgtgg tcgtggtggc actggggctg    120
accgtcagcg tgctggtgct gctgaccaat ctgctggtca tagcagccat cgcctccaac    180
cgccgcttcc accagcccat ctactacctg ctcggcaatc tggccgcggc tgacctcttc    240
gcgggcgtgg cctacctctt cctcatgttc cacactggtc cccgcacagc ccgactttca    300
cttgagggct ggttcctgcg gcagggcttg ctggacacaa gcctcactgc gtcggtggcc    360
acactgctgg ccatcgccgt ggagcggcac cgcagtgtga tggccgtgca gctgcacagc    420
cgcctgcccc gtggccgcgt ggtcatgctc attgtgggcg tgtgggtggc tgccctgggc    480
ctggggctgc tgcctgccca ctcctggcac tgcctctgtg ccctggaccg ctgctcacgc    540
atggcacccc tgctcagccg ctcctatttg gccgtctggg ctctgtcgag cctgcttgtc    600
ttcctgctca tggtggctgt gtacacccgc attttcttct acgtgcggcg gcgagtgcag    660
cgcatggcag agcatgtcag ctgccacccc cgctaccgag agaccacgct cagcctggtc    720
aagactgttg tcatcatcct gggggcgttc gtggtctgct ggacaccagg ccaggtggta    780
ctgctcctgg atggtttagg ctgtgagtcc tgcaatgtcc tggctgtaga aaagtacttc    840
ctactgttgg ccgaggccaa ctcactggtc aatgctgctg tgtactcttg ccgagatgct    900
gagatgcgcc gcaccttccg ccgccttctc tgctgcgcgt gcctccgcca gtccacccgc    960
gagtctgtcc actatacatc ctctgcccag ggaggtgcca gcactcgcat catgcttccc   1020
gagaacggcc acccactgat ggactccacc ctttag                             1056
```

<210> 46
<211> 353
<212> PRT
<213> Homo sapiens

<400> 46

```
Met Gly Ser Leu Tyr Ser Glu Tyr Leu Asn Pro Asn Lys Val Gln Glu
                5                   10                  15
His Tyr Asn Tyr Thr Lys Glu Thr Leu Glu Thr Gln Glu Thr Thr Ser
            20                  25                  30
Arg Gln Val Ala Ser Ala Phe Ile Val Ile Leu Cys Cys Ala Ile Val
        35                  40                  45
Val Glu Asn Leu Leu Val Leu Ile Ala Val Ala Arg Asn Ser Lys Phe
    50                  55                  60
His Ser Ala Met Tyr Leu Phe Leu Gly Asn Leu Ala Ala Ser Asp Leu
65                  70                  75                  80
Leu Ala Gly Val Ala Phe Val Ala Asn Thr Leu Leu Ser Gly Ser Val
                85                  90                  95
Thr Leu Arg Leu Thr Pro Val Gln Trp Phe Ala Arg Glu Gly Ser Ala
            100                 105                 110
Ser Ile Thr Leu Ser Ala Ser Val Phe Ser Leu Leu Ala Ile Ala Ile
        115                 120                 125
Glu Arg His Val Ala Ile Ala Lys Val Lys Leu Tyr Gly Ser Asp Lys
    130                 135                 140
Ser Cys Arg Met Leu Leu Leu Ile Gly Ala Ser Trp Leu Ile Ser Leu
145                 150                 155                 160
Val Leu Gly Gly Leu Pro Ile Leu Gly Trp Asn Cys Leu Gly His Leu
```

```
                165                 170                 175
Glu Ala Cys Ser Thr Val Leu Pro Leu Tyr Ala Lys His Tyr Val Leu
            180                 185                 190
Cys Val Val Thr Ile Phe Ser Ile Ile Leu Leu Ala Ile Val Ala Leu
        195                 200                 205
Tyr Val Arg Ile Tyr Cys Val Val Arg Ser Ser His Ala Asp Met Ala
    210                 215                 220
Ala Pro Gln Thr Leu Ala Leu Leu Lys Thr Val Thr Ile Val Leu Gly
225                 230                 235                 240
Val Phe Ile Val Cys Trp Leu Pro Ala Phe Ser Ile Leu Leu Leu Asp
                245                 250                 255
Tyr Ala Cys Pro Val His Ser Cys Pro Ile Leu Tyr Lys Ala His Tyr
            260                 265                 270
Phe Phe Ala Val Ser Thr Leu Asn Ser Leu Leu Asn Pro Val Ile Tyr
        275                 280                 285
Thr Trp Arg Ser Arg Asp Leu Arg Arg Glu Val Leu Arg Pro Leu Gln
    290                 295                 300
Cys Trp Arg Pro Gly Val Gly Val Gln Gly Arg Arg Arg Val Gly Thr
305                 310                 315                 320
Pro Gly His His Leu Leu Pro Leu Arg Ser Ser Ser Ser Leu Glu Arg
                325                 330                 335
Gly Met His Met Pro Thr Ser Pro Thr Phe Leu Glu Gly Asn Thr Val
            340                 345                 350
Val
```

<210> 47
<211> 1059
<212> DNA
<213> Homo sapiens

<400> 47

```
atgggcagct tgtactcgga gtacctgaac cccaacaagg tccaggaaca ctataattat     60
accaaggaga cgctggaaac gcaggagacg acctcccgcc aggtggcctc ggccttcatc    120
gtcatcctct gttgcgccat tgtggtggaa aaccttctgg tgctcattgc ggtggcccga    180
aacagcaagt tccactcggc aatgtacctg tttctgggca acctggccgc ctccgatcta    240
ctggcaggcg tggccttcgt agccaatacc ttgctctctg gctctgtcac gctgaggctg    300
acgcctgtgc agtggtttgc ccgggagggc tctgcctcca tcacgctctc ggcctctgtc    360
ttcagcctcc tggccatcgc cattgagcgc cacgtggcca ttgccaaggt caagctgtat    420
ggcagcgaca agagctgccg catgcttctg ctcatcgggg cctcgtggct catctcgctg    480
gtcctcggtg gcctgcccat ccttggctgg aactgcctgg gccacctcga ggcctgctcc    540
actgtcctgc ctctctacgc caagcattat gtgctgtgcg tggtgaccat cttctccatc    600
atcctgttgg ccatcgtggc cctgtacgtg cgcatctact gcgtggtccg ctcaagccac    660
gctgacatgg ccgccccgca gacgctagcc ctgctcaaga cggtcaccat cgtgctaggc    720
gtctttatcg tctgctggct gcccgccttc agcatcctcc ttctggacta tgcctgtccc    780
gtccactcct gcccgatcct ctacaaagcc cactactttt tcgccgtctc caccctgaat    840
tccctgctca accccgtcat ctacacgtgg cgcagccggg acctgcggcg ggaggtgctt    900
cggccgctgc agtgctggcg gccggggggtg ggggtgcaag gacggaggcg ggtcgggacc    960
ccgggccacc acctcctgcc actccgcagc tccagctccc tggagagggg catgcacatg   1020
cccacgtcac ccacgtttct ggagggcaac acggtggtc                          1059
```

<210> 48
<211> 384
<212> PRT
<213> Homo sapiens

<400> 48

```
Met Asn Ala Thr Gly Thr Pro Val Ala Pro Glu Ser Cys Gln Gln Leu
                5                   10                  15
Ala Ala Gly Gly His Ser Arg Leu Ile Val Leu His Tyr Asn His Ser
            20                  25                  30
Gly Arg Leu Ala Gly Arg Gly Gly Pro Glu Asp Gly Gly Leu Gly Ala
        35                  40                  45
Leu Arg Gly Leu Ser Val Ala Ala Ser Cys Leu Val Val Leu Glu Asn
    50                  55                  60
Leu Leu Val Leu Ala Ala Ile Thr Ser His Met Arg Ser Arg Arg Trp
65                  70                  75                  80
Val Tyr Tyr Cys Leu Val Asn Ile Thr Leu Ser Asp Leu Leu Thr Gly
                85                  90                  95
```

```
Ala Ala Tyr Leu Ala Asn Val Leu Leu Ser Gly Ala Arg Thr Phe Arg
            100                 105                 110
Leu Ala Pro Ala Gln Trp Phe Leu Arg Glu Gly Leu Leu Phe Thr Ala
        115                 120                 125
Leu Ala Ala Ser Thr Phe Ser Leu Leu Phe Thr Ala Gly Glu Arg Phe
    130                 135                 140
Ala Thr Met Val Arg Pro Val Ala Glu Ser Gly Ala Thr Lys Thr Ser
145                 150                 155                 160
Arg Val Tyr Gly Phe Ile Gly Leu Cys Trp Leu Leu Ala Ala Leu Leu
                165                 170                 175
Gly Met Leu Pro Leu Leu Gly Trp Asn Cys Leu Cys Ala Phe Asp Arg
            180                 185                 190
Cys Ser Ser Leu Leu Pro Leu Tyr Ser Lys Arg Tyr Ile Leu Phe Cys
        195                 200                 205
Leu Val Ile Phe Ala Gly Val Leu Ala Thr Ile Met Gly Leu Tyr Gly
    210                 215                 220
Ala Ile Phe Arg Leu Val Gln Ala Ser Gly Gln Lys Ala Pro Arg Pro
225                 230                 235                 240
Ala Ala Arg Arg Lys Ala Arg Arg Leu Leu Lys Thr Val Leu Met Ile
                245                 250                 255
Leu Leu Ala Phe Leu Val Cys Trp Gly Pro Leu Phe Gly Leu Leu Leu
            260                 265                 270
Ala Asp Val Phe Gly Ser Asn Leu Trp Ala Gln Glu Tyr Leu Arg Gly
        275                 280                 285
Met Asp Trp Ile Leu Ala Leu Ala Val Leu Asn Ser Ala Val Asn Pro
    290                 295                 300
Ile Ile Tyr Ser Phe Arg Ser Arg Glu Val Cys Arg Ala Val Leu Ser
305                 310                 315                 320
Phe Leu Cys Cys Gly Cys Leu Arg Leu Gly Met Arg Gly Pro Gly Asp
                325                 330                 335
Cys Leu Ala Arg Ala Val Glu Ala His Ser Gly Ala Ser Thr Thr Asp
            340                 345                 350
Ser Ser Leu Arg Pro Arg Asp Ser Phe Arg Gly Ser Arg Ser Leu Ser
        355                 360                 365
Phe Arg Met Arg Glu Pro Leu Ser Ser Ile Ser Ser Val Arg Ser Ile
    370                 375                 380
```

<210> 49
<211> 1152
<212> DNA
<213> Homo sapiens

<400> 49

```
atgaacgcca cggggacccc ggtggccccc gagtcctgcc aacagctggc ggccggcggg     60
cacagccggc tcattgttct gcactacaac cactcgggcc ggctggccgg gcgcgggggg    120
ccggaggatg gcggcctggg ggccctgcgg gggctgtcgg tggccgccag ctgcctggtg    180
gtgctggaga acttgctggt gctggcggcc atcaccagcc acatgcggtc gcgacgctgg    240
gtctactatt gcctggtgaa catcacgctg agtgacctgc tcacgggcgc ggcctacctg    300
gccaacgtgc tgctgtcggg ggcccgcacc ttccgtctgg cgcccgccca gtggttccta    360
cgggagggcc tgctcttcac cgccctggcc gcctccacct tcagcctgct cttcactgca    420
ggggagcgct ttgccaccat ggtgcggccg gtggccgaga gcggggccac caagaccagc    480
cgcgtctacg gcttcatcgg cctctgctgg ctgctggccg cgctgctggg gatgctgcct    540
ttgctgggct ggaactgcct gtgcgccttt gaccgctgct ccagccttct gcccctctac    600
tccaagcgct acatcctctt ctgcctggtg atcttcgccg gcgtcctggc caccatcatg    660
ggcctctatg gggccatctt ccgcctggtg caggccagcg ggcagaaggc cccacgccca    720
gcggcccgcc gcaaggcccg ccgcctgctg aagacggtgc tgatgatcct gctggccttc    780
ctggtgtgct ggggcccact cttcgggctg ctgctggccg acgtctttgg ctccaacctc    840
tgggcccagg agtacctgcg gggcatggac tggatcctgg ccctggccgt cctcaactcg    900
gcggtcaacc ccatcatcta ctccttccgc agcagggagg tgtgcagagc cgtgctcagc    960
ttcctctgct gcgggtgtct ccggctgggc atgcgagggc ccggggactg cctggcccgg   1020
gccgtcgagg ctcactccgg agcttccacc accgacagct ctctgaggcc aagggacagc   1080
tttcgcggct cccgctcgct cagctttcgg atgcgggagc ccctgtccag catctccagc   1140
gtgcggagca tc                                                       1152
```

<210> 50
<211> 353
<212> PRT
<213> Homo sapiens

<400> 50
Met Asn Glu Cys His Tyr Asp Lys His Met Asp Phe Phe Tyr Asn Arg
5 10 15
Ser Asn Thr Asp Thr Val Asp Asp Trp Thr Gly Thr Lys Leu Val Ile
20 25 30
Val Leu Cys Val Gly Thr Phe Phe Cys Leu Phe Ile Phe Phe Ser Asn
35 40 45
Ser Leu Val Ile Ala Ala Val Ile Lys Asn Arg Lys Phe His Phe Pro
50 55 60
Phe Tyr Tyr Leu Leu Ala Asn Leu Ala Ala Ala Asp Phe Phe Ala Gly
65 70 75 80
Ile Ala Tyr Val Phe Leu Met Phe Asn Thr Gly Pro Val Ser Lys Thr
85 90 95
Leu Thr Val Asn Arg Trp Phe Leu Arg Gln Gly Leu Leu Asp Ser Ser
100 105 110
Leu Thr Ala Ser Leu Thr Asn Leu Leu Val Ile Ala Val Glu Arg His
115 120 125
Met Ser Ile Met Arg Met Arg Val His Ser Asn Leu Thr Lys Lys Arg
130 135 140
Val Thr Leu Leu Ile Leu Leu Val Trp Ala Ile Ala Ile Phe Met Gly
145 150 155 160
Ala Val Pro Thr Leu Gly Trp Asn Cys Leu Cys Asn Ile Ser Ala Cys
165 170 175
Ser Ser Leu Ala Pro Ile Tyr Ser Arg Ser Tyr Leu Val Phe Trp Thr
180 185 190
Val Ser Asn Leu Met Ala Phe Leu Ile Met Val Val Val Tyr Leu Arg
195 200 205
Ile Tyr Val Tyr Val Lys Arg Lys Thr Asn Val Leu Ser Pro His Thr
210 215 220
Ser Gly Ser Ile Ser Arg Arg Arg Thr Pro Met Lys Leu Met Lys Thr
225 230 235 240
Val Met Thr Val Leu Gly Ala Phe Val Val Cys Trp Thr Pro Gly Leu
245 250 255
Val Val Leu Leu Leu Asp Gly Leu Asn Cys Arg Gln Cys Gly Val Gln
260 265 270
His Val Lys Arg Trp Phe Leu Leu Leu Ala Leu Leu Asn Ser Val Val
275 280 285
Asn Pro Ile Ile Tyr Ser Tyr Lys Asp Glu Asp Met Tyr Gly Thr Met
290 295 300
Lys Lys Met Ile Cys Cys Phe Ser Gln Glu Asn Pro Glu Arg Arg Pro
305 310 315 320
Ser Arg Ile Pro Ser Thr Val Leu Ser Arg Ser Asp Thr Gly Ser Gln
325 330 335
Tyr Ile Glu Asp Ser Ile Ser Gln Gly Ala Val Cys Asn Lys Ser Thr
340 345 350
Ser

<210> 51
<211> 1059
<212> DNA
<213> Homo sapiens

<400> 51
atgaatgagt gtcactatga caagcacatg gacttttttt ataataggag caacactgat 60
actgtcgatg actggacagg aacaaagctt gtgattgttt tgtgtgttgg gacgtttttc 120
tgcctgttta tttttttttc taattctctg gtcatcgcgg cagtgatcaa aaacagaaaa 180
tttcatttcc ccttctacta cctgttggct aatttagctg ctgccgattt cttcgctgga 240
attgcctatg tattcctgat gtttaacaca ggcccagttt caaaaacttt gactgtcaac 300
cgctggtttc tccgtcaggg gcttctggac agtagcttga ctgcttccct caccaacttg 360
ctggttatcg ccgtggagag gcacatgtca atcatgagga tgcgggtcca tagcaacctg 420
accaaaaaga gggtgacact gctcattttg cttgtctggg ccatcgccat ttttatgggg 480
gcggtcccca cactgggctg gaattgcctc tgcaacatct ctgcctgctc ttccctggcc 540
cccatttaca gcaggagtta ccttgttttc tggacagtgt ccaacctcat ggccttcctc 600
atcatggttg tggtgtacct gcggatctac gtgtacgtca agaggaaaac caacgtcttg 660
tctccgcata caagtgggtc catcagccgc cggaggacac ccatgaagct aatgaagacg 720
gtgatgactg tcttaggggc gtttgtggta tgctggaccc cgggcctggt ggttctgctc 780
ctcgacggcc tgaactgcag gcagtgtggc gtgcagcatg tgaaaaggtg gttcctgctg 840

ctggcgctgc tcaactccgt cgtgaacccc atcatctact cctacaagga cgaggacatg 900
tatggcacca tgaagaagat gatctgctgc ttctctcagg agaacccaga gaggcgtccc 960
tctcgcatcc cctccacagt cctcagcagg agtgacacag gcagccagta catagaggat 1020
agtattagcc aaggtgcagt ctgcaataaa agcacttcc 1059

<210> 52
<211> 398
<212> PRT
<213> Homo sapiens

<400> 52
Met Glu Ser Gly Leu Leu Arg Pro Ala Pro Val Ser Glu Val Ile Val
1 5 10 15
Leu His Tyr Asn Tyr Thr Gly Lys Leu Arg Gly Ala Arg Tyr Gln Pro
20 25 30
Gly Ala Gly Leu Arg Ala Asp Ala Val Val Cys Leu Ala Val Cys Ala
35 40 45
Phe Ile Val Leu Glu Asn Leu Ala Val Leu Leu Val Leu Gly Arg His
50 55 60
Pro Arg Phe His Ala Pro Met Phe Leu Leu Leu Gly Ser Leu Thr Leu
65 70 75 80
Ser Asp Leu Leu Ala Gly Ala Ala Tyr Ala Ala Asn Ile Leu Leu Ser
85 90 95
Gly Pro Leu Thr Leu Lys Leu Ser Pro Ala Leu Trp Phe Ala Arg Glu
100 105 110
Gly Gly Val Phe Val Ala Leu Thr Ala Ser Val Leu Ser Leu Leu Ala
115 120 125
Ile Ala Leu Glu Arg Ser Leu Thr Met Ala Arg Arg Gly Pro Ala Pro
130 135 140
Val Ser Ser Arg Gly Arg Thr Leu Ala Met Ala Ala Ala Ala Trp Gly
145 150 155 160
Val Ser Leu Leu Leu Gly Leu Leu Pro Ala Leu Gly Trp Asn Cys Leu
165 170 175
Gly Arg Leu Asp Ala Cys Ser Thr Val Leu Pro Leu Tyr Ala Lys Ala
180 185 190
Tyr Val Leu Phe Cys Val Leu Ala Phe Val Gly Ile Leu Ala Ala Ile
195 200 205
Cys Ala Leu Tyr Ala Arg Ile Tyr Cys Gln Val Arg Ala Asn Ala Arg
210 215 220
Arg Leu Pro Ala Arg Pro Gly Thr Ala Gly Thr Thr Ser Thr Arg Ala
225 230 235 240
Arg Arg Lys Pro Arg Ser Leu Ala Leu Leu Arg Thr Leu Ser Val Val
245 250 255
Leu Leu Ala Phe Val Ala Cys Trp Gly Pro Leu Phe Leu Leu Leu Leu
260 265 270
Leu Asp Val Ala Cys Pro Ala Arg Thr Cys Pro Val Leu Leu Gln Ala
275 280 285
Asp Pro Phe Leu Gly Leu Ala Met Ala Asn Ser Leu Leu Asn Pro Ile
290 295 300
Ile Tyr Thr Leu Thr Asn Arg Asp Leu Arg His Ala Leu Leu Arg Leu
305 310 315 320
Val Cys Cys Gly Arg His Ser Cys Gly Arg Asp Pro Ser Gly Ser Gln
325 330 335
Gln Ser Ala Ser Ala Ala Glu Ala Ser Gly Gly Leu Arg Arg Cys Leu
340 345 350
Pro Pro Gly Leu Asp Gly Ser Phe Ser Gly Ser Glu Arg Ser Ser Pro
355 360 365
Gln Arg Asp Gly Leu Asp Thr Ser Gly Ser Thr Gly Ser Pro Gly Ala
370 375 380
Pro Thr Ala Ala Arg Thr Leu Val Ser Glu Pro Ala Ala Asp
385 390 395

<210> 53
<211> 1194
<212> DNA
<213> Homo sapiens

<400> 53

```
atggagtcgg ggctgctgcg gccggcgccg gtgagcgagg tcatcgtcct gcattacaac     60
tacaccggca agctccgcgg tgcgcgctac cagccgggtg ccggcctgcg cgccgacgcc    120
gtggtgtgcc tggcggtgtg cgccttcatc gtgctagaga atctagccgt gttgttggtg    180
ctcggacgcc acccgcgctt ccacgctccc atgttcctgc tcctgggcag cctcacgttg    240
tcggatctgc tggcaggcgc cgcctacgcc gccaacatcc tactgtcggg gccgctcacg    300
ctgaaactgt cccccgcgct ctggttcgca cgggagggag gcgtcttcgt ggcactcact    360
gcgtccgtgc tgagcctcct ggccatcgcg ctggagcgca gcctcaccat ggcgcgcagg    420
gggcccgcgc ccgtctccag tcgggggcgc acgctggcga tggcagccgc ggcctggggc    480
gtgtcgctgc tcctcgggct cctgccagcg ctgggctgga attgcctggg tcgcctggac    540
gcttgctcca ctgtcttgcc gctctacgcc aaggcctacg tgctcttctg cgtgctcgcc    600
ttcgtgggca tcctggccgc gatctgtgca ctctacgcgc gcatctactg ccaggtacgc    660
gccaacgcgc ggcgcctgcc ggcacggccc gggactgcgg ggaccacctc gacccgggcg    720
cgtcgcaagc cgcgctcgct ggccttgctg cgcacgctca gcgtggtgct cctggccttt    780
gtggcatgtt ggggccccct cttcctgctg ctgttgctcg acgtggcgtg cccggcgcgc    840
acctgtcctg tactcctgca ggccgatccc ttcctgggac tggccatggc caactcactt    900
ctgaacccca tcatctacac gctcaccaac cgcgacctgc gccacgcgct cctgcgcctg    960
gtctgctgcg gacgccactc ctgcggcaga gacccgagtg gctcccagca gtcggcgagc   1020
gcggctgagg cttccggggg cctgcgccgc tgcctgcccc cgggccttga tgggagcttc   1080
agcggctcgg agcgctcatc gccccagcgc gacgggctgg acaccagcgg ctccacaggc   1140
agccccggtg cacccacagc cgcccggact ctggtatcag aaccggctgc agac          1194
```

<210> 54
<211> 22
<212> DNA
<213> Artificial Sequence

<220>
<223> Primer

<400> 54
ccaccgaccc atgtactatt tt 22

<210> 55
<211> 22
<212> DNA
<213> Artificial Sequence

<220>
<223> Primer

<400> 55
tgtaggctac tcctgccaac ag 22

<210> 56
<211> 22
<212> DNA
<213> Artificial Sequence

<220>
<223> Probe, labeled 5'-terminal with FAM and 3'-terminal with TAMRA

<400> 56
ttggcaatct ggccctctca ga 22

<210> 57
<211> 21
<212> DNA
<213> Artificial Sequence

<220>
<223> Primer

<400> 57
actgtcagca catggctcct t 21

<210> 58
<211> 21
<212> DNA

<213> Artificial Sequence

<220>
<223> Primer

<400> 58
accgtaatgt gcctctcgat t 21

<210> 59
<211> 22
<212> DNA
<213> Artificial Sequence

<220>
<223> Probe, labeled 5'-terminal with FAM and 3'-terminal with TAMRA

<400> 59
attgacacca gcctgacggc at 22

<210> 60
<211> 19
<212> DNA
<213> Artificial Sequence

<220>
<223> Primer

<400> 60
ccgtgctctt cttggtcat 19

<210> 61
<211> 19
<212> DNA
<213> Artificial Sequence

<220>
<223> Primer

<400> 61
ccagatggca atcaaaacc 19

<210> 62
<211> 26
<212> DNA
<213> Artificial Sequence

<220>
<223> Probe, labeled 5'-terminal with FAM and 3'-terminal with TAMRA

<400> 62
tgcagcttca tcgtcttgga gaacct 26

<210> 63
<211> 22
<212> DNA
<213> Artificial Sequence

<220>
<223> Primer

<400> 63
cctggtcaag actgttgtca tc 22

<210> 64
<211> 19
<212> DNA
<213> Artificial Sequence

<220>
<223> Primer

<400> 64
caggacattg caggactca 19

<210> 65
<211> 27
<212> DNA
<213> Artificial Sequence

<220>
<223> Probe, labeled 5'-terminal with FAM and 3'-terminal with TAMRA

<400> 65
tggtactgct cctggatggt ttaggct 27

<210> 66
<211> 19
<212> DNA
<213> Artificial Sequence

<220>
<223> Primer

<400> 66
ccaacaaggt ccaggaaca 19

<210> 67
<211> 19
<212> DNA
<213> Artificial Sequence

<220>
<223> Primer

<400> 67
aggttttcca ccacaatgg 19

<210> 68
<211> 28
<212> DNA
<213> Artificial Sequence

<220>
<223> Probe, labeled 5'-terminal with FAM and 3'-terminal with TAMRA

<400> 68
aattatacca aggagacgct ggaaacgc 28

<210> 69
<211> 19
<212> DNA
<213> Artificial Sequence

<220>
<223> Primer

<400> 69
gaactgcctg tgcgccttt 19

<210> 70
<211> 20
<212> DNA
<213> Artificial Sequence

<220>
<223> Primer

<400> 70
ccatagaggc ccatgatggt 20

<210> 71
<211> 28
<212> DNA
<213> Artificial Sequence

<220>
<223> Probe, labeled 5'-terminal with FAM and 3'-terminal with TAMRA

<400> 71
tctgcccctc tactccaagc gctacatc 28

<210> 72
<211> 19
<212> DNA
<213> Artificial Sequence

<220>
<223> Primer

<400> 72
tgactgcttc cctcaccaa 19

<210> 73
<211> 22
<212> DNA
<213> Artificial Sequence

<220>
<223> Primer

<400> 73
gcatcctcat gattgacatg tg 22

<210> 74
<211> 22
<212> DNA
<213> Artificial Sequence

<220>
<223> Probe, labeled 5'-terminal with FAM and 3'-terminal with TAMRA

<400> 74
ttgctggtta tcgccgtgga ga 22

<210> 75
<211> 19
<212> DNA
<213> Artificial Sequence

<220>
<223> Primer

<400> 75
cttgctccac tgtcttgcc 19

<210> 76
<211> 19
<212> DNA
<213> Artificial Sequence

<220>
<223> Primer

<400> 76

```
tagagtgcac agatcgcgg                                19

<210> 77
<211> 28
<212> DNA
<213> Artificial Sequence

<220>
<223> Probe, labeled 5'-terminal with FAM and 3'-terminal with TAMRA

<400> 77
ctctacgcca aggcctacgt gctcttct                      28
```